# EUROPEAN PATENT SPECIFICATION

(11) **EP 1 349 917 B1**
(45) Date of publication and mention of the grant of the patent: **10.03.2010**
(21) Application number: 01996076.4
(22) Date of filing: 07.12.2001
(51) Int. Cl.: C12N 1/21, C12N 15/66, C12N 15/70

(54) **METHODS AND COMPOSITIONS FOR SYNTHESIS OF NUCLEIC ACID MOLECULES USING MULTIPLE RECOGNITION SITES**
VERFAHREN UND ZUSAMMENSETZUNGEN ZUR SYNTHESE VON NUKLEINSÄUREMOLEKÜLEN UNTER VERWENDUNG MULTIPLER ERKENNUNGSSTELLEN
METHODES ET COMPOSITIONS DESTINEES A LA SYNTHESE DE MOLECULES D'ACIDE NUCLEIQUE A L'AIDE DE SITES DE RECONNAISSANCE MULTIPLES

(30) Priority: 08.12.2000 US 254510 P; 11.12.2000 US 732914; 14.09.2001 US 318902 P; 28.09.2001 US 326092 P; 27.11.2001 US 333124 P
(43) Date of publication of application: 08.10.2003
(60) Divisional application: 09156848.5
(73) Proprietor: Life Technologies Corporation, Carlsbad, CA 92008 (US)
(72) Inventor: CHESNUT, Jonathan, D., Carlsbad, CA 92008 (US); CARRINO, John, Carlsbad, CA 92008 (US); LEONG, Louis, Mission Viejo, CA 92691 (US); MADDEN, Knut, Carlsbad, CA 92008 (US); GLEESON, Martin, San Diego, CA 92130 (US); FAN, James, Carlsbad, CA 92008 (US); BRASCH, Michael, A., Gaithersburg, MD 20882 (US); CHEO, David, Kensington, MD 20895 (US); HARTLEY, James, L., Frederick, MD 21702 (US); BYRD, Devon, R., N., Fredericksburg, Virginia 22405 (US); TEMPLE, Gary, F., Washington Grove, MD 20880 (US)
(74) Representative: Williams, Richard Andrew Norman
(86) International application number: PCT/US2001/045773
(87) International publication number: WO 2002/046372

(56) References cited:
- WO-A-01/31039
- US-A- 5 766 891
- US-A- 5 888 732
- "Honing your cloning" THE SCIENTIST, [Online] vol. 14, no. 16, 21 August 2000 (2000-08-21), page 29, XP002287598 Retrieved from the Internet: URL:http://www.the-scientist.com/yr2000/au g/profile1_000821.html> [retrieved on 2004-07-08]
- RUSSELL MARIJANE: "A recombination-based cloning system that decreases time to protein analysis" AMERICAN BIOTECHNOLOGY LABORATORY, vol. 18, no. 7, June 2000 (2000-06), page 8, 10, XP002287580 ISSN: 0749-3223
- "The Echo(TM) cloning system: The Future of cloning is here" INVITROGEN ONLINE CATALOG, [Online] 11 July 2000 (2000-07-11), XP002287581 Retrieved from the Internet: URL:http://web.archive.org/web/20000711064 556/www.invitrogen.com/catalog_echo.html> [retrieved on 2004-07-07]
- HARTLEY JAMES L ET AL: "DNA cloning using in vitro site-specific recombination" GENOME RESEARCH, COLD SPRING HARBOR LABORATORY PRESS, US, vol. 10, no. 11, November 2000 (2000-11), pages 1788-1795, XP002187669 ISSN: 1088-9051
- QINGHUA LIU ET AL: "THE UNIVECTOR PLASMID-FUSION SYSTEM A METHOD FOR RAPID CONSTRUCTIONOF RECOMBINANT DNA WITHOUT RESTRICTION ENZYMES" CURRENT BIOLOGY, CURRENT SCIENCE,, GB, vol. 8, no. 24, 3 December 1998 (1998-12-03), pages 1300-1309, XP000941593 ISSN: 0960-9822
- HEYMAN J A ET AL: "GENOME-SCALE CLONING AND EXPRESSION OF INDIVIDUAL OPEN READING FRAMES USING TOPOISOMERASE I-MEDIATED LIGATION" GENOME RESEARCH, COLD SPRING HARBOR LABORATORY PRESS, US, vol. 9, February 1999 (1999-02), pages 383-392, XP002939944 ISSN: 1088-9051
- SHUMAN STEWART: "Novel approach to molecular cloning and polynucleotide synthesis using vaccinia DNA topoisomerase" JOURNAL OF BIOLOGICAL CHEMISTRY, AMERICAN SOCIETY OF BIOLOGICAL CHEMISTS, BALTIMORE, MD, US, vol. 269, no. 51, 23 December 1994 (1994-12-23), pages 32678-32684, XP002164562 ISSN: 0021-9258
- "The Echo(TM) cloning system: The Future of cloning is here" INVITROGEN ONLINE CATALOG, [Online] XP002287582 Retrieved from the Internet: URL:http://invitrogen.com/content.cfm?page id=3371&cfid=16767784&cftoken=62396683> [retrieved on 2004-07-07]
- CHENG C. ET AL.: "Conservation of structure and mechanism between eukaryotic topoisomerase I and site-specific recombinases" CELL, vol. 92, pages 841-850, XP002225937

## Description

### BACKGROUND OF THE INVENTION

### Field of the Invention

The present invention relates to the fields of biotechnology and molecular biology. In particular, the present invention relates to joining multiple nucleic acid molecules containing one or more recombination sites and/or one or more topoisomerase recognition sites. The present invention also relates to cloning such joined nucleic acid molecules using recombinational cloning methods such as those employing topoisomerase and/or recombination proteins. The invention also relates to joining multiple peptides, and combinations of peptides and nucleic acid molecules through the use of recombination sites and/or topoisomerase recognition sites. Other molecules and compounds or combinations of molecules and compounds may also be joined through recombination sites and/or topoisomerase recognition sites according to the invention. Such peptides, nucleic acids and other molecules and/or compounds (or combinations thereof) may also be joined or bound through recombination reactions and/or through topoisomerase joining reactions to one or a number of supports or structures in accordance with the invention.

### Related Art

### Site-specific Recombinases

Site-specific recombinases are proteins that are present in many organisms (e.g. viruses and bacteria) and have been characterized as having both endonuclease and ligase properties. These recombinases (along with associated proteins in some cases) recognize specific sequences of bases in a nucleic acid molecule and exchange the nucleic acid segments flanking those sequences. The recombinases and associated proteins are collectively referred to as "recombination proteins" (see, e.g., Landy, A., Current Opinion in Biotechnology 3:699-707 (1993)).

Numerous recombination systems from various organisms have been described. See, e.g., Hoess, et al., Nucleic Acids Research 14(6):2287 (1986); Abremski, et al., J. Biol. Chem. 261(1):391 (1986); Campbell, J. Bacteriol. 174(23):7495 (1992); Qian, et al., J. Biol. Chem. 267(11):7794 (1992); Araki, et al., J. Mol. Biol. 225(1):25 (1992); Maeser and Kahnmann, Mol. Gen. Genet. 230:170-176) (1991); Esposito, et al., Nucl. Acids Res. 25(18):3605 (1997). Many of these belong to the integrase family of recombinases (Argos, et al., EMBO J. 5:433-440 (1986); Voziyanov, et al., Nucl. Acids Res. 27:930 (1999)). Perhaps the best studied of these are the Integrase/*att* system from bacteriophage ( (Landy, A. Current Opinions in Genetics and Devel. 3:699-707 (1993)), the Cre/*loxP* system from bacteriophage P1 (Hoess and Abremski (1990) In Nucleic Acids and Molecular Biology, vol. 4. Eds.: Eckstein and Lilley, Berlin-Heidelberg: Springer-Verlag; pp. 90-109), and the FLP/FRT system from the Saccharomyces cerevisiae 2 µ circle plasmid (Broach, et al., Cell 29:227-234 (1982)).

### Recombination Sites

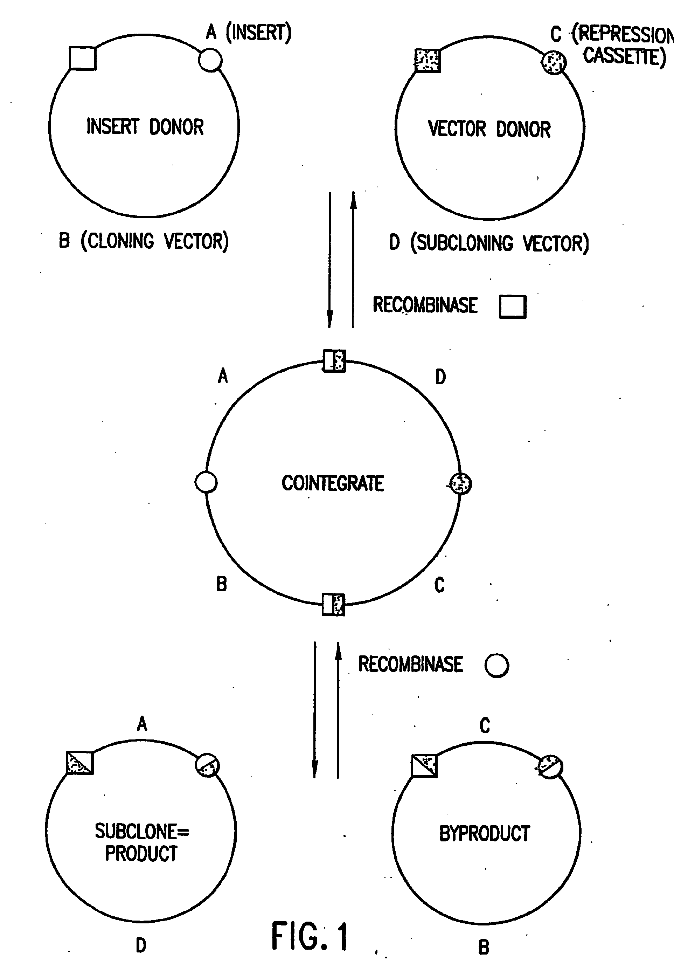

Whether the reactions discussed above are termed recombination, transposition or integration and are catalyzed by a recombinase, transposase or integrase, they share the key feature of specific recognition sequences, often termed "recombination sites," on the nucleic acid molecules participating in the reactions. These recombination sites are sections or segments of nucleic acid on the participating nucleic acid molecules that are recognized and bound by the recombination proteins during the initial stages of integration or recombination. For example, the recombination site for Cre recombinase is *loxP* which is a 34 base pair sequence comprised of two 13 base pair inverted repeats (serving as the recombinase binding sites) flanking an 8 base pair core sequence. See Figure 1 of Sauer, B., Curr. Opin. Biotech. 5:521-527 (1994). Other examples of recognition sequences include the *attB*, *attP*, *attL*, and *attR* sequences which are recognized by the recombination protein ( Int. *attB* is an approximately 25 base pair sequence containing two 9 base pair core-type Int binding sites and a 7 base pair overlap region, while *attP* is an approximately 240 base pair sequence containing core-type Int binding sites and arm-type Int binding sites as well as sites for auxiliary proteins integration host factor (IHF), FIS and excisionase (Xis). See Landy, Curr. Opin. Biotech. 3:699-707 (1993).

### Conventional Nucleic Acid Cloning

The cloning of nucleic acid segments currently occurs as a daily routine in many research labs and as a prerequisite step in many genetic analyses. The purpose of these clonings is various, however, two general purposes can be considered: (1) the initial cloning of nucleic acid from large DNA or RNA segments (chromosomes, YACs, PCR fragments, mRNA, etc.), done in a relative handful of known vectors such as pUC, pGem, pBlueScript, and (2) the subcloning of these nucleic acid segments into specialized vectors for functional analysis. A great deal of time and effort is expended both in the transfer of nucleic acid segments from the initial cloning vectors to the more specialized vectors. This transfer is called subcloning.

The basic methods for cloning have been known for many years and have changed little during that time. A typical cloning protocol is as follows:
(1) digest the nucleic acid of interest with one or two restriction enzymes;
(2) gel purify the nucleic acid segment of interest when known;
(3) prepare the vector by cutting with appropriate restriction enzymes, treating with alkaline phosphatase, gel purify etc., as appropriate;
(4) ligate the nucleic acid segment to the vector, with appropriate controls to eliminate background of uncut and self-ligated vector;
(5) introduce the resulting vector into an *E. coli* host cell;
(6) pick selected colonies and grow small cultures overnight;
(7) make nucleic acid minipreps; and
(8) analyze the isolated plasmid on agarose gels (often after diagnostic restriction enzyme digestions) or by PCR.

The specialized vectors used for subcloning nucleic acid segments are functionally diverse. These include but are not limited to: vectors for expressing nucleic acid molecules in various organisms; for regulating nucleic acid molecule expression; for providing tags to aid in protein purification or to allow tracking of proteins in cells; for modifying the cloned nucleic acid segment (*e.g.*, generating deletions); for the synthesis of probes (*e.g*., riboprobes); for the preparation of templates for nucleic acid sequencing; for the identification of protein coding regions; for the fusion of various protein-coding regions; to provide large amounts of the nucleic acid of interest, *etc*. It is common that a particular investigation will involve subcloning the nucleic acid segment of interest into several different specialized vectors.

As known in the art, simple subclonings can be done in one day (*e.g*., the nucleic acid segment is not large and the restriction sites are compatible with those of the subcloning vector). However, many other subclonings can take several weeks, especially those involving unknown sequences, long fragments, toxic genes, unsuitable placement of restriction sites, high backgrounds, impure enzymes, *etc*. One of the most tedious and time consuming type of subcloning involves the sequential addition of several nucleic acid segments to a vector in order to construct a desired clone. One example of this type of cloning is in the construction of gene targeting vectors. Gene targeting vectors typically include two nucleic acid segments, each identical to a portion of the target gene, flanking a selectable marker. In order to construct such a vector, it may be necessary to clone each segment sequentially, i.e., first one gene fragment is inserted into the vector, then the selectable marker and then the second fragment of the target gene. This may require a number of digestion, purification, ligation and isolation steps for each fragment cloned Subcloning nucleic acid fragments is thus often viewed as a chore to be done as few times as possible.

Several methods for facilitating the cloning of nucleic acid segments have been described, *e.g*., as in the following references.

Ferguson, J., et al., Gene 16:191 (1981), disclose a family of vectors for subcloning fragments of yeast nucleic acids. The vectors encode kanamycin resistance. Clones of longer yeast nucleic acid segments can be partially digested and ligated into the subcloning vectors. If the original cloning vector conveys resistance to ampicillin, no purification is necessary prior to transformation, since the selection will be for kanamycin.

Hashimoto-Gotoh, T., et al., Gene 41:125 (1986), disclose a subcloning vector with unique cloning sites within a streptomycin sensitivity gene; in a streptomycin-resistant host, only plasmids with inserts or deletions in the dominant sensitivity gene will survive streptomycin selection.

Notwithstanding the improvements provided by these methods, traditional subclonings using restriction and ligase enzymes are time consuming and relatively unreliable. Considerable labor is expended, and if two or more days later the desired subclone can not be found among the candidate plasmids, the entire process must then be repeated with alternative conditions attempted.

### Recombinational Cloning

Cloning systems that utilize recombination at defined recombination sites have been previously described in U.S. Patent Nos. 5,888,732, 6,143,557, 6,171,861, 6,270,969, and 6,277,608 which are specifically incorporated herein by reference. In brief, the Gateway™ Cloning System, described in this application and the applications referred to in the related applications section, utilizes vectors that contain at least one and preferably at least two different site-specific recombination sites based on the bacteriophage lambda system (e. g., *att*1 and *att*2) that are mutated from the wild type (*att*0) sites. Each mutated site has a unique specificity for its cognate partner *att* site of the same type (for example *att*B1 with *att*P1, or *att*L1 with *att*R1) and will not cross-react with recombination sites of the other mutant type or with the wild-type *att*0 site. Nucleic acid fragments flanked by recombination sites are cloned and subcloned using the Gateway™ system by replacing a selectable marker (for example, *ccd*B) flanked by *att* sites on the recipient plasmid molecule, sometimes termed the Destination Vector. Desired clones are then selected by transformation of a *ccd*B sensitive host strain and positive selection for a marker on the recipient molecule. Similar strategies for negative selection (e.g., use of toxic genes) can be used in other organisms such as thymidine kinase (TK) in mammals and insects.

Mutating specific residues in the core region of the *att* site can generate a large number of different *att* sites. As with the *att*1 and *att*2 sites utilized in Gateway™, each additional mutation potentially creates a novel *att* site with unique specificity that will recombine only with its cognate partner *att* site bearing the same mutation and will not cross-react with any other mutant or wild-type *att* site. Novel mutated *att* sites (e. g., *att*B 1-10, *att*P 1-10, *att*R 1-10 and *att*L 1-10) are described in previous patent application serial number 60/136,744, filed May 28, 1999. Other recombination sites having unique specificity (i.e., a first site will recombine with its corresponding site and will not recombine or not substantially recombine with a second site having a different specificity) may be used to practice the present invention. Examples of suitable recombination sites include, but are not limited to, *lox*P sites and derivatives such as *lox*P511 (see U.S. Patent No. 5,851,808), *frt* sites and derivatives, *dif* sites and derivatives, *psi* sites and derivatives and cer sites and derivatives. The present invention provides novel methods using such recombination sites to join or link multiple nucleic acid molecules or segments and more specifically to clone such multiple segments into one or more vectors containing one or more recombination sites (such as any Gateway^{™} Vector including Destination Vectors).

### SUMMARY OF THE INVENTION

The invention provides an isolated nucleic acid molecule as set forth in claims 1 to 19.
The invention also provides a host cell as set forth in claim 20.
The invention also provides a kit as set out in claims 21 and 22.
The invention provides a method of cloning a nucleic acid molecule as set out in claims 23 to 41.

Described herein are nucleic acid molecules which comprise one or more (e.g., one, two, three, four, five, etc.) recombination sites (e.g., one or more *att* sites, one or more *lox* sites, etc.) and/or one or more (e.g., one, two, three, four, five, etc.) topoisomerase recognition sites (e.g., one or more recognition sites for a type IA topoisomerase, a type IB topoisomerase, a type II topoisomerase, etc.), as well as nucleic acid molecules which have undergone cleavage with a topoisomerase (e.g., a site specific topoisomerase). Also described herein are nucleic acid molecules which comprise one or more recombination sites and/or one or more topoisomerases. More specifically the disclosure relates to combining or joining at least a first nucleic acid molecule which comprises at least a first nucleic acid molecule which comprises at least one recombination site and at least a second nucleic acid molecule which comprises at least one topoisomerase recognition site and/or at least one topoisomerase. Upon joining these at least first and second molecules, at least a third (or chimeric) molecule may be produced which comprises (1) at least one recombination site and (2) at least one topoisomerase recognition site and/or at least one topoisomerase. These nucleic acid molecules may be linear or closed circular (e.g., relaxed, supercoiled, etc.). Such recombination sites, topoisomerase recognition sites and topoisomerase can be located at any position on any number of nucleic acid molecules of the invention, including at or near the termini of the nucleic acid molecules and/or within the nucleic acid molecules. Moreover, any combination of the same or different recombination sites, topoisomerase recognition sites and/or topoisomerases may be used.

Described herein are nucleic acid molecules and compositions comprising nucleic acid molecules (*e*.*g*., reaction mixtures), wherein the nucleic acid molecules comprise (1) at least one (*e.g.*, one, two, three, four, five, six, seven eight, etc.) recombination site and (2) at least one (*e.g.,* one, two, three, four, five, six, seven eight, etc.) topoisomerase (*e.g.*, a covalently linked topoisomerase) or at least one (*e.g.*, one, two, three, four, five, six, seven eight, etc.) toposiomerase recognition site. The topoisomerases or toposiomerase recognition sites, as well as the recombination sites, of the nucleic acid molecules referred to above can be either internal or at or near one or both termini. For example, one or more (*e.g*., one, two, three, four, five, six, seven eight, etc.) of the at least one topoisomerase or the at least one topoisomerase recognition site, as well as one or more of the at least one recombination site, can be located at or near a 5' terminus, at or near a 3' terminus, at or near both 5' termini, at or near both 3' termini, at or near a 5' terminus and a 3' terminus, at or near a 5' terminus and both 3' termini, or at or near a 3' terminus and both 5' termini. Also described herein are methods for preparing and using nucleic acid molecules and compositions as herein described.

Described herein are nucleic acid molecules (1) to which topoisomerases of various types (*e.g.,* a type IA toposiomerase, a type IB toposiomerase, a type II topoisomerase, etc.) are attached (*e*.*g*., covalently bound) and/or (2) which contain two or more topoisomerase recognition sites which are recognized by various types of topoisomerases, as well as methods for preparing and using compositions comprising such nucleic acid molecules. These nucleic acid molecules may further comprise one or more (*e.g*., one, two, three, four, five, six, seven eight, etc.) recombination site.

Also described herein are methods for joining two or more nucleic acid segments, wherein at least one of the nucleic acid segments contains at least one toposiomerase or topoisomerase recognition site and/or one or more recombination sites. Further, when the nucleic acid segments used contain more than one (*e.g.*, two, three, four, five, six, seven eight, etc.) toposiomerase, either on the same or different nucleic acid segments, these toposiomerase may be of the same type or of different types. Similarly, when the nucleic acid segments used contain more than one toposiomerase recognition site, either on the same or different nucleic acid segments, these toposiomerase recognition sites may be recognized by topoisomerases of the same type or of different types. Additionally, when the nucleic acid segments used contain one or more recombination sites, these recombination sites may be able to recombine with one or more recombination sites on the same or different nucleic acid segments. Thus, described herein are methods for joining nucleic acid segments using methods employing any one toposiomerase or topoisomerase recognition site. Further methods are described herein for joining nucleic acid segments using methods employing (1) any combination of topoisomerases or topoisomerase recognition sites and/or (2) any combination of recombination sites. Also described herein are nucleic acid molecules produced by the methods described above, as well as uses of these molecules and compositions comprising these molecules.

Described herein are methods for joining any number of nucleic acid segments (*e.g*., two, three, four, five, six, seven, eight, nine, ten, etc.) which contain different functional or structural elements. Therefore methods for bringing together any number of nucleic acid segments (*e.g.*, two, three, four, five, six, seven, eight, nine, ten, etc.) which confer different properties upon a nucleic acid molecule product are also described. In many instances, use of the methods described herein will result in the formation of nucleic acid molecules wherein there is operable interaction between properties and/or elements of individual nucleic acid segments which are joined (*e.g*., operable interaction/linkage between an expression control sequence and an open reading frame). Examples of (1) functional and structural elements and (2) properties which may be conferred upon product molecules include, but are not limited to, multiple cloning sites (*e.g.*, nucleic acid regions which contain at least two restriction endonuclease cleavage sites), packaging signals (*e.g*., adenoviral packaging signals, alphaviral packaging signals, etc.), restriction endonuclease cleavage sites, open reading frames (e.g., intein coding sequence, affinity purification tag coding sequences, etc.), expression control sequences (*e.g*., promoters, operators, etc.), etc. Additional elements and properties which can be conferred by nucleic acid segments upon a product nucleic acid molecule are described elsewhere herein. Also described herein are nucleic acid molecules produced by the methods described above, as well as uses of these molecules and compositions comprising these molecules.

Described herein are methods for joining two or more (e.g., 2, 3, 4, 5, 6, 7, 8, etc.) nucleic acid segments, wherein at least one (*e.g*., 1, 2, 3, 4, 5, 6, 7, 8, etc.) of the nucleic acid segments comprises one or more (*e.g.*, 1, 2, 3, 4, 5, 6, 7, 8, etc.) topoisomerases and/or one or more topoisomerase recognition sites and at least one of the nucleic acid segments comprises one or more recombination sites. Also described herein are methods for joining at least two (*e.g*., 2, 3, 4, 5, 6, 7, 8, etc.) nucleic acid molecules (*e.g.*, methods employing recombination and/or mediated by one or more topoisomerases), wherein one of the nucleic acid segments comprises one or more topoisomerases or topoisomerase recognition sites but does not contain a recombination site and the other nucleic acid segments comprises one or more recombination site but does not contain a topoisomerase or topoisomerase recognition site. Thus, methods described herein can be used to prepare joined or chimeric nucleic acid molecules by the joining of nucleic acid segments, wherein the product nucleic acid molecules comprise (1) one or more (*e.g.*, 1, 2, 3, 4, 5, 6, 7, 8, etc.) topoisomerases and/or one or more (*e.g*., 1, 2, 3, 4, 5, 6, 7, 8, etc.) topoisomerase recognition sites and (2) one or more (*e.g.*, 1, 2, 3, 4, 5, 6, 7, 8, etc.) recombination sites. Also described herein are nucleic acid molecules prepared by such methods, compositions comprising such nucleic acid molecules, and methods for using such nucleic acid molecules.

Compositions comprising one or more nucleic acid segments and/or nucleic acid molecules are described herein. Such compositions may comprise one or a number of other components selected from the group consisting of one or more other nucleic acid molecules (which may comprise recombination sites, topoisomerase recognition sites, topoisomerases, etc.), one or more nucleotides, one or more polymerases, one or more reverse transcriptases, one or more recombination proteins, one or more topoisomerases, one or more buffers and/or salts, one or more solid supports, one or more polyamines, one or more vectors, one or more restriction enzymes and the like. For example, compositions described herein include, but are not limited to, mixtures (*e.g.*, reaction mixtures) comprising a nucleic acid segment which comprises at least one topoisomerase recognition site and at least one topoisomerase which recognizes at least one of the at least one topoisomerase recognition sites of the nucleic acid segment. Compositions described herein further include at least one nucleic acid segment comprising (1) at least one topoisomerase recognition site or at least one nucleic acid segment to which at least one topoisomerase is attached (*e.g.*, covalently bound) and (2) one or more additional components. Examples of such additional components include, but are not limited to, topoisomerases; additional nucleic acid segments, which may or may not comprise one or more topoisomerases or topoisomerase recognition sites; buffers; salts; polyamines (*e.g.*, spermine, spermidine, etc.); water; etc. Nucleic acid segments present in compositions as described herein may further comprise one or more recombination sites and/or one or more recombinase.

Nucleic acid molecules or segments produced by or used in conjunction with the methods described herein as well as nucleic acid molecules or segments thereof described herein, include those molecules or segments specifically described herein as well as those molecules or segments that have substantial sequence identity to those molecules or segments specifically described herein. By a molecule or segment having "substantial sequence identity" to a given molecule or segment is meant that the molecule or segment is at least 65%, at least 70%, at least 75%, at least 80%, at least 85%, at least about 90%, 91%, 92%, 93%, 94%, 95%, 96%, 97%, 98% or 99%, identical to the given (or "reference") molecule or segment. By a nucleic acid molecule or segment having a nucleotide sequence at least, for example, 65% "identical" to a reference nucleic acid molecule or segment is intended that the nucleotide sequence of the nucleic acid molecule or segment is identical to that of the reference sequence except that the nucleic acid molecule or segment may include up to 35 point mutations per each 100 nucleotides of the reference nucleotide sequence. In other words, to obtain a polynucleotide having a nucleotide sequence at least 65% identical to a reference nucleotide sequence, up to 5% of the nucleotides in the reference sequence may be deleted or substituted with another nucleotide, or a number of nucleotides up to 35% of the total nucleotides in the reference sequence may be inserted into the reference sequence. These mutations of the reference sequence may occur at the 5' or 3' terminal positions (or both) of the reference nucleotide sequence, or anywhere between those terminal positions, interspersed either individually among nucleotides in the reference sequence or in one or more contiguous groups within the reference sequence.

As a practical matter, whether any particular nucleic acid molecule or segment is at least about 65%, 70%, 75%, 80%, 85%, 90%, 95%, 96%, 97%, 98% or 99% identical to a given reference molecule or segment can be determined conventionally using known computer programs such as FASTA (Heidelberg, Germany), BLAST (Washington, DC) or BESTFIT (Wisconsin Sequence Analysis Package, Version 8 for Unix, Genetics Computer Group, University Research Park, 575 Science Drive, Madison, WI 53711), which employs a local homology algorithm (Smith and Waterman, Advances in Applied Mathematics 2: 482-489 (1981)) to find the best segment of homology between two sequences. When using FASTA, BLAST, BESTFIT or any other sequence alignment program to determine whether a particular sequence is, for instance, 65% identical to a reference sequence disclosed herein , the parameters are set such that the percentage of identity is calculated over the full length of the reference nucleotide sequence and that gaps in homology of up to 35% of the total number of nucleotides in the reference sequence are allowed.

Often, nucleic acid molecules which have undergone cleavage with a topoisomerase (e.g., a site specific topoisomerase) will further have a topoisomerase molecule covalently bound to a phosphate group of the nucleic acid molecules. Described herein are methods for preparing nucleic acid molecules described above and elsewhere herein, as well as recombinant methods for using such molecules.

The nucleic acid molecules described herein may be vectors. Also described herein are host cells which contain nucleic acid molecules described herein, as well as methods for making and using such host cells, for example, to produce expression products (*e.g.*, proteins, polypeptides, antigens, antigenic determinants, epitopes, and the like, or fragments thereof).

The nucleic acid molecules described herein may comprise two or more recombination sites with one or more (e.g., one, two, three, four, five, etc.) topoisomerase recognition site located between the recombination sites. Also described herein are nucleic acid molecules which may comprise two or more topoisomerase recognition sites with one or more (e.g., one, two, three, four, five, etc.) recombination sites located between the two or more topoisomerase recognition sites.

Described herein are nucleic acid molecules which comprise two recombination sites with two topoisomerase recognition sites located between the two recombination sites. Thus, if such molecules are linearized by cleavage between the topoisomerase recognition sites, the topoisomerase recognition sites in the resulting linear molecule will be located distal (*i.e*., closer to the two ends of the linear molecule) to the recombination sites. Therefore, also described herein are linear nucleic acid molecules which contain one or more recombination sites and one or more topoisomerase recognition sites. The one or more topoisomerase recognition sites may be located distal to the one or more recombination sites. Examples of such molecules are set out below in Example 8.

The positioning of recombination sites and topoisomerase recognition sites of a first nucleic acid molecule can be such that topoisomerase mediated linkage of this molecule to a second nucleic acid molecule results in the second nucleic acid molecule being positioned between the two or more recombination sites. As an example, a linear first nucleic acid molecule may contain one recombination site at or near each end and may further comprise a topoisomerase recognition site located distal to one of the two recombination sites. In such a case, incubation of the linear first nucleic acid molecule with a topoisomerase can be designed to result in the covalent linkage of the topoisomerase to the first nucleic acid molecule, wherein the topoisomerase is positioned at or near the end of the first nucleic acid molecule and distal to the adjacent/nearest recombination site. This end of the first nucleic acid molecule may be blunt or may have either a 5' or 3' overhang. When incubated with a suitable second nucleic acid molecule (e.g., a molecule with sequence complementarity to at least one strand of the topoisomerase modified end of the first nucleic acid molecule), one or both strands of one end of the second nucleic acid molecule can be covalently joined to one or both strands of one end of the first nucleic acid molecule. Further, if a circular nucleic acid molecule is desired, then the second end of the second nucleic acid molecule can be joined to the second end of the first nucleic acid molecule by a topoisomerase, a ligase or other method. The result of the process described above is the generation of a nucleic acid molecule which contains a nucleic acid insert positioned between two recombination sites. Specific examples of related processes are set out below in Example 8. Methods for covalently linking nucleic acid molecules using topoisomerase are described in more detail elsewhere herein.

Once a nucleic acid insert has been positioned between one or more recombination sites, this insert, as well as adjacent nucleic acid, may be transferred to other nucleic acid molecules by recombinational cloning. Thus methods for generating the nucleic acid molecules are described above and elsewhere herein.

The distance, in terms of the number of nucleotides, between recombination sites and topoisomerase recognition sites which reside in a nucleic acid molecule described herein will vary with the particular application for which the molecule is to be used, but can be zero, one, two, three, four, five, six, seven, eight, nine, ten, eleven, twelve, thirteen, fourteen, fifteen, sixteen, twenty, twenty-five, thirty, forty, fifty, sixty, eighty, one hundred, one hundred fifty, two hundred, three hundred, five hundred, seven hundred, nine hundred, one thousand, etc., or more, nucleotides. Further, the distance, in terms of the number of nucleotides, between recombination sites and topoisomerase recognition sites which reside in a nucleic acid molecule described herein may fall within the following ranges: 0-10 nucleotides, 10-30 nucleotides, 20-50 nucleotides, 40-80 nucleotides, 70-100 nucleotides, 90-200 nucleotides, 120-400 nucleotides, 200-400 nucleotides, 200-1000 nucleotides, 200-2,000 nucleotides, etc.

Described herein are materials and methods for joining or combining two or more (e.g., three or more, four or more, five or more etc.) segments or molecules of nucleic acid described herein. For such molecules to be combined, at least one of the segments or molecules may comprise at least one recombination site and at least one of the segments or molecules may comprise at least one topoisomerase recognition site. Such methods for joining multiple nucleic acid molecules may be conducted *in vivo* or *in vitro*. Accordingly, described herein are methods to create novel or unique combinations of sequences and the sequences created by such methods. The nucleic acid molecules created by the methods described herein may be used for any purpose known to those skilled in the art. As described herein at least one (and often two or more) of the nucleic acid molecules or segments to be joined by the methods described herein comprise at least one, and preferably at least two, recombination sites, although each molecule may comprise multiple recombination sites (e.g., three or more, four or more, five or more, etc.). The nucleic acid molecules may comprise at least one topoisomerase recognition site and/or at least one topoisomerase. The molecules may comprise (1) at least one recombination site and (2) at least one topoisomerase recognition site and/or at least one topoisomerase. Such recombination sites and topoisomerase recognition sites (which may be the same or different) may be located at various positions in each nucleic acid molecule or segment and the nucleic acid used may have various sizes and be in different forms including circular, supercoiled, linear, and the like. The nucleic acid' molecules used may also comprise one or more vectors or one or more sequences allowing the molecule to function as a vector in a host cell (such as an origin of replication). Nucleic acid molecules or segments for use as described herein are linear molecules having at least one recombination site at or near at least one termini of the molecule and preferably comprise at least one recombination site at or near both termini of the molecule. When multiple recombination sites are located on a nucleic acid molecule of interest, such sites may not substantially recombine or do not recombine with each other on that molecule. The corresponding binding partner recombination sites preferably are located on one or more other nucleic acid molecules to be linked or joined by the methods described herein. For instance, a first nucleic acid molecule may comprise at least a first and second recombination site and a second nucleic acid molecule may comprise at least a third and fourth recombination site, wherein the first and second sites do not recombine with each other and the third and fourth sites do not recombine with each other, although the first and third and/or the second and fourth sites may recombine.

The nucleic acid molecules to be joined by the methods described herein (e.g., the "starting molecules") may be used to produce one or more hybrid molecules containing all or a portion of the starting molecules (e.g., the "product nucleic acid molecules"). The starting molecules can be any nucleic acid molecule derived from any source or produced by any method. Such molecules may be derived from natural sources (such as cells, tissue, and organs from any animal or non-animal source) or may be non-natural (e.g., derivative nucleic acids) or synthetically derived. The segments or molecules may be produced by any means known to those skilled in the art including, but not limited to, amplification such as by PCR, isolation from natural sources, chemical synthesis, shearing or restriction digest of larger nucleic acid molecules (such as genomic or cDNA), transcription, reverse transcription and the like, and recombination sites and/or topoisomerase recognition sites and/or topoisomerases may be added to such molecules by any means known to those skilled in the art including ligation of adapters containing recombination sites and/or topoisomerase recognition sites and/or topoisomerases, amplification or nucleic acid synthesis using primers containing recombination sites and/or topoisomerase recognition sites and/or topoisomerases, insertion or integration of nucleic acid molecules (e.g., transponsons or integration sequences) containing recombination sites and/or topoisomerase recognition sites and/or topoisomerases, etc. The nucleic acid molecules used may be populations of molecules such as nucleic acid libraries or cDNA libraries.

Once nucleic acid molecules are joined by recombination using methods such as those described herein, these nucleic acid molecules may then be joined to other nucleic acid molecules using topoisomerase-mediated joining methods and/or recombination-mediated joining methods also described herein.

Recombination sites may be any recognition sequence on a nucleic acid molecule which participates in a recombination reaction catalyzed or facilitated by recombination proteins. In utilizing more than one recombination site, such recombination sites may be the same or different and may recombine with each other or may not recombine or not substantially recombine with each other. Recombination sites may also include mutants, derivatives or variants of wild-type or naturally occurring recombination sites. Recombination site modifications may include those that enhance recombination, such enhancement selected from the group consisting of substantially (i) favoring integrative recombination; (ii) favoring excisive recombination; (iii) relieving the requirement for host factors; (iv) increasing the efficiency of co-integrate or product formation; and (v) increasing the specificity of co-integrate or product formation. Modifications include those that enhance recombination specificity, remove one or more stop codons, and/or avoid hair-pin formation. Desired modifications can also be made to the recombination sites to include desired amino acid changes to the transcription or translation product (e.g., mRNA or protein) when translation or transcription occurs across the modified recombination site. Recombination sites that may be used include *att* sites. *frt* sites, *dif* sites, *psi* sites, *cer* sites, and *lox* sites or mutants, derivatives and variants thereof (or combinations thereof). Recombination sites may also include portions of such recombination sites.

Each starting nucleic acid molecule may comprise, in addition to one or more recombination sites and/or one or more topoisomerase recognition sites and/or one or more topoisomerases, a variety of sequences (or combinations thereof) including, but not limited to sequences suitable for use as primer sites (e.g., sequences which a primer such as a sequencing primer or amplification primer may hybridize to initiate nucleic acid synthesis, amplification or sequencing), transcription or translation signals or regulatory sequences such as promoters and/or operators, ribosomal binding sites, topoisomerase recognition sequences (or sites), Kozak sequences, and start codons, transcription and/or translation termination signals such as stop codons (which may be optimally suppressed by one or more suppressor tRNA molecules), tRNAs (*e*.*g*., suppressor tRNAs), origins of replication, selectable markers, and genes or portions of genes which may be used to create protein fusion (e.g., N-terminal or carboxy terminal) such as GST, GUS, GFP, open reading frame (orf) sequences, and any other sequence of interest which may be desired or used in various molecular biology techniques including sequences for use in homologous recombination (e.g., gene targeting).

Described herein are methods of generating a covalently linked recombinant nucleic acid molecule by contacting two or more (e.g., 2, 3, 4, 5, 6, 7, 8, 9, 10, etc) nucleic acid molecules (which may be alternatively and equivalently referred to herein as "nucleotide sequences"), *e*.*g*., double-stranded ("ds") or single-stranded ("ss") nucleic acid molecules, with at least one (e.g., 1, 2, 3, 4, 5, 6, 7, 8, 9, 10, etc.) topoisomerase. As will be understood by the ordinarily skilled artisan, any and all of the nucleic acid molecules or nucleotide sequences referred to herein, for example those used in or generated by the methods, compositions and kits disclosed herein, may be ss or ds nucleic acid molecules or nucleotide sequences, whether or not the molecules or sequences are specifically referred to herein as being ss and/or ds.

The methods described herein allow joining of such nucleic acid sequences in a desired orientation and/or order, which, if desired, can be further manipulated or used in a variety of assays or procedures, including, for example, for a transcription or transfection procedure, which can be performed *in vitro* or *in vivo*, a translation reaction or other protein expression procedure, recombination reactions, and the like. Three or more, four or more, five or more, etc., or a population or library of the same or different nucleic acid sequences can be linked according to a method described herein. The methods described herein can be used to link each end of a single nucleic acid molecule to form a covalently closed circular or supercoiled molecule.

The nucleic acid sequences to be linked can be derived from any source, and can be naturally occurring and chemically or recombinantly synthesized nucleic acid molecules such as cDNA, genomic DNA, vectors, oligonucleotides, and the like. Furthermore, the nucleic acid sequences can, but need not, contain one or more functional sequences such as gene regulatory elements, origins of replication, splice sites, polyadenylation sites, open reading frames, which can encode, for example, tag sequences, detectable or selectable markers, cell localization domains, or other peptide or polypeptide, and the like. As such, any number of nucleic acid sequences, which can be the same or different, may be linked, including, if desired, in a predetermined order or orientation or both.

The nucleic acid molecules (*e*.*g*., ds or ss nucleic acid molecules) to be linked can be in any form, for example, single-stranded or double-stranded, linear, circular, or supercoiled, and are characterized, in part, in that each nucleic acid molecule to be linked is a substrate for a topoisomerase or can be modified to be such a substrate. The topoisomerase can be any topoisomerase that can covalently link at least one strand of a nucleic acid molecule to at least one strand of another nucleic acid molecule, this may be through a phosphodiester bond. The topoisomerase can be a site specific topoisomerase or can have relaxed specificity, and may form a stable complex (e.g., a covalent complex) with one strand of the nucleic acid molecule at or near the site at which cleavage is effected.

Methods described herein generally are performed by contacting topoisomerase and the nucleic acid molecules (*e*.*g*., ds or ss nucleic acid molecules) to be joined under conditions such that both strands of an end of one nucleic acid molecule are ligated to both strands of an end of at least one (e.g., 1, 2, 3, 4, 5, 6, 7, 8, 9, 10, etc.) other nucleic acid molecule. As such, a method so described generates a covalently linked recombinant nucleic acid molecule (which may be either single-stranded or double-stranded), which does not contain a nick at the site or sites at which the substrate nucleic acid molecules are ligated. Recombinant nucleic acid molecules may be prepared by such a method. Such recombinant nucleic acid molecules may further comprise one or more recombination sites.

The methods described herein may be performed using various combinations of components. For example, the method can be performed by contacting two or more substrate nucleic acid molecules (*e*.*g*., ss nucleic acid molecules or ds nucleic acid molecules) to be covalently linked and at least one topoisomerase, wherein the topoisomerase cleaves one or both strands of the nucleic acid molecules and forms a stable complex with a nucleotide at a terminus of the cleavage site. The topoisomerase-charged ends or topoisomerase-charged nucleic acid molecules are then contacted with each other such that each strand of the substrate nucleic acid molecules are linked, thereby generating one or more covalently linked recombinant nucleic molecules. Preferably, the topoisomerase mediates the formation of phosphodiester bond at each linkage site. The method also can be performed by contacting two or more topoisomerase-charged nucleic acid molecules, either alone, or in the presence of excess topoisomerase, or by contacting one or more topoisomerase-charged nucleic acid molecules (which may be ss or ds) with one or more nucleic acid molecules (which may also be ss or ds) that contain a topoisomerase cleavage site, and a topoisomerase. Recombinant nucleic acid molecules may be prepared by such a method. Such recombinant nucleic acid molecules may further comprise one or more recombination sites. In various embodiments, the topoisomerase can have a relatively relaxed specificity such that it can bind to and cleave a variety of different nucleotide sequences, or the topoisomerase can be a site specific topoisomerase, which binds to and cleaves a specific nucleotide sequence. The topoisomerase also can be a type I topoisomerase, which cleaves one strand of a ds nucleic acid molecule, or can be a type II topoisomerase, which cleaves both strands of a ds nucleic acid molecule. Where the topoisomerase is a type II topoisomerase, cleavage is effected such that a linear ds nucleic acid molecule is produced, and is topoisomerase-charged at one or both ends. In certain such aspects, the strand of the ds nucleic acid molecule that is complementary to the strand containing the bound topoisomerase will form an overhanging sequence.

An advantage of performing a method described herein is that the ligation reaction performed by a topoisomerase occurs very quickly and over a wide range of temperatures. An additional advantage is that recombinant nucleic acid molecules generated according to the methods described herein do not contain nicks at the sites where two nucleic acid molecules are joined together. As such, the covalently linked recombinant nucleic acid molecules can be used directly in a subsequent procedure, for example, as a substrate for an amplification reaction such as a polymerase chain reaction (PCR).

By way of example, a method described herein may be performed by contacting 1) a first nucleic acid molecule (which may be ss or ds) having a first end and a second end, wherein, at the first end or second end or both, the first nucleic acid molecule has a topoisomerase recognition site at or near the 3' terminus; 2) at least a second nucleic acid molecule (which may also be ss or ds) having a first end and a second end, wherein, at the first end or second end or both, the at least second double stranded nucleotide sequence has a topoisomerase recognition site at or near the 3' terminus; and 3) a site specific topoisomerase, under conditions such that all components are in contact and the topoisomerase can effect its activity. The strand complementary to that containing the topoisomerase recognition sequence may comprise a 5' hydroxyl group and, upon cleavage by the topoisomerase, may further comprise a 5' overhanging sequence.

A method described herein also may be performed by contacting 1) a nucleic acid molecule (which may be ss or ds) having a first end and a second end, wherein each of the first end and second end contains a topoisomerase recognition site at or near the 3' terminus, and 2) a site specific topoisomerase, under conditions such that the components are in contact and the topoisomerase can effect its activity. For example, the topoisomerase can be a type IB topoisomerase such as a *Vaccinia* topoisomerase or an *S. cerevisiae* topoisomerase. Such a method provides a means to prepare a covalently closed circular or supercoiled ds nucleic acid molecule.

A method described herein also can be performed by contacting 1) a first nucleic acid molecule (which may be ss or ds) having a first end and a second end, wherein the first nucleic acid molecule has a topoisomerase recognition site at or near the 5' terminus of the first end or the second end or both; 2) at least a second nucleic acid molecule (which may also be ss or ds) having a first end and a second end, wherein the at least second double stranded nucleotide sequence has a topoisomerase recognition site at or near the 5' terminus of the first end or the second end or both; and 3) at least one site specific topoisomerase, under conditions such that all components are in contact and the at least one topoisomerase can effect its activity. For example, the topoisomerase can be a type IA topoisomerase such as an *E. coli* topoisomerase I or topoisomerase III, or eukaryotic topoisomerase III. Upon cleavage of a nucleic acid molecule, the topoisomerase preferably is stably bound to the 5' terminus. The 3' terminus of the end containing the topoisomerase recognition site, or bound topoisomerase, can comprise a 3' hydroxyl group, or can be modified to comprise a 3' hydroxyl group. Upon cleavage by the topoisomerase, the cleaved nucleic acid molecule may comprise a 3' overhanging sequence.

The methods as exemplified herein can be performed using two or more site specific topoisomerases, wherein the first, second or other nucleic acid substrates correspondingly have, at or near a 3' terminus or 5' terminus of an end, a topoisomerase recognition site for one of the two or more topoisomerases. The use of two or more topoisomerases, and corresponding topoisomerase recognition sites, can facilitate the joining of the nucleic acid molecules (which may be ss or ds) in a predetermined order, orientation, or combination thereof. Thus, it will be recognized that, where a method described herein is exemplified using a topoisomerase, the method similarly can be performed using two or more topoisomerases. In some cases, reference is made to the use of at least one topoisomerase, although, unless indicated otherwise, the methods can be performed using one, two, three or more topoisomerases, provided the substrate nucleic acid molecules contain the appropriate topoisomerase recognition sites. Similar considerations are relevant to topoisomerase-charged nucleic acid substrates, in that the topoisomerases can be the same or different.

A method described herein can be performed by contacting 1) a first nucleic acid molecule (which may be ss or ds) having a first end and a second end, wherein the first nucleic acid molecule has a topoisomerase recognition site at or near the 3' terminus and a topoisomerase recognition site at or near the 5' terminus of the first end or of the second end or of both ends; 2) at least a second nucleic acid molecule (which may also be ss or ds) having a first end and a second end; and 3) at least two (e.g., 2, 3, 4, 5, 6, 7, 8, 9, 10, etc.) site specific topoisomerases, under conditions such that all components are in contact and each of the topoisomerases can effect its activity. Upon cleavage of the termini of the substrate first nucleic acid molecule by the topoisomerases, the 5' terminus or the 3' terminus of one or both ends can comprise an overhanging sequence, or can be blunt ended, or one end can contain an overhang and the second end can be blunt ended. Where present, an overhanging sequence generally has sufficient complementarity to an overhanging sequence of the second (or other) nucleic acid molecule to allow for specific hybridization of the two molecules to each other.

Once nucleic acid molecules are joined by topoisomerase mediated joining methods described herein, the resulting nucleic acid molcules may then be used in recombination reactions, such as those described elsewhere herein.

The number of different topoisomerases useful in such an embodiment will depend, in part, on whether the first nucleic acid molecule contains topoisomerase recognition sites at only the first end or the second end, or contains topoisomerase recognition sites at both ends, and further, where the nucleic acid molecule contains topoisomerase recognition sites on both ends, whether at least the 3' recognition sites or the 5' recognition sites are different. In addition, the method can be performed such that one or more of the at least second nucleic acid molecule also can contain a topoisomerase recognition site at or near the 3' terminus and/or a topoisomerase recognition site at or near the 5' terminus of the first end or of the second end or of both ends, wherein the topoisomerase recognition sites at or near the 3' terminus or the 5' terminus or both of the other nucleic acid molecule can the same as or different from the topoisomerase recognition sites in the first nucleic acid molecule. As such, the number of different topoisomerase further will depend on the number of different substrate nucleic acid molecules being linked according to a method as described herein.

An advantage of performing a method described herein using a site specific topoisomerase is that the first nucleic acid molecule, the second nucleic acid molecule, and one or more additional nucleic acid molecules (which may be ss or ds) can be covalently linked in a predetermined directional orientation. An additional advantage is that a functional product can be selected *in vitro* by performing an amplification reaction using primers specific for the termini of the desired covalently linked recombinant nucleic acid molecule. As such, a covalently linked recombinant nucleic acid molecule (which may be ss or ds) generated according to a method described herein can be used directly in further procedures, for example, for transfecting a cell, or as a template for performing amplification (e.g., PCR), a recombination reaction (*e*.*g*., a recombination reaction such as those described herein), an *in vitro* transcription reaction, or a coupled transcription/translation reaction: Accordingly, the covalently linked recombinant nucleic acid molecule is useful, without further manipulation, for various purposes.

The first nucleic acid molecules, as well as other nucleic acids used in methods described herein, may be derived from at least a first population of nucleic acid molecules, for example, from a cDNA library or a combinatorial library such as a combinatorial library of synthetic oligonucleotides, and the second nucleic acid molecules, as well as other nucleic acids used in methods described herein, may be derived from at least a second population of source nucleic acid molecules. According to such a method, linking of first nucleic acid molecules with second nucleic acid molecules provides a means to generate combinatorial populations of covalently linked recombinant nucleic acid molecules (which may be ss or ds). In accordance with such a method, one or more target nucleic acid molecules also can be linked with the recombinant nucleic acid molecules of the population to produce additional populations. Such populations of combinatorial molecules can be further manipulated or analyzed, for example, by protein expression and screening for fusion proteins having desirable characteristics.

A method described herein is performed such that the first nucleic acid molecule (which may be ss or ds), as well as other nucleic acids used in methods described herein comprises an open reading frame, for example, an isolated cDNA or coding sequence of a gene, and a second nucleic acid molecule (which may be ss or ds) comprises a regulatory element such as a promoter, which can be operably covalently linked to the 5' end of the coding sequence such that the coding sequence can be transcribed therefrom. A second nucleic acid molecule, as well as other nucleic acids used in methods described herein, also can comprise two or more regulatory elements, for example, a promoter (*e*.*g*., a GAL4 promoter), an operator (*e*.*g*., a tet operator, a galactose operon operator, a lac operon operator, and the like), an internal ribosome entry site and an ATG initiator methionine codon, in operative linkage with each other, which can be operably covalently linked to the 5' end of a first nucleic acid molecule comprising a coding sequence. Such a method can further include contacting a third nucleic acid molecule (which may be ss or ds) comprising, for example, a polyadenylation signal, which can be operably covalently linked to the 3' end of the coding sequence. Such a method can be useful for generating an expressible nucleic acid molecule, which can be transcribed, translated, or both as a functional unit In addition, or alternatively, a nucleic acid molecule encoding a detectable marker, for example, an epitope tag, can be operably linked to a first or second (or other) nucleic acid molecule(s), according to a method described herein The generation of a recombinant nucleic acid molecule (which may be ss or ds) having a desired directional orientation of the nucleotide sequences in such a construct may be facilitated, for example, by including complementary 5' overhanging sequences at the termini of the nucleic acid molecules to be covalently linked together by the topoisomerase.

A method described herein may be performed such that at least the first nucleic acid molecule or the at least second nucleic acid molecule, as well as other nucleic acids used in methods described herein, is one of a plurality of nucleotide sequences, for example, a cDNA library, a combinatorial library of nucleotide sequences, or a variegated population of nucleotide sequences. A method described herein includes further contacting a generated covalently linked ds recombinant nucleic acid molecule (*e*.*g*., a recombinant nucleic acid molecule which is covalently linked in one or both strands) with a PCR primer pair, and amplifying all or a portion of the covalently linked recombinant nucleic acid molecule. In addition to generating a large amount of product, the amplification reaction can be selective for constructs comprising a desired covalently linked ds recombinant nucleic acid molecule, particularly where the nucleic acid molecules to be covalently linked comprise complementary overhanging sequences. As such, methods described herein provide an *in vitro* selection means that is suitable for high throughput analysis.

A method described herein is also exemplified by contacting 1) a first nucleic acid molecule (which may be ss or ds) having a first end and a second end, wherein, at the first end or second end or both, the first nucleic acid molecule has a topoisomerase covalently bound to the 3'terminus ("topoisomerase-charged"); and 2) at least a second topoisomerase-charged nucleic acid molecule (which may be ss or da). Preferably, the topoisomerase-charged nucleic acid molecules contain a 5' hydroxyl group at the ends containing the bound topoisomerase, although 5' hydroxy groups also can be generated using a phosphatase. The methods described herein can be performed using only a first nucleic acid molecule and a second nucleic acid molecule, or can include a third, fourth or more nucleic acid molecules (which may be ss or ds) as desired, wherein each nucleotide sequence is as defined. A first or second (or other) nucleic acid molecule independently can have a topoisomerase covalently bound to a 3' terminus of one end or at both ends of the nucleotide sequence, and, unless indicated otherwise, the first and second (or other) nucleic acid molecules can be the same or can be different. In certain such aspects, at least one of the nucleic acid molecules used in the methods described herein will comprise at least one recombination site. Further, nucleic acid molecules generated by methods described above may be used in recombination reactions, such as those described elsewhere herein.

The methods described herein are further exemplified by contacting 1) a first nucleic acid molecule (which may be ss or ds) having a first end and a second end, wherein, at the first end or second end or both, the first nucleic acid molecule has a topoisomerase covalently bound to a 5' terminus (i.e., a topoisomerase-charged 5' terminus); and 2) at least a second topoisomerase-charged nucleic acid molecule (which may be ss or ds) comprising at least one topoisomerase-charged 5' terminus. The topoisomerase-charged nucleic acid molecules can contain a 3' hydroxyl group at the ends containing the bound topoisomerase, or a 3' hydroxyl group can be generated using a phosphatase. As disclosed herein, such a method can be performed using only a first nucleic acid molecule and a second nucleic acid molecule, or can include a third, fourth or more nucleic acid molecules (which may be ss or ds) as desired, wherein each nucleotide sequence is as defined, including comprising at least one topoisomerase-charged 5' terminus. A first or second (or other) nucleic acid molecule independently can have a topoisomerase covalently bound to a 5' terminus of one end or at both ends of the nucleic acid molecule, and, unless indicated otherwise, the first and second (or other) nucleic acid molecules can be the same or can be different. In certain such aspects, at least one of the nucleic acid molecules used in the methods described herein will comprise at least one recombination site. Further, nucleic acid molecules generated by methods described above and elsewhere herein may also be used in recombination reactions such as those described elsewhere herein.

A method described herein is additionally exemplified by contacting 1) a first nucleic acid molecule having a first end and a second end, wherein, at the first end or second end or both, the first nucleic acid molecule has a first topoisomerase covalently bound to the 5' terminus and a second topoisomerase covalently bound to the 3' terminus of the first end or the second end or both (i.e., one or both ends contain a topoisomerase charged 5' terminus and a topoisomerase-charged 3' terminus); and 2) at least a second nucleic acid molecule, which, preferably, has or can be made to have hydroxyl groups at the 5' terminus and 3' terminus of an end to be covalently linked to an end of the first nucleic acid molecule containing the topoisomerases. The method also can be performed wherein either the 5' terminus or 3' terminus of the end containing a topoisomerase-charged 3'terminus or topoisomerase-charged 5' terminus, respectively, contains a topoisomerase recognition site, wherein the method further includes contacting the components with a topoisomerase that can effect its activity with respect to the topoisomerase recognition site. In certain such aspects, at least one of the nucleic acid molecules used in the methods described herein will comprise at least one recombination site. Further, nucleic acid molecules generated by methods described above and elsewhere herein may also be used in recombination reactions, such as those described elsewhere herein.

Such a method as described herein can be performed using only a first nucleic acid molecule and a second nucleic acid molecule, or can include a third, fourth or more nucleic acid molecule as desired, wherein the nucleic acid molecules are as defined for the first nucleic acid molecule, the second nucleic acid molecule, or a combination thereof. A first or second (or other) nucleic acid molecule independently can, but need not, have one or more topoisomerases covalently bound to a 5' terminus, 3' terminus, or both 5' and 3' termini of the second end (i.e., the undefined end). Further, one or more of these nucleic acid molecules may additionally comprise one or more recombination sites. Unless indicated otherwise, the first and second (or other) nucleic acid molecules can be the same or can be different.

Described herein is a method of generating a covalently linked ds recombinant nucleic acid molecule by 1) amplifying a portion of a first nucleic acid molecule using a PCR primer pair, wherein at least one primer of the primer pair encodes a complement of a topoisomerase recognition site, and, optionally, of one or more recombination sites, thereby producing an amplified first nucleic acid molecule having a first end and a second end, wherein the first end or second end or both has a topoisomerase recognition site at or near the 3'terminus; and 2) contacting a) the amplified first nucleic acid molecule; b) at least a second nucleic acid molecule having a first end and a second end, wherein the first end or second end or both has a topoisomerase recognition site, or cleavage product thereof, at or near the 3' terminus and has, or can be made to have, a hydroxyl group at the 5' terminus of the same end; and c) a site specific topoisomerase, under conditions such that the topoisomerase can cleave the end of the amplified first nucleic acid molecule having a topoisomerase recognition site and the end (or ends) of the at least second nucleic acid molecule having a topoisomerase recognition site, and can effect its ligating activity. The PCR primer that encodes a complement of topoisomerase recognition site can have a hydroxyl group at its 5' terminus, or the amplified first nucleic acid molecule generated using the primer can be contacted with a phosphatase to generate a hydroxyl group at its 5' terminus. The PCR primer encoding the complement of a topoisomerase recognition site also can comprise a nucleotide sequence at its 5' terminus such that, upon cleavage by a site specific topoisomerase of a first nucleic acid molecule amplified using the primer, the nucleic acid molecule contains a 5' overhanging sequence, which is complementary to a 5' overhanging sequence of a second (or other) nucleic acid molecule to which the first nucleic acid molecule is to be covalently linked according to a method described herein. At least one of the nucleic acid molecules used in the methods described herein may comprise at least one recombination site. Further, nucleic acid molecules generated by methods described above and elsewhere herein may also be used in recombination reactions, such as those described elsewhere herein.

Described herein is a method of generating a covalently linked ds recombinant nucleic acid molecule by 1) amplifying a portion of a first nucleic acid molecule using a PCR primer pair, wherein at least one primer of the primer pair encodes a topoisomerase recognition site, and, optionally, one or more recombination sites, thereby producing an amplified first nucleic acid molecule having a first end and a second end, wherein the first end or second end or both has a topoisomerase recognition site at or near the 5' terminus; and 2) contacting a) the amplified first nucleic acid molecule; b) at least a second nucleic acid molecule having a first end and a second end, wherein the first end or second end or both has a topoisomerase recognition site at or near the 5' terminus and has, or can be made to have, a hydroxyl group at the 3' terminus of the same end; and c) at least one site specific topoisomerase, under conditions such that the at least one topoisomerase can cleave the end of the amplified first nucleic acid molecule having a topoisomerase recognition site and the end (or ends) of the at least second nucleic acid molecule having a topoisomerase recognition site, and can effect its ligating activity. The amplified first nucleic acid molecule generally has a hydroxyl group at the 3' terminus of the end containing the topoisomerase recognition site, or can be modified to contain such a 3' hydroxyl group. The PCR primer encoding the topoisomerase recognition site can further comprise a nucleotide sequence at its 5' terminus, i.e., 5' to the topoisomerase recognition site, such that, upon cleavage of the amplified first nucleic acid molecule by a site specific topoisomerase, the nucleic acid molecule contains a 3' overhanging sequence, which is complementary to a 3' overhanging sequence of a second (or other) nucleic acid molecule to which the first nucleic acid molecule is to be covalently linked according to a method described herein. At least one of the nucleic acid molecules used in the methods described herein may comprise at least one recombination site. Further, nucleic acid molecules generated by methods described above and elsewhere herein may also be used in recombination reactions, such as those described elsewhere herein.

Described herein is a method of generating a covalently linked ds recombinant nucleic acid molecule by 1) amplifying a portion of a first nucleic acid molecule using a PCR primer pair, wherein at least one primer of the primer pair includes a topoisomerase recognition site, a nucleotide sequence complementary to a topoisomerase recognition site, and, optionally, a recombination site, thereby producing an amplified first nucleic acid molecule having a first end and a second end, wherein the amplified first nucleic acid molecule has a topoisomerase recognition site at or near the 5' terminus and a topoisomerase recognition site at or near the 3' terminus of the first end or of the second end or of both ends; and 2) contacting a) the amplified first nucleic acid molecule; b) at least a second nucleic acid molecule having a first end and a second end, wherein the second nucleic acid molecule has, or can be made to have, a 5' hydroxyl group and a 3' hydroxyl group at the first end or at second end or at both ends; and c) at least two site specific topoisomerases, under conditions such that i) at least one topoisomerase can cleave the topoisomerase recognition site at or near the 5' terminus of the end of the amplified first nucleic acid molecule, and can effect its ligating activity, and ii) at least one topoisomerase can cleave the topoisomerase recognition site at or near the 3' terminus of the end of the amplified first nucleic acid molecule, and can effect its ligating activity. Accordingly, described herein is a nucleic acid molecule containing, at one or both ends, a topoisomerase recognition site at or near the 5' terminus and a topoisomerase recognition site at or near the 3' terminus. In addition, described herein is such a nucleic acid molecule, which is topoisomerase charged at the 5' terminus or the 3' terminus or both. In certain such aspects, at least one of the nucleic acid molecules used in the methods described herein will comprise at least one recombination site. Further, nucleic acid molecules generated by methods described above and elsewhere herein may also be used in recombination reactions, such as those described elsewhere herein.

Described herein is an oligonucleotide containing at least one recognition site of one or more type IA site specific topoisomerases, at least one nucleotide sequence complementary to a recognition site of one or more type IB site specific topoisomerases and, optionally, at least one recombination site. Such an oligonucleotide is useful, for example, as a primer for a primer extension reaction or as one of a primer pair for performing an amplification reaction such as PCR. Such an oligonucleotide, referred to herein as an oligonucleotide primer, can be one of a primer pair, which can be useful for generating a ds nucleic acid amplification product that contains, at one end, a type IA topoisomerase recognition site at or near the 5' terminus and, at the same end, a type IB topoisomerase recognition site at or near the 3' terminus. The oligonucleotide primer can further contain a nucleotide sequence encoding (or complementary to) any other nucleotide sequence or peptide of interest, for example, a restriction endonuclease recognition site, a peptide tag, and, if desired, one or more additional type IA or type IB topoisomerase recognition sites, thereby allowing selection of one or more convenient or readily available topoisomerases for practicing a method described herein. The oligonucleotide primer can further comprise a nucleotide sequence at its 5' terminus, i.e., 5' to the type IA topoisomerase recognition site or to the nucleotide sequence complementary to the type IB topoisomerase recognition site, such that, upon cleavage of the amplified first nucleic acid molecule by as site specific topoisomerase, the nucleic acid molecule contains a 3' or 5' overhanging sequence, respectively, which is complementary to a 3' or 5' overhanging sequence, respectively, of a second (or other) nucleic acid molecule to which the first nucleic acid molecule is to be covalently linked according to a method described herein, or the oligonucleotide primer can be designed such that, upon cleavage of an amplified nucleic acid molecule generated therefrom, a blunt end topoisomerase charged nucleic acid molecule is generated.

Described herein is an oligonucleotide which contains at least one topoisomerase recognition site, or a nucleotide sequence complementary thereto, and at least one recombination site. Such an oligonucleotide may be used as described above, for example as one member of a primer pair.

Oligonucleotides described herein will often be between 15-20, 15-30, 15-50, 20-30, 20-50, 30-40, 30-50, 30-80, 30-100, 40-50, 40-70, 40-80, 40-100, 50-60, 50-80, 50-100, 15-80, 15-100, or 20-100 (or the like) nucleotides in length.

Described herein is a primer pair, which includes at least one oligonucleotide primer as defined above, wherein one of the primers is useful as a forward primer and the primer is useful as a reverse primer in an amplification reaction. The second primer in such a primer pair can, but need not, include a type IA topoisomerase recognition site, a nucleotide sequence complementary to a type IB topoisomerase recognition site, or both, and can include any other nucleotide sequence of interest and/or at least one recombination site. In one embodiment, the primer pair includes two oligonucleotide primers of the invention, wherein one oligonucleotide primer is useful as a forward primer and the second oligonucleotide primer is useful as a reverse primer, such a primer pair being useful, for example, for generating a nucleic acid molecule amplification product having topoisomerase recognition sites at both termini of both ends and/or one or more recombination sites, wherein the type IA or type IB or both topoisomerase recognition sites at the termini are the same or different.

Described herein is a nucleic acid molecule, which has a first end and a second end, and which contains a type IA topoisomerase recognition site at or near the 5' terminus and a type IB topoisomerase recognition site at or near the 3' terminus of the first end or of the second end or of both ends. In addition, described herein is a nucleic acid molecule as defined above, except wherein the nucleic acid molecule is a topoisomerase charged molecule, comprising a stably bound type IA topoisomerase or a type IB topoisomerase or both, at one or both ends, as desired. These nucleic acid molcules may further comprise one or more recombination sites.

The first nucleic acid molecule, as well as other nucleic acids used in methods described herein, may comprise an expressible nucleotide sequence which encodes molecules such as a polypeptide (which may be, *e*.*g*., a polypeptide with an intein), an antisense nucleotide sequence, interference RNA (*i.e.,* "RNAi") molecule(s), a ribozyme, a transfer RNA (*i.e.,* a tRNA, including but not limited to a supressor tRNA), a triplexing nucleotide sequence, and the like, and the second (or other) nucleic acid molecule comprises a transcription regulatory element such as a promoter (*e*.*g.,* a GAL4 operator), an operator (*e*.*g*., a tet operator, a galactose operon operator, a lac operon operator, and the like), an enhancer, a silencer, a translation start site, or a polyadenylation signal, or encodes a translation regulatory element such as an initiator methionine, a STOP codon, a cell compartmentalization domain, a homology domain, or the like, or a combination thereof in operative linkage. A second (or other) nucleic acid molecule, as well as other nucleic acids used in methods described herein, which can be an amplified second (or other) nucleic acid molecule prepared as for the amplified first nucleic acid molecule, also can comprise one or more multiple cloning sites ("MCS"), a detectable label, for example, an enzyme, a substrate for an enzyme, a fluorescent compound, a luminescent compound, a chemiluminescent compound, a radionuclide, a paramagnetic compound, and biotin; or can include a tag, which can be an oligonucleotide tag or can be a peptide tag, for example, a polyhistidine tag, a V5 epitope, or a myc epitope.

A method described herein may be performed using a first nucleic acid molecule that encodes a polypeptide (*e*.*g.,* a polypeptide which contains an intein), or a domain thereof, and a second (or other) nucleic acid molecule that encodes a transcription activation domain or a DNA binding domain. Such a method can be used to generate covalently linked ds recombinant nucleic acid molecules that encode chimeric polypeptides useful for performing a two hybrid assay system, particularly a high throughput two hybrid assay. In still another embodiment, the first nucleic acid molecules comprises a plurality of nucleotide sequences, which can be a cDNA library, a combinatorial library of nucleotide sequences, a variegated population of nucleotide sequences, or the like.

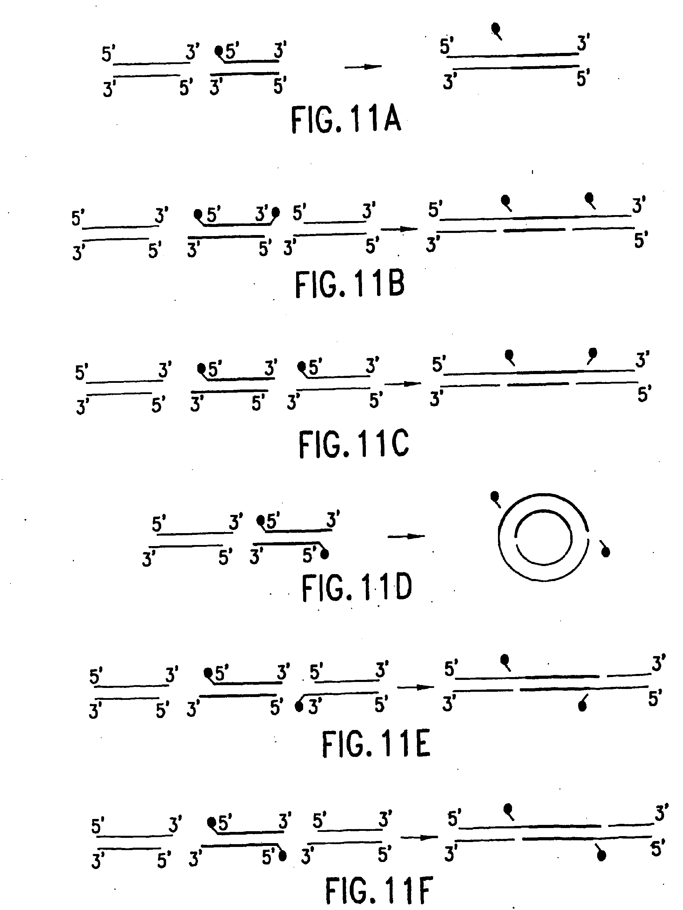

A method described herein provides a means to generate a covalently linked ds recombinant nucleic acid molecule useful for site specific insertion into a target genomic DNA sequence. The target genomic DNA sequence can be any genomic sequence, particularly a gene, and preferably a gene for which some or all of the nucleotide sequence is known. The method can be performed utilizing two sets of PCR primer pairs and a nucleic acid molecule. The nucleic acid molecule has a first end and a second end and encodes a polypeptide, for example, a selectable marker, wherein the nucleic acid molecule comprises a topoisomerase recognition site or cleavage product thereof at the 3' terminus of each end and, optionally, a hydroxyl group at the 5' terminus of each end, and wherein, preferably, the 5' termini comprise overhanging sequences, which are different from each other. Similarly, the nucleic acid molecule can comprise a topoisomerase recognition site or cleavage product thereof at or near the 5' terminus of one or both ends and, optionally, a hydroxyl group at the 3' terminus of one or both end, and wherein one or both the 3' termini can comprise overhanging sequences, which can be the same as or, preferably, different from each other; or the 5' terminus and 3' terminus of one or both ends of the nucleic acid molecule each can comprise a topoisomerase recognition site or cleavage product thereof (see Figure 11). In certain such aspects, at least one of the nucleic acid molecules used in the methods described herein will comprise at least one recombination site. Further, nucleic acid molecules generated by methods described above and elsewhere herein may also be used in recombination reactions, such as those described elsewhere herein.

The two sets of PCR primer pairs will generally be selected such that, in the presence of an appropriate DNA polymerase such as Taq polymerase and a template comprising the sequences to be amplified, the primers amplify portions of a genomic DNA sequence that are upstream (and adjacent to) and downstream (and adjacent to) of the target site for insertion of the polypeptide (e.g., selectable marker). The sets of PCR primer pairs also are designed such that the amplification products contain a topoisomerase recognition site at least at the end to be covalently linked to the selectable marker, including at or near the 5' terminus, or the 3' terminus, or both, as appropriate for the particular method described herein being practiced. As such, the first PCR primer pair can include, for example; 1) a first primer, which comprises, in an orientation from 5' to 3', a nucleotide sequence complementary to a 5' overhanging sequence of the end of the selectable marker to which the amplification product is to be covalently linked, a nucleotide sequence complementary to a topoisomerase recognition site, and a nucleotide sequence complementary to a 3' sequence of a target genomic DNA sequence; and 2) a second primer, which comprises a nucleotide sequence of the target genomic DNA upstream of the 3' sequence to which the first primer is complementary. The second PCR primer pair includes 1) a first primer, which comprises, from 5' to 3', a nucleotide sequence complementary to the 5' overhanging sequence of the end of the selectable marker to which it is to be covalently linked, a nucleotide sequence complementary to a topoisomerase recognition site, and a nucleotide sequence of a 5' sequence of a target genomic DNA sequence, wherein the 5' sequence of the target genomic DNA is downstream of the 3' sequence of the target gnomic DNA to which the first primer of the first PCR primer pair is complementary; and 2) a second primer, which comprises a nucleotide sequence complementary to a 3' sequence of the target genomic DNA that is downstream of the 5' sequence of the target genomic DNA contained in the first primer.

Upon contact of the nucleic acid molecule comprising the selectable marker, the PCR amplification products, and at least one topoisomerase, a covalently linked ds recombinant nucleic acid molecule is generated according to a method described herein. The generated ds recombinant nucleic acid molecule is useful for performing homologous recombination in a genome, for example, to knock-out the function of a gene in a cell, or to confer a novel phenotype on the cell containing the generated ds recombinant nucleic acid molecule. The method can further be used to produce a transgenic non-human organism having the generated recombinant nucleic acid molecule stably maintained in its genome.

Described herein are compositions prepared according to the methods described herein, and to compositions useful for practicing the methods. Such compositions can include one or more reactants used in the methods described herein and/or one or more ds recombinant nucleic acid molecules produced according to a method described herein. Such compositions can include, for example, one or more nucleic acid molecules with one or more topoisomerase recognition sites; one or more topoisomerase-charge nucleic acid molecules; one or more nucleic acid molecules comprising one or more recombination sites; one or more primers useful for preparing a nucleic acid molecule containing a topoisomerase recognition site at one or both termini of one or both ends of an amplification product prepared using the primer; one or more topoisomerases; one or more substrate nucleic acid molecules, including, for example, nucleotide sequences encoding tags, markers, regulatory elements, or the like; one or more covalently linked ds recombinant nucleic acid molecules produced according to a method described herein; one or more cells containing or useful for containing a nucleic acid molecule, primer, or recombinant nucleic acid molecule as disclosed herein; one or more polymerases for performing a primer extension or amplification reaction; one or more reaction buffers; and the like. A composition as described herein may comprise two or more different topoisomerase-charged nucleic acid molecules and/or two or more different recombination sites. The composition can further comprise at least one topoisomerase. A composition as described herein also can comprise a site specific topoisomerase and a covalently linked ds recombinant nucleic acid molecule, wherein the recombinant nucleic acid molecule contains at least one topoisomerase recognition site for the site specific topoisomerase in each strand, and wherein a topoisomerase recognition site in one strand is within about 100 nucleotides of a topoisomerase recognition site in the complementary strand, generally within about five, ten, twenty or thirty nucleotides.

Product molecules produced by methods described herein may comprise any combination of starting molecules (or portions thereof) and can be any size and be in any form (e.g., circular, linear, supercoiled, etc.), depending on the starting nucleic acid molecule or segment, the location of the recombination sites on the molecule, and the order of recombination of the sites.

Any of the product molecules described herein may be further manipulated, analyzed or used in any number of standard molecular biology techniques or combinations of such techniques (*in vitro* or *in vivo*). These techniques include sequencing, amplification, nucleic acid synthesis, protein or peptide expression (for example, fusion protein expression, antibody expression, hormone expression etc.), protein-protein interactions (2-hybrid or reverse 2-hybrid analysis), homologous recombination or gene targeting, and combinatorial library analysis and manipulation. Also described herein is the cloning of the nucleic acid molecules (preferably by recombination) into one or more vectors or converting the nucleic acid molecules described herein into a vector by the addition of certain functional vector sequences (e.g., origins of replication). Recombination and/or topoisomerase-mediated joining may be accomplished *in vitro* and further manipulation or analysis is performed directly *in vitro.* Thus, further analysis and manipulation will not be constrained by the ability to introduce the molecules described herein into a host cell and/or maintained in a host cell. Thus, less time and higher throughput may be accomplished by further manipulating or analyzing the molecules of the invention directly in *in vitro,* although *in vitro* analysis or manipulation can be done after passage through host cells or can be done directly *in vivo* (while in the host cells).

Nucleic acid synthesis steps, according to the disclosure herein, may comprise:
(a) mixing a nucleic acid molecule of interest or template with one or more primers and one or more nucleotides to form a mixture; and
(b) incubating said mixture under conditions sufficient to synthesize a nucleic acid molecule complementary to all or a portion of said molecule or template.

The synthesized molecule may then be used as a template for further synthesis of a nucleic acid molecule complementary to all or a portion of the first synthesized molecule. Accordingly, a double stranded nucleic acid molecule (e.g., DNA) may be prepared. Preferably, such second synthesis step is preformed in the presence of one or more primers and one or more nucleotides under conditions sufficient to synthesize the second nucleic acid molecule complementary to all or a portion of the first nucleic acid molecule. Typically, synthesis of one or more nucleic acid molecules is performed in the presence of one or more polymerases (preferably DNA polymerases which may be thermostable or mesophilic), although reverse transcriptases may also be used in such synthesis reactions. Accordingly, the nucleic acid molecules used as templates for the synthesis of additional nucleic acid molecules may be RNA, mRNA, DNA or non-natural or derivative nucleic acid molecules. Nucleic acid synthesis, according to the disclosure herein, may be facilitated by incorporating one or more primer sites into the product molecules through the use of starting nucleic acid molecules containing such primer sites. Thus, by the methods described herein, primer sites may be added at one or a number of desired locations in the product molecules, depending on the location of the primer site within the starting molecule and the order of addition of the starting molecule in the product molecule.

Sequencing steps, according to the disclosure herein, may comprise:
(a) mixing a nucleic acid molecule to be sequenced with one or more primers, one or more nucleotides and one or more termination agents to form a mixture;
(b) incubating said mixture under conditions sufficient to synthesize a population of molecules complementary to all or a portion of said molecules to be sequenced; and
(c) separating said population to determine the nucleotide sequence of all or a portion of said molecule to be sequenced.

Such sequencing steps are preferably performed in the presence of one or more polymerases (e.g., DNA polymerases and/or reverse transcriptases) and one or more primers. Preferred terminating agents for sequencing include derivative nucleotides such as dideoxynucleotides (ddATP, ddTTP, ddGTP, ddCTP and derivatives thereof). Nucleic acid sequencing, as described herein, may be facilitated by incorporating one or more sequencing primer sites into the product molecules through the use of starting nucleic acid molecules containing such primer sites. Thus, by the methods described herein sequencing primer sites may be added at one or a number of desired locations in the product molecules, depending on the location of the primer site within the starting molecule and the order of addition of the starting molecule in the product molecule.

Protein expression steps, according to the disclosure herein, may comprise:
(a) obtaining a nucleic acid molecule to be expressed which comprises one or more expression signals; and
(b) expressing all or a portion of the nucleic acid molecule under control of said expression signal thereby producing a peptide or protein encoded by said molecule or portion thereof.

In this context, the expression signal may be said to be operably linked to the sequence to be expressed. The protein or peptide expressed is preferably expressed in a host cell (*in vivo*), although expression may be conducted *in vitro* using techniques well known in the art. Upon expression of the protein or peptide, the protein or peptide product may optionally be isolated or purified. Moreover, the expressed protein or peptide may be used in various protein analysis techniques including 2-hybrid interaction, protein functional analysis and agonist/antagonist-protein interactions (e.g., stimulation or inhibition of protein function through drugs, compounds or other peptides). The novel and unique hybrid proteins or peptides (e.g., fusion proteins) produced as described herein and particularly from expression of the combinatorial molecules described herein may generally be useful for therapeutics. Protein expression, according to the disclosure herein, may be facilitated by incorporating one or more transcription or translation signals or regulatory sequences, start codons, termination signals, splice donor/acceptor sequences (e.g., intronic sequences) and the like into the product molecules through the use of starting nucleic acid molecules containing such sequences. Thus, by the methods described herein, expression sequences may be added at one or a number of desired locations in the product molecules, depending on the location of such sequences within the starting molecule and the order of addition of the starting molecule in the product molecule.

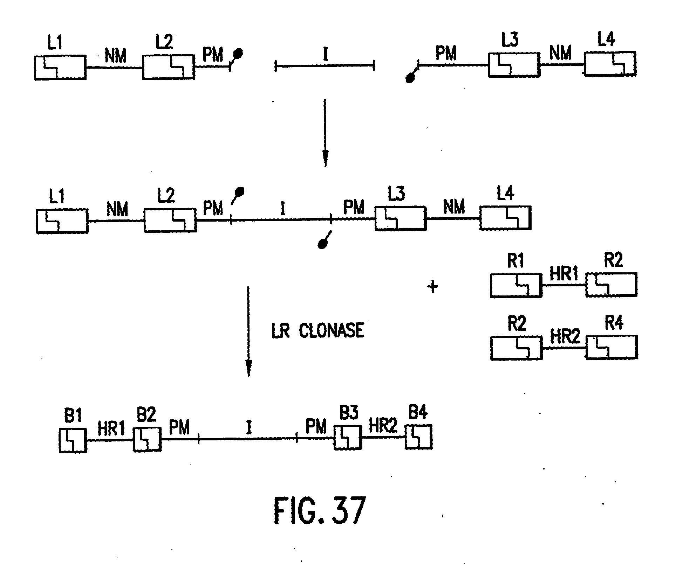

Homologous recombination, according to the disclosure herein, may comprise:
(a) mixing at least a first nucleic acid molecule as described herein (which is preferably a product molecule) comprising one or more recombination sites and/or one or more toposiomerase recognition sites with at least one target nucleic molecule, wherein said first and target molecules have one or more homologous sequences; and
(b) causing said first and target nucleic acid molecules to recombine by homologous recombination. One example of a nucleic acid construct that can be used for homologous recombination is depicted in Figure 37. Methods for preparing nucleic acid molecules which can be used for homologous recombination are described herein, and nucleic acid molecules prepared by such methods, as well as cells which have undergone homologous recombination according to methods described herein.

Such homologous recombination may occur *in vitro*, but preferably is accomplished *in vivo* (e.g., in a host cell). Preferably, homologous recombination causes transfer of all or a portion of a nucleic acid molecule described herein containing recombination sites (the first nucleic acid molecule) into one or more positions of the target nucleic acid molecule containing homologous sequences. Selection of such homologous recombination may be facilitated by positive or negative selection (e.g., using selectable markers) to select for a desired product and/or against an undesired product. In a preferred aspect, the nucleic acid molecule described herein may comprise at least one selectable marker and at least two sequences which are homologous to the target molecule. Preferably, the first molecule comprises at least two homologous sequences flanking at least one selectable marker.

The present invention thus facilitates construction of gene targeting nucleic acid molecules or vectors which may be used to knock-out or mutate a sequence or gene of interest (or alter existing sequences, for example to convert a mutant sequence to a wild type sequence), particularly genes or sequences within a host or host cells such as animal, plant, human, insect, bacteria, and the like or sequences of adventitious agents such as viruses within such host or host cells. Such gene targeting may preferably comprise targeting a sequence on the genome of such host cells. Such gene targeting may be conducted *in vitro* or *in vivo.* Thus, described herein is a method of targeting or mutating a sequence or a gene comprising:
(a) obtaining at least one nucleic acid molecule described herein comprising one or more recombination sites and/or one or more topoisomerase recognition sites (and preferably one or more selectable markers), wherein said molecule comprises one or more sequences homologous to the target gene or sequence of interest (said one or more homologous sequences preferably flank one or more selectable markers on the molecule described herein); and
(b) contacting said molecule with one or more target genes or sequences of interest under conditions sufficient to cause homologous recombination at one or more sites between said target sequence or gene of interest and said molecule described herein, thereby causing insertion of all or a portion of the molecule described herein within the target sequence or gene.

Such targeting method may cause deletion, inactivation or partial inactivation of the sequence or target gene such that an expression product (typically a protein or peptide) normally expressed by such sequence is not produced or produced at a higher or lower level or to the extent produced is has an altered protein sequence which may result in more or less activity or in an inactive or partially inactive expression product. The selectable marker preferably present on the molecule described herein facilitates selection of candidates (for example host cells) in which the homologous recombination event was successful. Thus, described herein is a method to produce host cells, tissues, organs, and non-human animals (e.g., transgenic animals) containing the modified gene or sequence produced by the targeting methods described herein. The modified sequence or gene preferably comprise at least one recombination site and/or at least one selectable marker provided by the molecule described herein.

Thus, described herein is a method of targeting or mutating a sequence or a gene comprising:
(a) obtaining at least one nucleic acid molecule described herein comprising one or more recombination sites, at least one selectable marker flanked by one or more sequences homologous to the target gene or sequence of interest and, optionally, one or more topoisomerase recognition sites;
(b) contacting said molecule with one or more target genes or sequences of interest under conditions sufficient to cause homologous recombination at one or more sites between said target sequence or gene of interest and said molecule, thereby causing insertion of all or a portion of the molecule described herein (and preferably causing insertion of at least one selectable marker and/or at least one recombination site) within the target sequence or gene; and
(c) optionally selecting for said sequence or gene comprising all or a portion of the molecule described herein or for a host cell containing said gene or sequence containing all or a portion of said molecule described herein.

Recombination sites introduced into targeted sequences according to the disclosure herein may be used to excise or remove all or a portion of the molecule inserted into the target sequence. Thus, the disclosure herein allows for *in vitro* or *in vivo* removal of such sequences and thus may allow for reactivation of the target gene or sequence. After identification and isolation of a sequence containing the alterations introduced as above, a selectable marker present on the molecule described herein may be removed.

Described herein are methods for cloning the starting or product nucleic acid molecules described herein into one or more vectors or converting the product molecules described herein into one or more vectors. In one aspect, the starting molecules are recombined to make one or more product molecules and such product molecules are cloned (preferably by recombination) into one or more vectors. In another aspect, the starting - , molecules are cloned directly into one or more vectors such that a number of starting molecules are joined within the vector, thus creating a vector containing the product molecules described herein. In another aspect, the starting molecules are cloned directly into one or more vectors such that the starting molecules are not joined within the vector (i.e., the starting molecules are separated by vector sequences). In yet another aspect, a combination of product molecules and starting molecules may be cloned in any order into one or more vectors, thus creating a vector comprising a new product molecule resulting from a combination of the original starting and product molecules.

Thus, also described herein is a method of cloning comprising:
(a) at least one nucleic acid molecule described herein comprising one or more recombination sites and/or one or more topoisomerase recognition sites; and
(b) transferring all or a portion of said molecule into one or more vectors. Also described herein are vectors prepared by such methods, compositions comprising these vectors, and methods using these vectors.

Such vectors will often comprise one or more recombination sites and/or one or more topoisomerase recognition sites, and the transfer of the molecules into such vectors is preferably accomplished by recombination between one or more sites on the vectors and one or more sites on the molecules described herein. The product molecules described herein may be converted to molecules which function as vectors by including the necessary vector sequences (e.g., origins of replication). Thus, according to the disclosure herein, such vectors sequences may be incorporated into the product molecules through the use of starting molecules containing such sequences. Such vector sequences may be added at one or a number of desired locations in the product molecules, depending on the location of the sequence within the starting molecule and the order of addition of the starting molecules in the product molecule. The product molecule containing the vector sequences may be in linear form or may be converted to a circular or supercoiled form by causing recombination of recombination sites within the product molecule or by a topoisomerase-mediated joining reaction. Often, circularization of such product molecule is accomplished by recombining recombination sites at or near both termini of the product molecule.

The vector sequences described herein may comprise one or a number of elements and/or functional sequences and/or sites (or combinations thereof) including one or more sequencing or amplification primer sites, one or more multiple cloning sites, one or more selectable markers (e.g., toxic genes, antibiotic resistance genes, selectable markers etc.), one or more transcription or translation sites or signals, one or more transcription or translation termination sites, one or more topoisomerase recognition sites, one or more topoisomerases, one or more origins of replication, one or more recombination sites (or portions thereof), etc. The vector sequences used as described herein may also comprise stop codons which may be suppressed to allow expression of desired fusion proteins as described herein. Thus, according to the disclosure herein, vector sequences may be used to introduce one or more of such elements, functional sequences and/or sites into any of the nucleic acid molecule described herein, and such sequences may be used to further manipulate or analyze any such nucleic acid molecule cloned into such vectors. For example, primer sites provided by a vector (preferably located on both sides of the insert cloned in such vector) allow sequencing or amplification of all or a portion of a product molecule cloned into the vector. Additionally, transcriptional or regulatory sequences contained by the vector allows expression of peptides, polypeptides or proteins encoded by all or a portion of the product molecules cloned to the vector. Likewise, genes, portion of genes or sequence tags (such as GUS, GST, GFP, His tags, epitope tags and the like) provided by the vectors allow creation of populations of gene fusions with the product molecules cloned in the vector or allows production of a number of peptide, polypeptide or protein fusions encoded by the sequence tags provided by the vector in combination with the product sequences cloned in such vector. Such genes, portions of genes or sequence tags may be used in combination with optionally suppressed stop codons to allow controlled expression of fusion proteins encoded by the sequence of interest being cloned into the vector and the vector supplied gene or tag sequence. In a construct, the vector may comprise one or more recombination sites, one or more stop codons and one or more tag sequences. In some embodiments, the tag sequences may be adjacent to a recombination site. Optionally, a stop codon may be incorporated into the sequence of the tag or in the sequence of the recombination site in order to allow controlled addition of the tag sequence to the gene of interest. In embodiments of this type, the gene of interest may be inserted into the vector by recombinational cloning such that the tag and the coding sequence of the gene of interest are in the same reading frame. The gene of interest may be provided with translation initiation signals, *e*.*g*., Shine-Delgamo sequences, Kozak sequences and/or IRES sequences, in order to permit the expression of the gene with a native N-terminal when the stop codon is not suppressed. The gene of interest may also be provided with a stop codon at the 3'-end of the coding sequence. In some embodiments, a tag sequence may be provided at both the N- and C-terminals of the gene of interest. Optionally, the tag sequence at the N-terminal may be provided with a stop codon and the gene of interest may be provided with a stop codon and the tag at the C-terminal may be provided with a stop codon. The stop codons may be the same or different. In some embodiments, the stop codon of the N-terminal tag is different from the stop codon of the gene of interest. In embodiments of this type, suppressor tRNAs corresponding to one or both of the stop codons may be provided. When both are provided, each of the suppressor tRNAs may independently be provided on the same vector, a different vector or in the host cell genome. The suppressor tRNAs need not both be provided in the same way, for example; one may be provided on the vector containing the gene of interest while the other may be provided in the host cell genome. In this way, the nucleic acid molecules described herein may comprise a suppressible stop codon that separates two coding regions. Depending on the location of the expression signals (e.g., promoters), expression of the suppressor tRNA results in suppression of the stop codon(s), thereby allowing the production of a fusion peptide, for example a fusion peptide having an affinity tag sequence at the N-and/or C-terminus of the expressed protein. By not suppressing the stop codon(s), expression of the sequence of interest without the N- and/or C-terminal tag sequence may be accomplished. Thus, the disclosure herein allows through recombination efficient construction of vectors containing a gene or sequence of interest (e.g., one or more open reading frames or "orfs") for controlled expression of fusion proteins depending on the need. Preferably, the starting nucleic acid molecules or product molecules described herein which are cloned into one or more vectors comprise at least one open reading frame (orf). Such starting or product molecules may also comprise functional sequences (e.g., primer sites, transcriptional or translation sites or signals, termination sites (e.g., stop codons which may be optionally suppressed), origins of replication, and the like) and preferably comprise sequences that regulate gene expression including transcriptional regulatory sequences and sequences that function as internal ribosome entry sites (IRES). Preferably, at least one of the starting or product molecules and/or vectors may comprise sequences that function as a promoter. Such starting or product molecules and/or vectors may also comprise transcription termination sequences, selectable markers, restriction enzyme recognition sites, and the like.

In some embodiments, the vector comprise two copies of the same selectable marker, each copy flanked by recombination sites and/or topoisomerase recognition sites. In other embodiments, the vector comprises two different selectable markers each flanked by two recombination sites. In some embodiments, one or more of the selectable markers may be a negative selectable marker.

Described herein is a method of cloning comprising providing at least a first nucleic acid molecule comprising at least a first and a second recombination site and at least a second nucleic acid molecule comprising at least a third and a fourth recombination site, wherein either the first or the second recombination site is capable of recombining with either the third or the fourth recombination site and conducting a recombination reaction such that the two nucleic acid molecules are recombined into one or more product nucleic acid molecules and cloning the product nucleic acid molecules into one or more vectors. In certain such embodiments, the recombination sites flank the first and/or second nucleic acid molecules. Moreover, the cloning step is often accomplished by the recombination reaction of the product molecule into a vector comprising one or more recombination sites. In one aspect, the cloning step comprises conducting a recombination reaction between the sites in the product nucleic acid molecule that did not react in the first recombination reaction with a vector having recombination sites capable of recombining with the unreacted sites.

In some embodiments, a recombination site and/or a topoisomerase recognition site may be attached to a molecule of interest using conventional conjugation technology. For example, oligonucleotides comprising the recombination site and/or topoisomerase recognition site can be synthesized so as to include one or more reactive functional moieties which may be the same or different. Suitable reactive functional moieties include, but are not limited to, amine groups, epoxy groups, vinyl groups, thiol groups and the like. The synthesis of oligonucleotides comprising one or more reactive functional moieties is routine in the art. Once synthesized, oligonucleotides comprising one or more reactive functional moieties may be attached to one or more reactive groups present on the molecule or compound of interest. The oligonucleotides may be attached directly by reacting one or more of the reactive functional moieties with one or more of the reactive functional groups. In some embodiments, the attachment may be effected using a suitable linking group capable of reacting with one or more of the reactive functional moieties present on the oligonucleotide and with one or more of the reactive groups present on the molecule of interest. In other embodiments, both direct attachment and attachment through a linking group may be used. Those skilled in the art will appreciate that the reactive functional moieties on the oligonucleotide may be the same or different as the reactive functional moieties on the molecules and/or compounds of interest Suitable reagents and techniques for conjugation of the oligonucleotide to the molecule of interest may be found in Hermanson, Bioconjugate Techniques, Academic Press Inc., San Diego, CA, 1996.

Described herein are compositions for carrying out the methods described herein, and kits comprising such compositions, and to compositions created while carrying out the methods described herein.

Compositions, methods and kits as described herein may be prepared and carried out using a phage-lambda site-specific recombination system. Further, such compositions, methods and kits may be prepared and carried out using the GATEWAY™ Recombinational Cloning System and/or the TOPO^{®} Cloning System and/or the pENTR Directional TOPO^{®} Cloning System, which are available from Invitrogen Corporation (Carlsbad, California).

Described herein are isolated nucleic acid molecules comprising one or more (*e.g.,* one, two, three, four, five, etc.) recombination sites and/or one or more (*e*.*g*., one, two, three, four, five, etc.) topoisomerase recognition sites. One such molecule may contain two or more recombination sites flanking one topoisomerase recognition site. Another such molecule may contain two or more recombination sites and two or more topoisomerase recognition sites, wherein each recombination site may flank a topoisomerase recognition site. Nucleic acid molecules as described herein may be linear, circular, or have any of a variety of geometries and structures, such as coiled, supercoiled, etc. Recombination sites advantageously used in nucleic acid molecules as described herein include, but are not limited to, *att* sites (including, but not limited to, *att*B sites, *att*P sites, *att*L sites, *att*R sites, and the like), *lox* sites (including, but not limited to, *lox*P sites, *lox*P511 sites, and the like), *psi* sites, *dif* sites, *cer* sites, *frt* sites, and mutants, variants, and derivatives of these recombination sites that retain the ability to undergo recombination. Topoisomerase recognition sites advantageously used in the nucleic acid molecules of this aspect of the invention are preferably recognized and bound by a type I topoisomerase (such as type IA topoisomerases (including but not limited to *E. coli* topoisomerase I, *E. coli* topoisomerase III, eukaryotic topoisomerase II, archeal reverse gyrase, yeast topoisomerase III, *Drosophila* topoisomerase III, human topoisomerase III, *Streptococcus pneumoniae* topoisomerase III, and the *tra*E protein of plasmid RP4) and type IB topoisomerases (including but not limited to eukaryotic nuclear type I topoisomerase and a poxvirus (such as that isolated from or produced by vaccinia virus, Shope fibroma virus, ORF virus, fowlpox virus, molluscum contagiosum virus and *Amsacta moorei* entomopoxvirus)), and type II topoisomerase (including, but not limited to, bacterial gyrase, bacterial DNA topoisomerase IV, eukaryotic DNA topoisomerase II (such as calf thymus type II topoisomerase), and T-even phage- encoded DNA topoisomerase).

Described herein are vectors (which may be expression vectors) comprising such isolated nucleic acid molecules. Exemplary vectors described herein include, but are not limited to, pcDNAGW-DT(sc), pBNTR-DT(sc), pcDNA-DEST41, pENTR/D-TOPO, pENTWSD/D-TOPO, pcDNA3.2/V5/GWD-TOPO and pcDNA6.2/V5/GWD-TOPO. Also described herein are host cells comprising such the isolated nucleic acid molecules or vectors described herein.

Described herein are *in vitro* methods of cloning a nucleic acid molecule. Methods disclosed herein may comprise one or more steps, including:
(a) obtaining a nucleic acid molecule to be cloned (which may be a linear molecule (and which may be blunt-ended or not) such as a PCR product, and which may optionally comprise one or more genes or open reading frames);
(b) mixing the nucleic acid molecule to be cloned *in vitro* with a vector (which may be an expression vector) comprising at least a first topoisomerase recognition site flanked by at least a first recombination site and at least a second recombation site, wherein the first and second recombination sites do not recombine with each other, and with at least one topoisomerase; and
(c) incubating the mixture under conditions such that the nucleic acid molecule to be cloned is inserted into the vector between the first and second topoisomerase recognition sites, thereby producing a first product molecule comprising the nucleic acid molecule localized between the first and second recombination sites. Also described herein are nucleic acid molecules prepared by the above methods.

Methods described herein may comprise one or more additional steps, including, for example, contacting the first product molecule with at least one vector comprising at least a third and fourth recombination sites that do not recombine with each other, under conditions favoring recombination between the first and third and between the second and fourth recombination sites, thereby producing at least one second product molecule. The first and/or second product molecules produced by these methods may be inserted into a host cell. The vectors may comprise at least one additional nucleic acid sequence selected from the group consisting of a selectable marker, a cloning site, a restriction site, a promoter, an operon, an origin of replication, and a gene or partial gene (*i*.*e*., a gene fragment or element).

Recombination sites and topoisomerase recognition sites used in the methods described herein include, but are not limited to, those described elsewhere herein. In particular methods, the second product nucleic acid molecule and the vector are combined in the presence of at least one recombination protein, which may be but is not limited to Cre, Int, IHF, Xis, Fis, Hin, Gin, Cin, Tn3 resolvase, TndX, XerC, or XerD. In certain such embodiments, the recombination protein is Cre, Int, Xis, IHF or Fis.

Described herein are kits comprising these isolated nucleic acid molecules of the invention, which may optionally comprise one or more additional components selected from the group consisting of one or more topoisomerases, one or more recombination proteins, one or more vectors, one or more polypeptides having polymerase activity, and one or more host cells.

Other preferred embodiments of the invention will be apparent to one or ordinary skill in the art in light of what is known in the art, in light of the following drawings and description of the invention, and in light of the claims.

### BRIEF DESCRIPTION OF THE DRAWINGS/FIGURES

Figure 1 is a schematic representation of a basic recombinational cloning reaction.

Figure 2 is a schematic representation of the use of the disclosure herein to clone two nucleic acid segments by performing an LR recombination reaction.

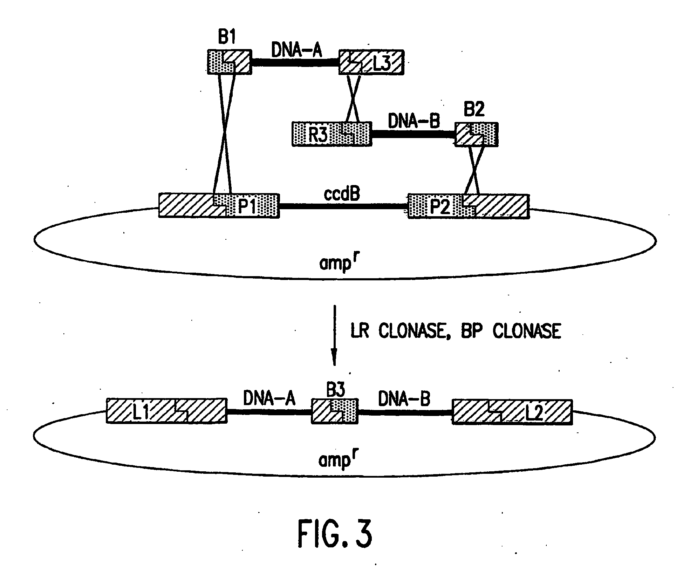

Figure 3 is a schematic representation of the use of the disclosure herein present to clone two nucleic acid segments by joining the segments using an LR reaction and then inserting the joined fragments into a Destination Vector using a BP recombination reaction.

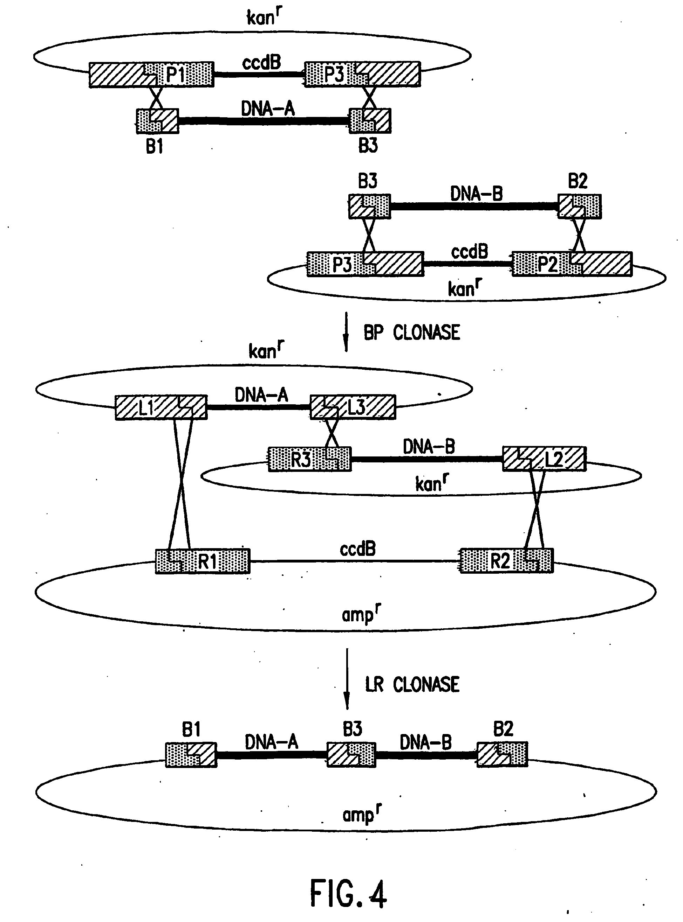

Figure 4 is a schematic representation of the use of the disclosure herein to clone two nucleic acid segments by performing a BP reaction followed by an LR reaction.

Figure 5 is a schematic representation of two nucleic acid segments having *att*B sites being cloned by performing a first BP reaction to generate an *att*L site on one segment and an *att*R on the other followed by an LR reaction to combine the segments. In variations of this process, P1, P2, and/or P3 can be oligonucleotides or linear stretches of nucleotides.

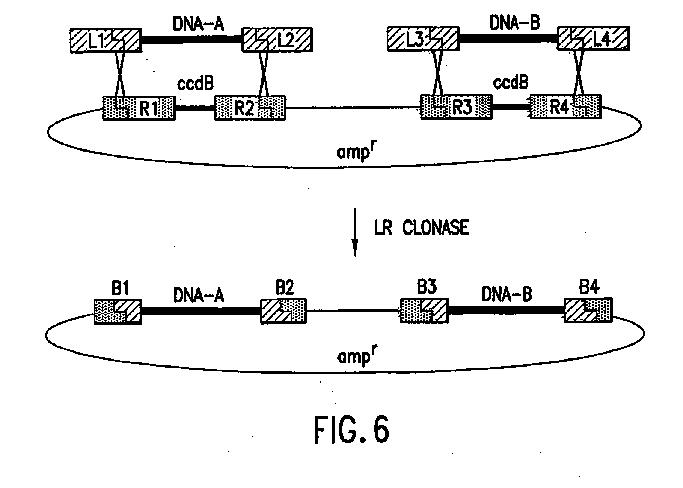

Figure 6 is a schematic representation of the cloning of two nucleic acid segments into two separate sites in a Destination Vector using an LR reaction.

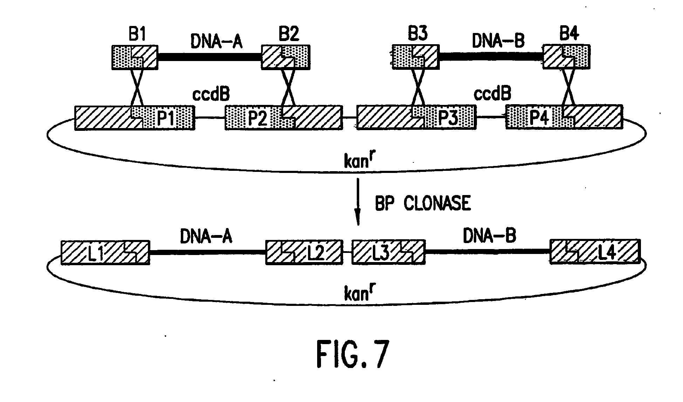

Figure 7 is a schematic representation of the cloning of two nucleic acid segments into two separate sites in a Destination Vector using a BP reaction.

Figures 8A and 8B depict generating a covalently linked double stranded nucleotide sequence containing an element on each end according to a method described herein. "PCR" indicates polymerase chain reaction; "TOPO" indicates topoisomerase; topoisomerase shown as circle attached to sequence; "P1" and "P2" indicate PCR primers. Topoisomerase recognition site is indicated in bold print.

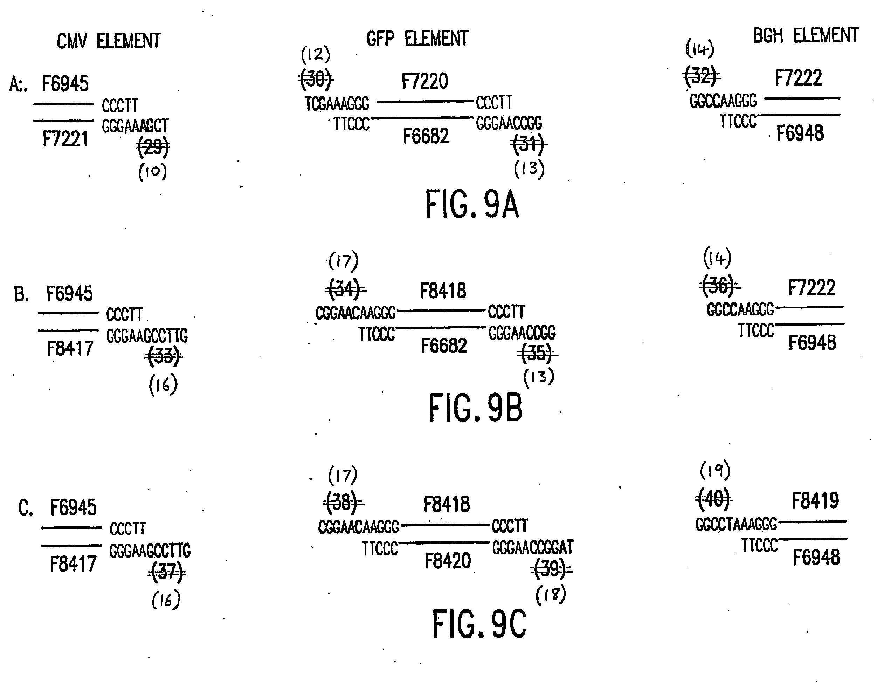

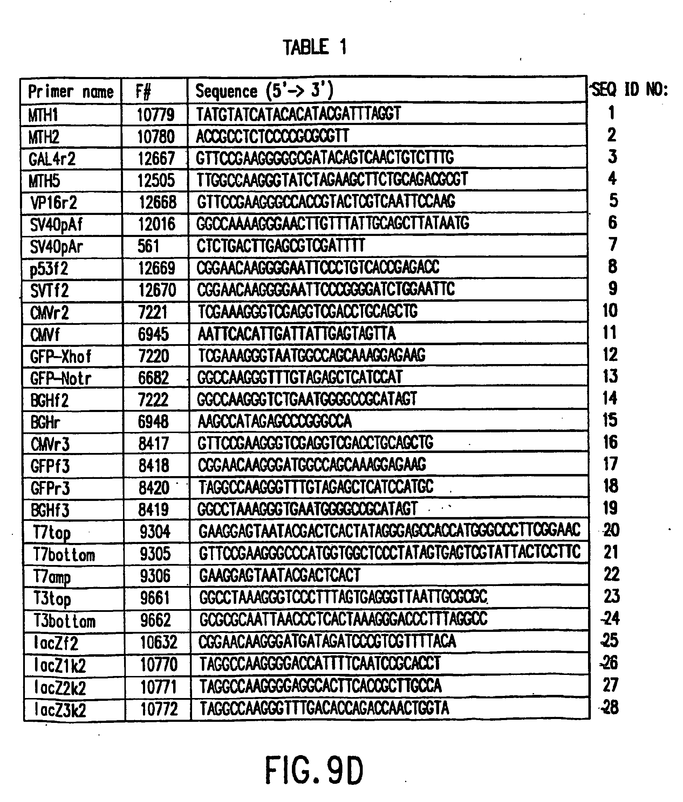

Figures 9A-9C show the ends of PCR products representing a cytomegalovirus promoter element ("CMV"), a green fluorescent protein element ("GFP"), and a bovine growth hormone polyadenylation signal ("BGH") element. Primers used to construct the PCR products of Figures 9A, 9B and 9C are indicated by an "F" number (see Figure 9D). The portion of one or both ends including the topoisomerase recognition site (CCCTT) is shown. Bold print indicates overhanging sequences. In Figures 9A and 9B, one (Figure 9B) or both (Figure 9A) of the overhang sequences are palindromic in nature. Sequences are shown in conventional orientation, with the top strand in a 5' to 3' orientation from left to right, and the bottom strand in a 3' to 5' orientation from left to right. Number in parentheses above or below sequences indicates SEQ ID NOs.

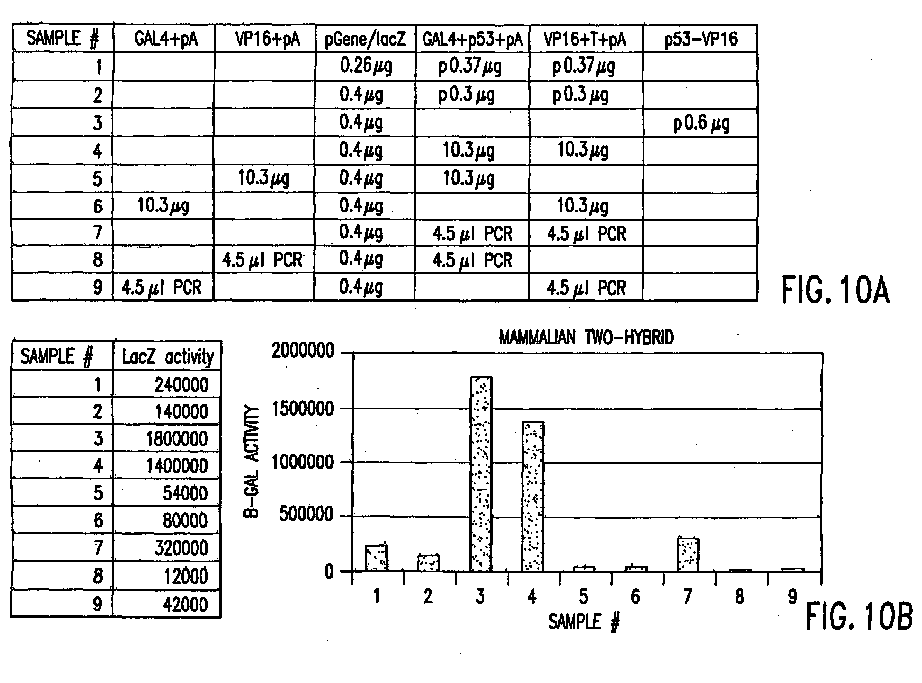

Figures 10A and 10B show constructs (Figure 10A) and results (Figure 10B) of experiments examining the ability to use covalently linked ds recombinant nucleic acid molecules that encode polypeptides for performing a two hybrid assay. Figure 10A shows the amount of each construct used for transfection. A "p" preceding an amount or volume of reactant indicates plasmid form, "1" indicates linear form, and "PCR," indicates PCR amplification reaction mixture. Figure 10B shows the level of β-galactosidase activity ("LacZ activity") associated with each transfected sample. Increased LacZ activity is indicative of a positive interaction.

Figures 11A to 11F represents variants of the composition and methods for generating a ds recombinant nucleic acid molecule covalently linked in one strand. Note nicks in one or both strands of the molecules shown in Figures 11B-11F.

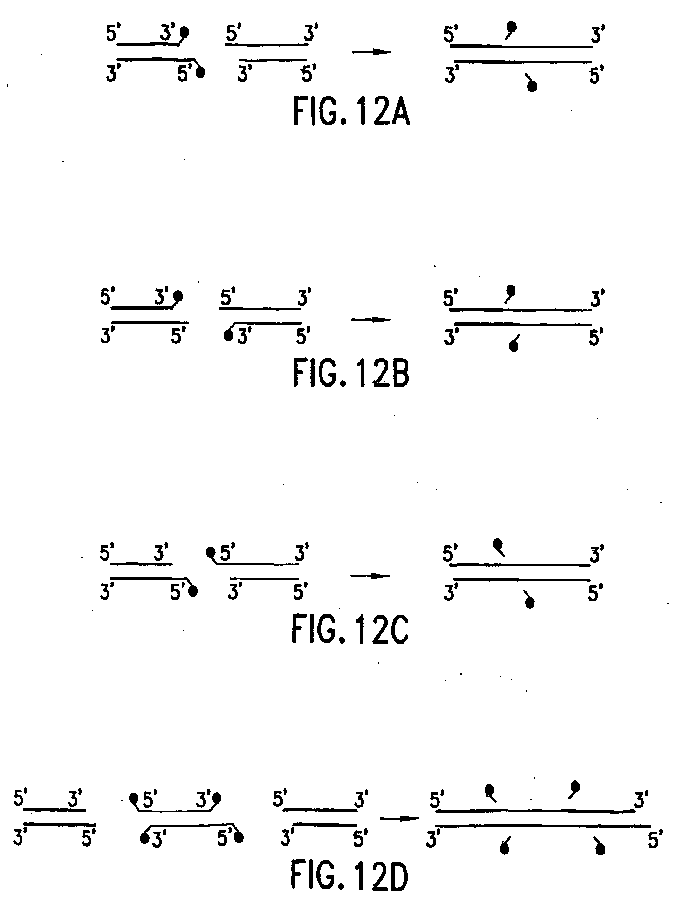

Figures 12A to 12D illustrates variants of compositions and methods described herein for generating a covalently linked ds recombinant nucleic acid molecule. Topoisomerase is shown as a solid circle, and is either attached to a terminus of a substrate nucleic acid molecule or is released following a linking reaction. As illustrated, the substrate nucleic acid molecules have 5' overhangs, although they similarly can have 3' overhangs or can be blunt ended. In addition, while the illustrated nucleic acid molecules are shown having the topoisomerases bound thereto (topoisomerase-charged), one or more of the termini shown as having a topoisomerase bound thereto also can be represented as having a topoisomerase recognition site, in which case the joining reaction would further require addition of one or more site specific topoisomerases, as appropriate.

Figure 12A shows a first nucleic acid molecule having a topoisomerase linked to each of the 5' terminus and 3' terminus of one end, and further shows linkage of the first nucleic acid molecule to a second nucleic acid molecule.

Figure 12B shows a first nucleic acid molecule having a topoisomerase bound to the 3' terminus of one end, and a second nucleic acid molecule having a topoisomerase bound to the 3' terminus of one end, and further-shows a covalently linked ds recombinant nucleic acid molecule generated due to contacting the ends containing the topoisomerase-charged substrate nucleic acid molecules.

Figure 12C shows a first nucleic acid molecule having a topoisomerase bound to the 5' terminus of one end, and a second nucleic acid molecule having a topoisomerase bound to the 5' terminus of one end, and further shows a covalently linked ds recombinant nucleic acid molecule generated due to contacting the ends containing the topoisomerase-charged substrate nucleic acid molecules.

Figure 12D shows a nucleic acid molecule having a topoisomerase linked to each of the 5' terminus and 3' terminus of both ends, and further shows linkage of the topoisomerase-charged nucleic acid molecule to two nucleic acid molecules, one at each end. The topoisomerases at each of the 5' termini and/or at each of the 3' termini can be the same or different.

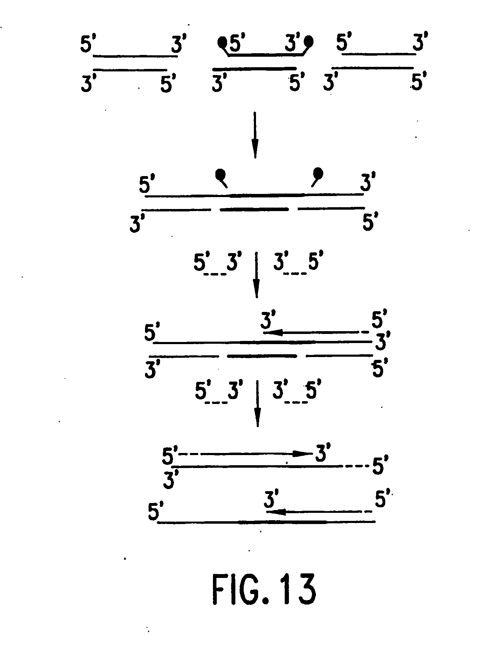

Figure 13 illustrates the generation of an expressible ds recombinant nucleic acid molecule and amplification of the expressible ds recombinant nucleic acid molecule. The expressible ds recombinant nucleic acid molecule is generated from three nucleic acid molecules, including a nucleotide sequence comprising a promoter, a nucleotide sequence comprising a coding sequence, and a nucleotide sequence comprising a polyadenylation signal. Generation of the nucleic acid molecule can be facilitated by the incorporation of complementary 5' and/or 3' overhanging sequences at the ends of the ds nucleotides sequences to be joined. The expressible ds recombinant nucleic acid molecule is generated by contacting a first nucleic acid molecule having a type IA topoisomerase at a 5' terminus of a first end and a type IB topoisomerase at a 3' terminus of a second end, with a second nucleic acid molecule and a third double stranded nucleotide sequence. The expressible ds recombinant nucleic acid molecule is amplified using a first primer that hybridizes to the second ds recombinant nucleic acid molecule upstream of the promoter, and a second primer that hybridizes to the third ds recombinant nucleic acid molecule downstream of the polyadenylation signal.

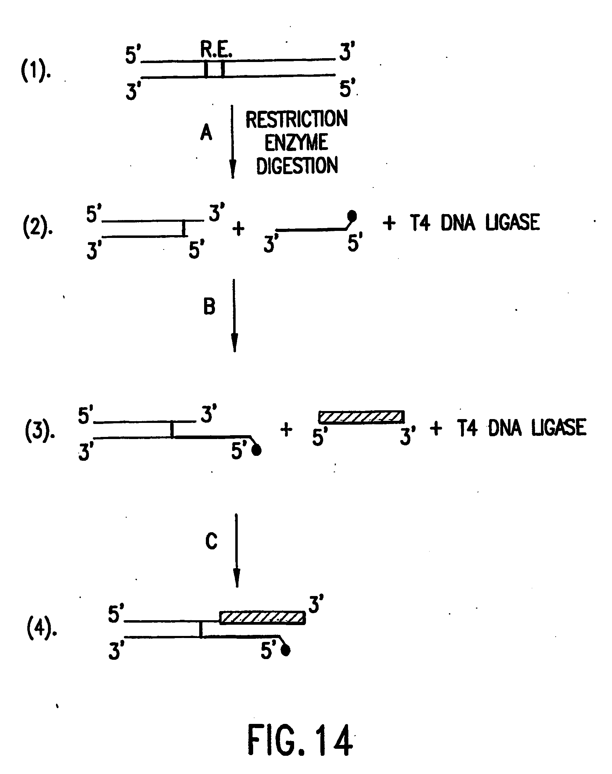

Figure 14 shows one example of a process for preparing a double stranded nucleic acid molecule which contains a topoisomerase (e.g., a type IA topoisomerase) bound to the 5' terminus of one end of the molecule, wherein the same end of the molecule further comprises a 3' overhang (see (4) in this figure).

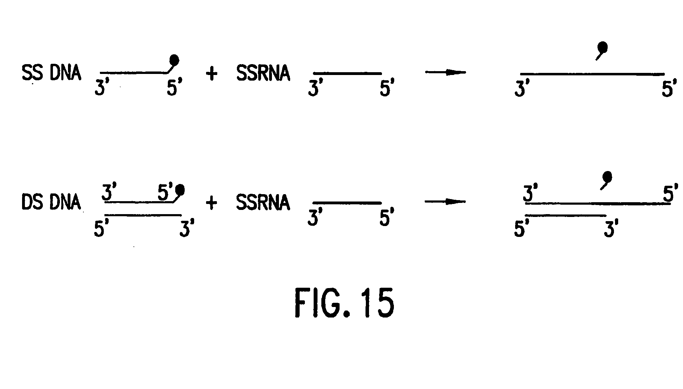

Figure 15 shows how a single stranded or double stranded DNA nucleotide sequence may be joined with single stranded RNA nucleotide sequence.

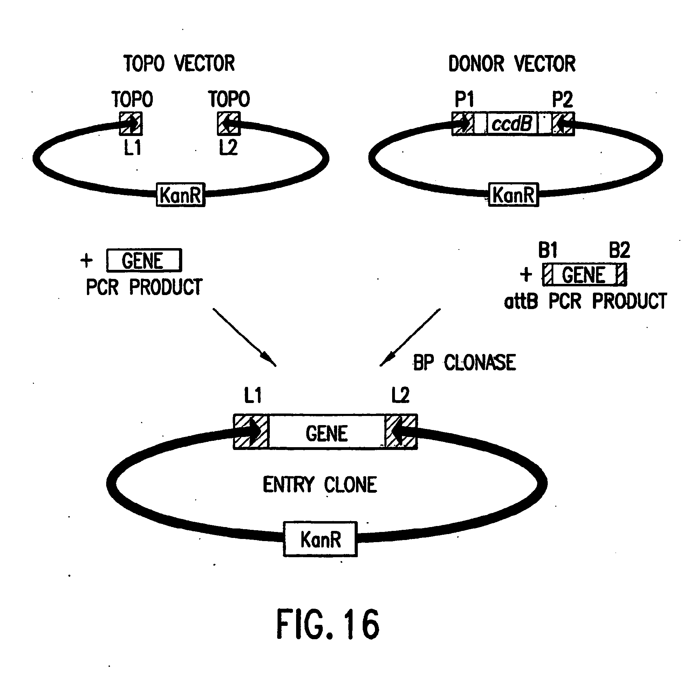

Figure 16 is a schematic demonstrating the flexibility in entry point for PCR cloning using the TOPO-Gateway™ or standard Gateway™ cloning methodologies.

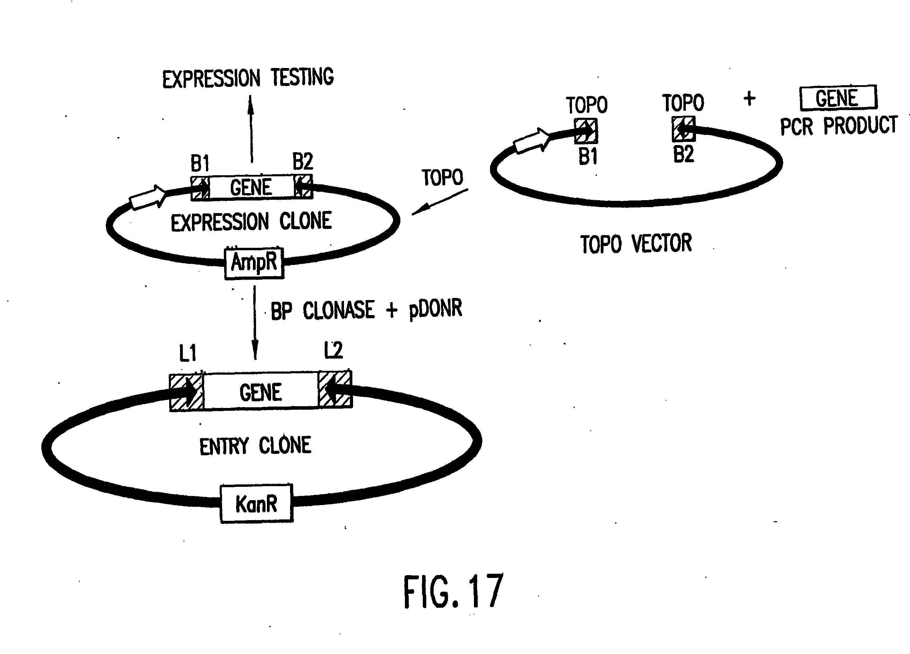

Figure 17 is a schematic diagram of the production of expression clones using the Gateway™ system and a directional TOPO-Gateway™ expression vector.

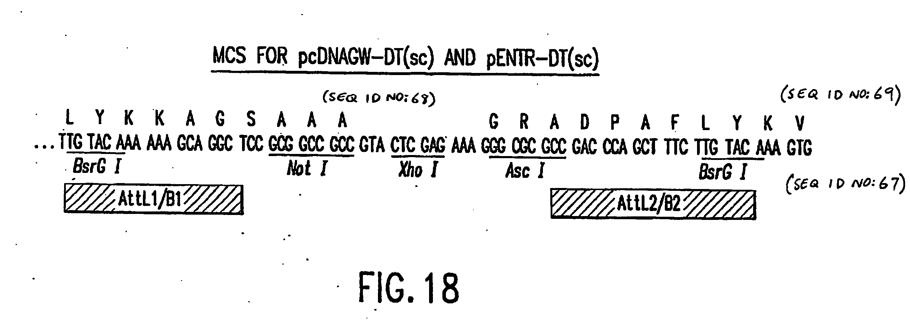

Figure 18 is a map of the multiple cloning site in plasmids pcDNAGW-DT(sc) and pENTR-DT(sc).

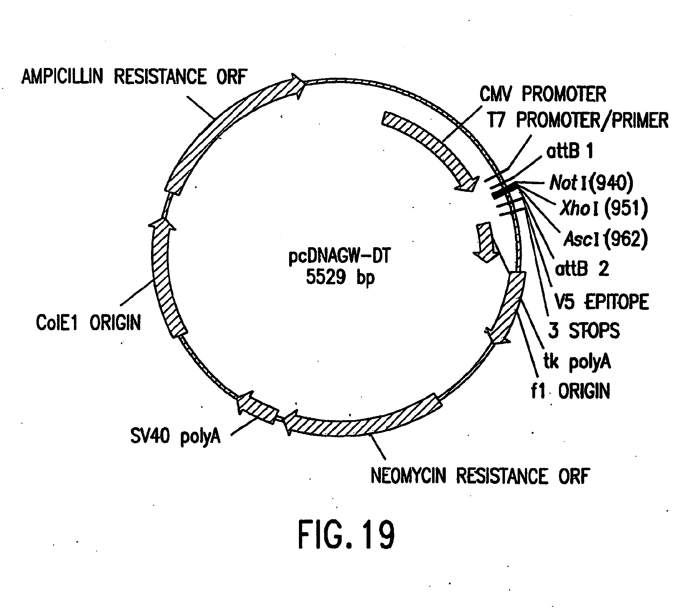

Figure 19 is a physical map of plasmid pcDNAGW-DT.

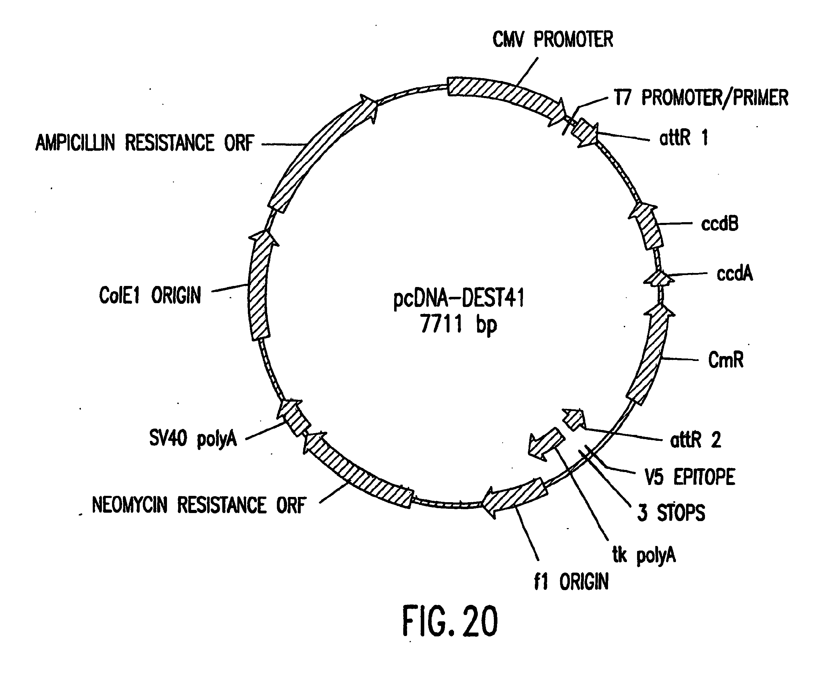

Figure 20 is a physical map of plasmid pcDNA-DEST41.

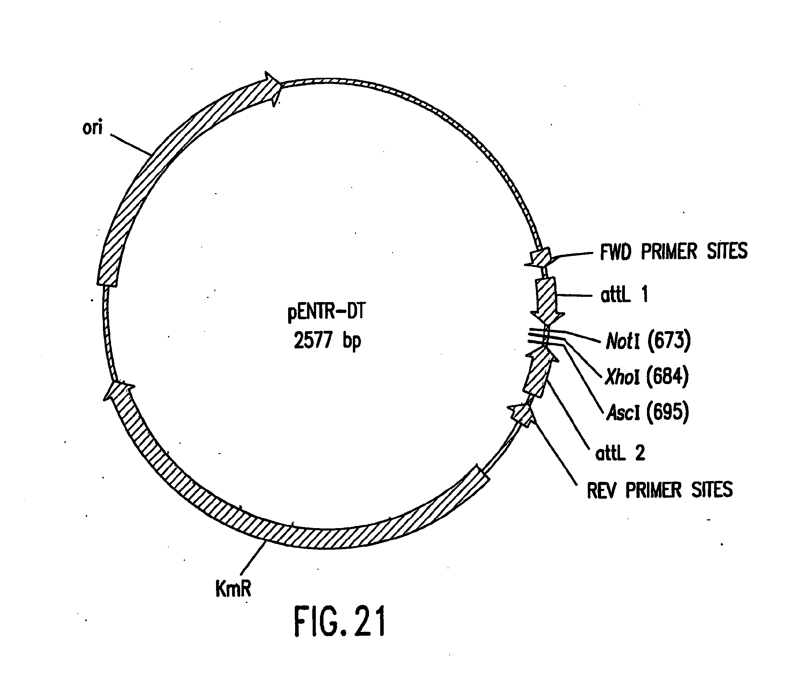

Figure 21 is a physical map of plasmid pENTR-DT.

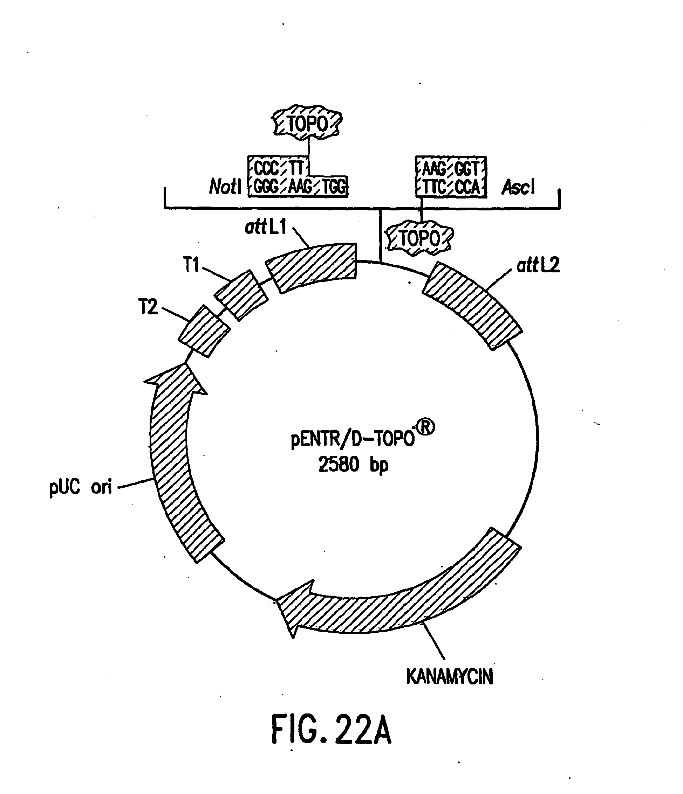

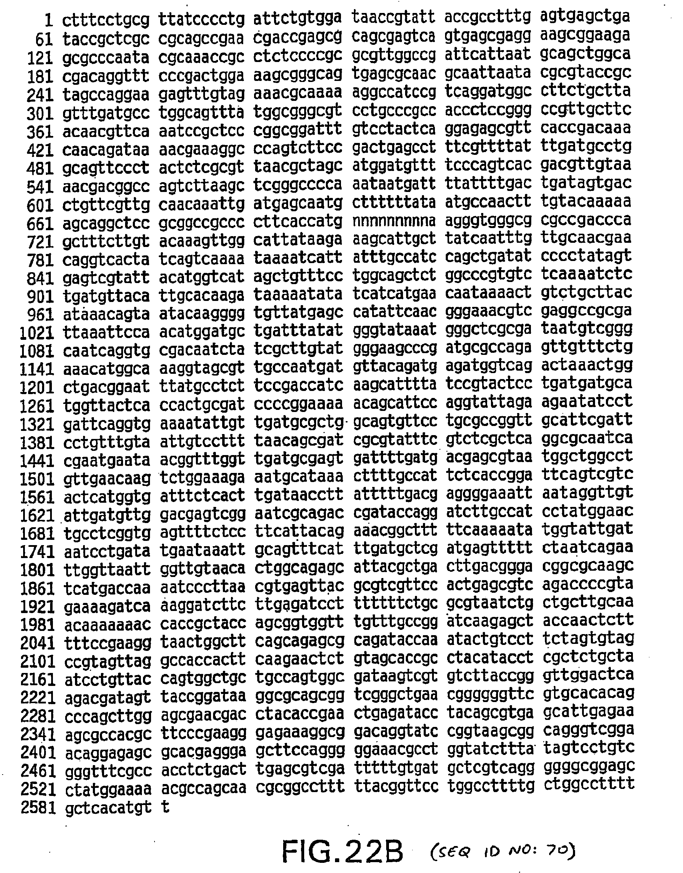

Figure 22 is a depiction of the physical map (Fig. 22A) showing the TOPO cloning site in; and the nucleotide sequence (Fig. 22B) of, plasmid pENTR/D-TOPO. The physical map depicts the adapted, supercoiled form of the vector, while the nucleotide sequence depicts the vector containing a start codon and an open reading frame (atgnnnnnn...). Restriction sites are labeled to indicate the actual cleavage site. The boxed region indicates *att*L sequences in the entry clone that will be transferred into the destination vector following recombination. The sequence of pENTR/D-TOPO depicted in Figure 22B is also available for downloading from the Invitrogen Corporation web site at http://www.invitrogen.com/content/vectors/pentr dtopo seq.txt.

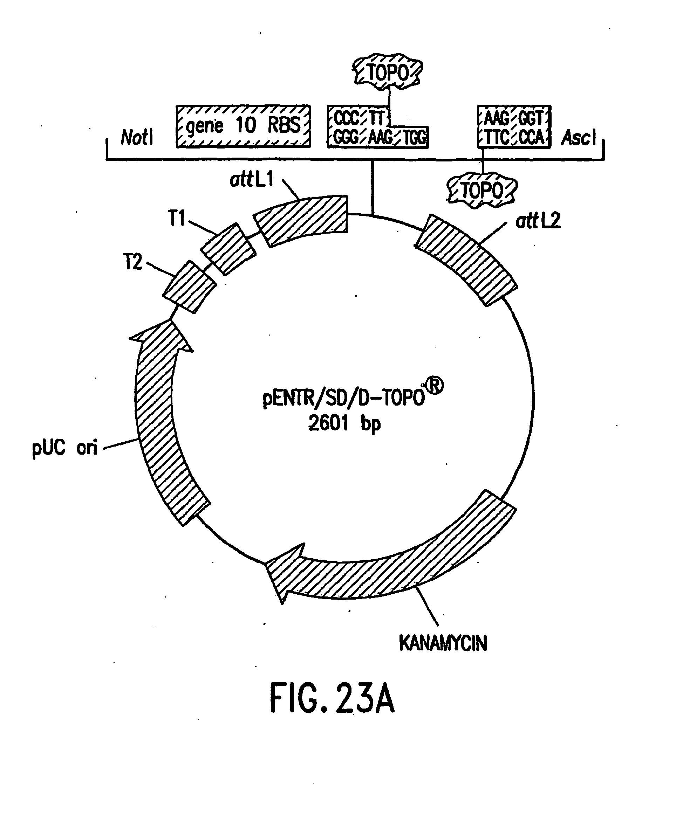

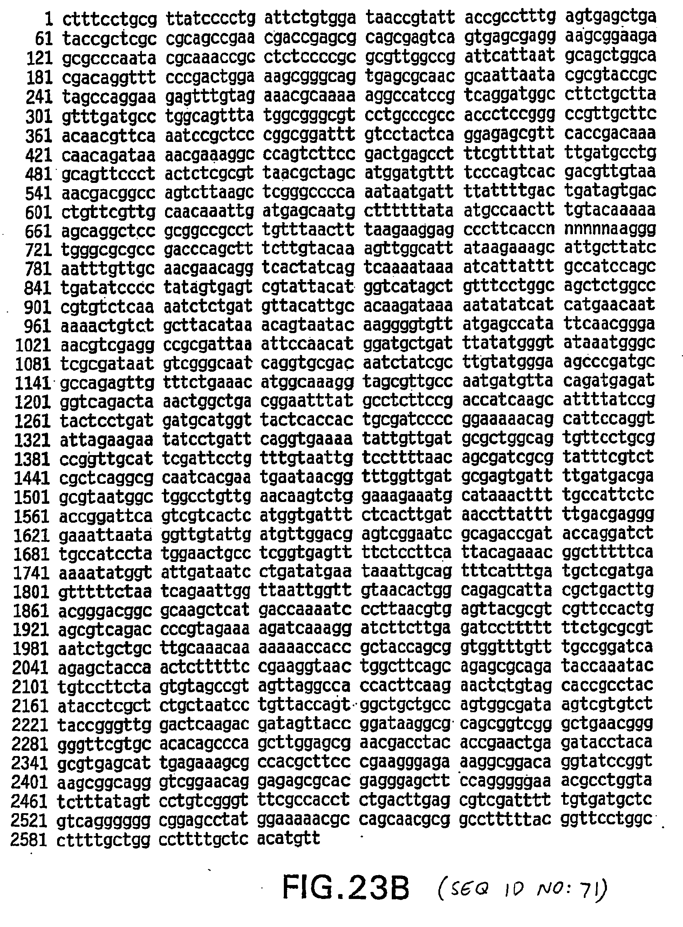

Figure 23 is a depiction of the physical map (Fig. 23A) showing the TOPO cloning site in, and the nucleotide sequence (Fig. 23B) of, plasmid pENTR/SD/D-TOPO. The physical map depicts the adapted, super-coiled form of the vector, while the nucleotide sequence depicts the vector containing a start codon and an open reading frame (atgnnnnnn...). Restriction sites are labeled to indicate the actual cleavage site. The boxed region indicates *att*L sequences in the entry clone that will be transferred into the destination vector following recombination. The nucleotide sequence of pENTR/SD/D-TOPO depicted in Figure 23B is also available for downloading from http://www.invitrogen.com./content/vectors/pentrsd_dtopo_seq.txt.

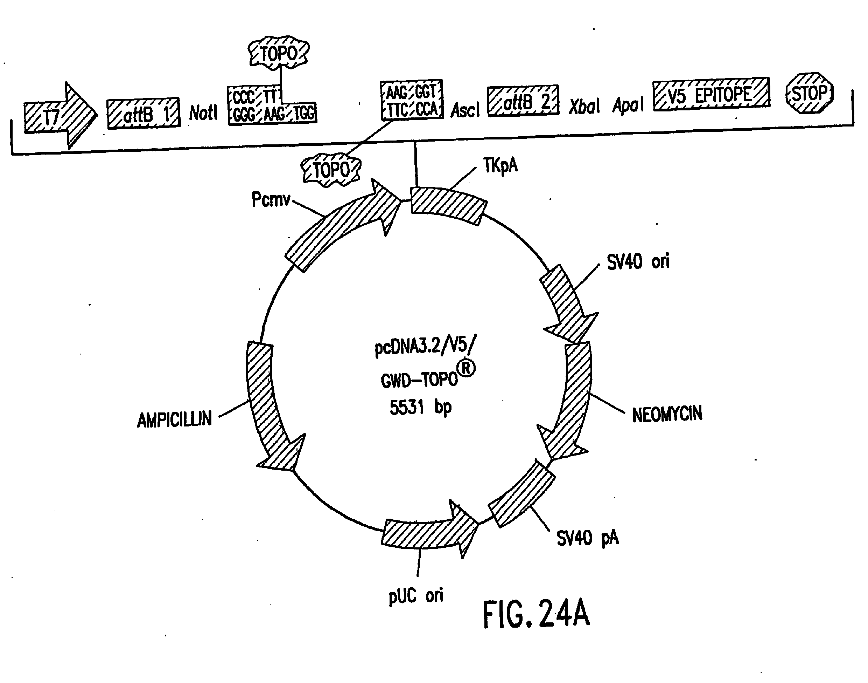

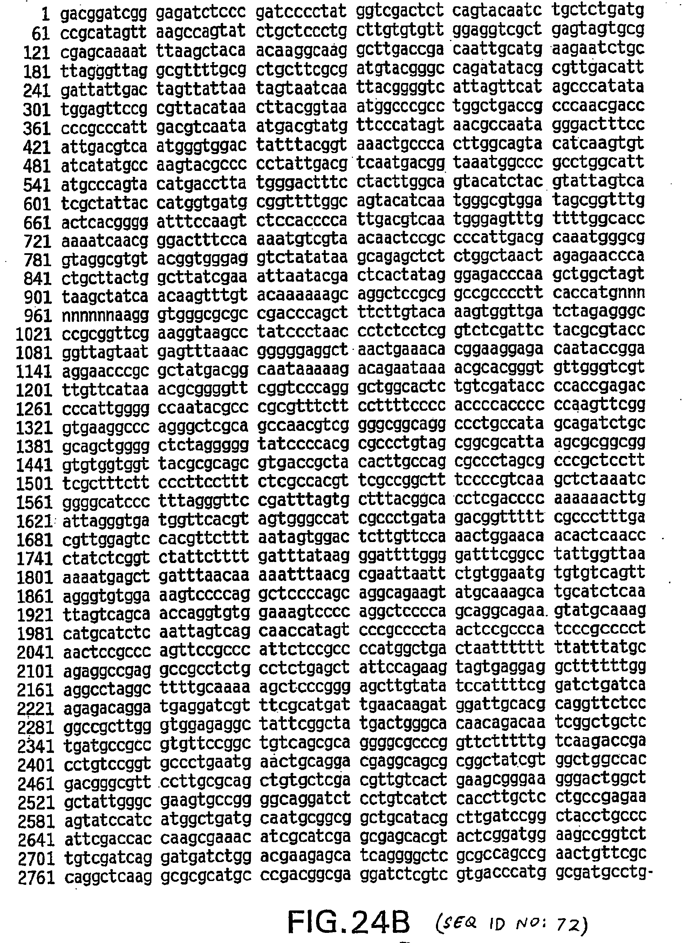

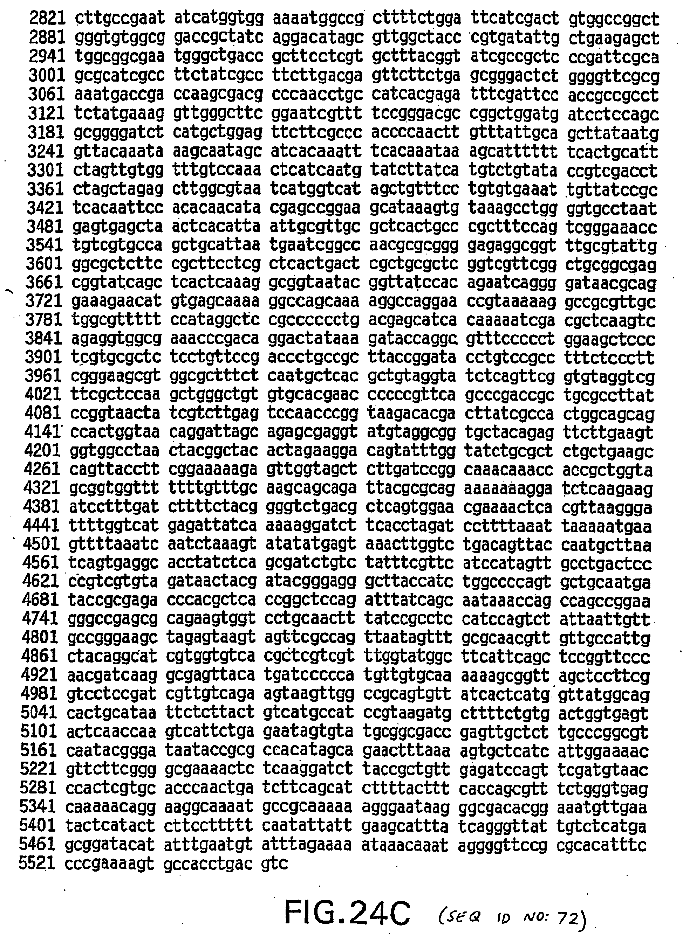

Figure 24 is a depiction of the physical map (Fig. 24A) and the nucleotide sequence (Fig. 24B-C) of plasmid pcDNA3.2/V5/GWD-TOPO®. The physical map depicts the adapted, supercoiled form of the vector, while the nucleotide sequence depicts the vector containing a start codon and an open reading frame (atgnnnnnn...).

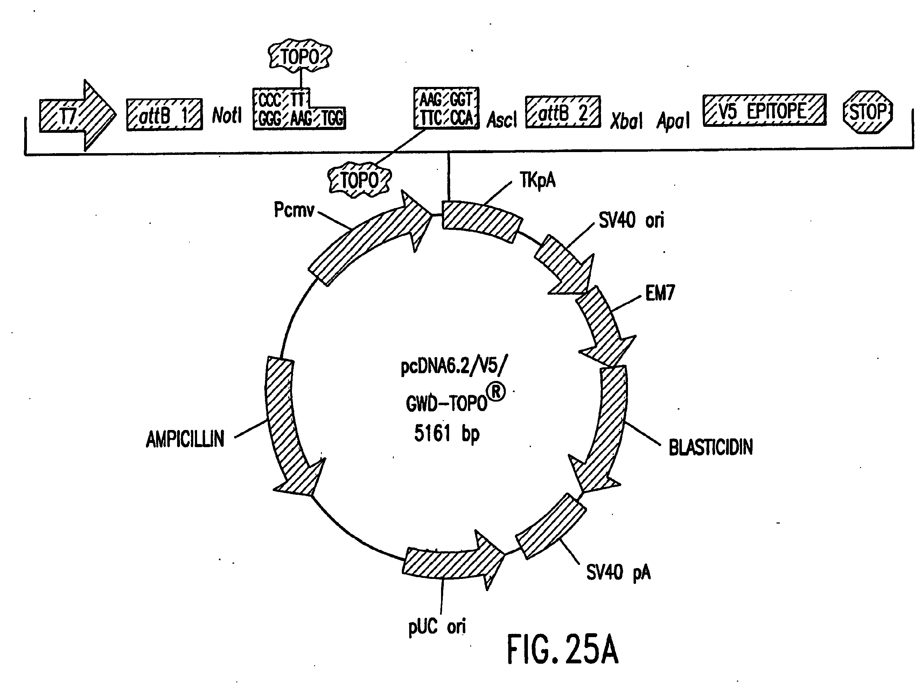

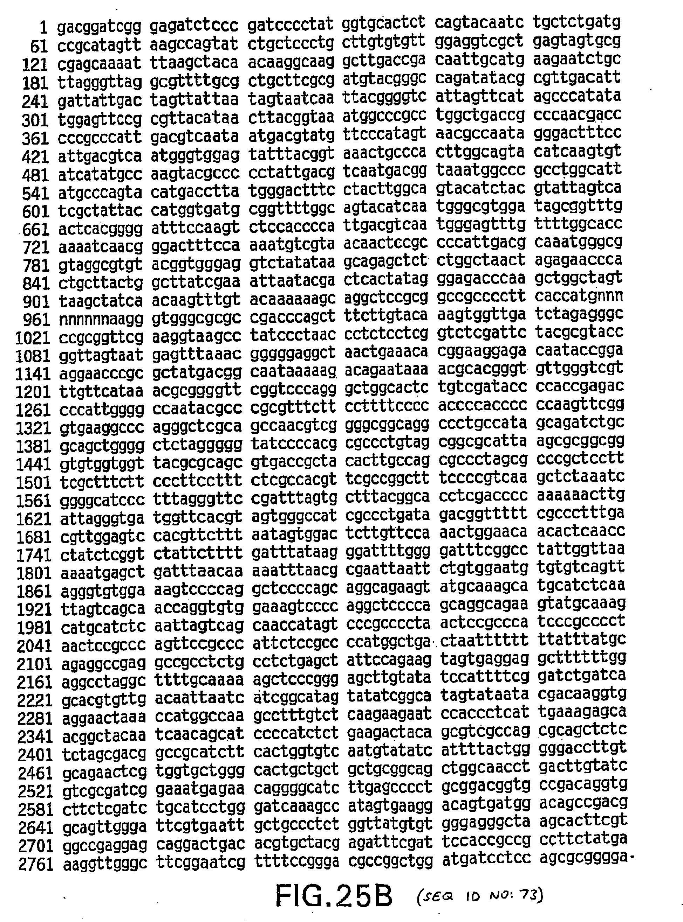

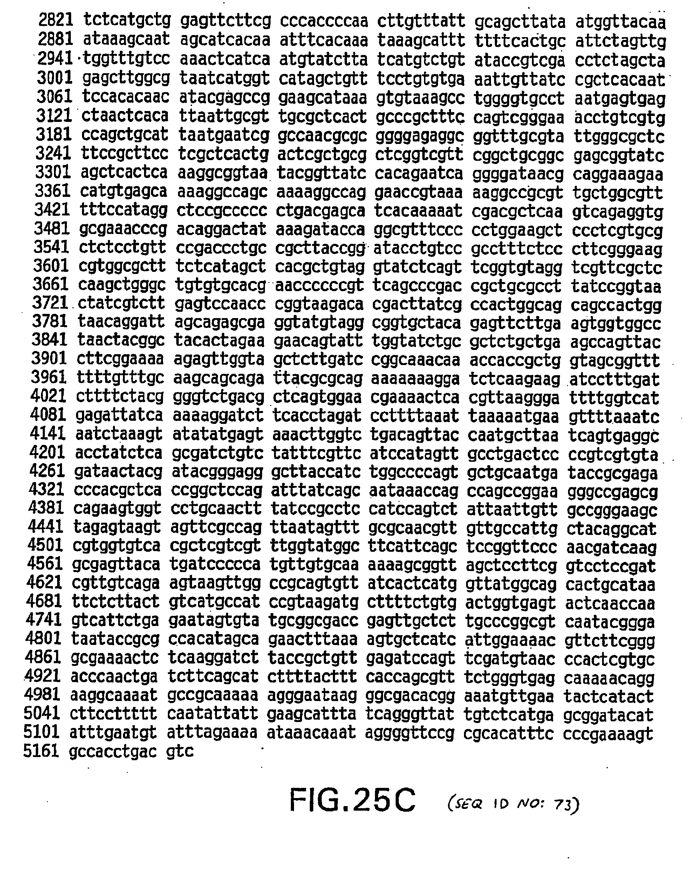

Figure 25 is a depiction of the physical map (Fig. 25A) and the nucleotide sequence (Fig. 25B-C) of plasmid pcDNA6.2/V5/GWD-TOPO®. The physical map depicts the adapted, supercoiled form of the vector, while the nucleotide sequence depicts the vector containing a start codon and an open reading frame (atgnnnnnn...).

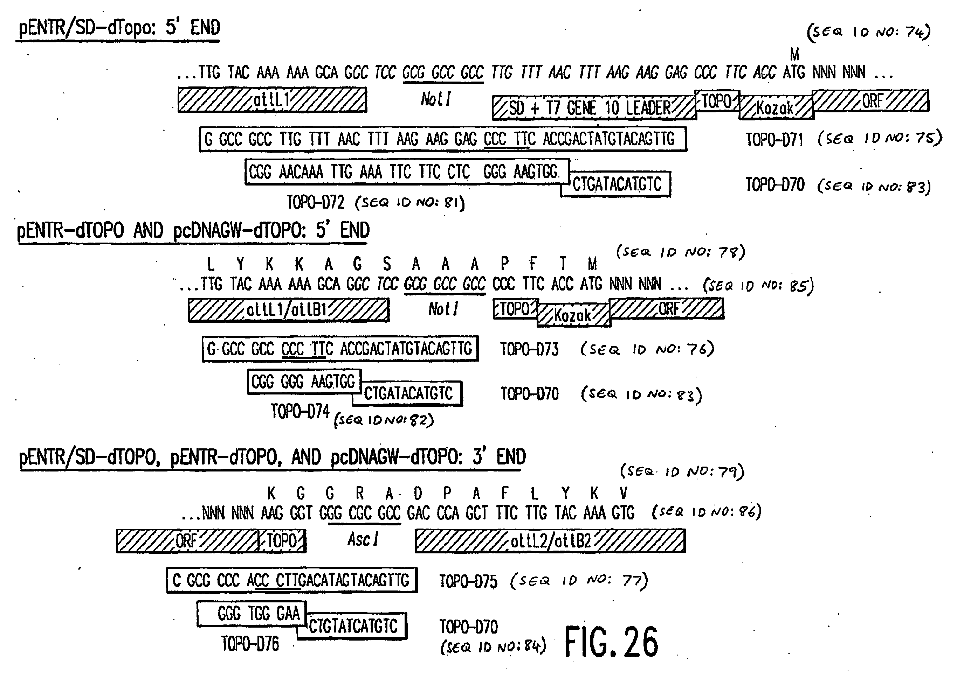

Figure 26 is a depiction of an exemplary adaptation strategy for pENTR/SD-dTopo, pENTR-dTopo, and pcDNAGW-dTopo.

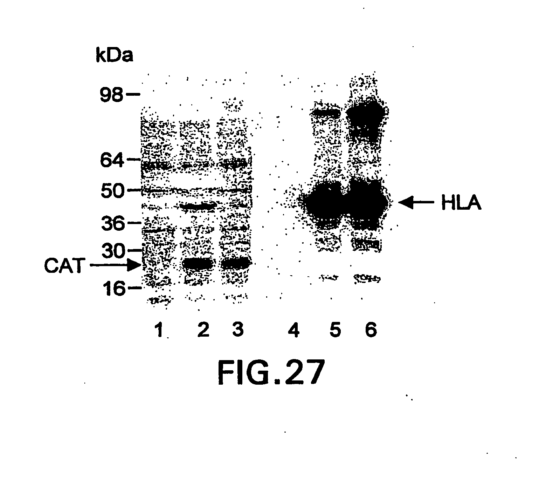

Figure 27 is a photograph of a Western blot analysis of HLA and CAT expresed in COS cells. The genes encoding CAT (26 kDa) and HLA (41 kDa) were amplified by PCR and either Topo-cloned into pENTRd-Topo and transferred into pcDNA-DEST40 (lanes 2 and 5, respectively), or cloned directly into pcDNAGW-dTopo (lanes 3 and 6, respectively). These constructs were used to transfect COS cells and the lysates probed for recombinant VStagged protein by Western blot, using V5-HRP antibody conjugate. Lanes 1 and 4 represent cells only controls.

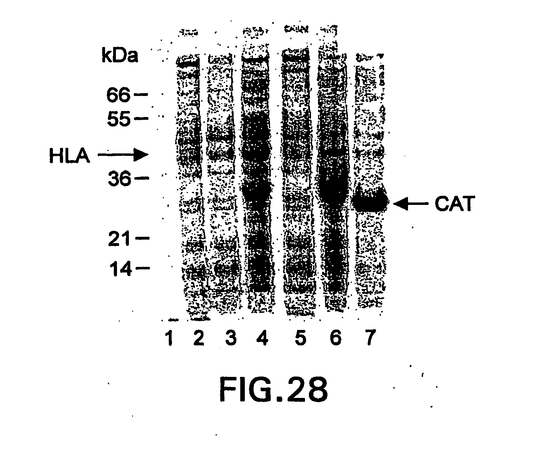

Figure 28 is a photograph of a gel depicting HLA and CAT expression in E. coli. The genes encoding HLA (41 kDa) and CAT (26 kDa) were amplified by PCR and either topo cloned into pENTR/SD-dTopo and transferred into pET-DEST42 (lanes 3 and 6, respectively) or cloned directly into pET101-dTopo (lanes 4 and 7, respectively). These constructs were used to transform BL21(DE3) cells and induced to express by addition of IPTG to 1 mM for 3 hours at 37C. Cell lysates were run on a NuPage and stained with SafeStain™. Lanes 2 and 5 represent cells uninduced cell lysates from the respective pET-DEST42 cultures.

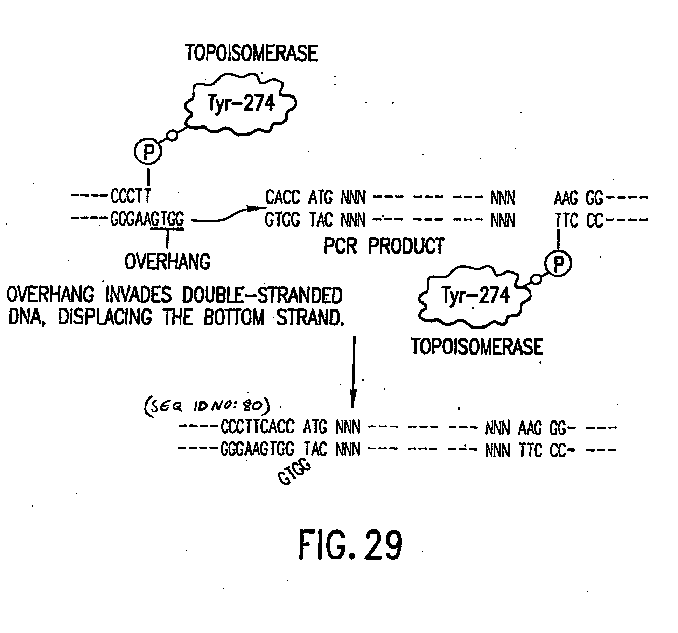

Figure 29 is a schematic depiction of the binding of a topoisomerase to a recognition site near the 3' terminus of a target nucleic acid molecule. Upon binding of the topoisomerase, the downstream sequence (3' to the cleavage site) can dissociate, leaving a nucleic acid molecule having the topoisomerase covalently bound to the newly generated 3' end.

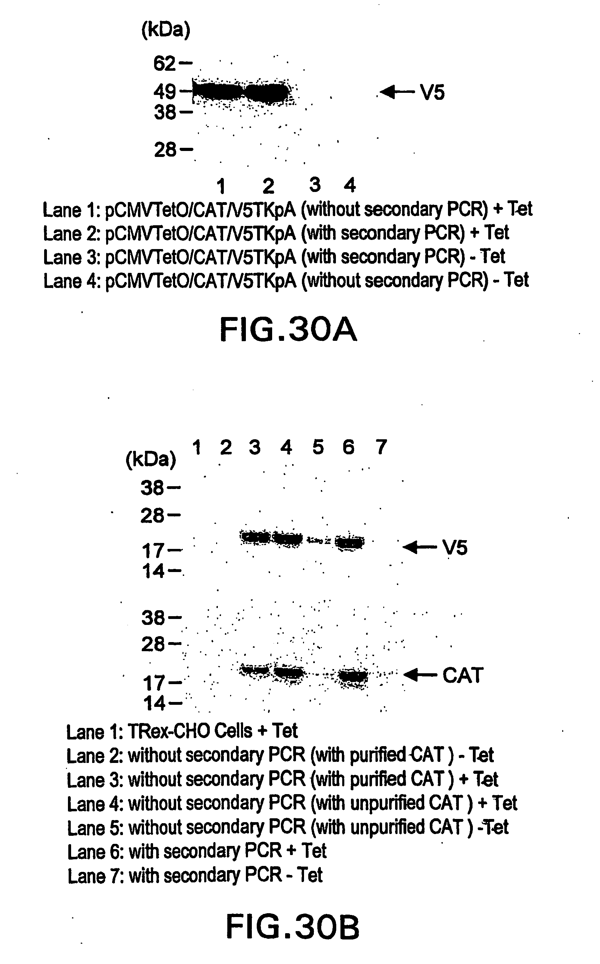

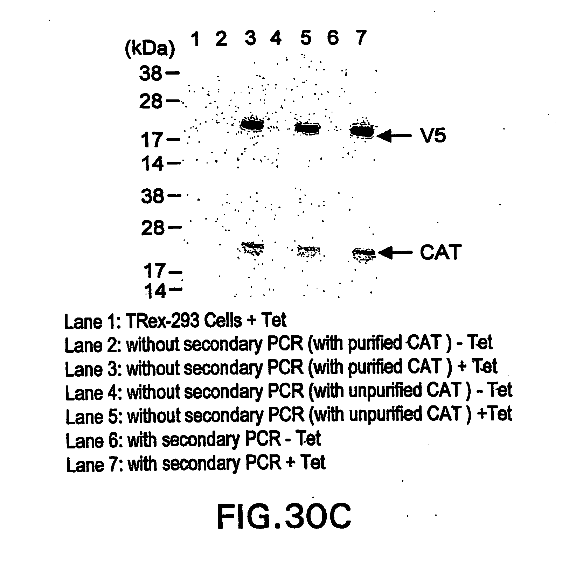

Figure 30 depicts protein expression results (Western blot) for mammalian expression cassettes that were constructed by PCR amplification of expression elements and a gene of interest (CAT or V5) followed by a TOPO joining reaction performed with or without secondary PCR. Protein expression data from the expression cassette transfected into suspension TRex-CHO cells (Figure 30A), adherent TRex-CHO cells (Figure 30B), and adherent TRex-293 cells (Figure 30C). For the Western blot, anti-V5 or anti-CAT antibodies were used for detection. Arrows indicate the position of the bands corresponding to the V5 or CAT proteins.

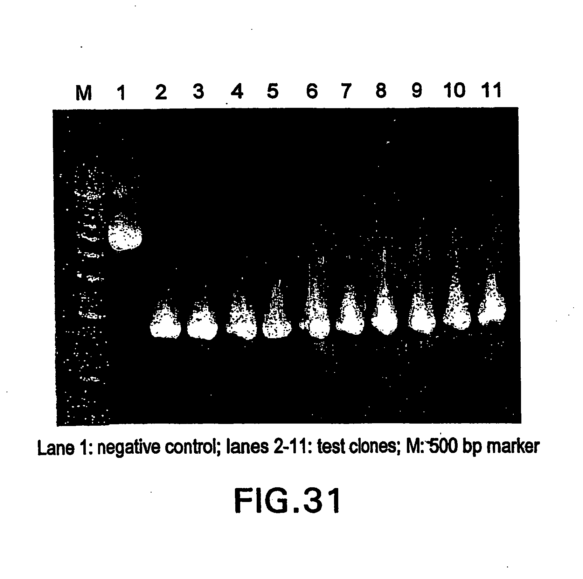

Figure 31 is a photograph of an ethidium bromide-stained agarose gel containing PCR products showing that the Gateway-compatible cassette contained inserts of the expected size. The Gateway-compatible cassette was constructed by first generating a CAT insert by PCR and then using a TOPO joining reaction to introduce attB1 and attB2 adaptors. The purified DNA product was inserted into pDONR 222 using a BP reaction. Following transformation into *E. coli*, PCR was performed on the colonies and the PCR product was checked on an ethidium bromide-stained agarose gel.

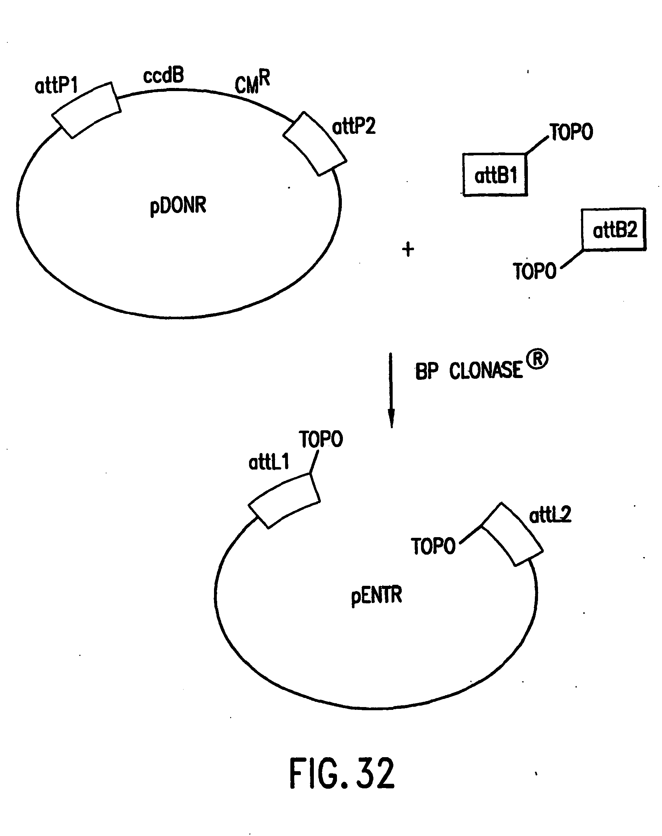

Figure 32 is a schematic diagram depicting the preparation of topoisomerase-charged pENTR vectors, by charging pDONR vectors with topoisomerase and carrying out a BxP GATEWAY cloning reaction according to methods described herein.

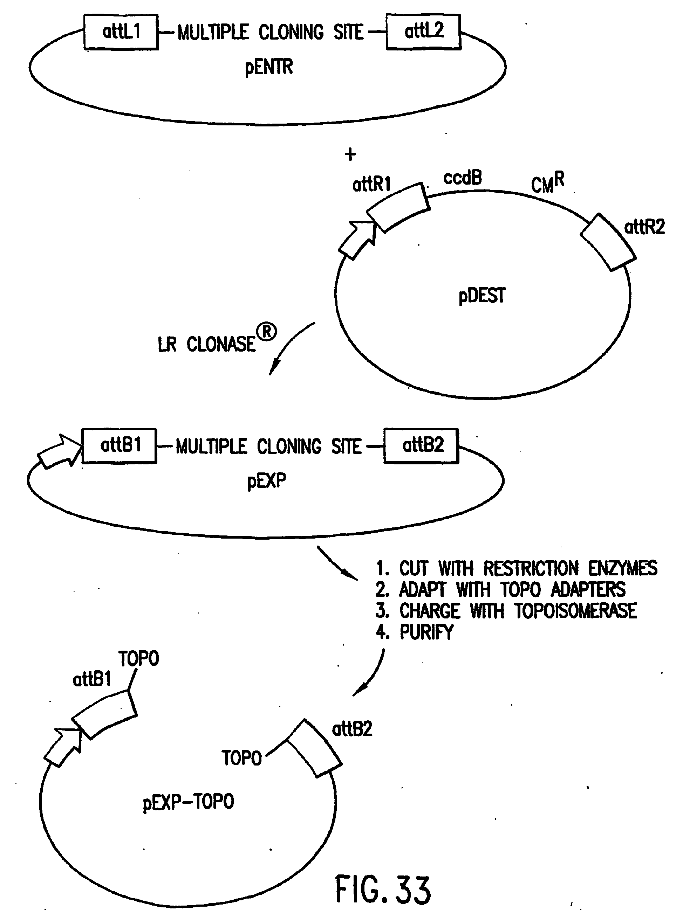

Figure 33 is a schematic diagram depicting the preparation of topoisomerase-charged pEXP vectors, by charging pDEST vectors with topoisomerase and carrying out an LxR GATEWAY cloning reaction, then adding TOPO adaptors to the cut ends of the pEXP vector, according to methods described herein.

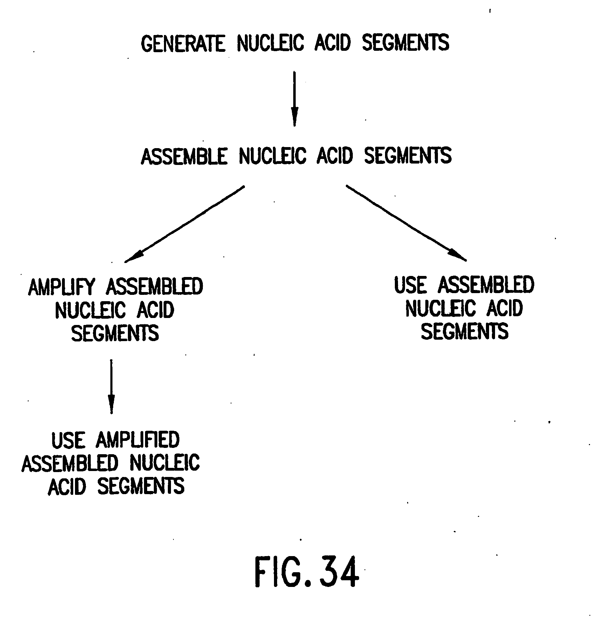

Figure 34 shows a schematic outline of methods described herein. In the first step, nucleic acid molecules to be assembled are generated using, for example, PCR. In the second step, nucleic acid molecules of the first step are assembled using methods described herein (*e*.*g*., methods involving the use of topoisomerase to covalently linking at least one strand of one nucleic acid segments to another nucleic acid segment). In the third step, assembled nucleic acid molecules generated in the second step either may be used directly or may be amplified and then used. Examples of uses of the assembled molecules are described elsewhere herein.

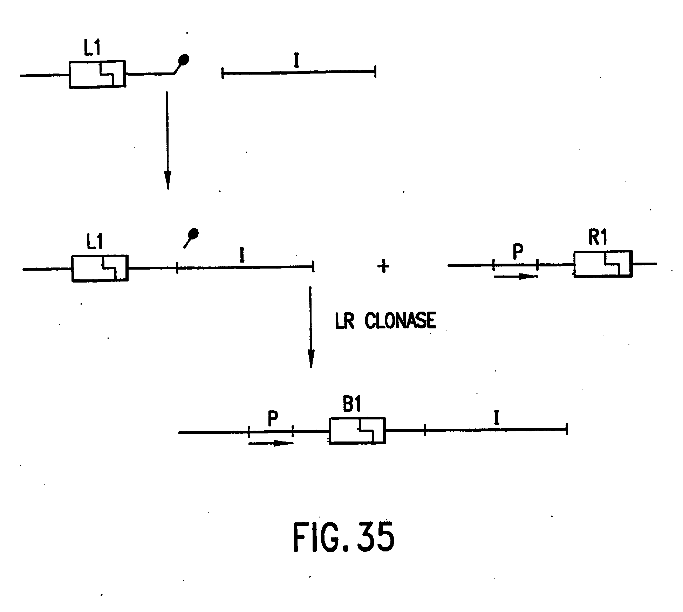

Figure 35 shows a schematic representation of a process for using topoisomerase to link two nucleic acid segments, followed by single site recombination to recombine the linked nucleic acid segment with another nucleic acid segment. In the first step, a topoisomerase adapted nucleic acid segment which contains an *att*L1 recombination site is linked to another nucleic acid segment, referred to here as an insert (labeled "I"), using any of the topoisomerase mediated methods described herein for connecting nucleic acid molecules. The topoisomerase assembled nucleic acid segments are then contacted with another nucleic acid segment which contains a promoter, labeled "P", and an *att*R1 recombination site in the presence of LR CLONASE™ under conditions which allow for recombination between the two recombination sites. Recombination results in the formation of a nucleic acid molecule which contains the insert nucleic acid segment in operable linkage with the promoter. Further, an *att*B1 recombination site is located between the promoter and the insert in the end product. The recombination sites shown in this figure are *att*L and *att*B sites, but any suitable recombination sites could be used.

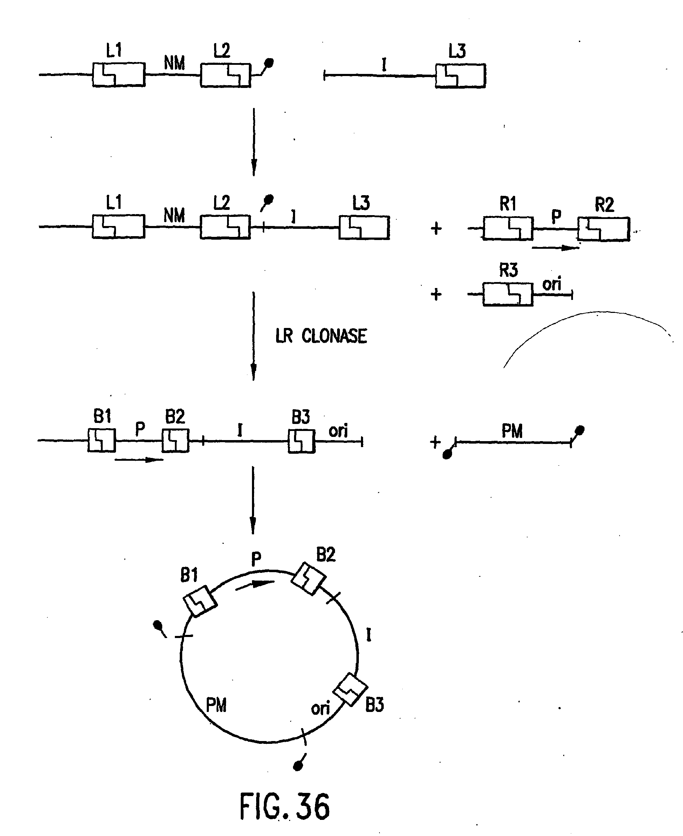

Figure 36 shows a schematic representation of a process for using topoisomerase and recombination to recombine and/or link five separate nucleic acid segments and circularize the resulting product. In the first step, a topoisomerase adapted nucleic acid segment which contains *att*L1 and *att*L2 recombination sites and a negative selection marker (labeled "NM") is linked to another nucleic acid segment, referred to here as an insert (labeled "I"), using any of the topoisomerase mediated methods described herein for connecting nucleic acid molecules. The topoisomerase assembled nucleic acid segments are then contacted with two additional nucleic acid segments, each of which contains at least one *att*R recombination site, in the presence of LR CLONASE^{™} (Invitrogen Corporation, Carlsbad, CA) under conditions which allow for recombination between the various recombination sites. In certain such methods, for example, TOPO-adapted vectors are incubated with one or more nucleic acid segments (*e*.*g*., one or more PCR products) at room temperature (*e*.*g*., about 20-20°C) for about 5-30 (and preferably about 10) minutes; the reaction is then heat-treated by incubation at about 80°C for about 20 minutes, and the reaction mixture then used in a standard LR reaction according to manufacturer's instructions (Invitrogen Corporation), except the incubation time for the LR reaction is increased to about 3 hours. The recombination reactions result in the formation of a product molecule in which the promoter is linked to (1) the insert molecule and (2) an origin of replication (labeled "ori"). This product molecule is then connected to a nucleic acid segment which is topoisomerase adapted at both termini and contains a positive selection marker (labeled "PM"). Further, the final topoisomerase linkage step results in the formation of a circular nucleic acid molecule. The recombination sites shown in this figure are *att*L and *att*B sites, but any suitable recombination sites could be used.

Figure 37 shows a schematic representation of a process for the preparation of nucleic acid molecules for performing homologous recombination. In this instance, three nucleic acid segments are connected to each other using methods which involve topoisomerase mediated covalent linkage of nucleic acid strands of the individual segments. Two of these nucleic acid segments each contain a positive selection marker and two *att*L sites which flank a negative selection marker. Thus, the nucleic acid molecule which results from the first step contains a nucleic acid segment, referred to here as an insert. On each side of the insert is (1) a positive selection marker and (2) two recombination sites which flank a negative selection marker. LR CLONASE^{™} catalyzed recombination in the presence of two nucleic acid segments which contain regions that share homology to a chromosomal locus where the nucleic acid end product is designed to integrate (labeled "HR1" and HR2") results in the formation of the end product nucleic acid molecule shown. As one skilled in the art would recognize, any suitable recombination sites could be used in the process set out in this figure.

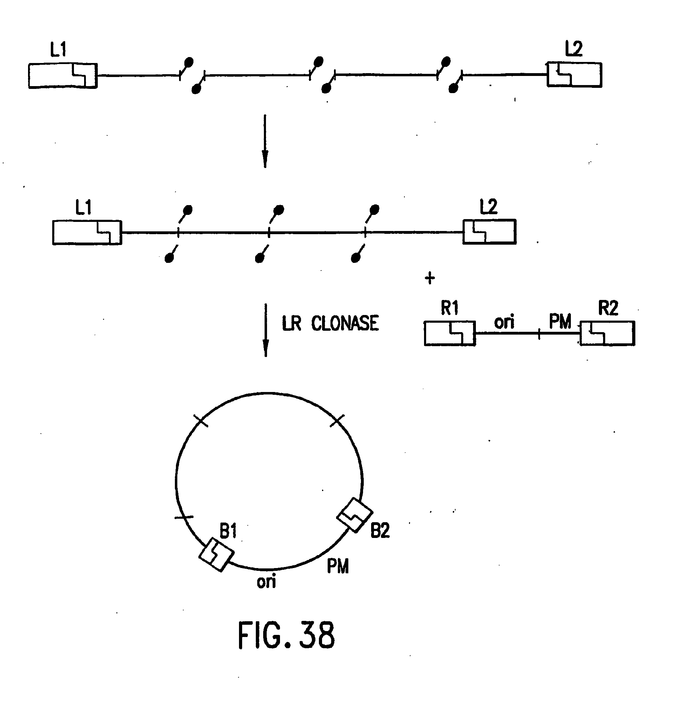

Figure 38 shows a schematic representation of the linking of four nucleic acid segments using toposiomerase to generate a linear nucleic acid molecule with recombination sites (labeled "L1" and "L2") located near the termini. Upon toposiomerase mediated linkage of the nucleic acid strands, no nicks are present at the junction points. In a second step, the topoisomerase assembled nucleic acid segments are contacted with another nucleic acid segment which contains an origin of replication (labeled "ori"), a positive selection marker (labeled "PM"), an *att*R1 recombination site, and an *att*R2 recombination site in the presence of LR CLONASE^{™} under conditions which allow for recombination between the recombination sites. Recombination results in the formation of a circular nucleic acid molecule as shown. The recombination sites shown in this figure are *att*L and *att*B sites, but any suitable recombination sites could be used.

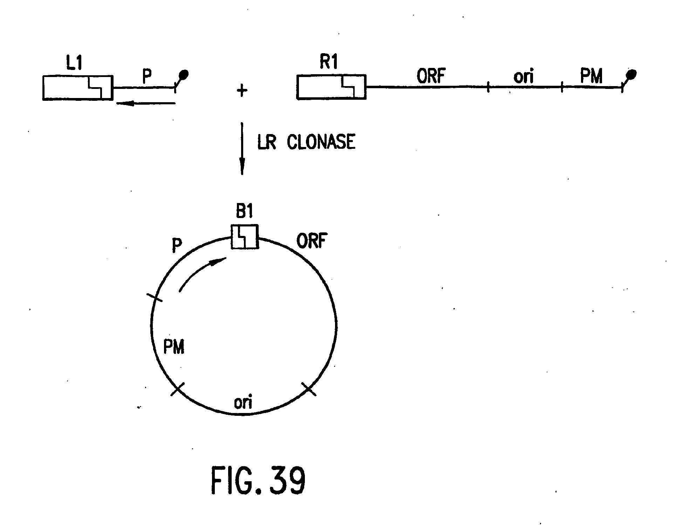

Figure 39 shows a schematic representation of the linking of two nucleic acid segments in a single step process using toposiomerase and recombination sites to generate a circular nucleic acid molecule. One of the nucleic acid segments contains an *att*L1 recombination site (labeled "L1"), a promoter (labeled "P"), and toposiomerase molecule covalently linked to one terminus. The other nucleic acid segment contains an *att*R1 recombination site (labeled "R1"), an open reading frame (labeled "ORF"), an origin of replication (labeled "ORI"), a positive selection marker (labeled "PM"), and topoisomerase molecule covalently linked to one terminus. Thus, when these two nucleic acid segments are contacted with each other in the presence of LR CLONASE^{™} under conditions which allow for recombination between the *att*L and *att*R recombination sites and topoisomerase mediated linkage of nucleic acid strands, a circular molecule is formed having the structure indicated. The recombination sites shown in this figure are *att*L and *att*B sites, but any suitable recombination sites could be used.

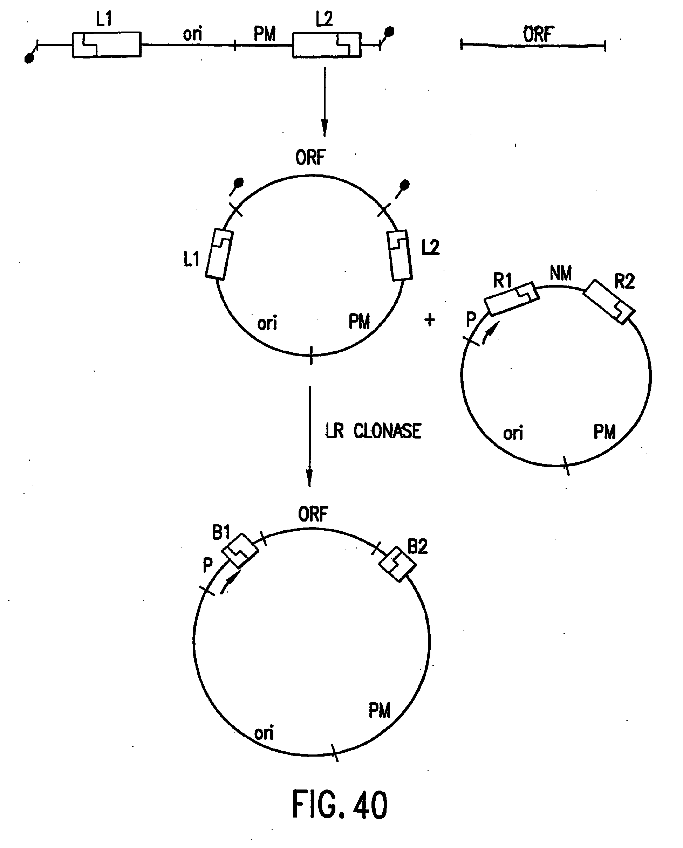

Figure 40 shows a schematic representation of the linking of two nucleic acid segments using toposiomerase mediated methods to generate a circular nucleic acid molecule. This circular molecule contains an open reading frame (labeled "ORF") positioned between *att*L1 and *att*L2 recombination site (labeled "L1" and "L2"). The topoisomerase assembled product then undergoes recombination with another circular molecule which contains *att*R1 and *att*R2 recombination sites to generate a third circular nucleic acid molecule which contains the open reading frame positioned between *att*B1 and *att*B2 recombination sites. Further, the open reading frame is operably linked to a promoter. The recombination sites shown in this figure are *att*L and *att*B sites, but any suitable recombination sites could be used.

### DETAILED DESCRIPTION OF THE INVENTION

### Definitions

In the description that follows, a number of terms used in recombinant nucleic acid technology are utilized extensively. In order to provide a clear and more consistent understanding of the specification and claims, including the scope to be given such terms, the following definitions are provided.

Gene: As used herein, a gene is a nucleic acid sequence that-contains information necessary for expression of a polypeptide, protein or functional RNA (*e*.*g*., a ribozyme, tRNA, rRNA, mRNA, etc.). It includes the promoter and the structural gene open reading frame sequence (orf) as well as other sequences involved in expression of the protein.

Structural gene: As used herein, a structural gene refers to a nucleic acid sequence that is transcribed into messenger RNA that is then translated into a sequence of amino acids characteristic of a specific polypeptide.

Host: As used herein, a host is any prokaryotic or eukaryotic organism that is a recipient of a replicable expression vector, cloning vector or any nucleic acid molecule. The nucleic acid molecule may contain, but is not limited to, a structural gene, a transcriptional regulatory sequence (such as a promoter, enhancer, repressor, and the like) and/or an origin of replication. As used herein, the terms "host," "host cell," "recombinant host" and "recombinant host cell" may be used interchangeably. For examples of such hosts, see Maniatis et al., Molecular Cloning: A Laboratory Manual, Cold Spring Harbor Laboratory, Cold Spring Harbor, New York (1982).

Transcriptional Regulatory Sequence: As used herein, transcriptional regulatory sequence is a functional stretch of nucleotides contained on a nucleic acid molecule, in any configuration or geometry, that acts to regulate the transcription of one or more structural genes into messenger RNA. Examples of transcriptional regulatory sequences include, but are not limited to, promoters, operators, enhancers, repressers, and the like. Transcriptional regulatory sequences may also regulate the transcription of nucleic acid molecules which encode functional RNAs (*e*.*g*., ribozymes, tRNAs, rRNAs, mRNAs, etc.).

Promoter: As used herein, a promoter is an example of a transcriptional regulatory sequence, and is specifically a nucleic acid sequence generally described as the 5'-region of a gene located proximal to the start codon. The transcription of an adjacent nucleic acid segment is initiated at the promoter region. A repressible promoter's rate of transcription decreases in response to a repressing agent. An inducible promoter's rate of transcription increases in response to an inducing agent. A constitutive promoter's rate of transcription is not specifically regulated, though it can vary under the influence of general metabolic conditions.

Insert: As used herein, an insert is a desire nucleic acid segment that is a part of a larger nucleic acid molecule.

Target Nucleic Acid Molecule: As used herein, target nucleic acid molecule is a nucleic acid segment of interest preferably nucleic acid which is to be acted upon using the compounds and methods of the present invention. Such target nucleic acid molecules preferably contain one or more genes or portions of genes.

Insert Donor: As used herein, an insert donor is one of the two parental nucleic acid molecules (e.g. RNA or DNA) as described herein which carries the Insert. The Insert Donor molecule comprises the Insert flanked on both sides with recombination sites. The Insert Donor can be linear or circular. The Insert Donor may be a circular nucleic acid molecule, optionally supercoiled, and further comprises a cloning vector sequence outside of the recombination signals (see Figure 1). When a population of Inserts or population of nucleic acid segments are used to make the Insert Donor, a population of Insert Donors result and may be used in accordance with disclosure herein.

Product: As used herein, a product is one the desired daughter molecules comprising the A and D sequences which is produced after the second recombination event during the recombinational cloning process (see Figure 1). The Product contains the nucleic acid which was to be cloned or subcloned. When a population of Insert Donors are used, the resulting population of Product molecules will contain all or a portion of the population of Inserts of the Insert Donors and preferably will contain a representative population of the original molecules of the Insert Donors.

Recognition sequence: As used herein, a recognition sequence (alternatively and equivalently referred to herein as a "recognition site") is a particular sequence to which a protein, chemical compound, DNA, or RNA molecule (e.g., restriction endonuclease, a topoisomerase, a modification methylase, or a recombinase) recognizes and binds. In the present invention, a recognition sequence will usually refer to a recombination site (which may alternatively be referred to as a recombinase recognition site) or a topoisomerase recognition site. For example, the recognition sequence for Cre recombinase is loxP which is a 34 base pair sequence comprised of two 13 base pair inverted repeats (serving as the recombinase binding sites) flanking an 8 base pair core sequence. See Figure 1 of Sauer, B., Current Opinion in Biotechnology 5:521-527 (1994). Other examples of such recognition sequences are the attB, attP, attL, and attR sequences which are recognized by the recombinase enzyme (Integrase. attB is an approximately 25 base pair sequence containing two 9 base pair core-type Int binding sites and a 7 base pair overlap region. attP is an approximately 240 base pair sequence containing core-type Int binding sites and arm-type Int binding sites as well as sites for auxiliary proteins integration host factor (IHF), FIS and excisionase (Xis). See Landy, Current Opinion in Biotechnology 3:699-707 (1993). Such sites may also be engineered to enhance production of products in the methods of the invention. When such engineered sites lack the P1 or H1 domains to make the recombination reactions irreversible (e.g., attR or attP), such sites may be designated attR' or attP' to show that the domains of these sites have been modified in some way. Examples of topoisomerase recognitions sites include, but are not limited to, the sequence 5'-GCAACTT-3' that is recognized by E. coli topoisomerase III (a type I topoisomerase); the sequence 5-(C/T)CCTT-3' which is a topoisomerase recognition site that is bound specifically by most poxvirus topoisomerases, including vaccinia virus DNA topoisomerase I; and others that are known in the art as discussed elsewhere herein.

Recombination proteins: As used herein, recombination proteins include excisive or integrative proteins, enzymes, co-factors or associated proteins that are involved in recombination reactions involving one or more recombination sites, which may be wild-type proteins (See Landy, Current Opinion in Biotechnology 3:699-707 (1993)), or mutants, derivatives (e.g., fusion proteins containing the recombination protein sequences or fragments thereof), fragments, and variants thereof.

Recombination site: A used herein, a recombination site is a recognition sequence on a nucleic acid molecule participating in an integration/recombination reaction by recombination proteins. Recombination sites are discrete sections or segments of nucleic acid on the participating nucleic acid molecules that are recognized and bound by a site-specific recombination protein during the initial stages of integration or recombination. For example, the recombination site for Cre recombinase is loxP which is a 34 base pair sequence comprised of two 13 base pair inverted repeats (serving as the recombinase binding sites) flanking an 8 base pair core sequence. See Figure 1 of Sauer, B., Curr. Opin. Biotech. 5:521-527 (1994). Other examples of recognition sequences include the attB, attP, attL, and attR sequences described herein, and mutants, fragments, variants and derivatives thereof, which are recognized by the recombination protein ( Int and by the auxiliary proteins integration host factor (IHF), FIS and excisionase (Xis). See Landy, Curr. Opin. Biotech. 3:699-707 (1993).

Recombinational Cloning: As used herein, recombinational cloning is a method, such as that described in U.S. Patent Nos. 5,888,732, 6,143,557, 6,171,861, 6,270,969, and 6,277,608, and as also described herein, whereby segments of nucleic acid molecules or populations of such molecules are exchanged, inserted, replaced, substituted or modified, in vitro or in vivo. Preferably, such cloning method is an in vitro method.

Repression cassette: As used herein, repression cassette is a nucleic acid segment that contains a repressor or a Selectable marker present in the subcloning vector.

Selectable marker: As used herein, selectable marker is a nucleic acid segment that allows one to select for or against a molecule (e.g., a replicon) or a cell that contains it, often under particular conditions. These markers can encode an activity, such as, but not limited to, production of RNA, peptide, or protein, or can provide a binding site for RNA, peptides, proteins, inorganic and organic compounds or compositions and the like. Examples of selectable markers include but are not limited to: (1) nucleic acid segments that encode products which provide resistance against otherwise toxic compounds (e.g., antibiotics); (2) nucleic acid segments that encode products which are otherwise lacking in the recipient cell (e.g., tRNA genes, auxotrophic markers); (3) nucleic acid segments that encode products which suppress the activity of a gene product; (4) nucleic acid segments that encode products which can be readily identified (e.g., phenotypic markers such as (-galactosidase, green fluorescent protein (GFP), and cell surface proteinn); (5) nucleic acid segments that bind products which are otherwise detrimental to cell survival and/or function; (6) nucleic acid segments that otherwise inhibit the activity of any of the nucleic acid segments described in Nos. 1-5 above (e.g., antisense oligonucleotides); (7) nucleic acid segments that bind products that modify a substrate (e.g. restriction endonucleases); (8) nucleic acid segments that can be used to isolate or identify a desired molecule (e.g. specific protein binding sites); (9) nucleic acid segments that encode a specific nucleotide sequence which can be otherwise non-functional (e.g., for PCR amplification of subpopulations of molecules); (10) nucleic acid segments, which when absent, directly or indirectly confer resistance or sensitivity to particular compounds; and/or (11) nucleic acid segments that encode products which are toxic in recipient cells.

Selection scheme: As used herein, selection scheme is any method which allows selection, enrichment, or identification of a desired Product or Product(s) from a mixture containing an Entry Clone or Vector, a Destination Vector, a Donor Vector, an Expression Clone or Vector, any intermediates (e.g. a Cointegrate or a replicon), and/or Byproducts. The selection schemes of one preferred embodiment have at least two components that are either linked or unlined during recombinational cloning. One component is a Selectable marker. The other component controls the expression in vitro or in vivo of the Selectable marker, or survival of the cell (or the nucleic acid molecule, e.g., a replicon) harboring the plasmid carrying the Selectable marker. Generally, this controlling element will be a repressor or inducer of the Selectable marker, but other means for controlling expression or activity of the Selectable marker can be used. Whether a repressor or activator is used will depend on whether the marker is for a positive or negative selection, and the exact arrangement of the various nucleic acid segments, as will be readily apparent to those skilled in the art. In some preferred embodiments, the selection scheme results in selection of or enrichment for only one or more desired Products. As defined herein, selecting for a nucleic acid molecule includes (a) selecting or enriching for the presence of the desired nucleic acid molecule, and (b) selecting or enriching against the presence of nucleic acid molecules that are not the desired nucleic acid molecule.

In one embodiment, the selection schemes (which can be carried out in revere) will take one of three forms, which will be discussed in terms of Figure 1. The first, exemplified herein with a Selectable marker and a repressor therefore, selects for molecules having segment D and lacking segment C. The second selects against molecules having segment C and for molecules having segment D. Possible embodiments of the second form would have a nucleic acid segment carrying a gene toxic to cells into which the in vitro reaction products are to be introduced. A toxic gene can be a nucleic acid that is expressed as a toxic gene product (a toxic protein or RNA), or can be toxic in and of itself. (In the latter case, the toxic gene is understood to carry its classical definition of "heritable trait".)

Examples of such toxic gene products are well known in the art, and include, but are not limited to, restriction endonucleases (e.g., DpnI), apoptosis-related genes (e.g. ASK1 or members of the bcl-2/ced-9 family), retroviral genes including those of the human immunodeficiency virus (HIV), defensins such as NP-1, inverted repeats or paired palindromic nucleic acid sequences, bacteriophage lytic genes such as those from (X174 or bacteriophage T4; antibiotic sensitivity genes such as rpsL, antimicrobial sensitivity genes such as pheS, plasmid killer genes, eukaryotic transcriptional vector genes that produce a gene product toxic to bacteria, such as GATA-1, and genes that kill hosts in the absence of a suppressing function, e.g., kicB, ccdB, (X174 E (Liu, Q. et al., Curr. Biol. 8:1300-1309 (1998)), and other genes that negatively affect replicon stability and/or replication. A toxic gene can alternatively be selectable in vitro, e.g., a restriction site.

Many genes coding for restriction endonucleases operably linked to inducible promoters are known, and may be used following the disclosure herein. See, e.g. U.S. Patent Nos. 4,960,707 (DpnI and DpnII); 5,000,333, 5,082,784 and 5,192,675 (KpnI); 5,147,800 (NgoAIII and NgoAI); 5,179,015 (FspI and HaeIII): 5,200,333 (HaeII and TaqI); 5,248,605 (HpaII); 5,312,746°ClaI); 5,231,021 and 5,304,480 (XhoI and XhoII); 5,334,526 (AluI); 5,470,740 (NsiI); 5,534,428 (SstI/SacI); 5,202,248 (NcoI); 5,139,942 (NdeI); and 5,098,839 (PacI). See also Wilson, G.G., Nucl. Acids Res. 19:2539-2566 (1991); and Lunnen, K.D., et al., Gene 74:25-32 (1988).

In the second form, segment D carries a Selectable marker. The toxic gene would eliminate transformants harboring the Vector Donor, Cointegrate, and Byproduct molecules, while the Selectable marker can be used to select for cells containing the Product and against cells harboring only the Insert Donor.

The third form selects for cells that have both segments A and D in cis on the same molecule, but not for cells that have both segments in trans on different molecules. This could be embodied by a Selectable marker that is split into two inactive fragments, one each on segments A and D.

The fragments are so arranged relative to the recombination sites that when the segments are brought together by the recombination event, they reconstitute a functional Selectable marker. For example, the recombinational event can link a promoter with a structural nucleic acid molecule (e.g., a gene), can link two fragments of a structural nucleic acid molecule, or can link nucleic acid molecules that encode a heterodimeric gene product needed for survival, or can link portions of a replicon.

Site-specific recombinase: As used herein, a site specific recombinase is a type of recombinase which typically has at least the following four activities (or combinations thereof): (1) recognition of one or two specific nucleic acid sequences; (2) cleavage of said sequence or sequences; (3) topoisomerase activity involved in strand exchange; and (4) ligase activity to reseal the cleaved strands of nucleic acid. See Sauer, B., Current Opinions in Biotechnology 5:521-527 (1994). Conservative site-specific recombination is distinguished from homologous recombination and transposition by a high degree of specificity for both partners. The strand exchange mechanism involves the cleavage and rejoining of specific nucleic acid sequences in the absence of DNA synthesis (Landy, A. (1989) Ann. Rev. Biochem. 58:913-949).

Vector: As used herein, a vector is a nucleic acid molecule (preferably DNA) that provides a useful biological or biochemical property to an Insert. Examples include plasmids, phages, autonomously replicating sequences (ARS), centromeres, and other sequences which are able to replicate or be replicated in vitro or in a host cell, or to convey a desired nucleic acid segment to a desired location within a host cell. A Vector can have one or more restriction endonuclease recognition sites at which the sequences can be cut in a determinable fashion without loss of an essential biological function of the vector, and into which a nucleic acid fragment can be spliced in order to bring about its replication and cloning. Vectors can further provide primer sites, e.g., for PCR, transcriptional and/or translational initiation and/or regulation sites, recombinational signals, replicons, Selectable markers, etc. Clearly, methods of inserting a desired nucleic acid fragment which do not require the use of recombination, transpositions or restriction enzymes (such as, but not limited to, UDG cloning of PCR fragments (U.S. Patent No. 5,334,575), TA Cloning^{®} brand PCR cloning (Invitrogen Corporation, Carlsbad, CA) (also known as direct ligation cloning), and the like) can also be applied to clone a fragment into a cloning vector to be used as described herein. The cloning vector can further contain one or more selectable markers suitable for use in the identification of cells transformed with the cloning vector.

Subcloning vector: As used herein, a subcloning vector is a cloning vector comprising a circular or linear nucleic acid molecule which may include an appropriate replicon. In the present invention, the subcloning vector (segment D in Figure 1) can also contain functional and/or regulatory elements that are desired to be incorporated into the final product to act upon or with the cloned nucleic acid Insert (segment A in Figure 1). The subcloning vector can also contain a Selectable marker (preferably DNA).

Vector Donor: As used herein, a Vector Donor is one of the two parental nucleic acid molecules (e.g. RNA or DNA) described herein which carries the nucleic acid segments comprising the nucleic acid vector which is to become part of the desired Product. The Vector Donor comprises a subcloning vector D (or it can be called the cloning vector if the Insert Donor does not already contain a cloning vector) and a segment C flanked by recombination sites (see Figure 1). Segments C and/or D can contain elements that contribute to selection for the desired Product daughter molecule, as described above for selection schemes. The recombination signals can be the same or different, and can be acted upon by the same or different recombinases. In addition, the Vector Donor can be linear or circular.

Primer: As used herein, a primer is a single stranded or double stranded oligonucleotide, that is extended by covalent bonding of nucleotide monomers during amplification or polymerization of a nucleic acid molecule (e.g. a DNA molecule). In one aspect, the primer may be a sequencing primer (for example, a universal sequencing primer). In another aspect, the primer may comprise a recombination site or portion thereof.

Template: As used herein, a template is a double stranded or single stranded nucleic acid molecule which is to be amplified, synthesized or sequenced. In the case of a double-stranded DNA molecule, denaturation of its strands to form a first and a second strand is preferably performed before these molecules may be amplified, synthesized or sequenced, or the double stranded molecule may be used directly as a template. For single stranded templates, a primer complementary to at least a portion of the template is hybridized under appropriate conditions and one or more polypeptides having polymerase activity (e.g. DNA polymerases and/or reverse transcriptases) may then synthesize a molecule complementary to all or a portion of the template. Alternatively, for double stranded templates, one or more transcriptional regulatory sequences (e.g., one or more promoters) may be used in combination with one or more polymerases to make nucleic acid molecules complementary to all or a portion of the template. The newly synthesized molecule, may be of equal or shorter length compared to the original template. Mismatch incorporation or strand slippage during the synthesis or extension of the newly synthesized molecule may result in one or a number of mismatched base pairs. Thus, the synthesized molecule need not be exactly complementary to the template. Additionally, a population of nucleic acid templates may be used during synthesis or amplification to produce a population of nucleic acid molecules typically representative of the original template population.

Incorporating: As used herein, incorporating means becoming a part of a nucleic acid (e.g., DNA) molecule or primer.

Library: As used herein, a library is a collection of nucleic acid molecules (circular or linear). In one embodiment, a library may comprise a plurality (i.e., two or more) of nucleic acid molecules, which may or may not be from a common source organism, organ, tissue, or cell. In another embodiment, a library is representative of all or a portion or a significant portion of the nucleic acid content of an organism (a "genomic" library), or a set of nucleic acid molecules representative of all or a portion or a significant portion of the expressed nucleic acid molecules (a cDNA library or segments derived therefrom) in a cell, tissue, organ or organism. A library may also comprise random sequences made by de novo synthesis, mutagenesis of one or more sequences and the like. Such libraries may or may not be contained in one or more vectors.

Amplification: As used herein, amplification is any in vitro method for increasing a number of copies of a nucleotide sequence with the use of one or more polypeptides having polymerase activity (e.g., one or more nucleic acid polymerases or one or more reverse transcriptases). Nucleic acid amplification results in the incorporation of nucleotides into a DNA and/or RNA molecule or primer thereby forming a new nucleic acid molecule complementary to a template. The formed nucleic acid molecule and its template can be used as templates to synthesize additional nucleic acid molecules. As used herein, one amplification reaction may consist of many rounds of nucleic acid replication. DNA amplification reactions include, for example, polymerase chain reaction (PCR). One PCR reaction may consist of 5 to100 cycles of denaturation and synthesis of a DNA molecule.

Nucleotide: As used herein, a nucleotide is a base-sugar-phosphate combination. Nucleotides are monomeric units of a nucleic acid molecule (DNA and RNA). The term nucleotide includes ribonucleoside triphosphates ATP, UTP, CTG, GTP and deoxyribonucleoside triphosphates such as dATP, dCTP, dITP, dUTP, dGTP, dTTP, or derivatives thereof. Such derivatives include, for example, [(S]dATP, 7-deaza-dGTP and 7-deaza-dATP. The term nucleotide as used herein also refers to dideoxyribonucleoside triphosphates (dNTPs) and their derivatives. Illustrated examples of dideoxyribonucleoside triphosphates include, but are not limited to, ddATP, ddCTP, ddGTP, ddITP, and ddTTP. A "nucleotide" may be unlabeled or detectably labeled by well known techniques. Detectable labels include, for example, radioactive isotopes, fluorescent labels, chemiluminescent labels, bioluminescent labels and enzyme labels.

Nucleic acid molecule: As used herein, a nucleic acid molecule is a sequence of contiguous nucleotides (riboNTPs, dNTPs or ddNTPs, or combinations thereof) of any length which may encode a full-length polypeptide or a fragment of any length thereof, or which may be non-coding As used herein, the terms "nucleic acid molecule" and "polynucleotide" may be used interchangeably.

Oligonucleotide: As used herein, an oligonucleotide is a synthetic or natural molecule comprising a covalently linked sequence of nucleotides which are joined by a phosphodiester bond between the 3' position of the pentose of one nucleotide and the 5' position of the pentose of the adjacent nucleotide.

Polypeptide: As used herein, a polypeptide is a sequence of contiguous amino acids, of any length. As used herein, the terms "peptide," "oligopeptide," or "protein" may be used interchangeably with the term "polypeptide."

Hybridization: As used herein, the terms hybridization and hybridizing refer to base pairing of two complementary single-stranded nucleic acid molecules (RNA and/or DNA) to give a double stranded molecule. As used herein, two nucleic acid molecules may be hybridized, although the base pairing is not completely complementary. Accordingly, mismatched bases do not prevent hybridization of two nucleic acid molecules provided that appropriate conditions, well known in the art, are used. hybridization may be said to be under "stringent conditions." By "stringent conditions" as used herein is meant overnight incubation at 42°C in a solution comprising: 50% formamide, 5x SSC (150 mM NaCl, 15mM trisodium citrate), 50 mM sodium phosphate (pH 7.6), 5x Denhardt's solution, 10% dextran sulfate, and 20 g/ml denatured, sheared salmon sperm DNA, followed by washing the filters in O.lx SSC at about 65°C.

Other terms used in the fields of recombinant nucleic acid technology and molecular and cell biology as used herein will be generally understood by one of ordinary skill in the applicable arts.

### Overview

Described herein are methods, compositions and kits for the recombinational and/or topoisomerase-mediated joining of two or more segments or molecules of nucleic acid or other molecules and/or compounds (or combinations thereof). Attaching such linked nucleic acids or other molecules and/or compounds to one or more supports or structures preferably through recombination sites (which may include recombination protein recognition sequences, topoisomerase recognition sequences, etc.) or portions thereof is also described herein. Thus, the disclosure herein generally relates to linking any number of nucleic acids or other molecules and/or compounds via nucleic acid linkers comprising one or more topoisomerase recognition sites and/or one or more recombination sites or portions thereof. The linked products produce as described herein may comprise any number of the same or different nucleic acids or other molecules and/or compounds, depending on the starting materials. Such starting materials inlcude, but are not limited to, any nucleic acids (or derivatives thereof such as peptide nucleic acids (PNAs)), chemical compounds, detectably labeled molecules (such as fluorescent molecules and chemiluminescent molecules), drugs, peptides or protesins, lipids, carbohydrates and other molecules and/or compounds comprising one or more recombination sites or portions thereof. Through recombination of such recombination sites and/or topoisomerase-mediated joining reactions as described herein, any number or combination of such starting molecules and/or compounds can be linked to make linked products as described herein. In addition, deletion or replacement of certain portions or components of the linked products described herein can be accomplished by recombination.

The joined segments may be inserted into a different nucleic acid molecule such as vectors, such as by recombinational cloning methods and/or topoisomerase-mediated joining methods as described herein. Thus, the construction of nucleic acid molecules (RNA or DNA) by combining two or more segments of nucleic acid by a recombination reaction and/or a topoisomerase-mediated joining reaction and inserting the joined two or more segments into a vector by recombinational cloning is described herein. Where the joined nucleic acid molecules may be further combined with an additional nucleic acid molecule by a recombination reaction, the timing of the two recombination events, i.e. the joining of the segments and the insertion of the segments into a vector, is not critical. That is to say, it is not critical whether the two or more nucleic acid segments are joined together before insertion into the vector or whether, for example, one recombination site on each segment first reacts with a recombination site on the vector and subsequently the recombination sites on the nucleic acid segments react with each other to join the segments. Moreover, the nucleic acid segments can be cloned in any one or a number of positions within the vector and do not need to be inserted adjacent to each other, although, in some embodiments, joining of two or more of such R segments within the vector is preferred. Recombinational cloning allows efficient selection and identification of molecules (particularly vectors) containing the combined nucleic acid segments. Thus, two or more nucleic acid segments of interest can be combined and, optionally, inserted into a single vector suitable for further manipulation of the combined nucleic acid molecule.

In additional embodiments, at least two (*e*.*g*., 2, 3, 4, 5, 6, 7, 8, etc.) nucleic acid segments, each comprising at least one (*e*.*g*., 1, 2, 3, 4, 5, 6, 7, 8, etc.) recombination site and optionally with at least one (*e*.*g*., 1, 2, 3, 4, 5, 6, 7, 8, etc.) topoisomerase recognition site, are contacted with suitable recombination proteins and/or with topoisomerase to effect the joining all or a portion of the two molecules, depending on the position of the recombination sites in the molecules. In certain such embodiments, such as in nucleic acid molecules comprising at least two recombination sites, at least one of the two recombination site flanks each end of a topoisomerase recognition site in the molecule. By a recombination site (or a topoisomerase recognition site) that "flanks" another recognition site (*e*.*g*., another recombination site or topoisomerase recognition site) is meant that the two sites are within about 20 nucleotides of each other, or within about 20, 19, 18, 17, 16, 15, 14, 13, 12, 11, 10, 9, 8, 7, 6, 5, 4, 3, 2, 1 or 0 nucleotides of each other. Each individual nucleic acid segment may comprise a variety of sequences including, but not limited to sequences suitable for use as primer sites (e.g., sequences for which a primer such as a sequencing primer or amplification primer may hybridize to initiate nucleic acid synthesis, amplification or sequencing), transcription or translation signals or regulatory sequences such as promoters, ribosomal binding sites, Kozak sequences, and start codons, termination signals such as stop codons, origins of replication, recombination sites (or portions thereof), topoisomerase recognition sites (or portions thereof), selectable markers, and genes or portions of genes to create protein fusions (e.g., N-terminal or carboxy terminal) such as GST, GUS, GFP, 6 histidines, epitopes haptens and the like and combinations thereof. The vectors used for cloning such segments may also comprise these functional sequences (e.g., promoters, primer sites etc.). After combination of the segments comprising such sequences and optimally the cloning of the sequences into one or more vectors, the molecules may be manipulated in a variety of ways including sequencing or amplification of the target sequence (i.e., by using at least one or the primer sites introduced by the integration sequence), mutation of the target sequence (i.e., by insertion, deletion or substitution in or on the target sequences), and protein expression from the target sequence or portions thereof (i.e., by expression of translation and/or transcription signals contained by the segments and/or vectors).

Described herein is the generation of combinatorial libraries using the recombinational cloning methods disclosed. Thus, one or more of the nucleic acid segments joined may comprise a nucleic acid library. Such a library may comprise, for example, nucleic acid sequences corresponding to permutations of a sequence coding for a peptide, polypeptide or protein sequence. The permutations can be joined to another nucleic acid segment consisting of a single sequence or, alternatively, the second nucleic acid segment may also be a library corresponding to permutation of another peptide, polypeptide or protein sequence such that joining of the two segments may produce a library representing all possible combinations of all the permutations of the two peptide, polypeptide or proteins sequences. Numerous examples of the use of combinatorial libraries are known in the art. See, for example, Waterhouse, et al., Nucleic Acids Research, 1993, Vol. 21, No. 9, 2265-2266, Tsurushita, et al., Gene, 1996, Vol. 172 No. 1, 59-63, Persson, Int Rev Immunol 1993 10:2-3 153-63, Chanock, et al., Infect Agents Dis 1993 Jun 2:3 118-31, Burioni, et al., Res Virol 1997 Mar-Apr 148:2 161-4, Leung, Thromb Haemost 1995 Jul 74:1 373-6, Sandhu, Crit Rev Biotechnol 1992 12:5-6 437-62 and United States patents 5,733,743, 5,871,907 and 5,858,657.

### Recombination Sites

Recombination sites for use as described herein may be any nucleic acid sequence that can serve as a substrate in a recombination reaction. Such recombination sites may be wild-type or naturally occurring recombination sites or modified or mutant recombination sites. Examples of recombination sites for use as described herein include, but are not limited to, phage-lambda recombination sites (such as attP, attB, attL, and attR and mutants or derivatives thereof) and recombination sites from other bacteriophage such as phi80, P22, P2, 186, P4 and P1 (including lox sites such as loxP and loxP511). Novel mutated att sites (e. g., attB 1-10, attP 1-10, attR 1-10 and attL 1-10) are described in previous patent application serial number 60/136,744, filed May 28, 1999. Other recombination sites having unique specificity (i.e., a first site will recombine with its corresponding site and will not recombine with a second site having a different specificity) are known to those skilled in the art and may be used as described herein.

Corresponding recombination proteins for these systems may be used as described herein with the indicated recombination sites. Other systems providing recombination sites and recombination proteins for use as described herein include the FLP/FRT system from Saccharomyces cerevisiae, the resolvase family (e.g., ((, Tn3 resolvase, Hin, Gin and Cin), and IS231 and other Bacillus thuringiensis transposable elements. Other suitable recombination systems for use as described herein include the XerC and XerD recombinases and the psi, dif and cer recombination sites in *E. coli*. Other suitable recombination sites may be found in United States patent no. 5,851,808 issued to Elledge and Liu. Preferred recombination proteins and mutant or modified recombination sites for use in the invention include those described in U.S. Patent Nos. 5,888,732, 6,171,861, 6,143,557, 6,270,969 and 6,277,608, and commonly owned, co-pending U.S. Application Nos. 09/438,358 (filed 11/12/99), 09/517,466 (filed 03/02/00), 09/695,065 (filed 10/25/00) and 09/732,914 (filed 12/11/00), as well as those associated with the GATEWAY^{™} Cloning Technology available from Invitrogen Corporation (Carlsbad, CA).

### Topoisomerase Cloning

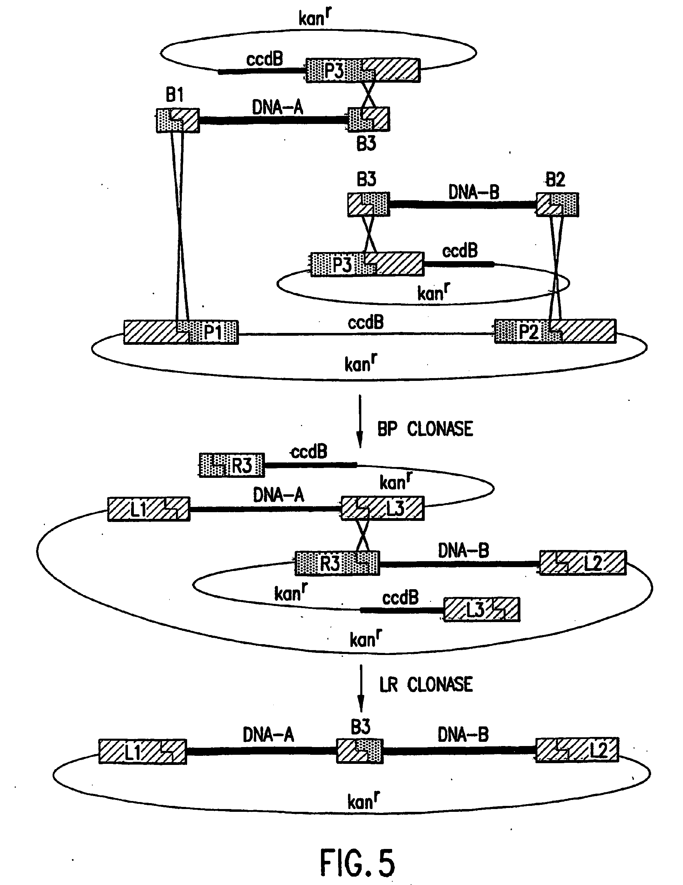

Described herein are methods of using one or more topoisomerases to generate a recombinant nucleic acid molecule from two or more nucleotide sequences. Described herein is a method for generating a ds recombinant nucleic acid molecule that is covalently linked in one strand. Such a method is directed to linking a first and at least a second nucleotide sequence with at least one (e.g., 1, 2, 3, 4, 5, 6, 7, 8, 9, 10, etc.) topoisomerase (e.g., a type IA, type IB, and/or type II topoisomerase) such that one strand, but not both strands, is covalently linked (see, for example, Figure 11). Also described herein is a method for generating a ds recombinant nucleic acid molecule covalently linked in both strands. Such a method is directed to linking a first and at least a second nucleotide sequence with at least one topoisomerase, such that ligated ends are covalently linked in both strands (i.e., the ds recombinant nucleic acid molecule contain no nicks at the positions where ends were ligated; see, for example, Figure 5). Also described herein is a method for generating a recombinant nucleic acid molecule covalently linked in one strand, wherein the substrate nucleotide sequences linked according to the method include at least one single stranded nucleotide sequence, which can be covalently linked to a second (or more) single stranded nucleotide sequence or to a nucleic acid molecule (see, for example, Figure 15).

A method for generating a ds recombinant nucleic acid molecule covalently linked in one strand can be performed by contacting a first nucleic acid molecule which has a site-specific topoisomerase recognition site (e.g., a type IA or a type II topoisomerase recognition site), or a cleavage product thereof, at a 5' or 3' terminus, with a second (or other) nucleic acid molecule, and optionally, a topoisomerase (e.g., a type IA, type IB, and/or type II topoisomerase), such that the second nucleotide sequence can be covalently attached to the first nucleotide sequence. The methods disclosed herein can be performed using any number of nucleotide sequences, typically nucleic acid molecules wherein at least one of the nucleotide sequences has a site-specifc topoisomerase recognition site (e.g., a type IA, or type II topoisomerase), or cleavage product thereof, at one or both 5' termini (see, for example, Figures 11A-11F).

A method for generating a ds recombinant nucleic acid molecule covalently linked in both strands can be performed, for example, by contacting a first nucleic acid molecule having a first end and a second end, wherein, at the first end or second end or both, the first nucleic acid molecule has a topoisomerase recognition site (or cleavage product thereof) at or near the 3' terminus; at least a second nucleic acid molecule having a first end and a second end, wherein, at the first end or second end or both, the at least second double stranded nucleotide sequence has a topoisomerase recognition site (or cleavage product thereof) at or near a 3' terminus; and at least one site specific topoisomerase (e.g., a type IA and/or a type IB topoisomerase), under conditions such that all components are in contact and the topoisomerase can effect its activity. A covalently linked ds recombinant nucleic acid generated according to a method described herein is characterized, in part, in that it does not contain a nick in either strand at the position where the nucleic acid molecules are joined. In one embodiment, the method is performed by contacting a first nucleic acid molecule and a second (or other) nucleic acid molecule, each of which has a topoisomerase recognition site, or a cleavage product thereof, at the 3' termini or at the 5' termini of two ends to be covalently linked. In another embodiment, the method is performed by contacting a first nucleic acid molecule having a topoisomerase recognition site, or cleavage product thereof, at the 5' terminus and the 3' terminus of at least one end, and a second (or other) nucleic acid molecule having a 3' hydroxyl group and a 5' hydroxyl group at the end to be linked to the end of the first nucleic acid molecule containing the recognition sites. As disclosed herein, the methods can be performed using any number of nucleic acid molecules having various combinations of termini and ends (see, for example, Figure 12A-12D).

Topoisomerases are categorized as type I, including type IA and type IB topoisomerases, which cleave a single strand of a double stranded nucleic acid molecule, and type II topoisomerases (gyrases), which cleave both strands of a nucleic acid molecule. Type IA and IB topoisomerases cleave one strand of a nucleic acid molecule. Cleavage of a nucleic acid molecule by type IA topoisomerases generates a 5' phosphate and a 3' hydroxyl at the cleavage site, with the type IA topoisomerase covalently binding to the 5' terminus of a cleaved strand. In comparison, cleavage of a nucleic acid molecule by type IB topoisomerases generates a 3' phosphate and a 5' hydroxyl at the cleavage site, with the type IB topoisomerase covalently binding to the 3' terminus of a cleaved strand. As disclosed herein, type I and type II topoisomerases, as well as catalytic domains and mutant forms thereof, are useful for generating ds recombinant nucleic acid molecules covalently linked in both strands according to a method described herein.

Type IA topoisomerases include *E. coli* topoisomerase I, *E. coli* topoisomerase III, eukaryotic topoisomerase II, archeal reverse gyrase, yeast topoisomerase III, Drosophila topoisomerase III, human topoisomerase III, Streptococcus pneumoniae topoisomerase III, and the like, including other type IA topoisomerases (see Berger, Biochim. Biophys. Acta 1400:3-18, 1998; DiGate and Marians, J. Biol. Chem. 264:17924-17930, 1989; Kim and Wang, J. Biol. Chem. 267:17178-17185, 1992; Wilson et al., J. Biol. Chem. 275:1533-1540, 2000; Lanai et al., Proc. Natl. Acad. Sci., USA 93:3653-3657, 1996, U.S. Pat. No. 6,277,620). *E. coli* topoisomerase III, which is a type IA topoisomerase that recognizes, binds to and cleaves the sequence 5'-GCAACTT-3', can be particularly useful in a method of the invention (Zhang et al., J. Biol. Chem. 270:23700-23705, 1995). A homolog, the traE protein of plasmid RP4, has been described by Li et al., J. Biol. Chem. 272:19582-119587 (1997) and can also be used in the practice of the invention: A DNA-protein adduct is formed with the enzyme covalently binding to the 5'-thymidine residue, with cleavage occurring between the two thymidine residues.

Type IB topoisomerases include the nuclear type I topoisomerases present in all eukaryotic cells and those encoded by vaccinia and other cellular poxviruses (see Cheng et al., Cell 92:841-850, 1998). The eukaryotic type IB topoisomerases are exemplified by those expressed in yeast, Drosophila and mammalian cells, including human cells (see Caron and Wang, Adv. Pharmacol. 29B,:271-297, 1994; Gupta et al., Biochim. Biophys. Acta 1262:1-14, 1995; see, also, Berger, supra, 1998). Viral type IB topoisomerases are exemplified by those produced by the vertebrate poxviruses (vaccinia, Shope fibroma virus, ORF virus, fowlpox virus, and molluscum contagiosum virus), and the insect poxvirus (*Amsacta moorei* entomopoxvirus) (see Shuman, Biochim. Biophys. Acta 1400:321-337, 1998; Petersen et al., Virology 230:197-206, 1997; Shuman and Prescott, Proc. Natl. Acad. Sci., USA 84:7478-7482, 1987; Shuman, J. Biol. Chem. 269:32678-32684, 1994; U.S. Pat. No. 5,766,891; WO 9619497 (PCT/US95116099); WO 9856943 (PCT/US98/12372); see, also, Cheng et al., *supra*, 1998).

Type II topoisomerases include, for example, bacterial gyrase, bacterial DNA topoisomerase IV, eukaryotic DNA topoisomerase II, and T-even phage encoded DNA topoisomerases (Roca and Wang, Cell 71:833-840, 1992; Wang, J. Biol. Chem. 266:6659-6662, 1991; Berger, supra, 1998;). Like the type IB topoisomerases, the type II topoisomerases have both cleaving and ligating activities. In addition, like type IB topoisomerase, substrate nucleic acid molecules can be prepared such that the type II topoisomerase can form a covalent linkage to one strand at a cleavage site. For example, calf thymus type II topoisomerase can cleave a substrate nucleic acid molecule containing a 5'recessed topoisomerase recognition site positioned three nucleotides from the 5' end, resulting in dissociation of the three nucleotide sequence 5' to the cleavage site and covalent binding the of the topoisomerase to the 5' terminus of the nucleic acid molecule (Andersen et al., *supra,* 1991). Furthermore, upon contacting such a type II topoisomerase charged nucleic acid molecule with a second nucleotide sequence containing a 3' hydroxyl group, the type II topoisomerase can ligate the sequences together, and then is released from the recombinant nucleic acid molecule. As such, type II topoisomerases also are useful for performing methods described herein.

Structural analysis of topoisomerases indicates that the members of each particular topoisomerase families, including type IA, type IB and type II topoisomerases, share common structural features with other members of the family (Berger, *supra,* 1998). In addition, sequence analysis of various type IB topoisomerases indicates that the structures are highly conserved, particularly in the catalytic domain (Shuman, *supra,* 1998; Cheng et al., *supra*, 1998; Petersen et al., *supra,* 1997). For example, a domain comprising amino acids 81 to 314 of the 314 amino acid vaccinia topoisomerase shares substantial homology with other type IB topoisomerases, and the isolated domain has essentially the same activity as the full length topoisomerase, although the isolated domain has a slower turnover rate and lower binding affinity to the recognition site (see Shuman, *supra*, 1998; Cheng et al., *supra*, 1998). In addition, a mutant vaccinia topoisomerase, which is mutated in the amino terminal domain (at amino acid residues 70 and 72) displays identical properties as the full length topoisomerase (Cheng et al., *supra*, 1998). In fact, mutation analysis of vaccinia type IB topoisomerase reveals a large number of amino acid residues that can be mutated without affecting the activity of the topoisomerase, and has identified several amino acids that are required for activity (Shuman, *supra,* 1998). In view of the high homology shared among the vaccinia topoisomerase catalytic domain and the other type IB topoisomerases, and the detailed mutation analysis of vaccinia topoisomerase, it will be recognized that isolated catalytic domains of the type IB topoisomerases and type IB topoisomerases having various amino acid mutations can be used in the methods described herein.

The various topoisomerases exhibit a range of sequence specificity For example, type II topoisomerases can bind to a variety of sequences, but cleave at a highly specific recognition site (see Andersen et al., J. Biol. Chem. 266:9203-9210, 1991). In comparison, the type IB topoisomerases include site specific topoisomerases, which bind to and cleave a specific nucleotide sequence ("topoisomerase recognition site"). Upon cleavage of a nucleic acid molecule by a topoisomerase, for example, a type IB topoisomerase, the energy of the phosphodiester bond is conserved via the formation of a phosphotyrosyl linkage between a specific tyrosine residue in the topoisomerase and the 3' nucleotide of the topoisomerase recognition site. Where the topoisomerase cleavage site is near the 3' terminus of the nucleic acid molecule, the downstream sequence (3' to the cleavage site) can dissociate, leaving a nucleic acid molecule having the topoisomerase covalently bound to the newly generated 3' end (see Figure 29).

A method is described herein, for generating a ds recombinant nucleic acid molecule covalently linked in one strand, can be performed by contacting 1) a first nucleic acid molecule having a first end and a second end, wherein the first nucleic acid molecule has a site-specific topoisomerase recognition site (e.g., a type IA or a type II topoisomerase recognition site) at or near the 5' terminus of the first end or the second end or both and, optionally, comprising one or more recombination sites; 2) at least a second nucleic acid molecule that has, or can be made to have, a first end and a second end; and 3) at least one (e.g., 1, 2, 3, 4, 5, 6, 7, 8, 9, 10, etc.) site-specific topoisomerase (e.g., a type IA or a type IB topoisomerase recognition site), under conditions such that all components are in contact and the at least one topoisomerase can effect its activity. For example, the topoisomerase can be a type IA topoisomerase such as *E. coli* topoisomerase I, *E. coli* topoisomerase III, or a eukaryotic topoisomerase III. Upon cleavage of a nucleic acid molecule, the topoisomerase preferably is stably bound to the 5' terminus. Upon cleavage by the topoisomerase, the cleaved nucleic acid molecule often may comprise a 3' overhanging sequence. Once nucleic acid molecules are joined by the methods described above, the resulting molecules may then be used in recombination reactions, such as those described elsewhere herein.

A method, is described herein for generating a ds recombinant nucleic acid molecule covalently linked in one strand can be performed such that any combination of ends are linked, and wherein one strand at the ends being linked is covalently linked and the other strand is not covalently linked, but contains a nick. For example, the first nucleic acid molecule can comprise a coding sequence, wherein the ATG start codon is at or near the first end and a poly A signal is encoded at or near the second end; and a second nucleic acid molecule can comprise a promoter element, which functions when positioned upstream of a coding sequence, and the first end is upstream of the second end, the method can be performed wherein a site-specific topoisomerase recognition site (e.g., a type IA or a type II topoisomerase recognition site) is at or near the 5' terminus of the first end of the first nucleic acid molecule, and wherein the contacting is performed under conditions such that the topoisomerase (e.g., a type IA or a type II topoisomerase) can covalently link the 5' terminus of the first end of the first nucleic acid molecule to the 3' terminus of the first end of the second nucleic acid molecule, thereby generating a ds recombinant nucleic acid molecule, in which a polypeptide can be expressed from the coding sequence. Alternatively, the method can be performed wherein the topoisomerase recognition site (e.g., a type IA or a type II topoisomerase recognition site) is at or near the 5' terminus of the second end of the first nucleic acid molecule, and wherein the contacting is performed under conditions such that the topoisomerase (e.g., a type IA or a type II topoisomerase recognition site) can covalently link the 5' terminus of the second end of the first nucleic acid molecule to the 3' terminus of the first end of the second nucleic acid molecule, thereby generating a ds recombinant nucleic acid molecule from which an antisense molecule can be expressed. Once nucleic acid molecules are joined by the methods described above, the resulting molecules may then be used in recombination reactions, such as those described elsewhere herein.

As another example using the first nucleic acid molecule and second nucleic acid molecule described above, the method can be performed, wherein the topoisomerase recognition site (e.g., a type IA or a type II topoisomerase recognition site) is at or near the 5' terminus of each of the first end and the second end of the first nucleic acid molecule, and wherein the contacting is performed under conditions such that the type IA topoisomerase can covalently link the 5' terminus of the first end of the first nucleic acid molecule to the 3' terminus of the first end of the second nucleic acid molecule, and the 5' terminus of the second end of the first nucleic acid molecule to the 3' terminus of the second end of the second nucleic acid molecule. As such, the ds recombinant nucleic acid molecule generated by the method is circularized, and includes a nick in each strand opposite the location where a strand was covalently linked by a topoisomerase (e.g., a type IA or a type II topoisomerase). Furthermore, the promoter of the second nucleic acid molecule can initiate expression of the first nucleic acid molecule. In one embodiment, the circularized ds recombinant nucleic acid molecule comprises a vector. Once nucleic acid molecules are joined by the methods described above, the resulting molecules may then be used in recombination reactions, such as those described elsewhere herein.

As another example using the first nucleic acid molecule and second nucleic acid molecule described above, the method can be performed, wherein the topoisomerase recognition site (e.g., a type IA or a type II topoisomerase recognition site) is at or near the 5' terminus of each of the first end and the second end of the first nucleic acid molecule, and wherein the contacting is performed under conditions such that the topoisomerase (e.g., a type IA or a type II topoisomerase) can covalently link the 5' terminus of the first end of the first nucleic acid molecule to the 3' terminus of the second -end of the second nucleic acid molecule, and the 5' terminus of the second end of the first nucleic acid molecule to the 3' terminus of the first end of the second nucleic acid molecule. As such, the ds recombinant nucleic acid molecule generated by the method is circularized, and includes a nick in each strand opposite the location where a strand was covalently linked by topoisomerase (e.g., a type IA or a type II topoisomerase recognition site). Furthermore, the promoter of the second nucleic acid molecule can initiate expression of an antisense sequence. In one embodiment, the circularized ds recombinant nucleic acid molecule comprises a vector. Once nucleic acid molecules are joined by the methods described above, the resulting molecules may then be used in recombination reactions, such as those described elsewhere herein.

As disclosed herein, a method of generating a ds recombinant nucleic acid molecule covalently linked in one strand, involving a first nucleic acid molecule and at least a second nucleic acid molecule, can further include a step for amplifying the ds Recombinant nucleic acid molecule covalently linked in one strand. The amplification reaction can be carried out by contacting the ds recombinant nucleic acid molecule with an amplification reaction primer pair, wherein a first primer of the pair is capable of binding to the covalently linked strand, at or near one end of the first or second nucleic acid molecule, and priming an amplification reaction toward the other nucleic acid molecule to generate a first extension product that is identical in nucleotide sequence to the nicked strand of the ds recombinant nucleic acid molecule; and the second primer of the pair is capable of binding to the first extension product, typically at or near the 3' terminus, and, in the presence of the first primer, can generate an amplification product using the covalently linked strand and the extension product (or extension products generated therefrom) as templates. For example, the method can be performed such that the type IA topoisomerase recognition site is at or near a first end of the first nucleic acid molecule, and the method further includes contacting the ds recombinant nucleic acid molecule with an amplification reaction primer pair, wherein a forward primer is capable of binding at or near the second-end of the first nucleic acid molecule, and wherein a reverse primer is capable of binding to a nucleotide sequence complementary to at least a portion of the second end of the second nucleic acid molecule; and amplifying the ds recombinant nucleic acid molecule. The first nucleic acid molecule can include a coding region and the second nucleic acid molecule can include a regulatory element. Once nucleic acid molecules are joined by the methods described above, the resulting molecules may then be used in recombination reactions, such as those described elsewhere herein.

A method of generating a ds recombinant nucleic acid molecule covalently linked in one strand also can be performed by contacting 1) a first nucleic acid molecule having a first end and a second end, wherein the first nucleic acid molecule has a site-specific topoisomerase recognition site (e.g., a type IA or a type II topoisomerase recognition site) at or near the 5' terminus of the first end or the second end or both; 2) at least a second nucleic acid molecule that has, or can be made to have, a first end and a second end; 3) at least a third nucleic acid molecule which has, or can be made to have, a first end and a second end, each end further comprising a 5' terminus and a 3' terminus; and 4) at least one (e.g., 1, 2, 3, 4, 5, 6, 7, 8, 9, 10, etc.) site-specific topoisomerase (e.g., a type IA or a type II topoisomerase recognition site), under conditions such that all components are in contact and the at least one topoisomerase can effect its activity. For example, the topoisomerase can be a type IA topoisomerase such as *E. coli* topoisomerase I, *E. coli* topoisomerase III, or a eukaryotic topoisomerase III. Upon cleavage of a nucleic acid molecule, the topoisomerase preferably is stably bound to the 5' terminus. Preferably, upon cleavage by the topoisomerase, the cleaved nucleic acid molecule comprises a 3' overhanging sequence. Once nucleic acid molecules are joined by the methods described above, the resulting molecules may then be used in recombination reactions, such as those described elsewhere herein.

A method is described herein for generating a ds recombinant nucleic acid molecule covalently linked in one strand, involving a first nucleic acid molecule that contains a site-specific topoisomerase recognition site (e.g., a type IA or a type IB topoisomerase recognition site), or cleavage product thereof, at least a second nucleic acid molecule, and at least a third nucleic acid molecule can be performed such that any combination of ends are linked, and one strand at the ends being linked is covalently linked and one strand is nicked. According to this embodiment, any of the ends can contain a type IA, type II, or type IB topoisomerase recognition site, or can comprise a cleavage product thereof, provided that the first ds recombinant nucleotide molecule contains a topoisomerase recognition site (e.g., a type IA or a type II topoisomerase recognition site) at or near a 5' terminus, or a cleavage product thereof, and only one topoisomerase or topoisomerase recognition site is present at the ends that are to be linked. For example, where the first nucleic acid molecule comprises a site-specific type IA topoisomerase recognition site at or near each of the first end and the second end, the method further can include contacting the first nucleic acid molecule and the second nucleic acid molecule with at least a third nucleic acid molecule which has, or can be made to have, a first end and a second end, each end further comprising a 5' terminus and a 3' terminus, under conditions such that the topoisomerase (e.g., a type IA or a type II topoisomerase) can covalently link the 5' terminus of the first end of the first nucleic acid molecule with the 3' terminus of the first end of the second nucleotide sequence, and the 5' terminus of the second end of the first nucleic acid molecule with the 3' terminus of the first end of the third nucleotide sequence. It will be recognized that other combinations of ends and topoisomerase recognition sites, or cleavage products thereof, can be used to perform such a method as described herein. Once nucleic acid molecules are joined by the methods described above, the resulting molecules may then be used in recombination reactions, such as those described elsewhere herein.

A method is described herein that also can be performed by contacting a first nucleic acid molecule and a second nucleic acid molecule with at least a third nucleic acid molecule, which comprises a first end and a second end, each end further comprising a 5' terminus and a 3' terminus, wherein the third nucleic acid molecule comprises a type IB topoisomerase recognition site at or near the 3' terminus of said first end, or said second end, or both said first end and said second end; and at least one (e.g., 1, 2, 3, 4, 5, 6, 7, 8, 9, 10, etc.) type IB topoisomerase under conditions such that the type IB topoisomerase can covalently link the 3' terminus of the first end or second end of the third nucleic acid molecule to the 5' terminus of the first end or second end of the second nucleic acid molecule. In such a method, where the third nucleic acid molecule comprises a type IB topoisomerase recognition site at or near the 3' terminus of the first end, the contacting can be performed under conditions such that the type IB topoisomerase can covalently link the 3' terminus of the first end of the third nucleic acid molecule to the 5' terminus of the first end of the second nucleic acid molecule. It will be recognized that other combinations of ends and topoisomerase recognition sites, or cleavage products thereof, can be used to perform such a method as described herein. Once nucleic acid molecules are joined by the methods described above, the resulting molecules may then be used in recombination reactions, such as those described elsewhere herein.

In another embodiment, a method for generating a ds recombinant nucleic acid molecule covalently linked in one strand can be performed by contacting 1) a first nucleic acid molecule having a first end and a second end, wherein the first nucleic acid molecule has a site-specific topoisomerase recognition site (e.g., a type IA or a type II topoisomerase recognition site) at or near the 5' terminus of an end and a type IB topoisomerase recognition site at or near the 3' terminus of the other end; 2) at least a second nucleic acid molecule that has, or can be made to have, a first end and a second end; 3) at least one (e.g., 1, 2, 3, 4, 5, 6, 7, 8, 9, 10, etc.) site-specific topoisomerase (e.g., a type IA or a type II topoisomerase); and 4) at least one (e.g., 1, 2, 3, 4, 5, 6, 7, 8, 9, 10, etc.) type IB topoisomerase under conditions such that all components are in contact and the at least one topoisomerase can effect its activity. For example, the topoisomerase, for which a recognition site is at or near the 5' terminus, can be a type IA topoisomerase such as *E. coli* topoisomerase I, *E. coli* topoisomerase III, or a eukaryotic topoisomerase III. Upon cleavage of a nucleic acid molecule, the type IA topoisomerase preferably is stably bound to the 5' terminus, and the type IB topoisomerase preferably is stably bound at the 3' terminus. Preferably, upon cleavage by the topoisomerases, the cleaved nucleic acid molecule comprises a 3' overhanging sequence and a 5' overhanging sequence. The method can further include contacting the ds recombinant nucleic acid molecule with a DNA ligase, thereby generating a ds recombinant nucleic acid molecule covalently linked in both strands. Once nucleic acid molecules are joined by the methods described above, the resulting molecules may then be used in recombination reactions, such as those described elsewhere herein.

A method of generating a ds recombinant nucleic acid molecule covalently linked in one strand by contacting a first nucleic acid molecule, a second nucleic acid molecule, and at least a third nucleic acid molecule, can further include a step for amplifying the ds recombinant nucleic acid molecule, particularly the covalently linked strand. The amplification can be carried out by contacting the ds recombinant nucleic acid molecule with an amplification reaction primer pair, wherein a first primer of the pair can bind selectively to the covalently linked strand at or near one end of the first or second nucleic acid molecule and prime an amplification reaction toward the other nucleic acid molecule to generate a first extension product that is complementary to the covalently-linked strand; and the second primer of the pair can bind selectively to the first extension product, typically at or near the 3' terminus, and, in the presence of the first primer, can generate an amplification product using the covalently linked strand and the extension product (or extension products derived therefrom) as templates. The method can be performed such that the topoisomerase recognition site (e.g., a type IA or a type IB topoisomerase recognition site) is at or near the first end of the first nucleic acid molecule, and can further include contacting the ds recombinant nucleic acid molecule with an amplification reaction primer pair, wherein a forward primer is capable of binding to a nucleotide sequence at or near the second end of the first nucleic acid molecule and wherein a reverse primer is capable of binding to a nucleotide sequence complementary to at least a portion of the third nucleic acid molecule; and amplifying the ds recombinant nucleic acid molecule. The first nucleic acid molecule can include a coding region and the third nucleic acid molecule can include a regulatory element Furthermore, the ends being linked can contain complementary overhanging sequences. Once nucleic acid molecules are joined by the methods described above, the resulting molecules may then be used in recombination reactions, such as those described elsewhere herein.

Representative embodiments of the disclosed methods for generating a ds recombinant nucleic acid molecule covalently linked in one strand and, optionally, comprising one or more recombination sites, are illustrated in Figures 11A-11F. In Figure 11A, one of the nucleic acid molecules has a topoisomerase attached to the 5' terminus of one end such that, when this molecule, which has a 3' overhang, is contacted with a second nucleic acid molecule having a substantially complementary 3' overhang, under suitable conditions, the nucleotides comprising the 3' overhangs can hybridize and the topoisomerases can catalyze ligation. Figure 11B shows a first nucleic acid molecule having topoisomerase molecules linked to the 5' terminus and 3' terminus of two different ends of one nucleotide sequence, and further shows linkage of the first nucleic acid molecule to two other nucleotide sequences to generate a nucleic acid molecule which has one strand without any nicks and another strand with two nicks. Figure 11C shows a first nucleic acid molecule having a topoisomerase molecule linked to the 5' terminus of one end and a second nucleic acid molecule having a topoisomerase molecule linked to the 5' terminus of one end, and further shows linkage of the first and second nucleic acid molecule to one other nucleotide sequence to generate a nucleic acid molecule which has one strand without any nicks and another strand with two nicks. In Figure 11D, one of the nucleic acid molecules to be linked has site-specific type IA topoisomerases attached to the 5' terminus of both ends such that, when the nucleotide sequences are contacted the complementary 3' overhangs can hybridize and the topoisomerases catalyze ligation. Figure 11E shows another example of linking three nucleic acid molecules together, using one nucleic acid molecule that is topoisomerase-charged with a type IA topoisomerase at a 5' terminus and another nucleic acid molecule that is topoisomerase-charged with a type IB topoisomerase at a 3' terminus of the opposite strand to be linked, such that when the nucleotide sequences are contacted the complementary 3' overhangs can hybridize and the topoisomerases catalyze ligation. Figure 11F illustrates another example of linking three nucleic acid molecules together, in this case using one nucleic acid molecule that is topoisomerase-charged with a topoisomerase (e.g., a type IA or a type II topoisomerase) at a 5' terminus and with a type IB topoisomerase at a 3' terminus of the opposite strand, such that when the nucleotide sequences are contacted under suitable conditions, the complementary 3' overhangs can hybridize and the topoisomerases catalyze ligation. Once nucleic acid molecules are joined by the methods described above, the resulting molecules may then be used in recombination reactions, such as those described elsewhere herein.

The examples set forth in Figures 11A-11F show the ends of the nucleic acid molecules opposite those being linked as having blunt ends, and shows the being linked as having 3' overhanging sequences. However, the substrate nucleic acid molecules can have any ends and overhangs as desired, including both ends being blunt and/or complementary, or combinations thereof, such that the ends can be ligated to each other, for example, to form circular molecules or to other nucleic acid molecules having an appropriate end. Thus, one or more of the blunt ends as shown in Figures 11A-11F can be substituted with a nucleotide sequence comprising a 5' overhang or a 3' overhang, either of which can constitute a single nucleotide such as a thymidine residue or multiple nucleotides (e.g., two, three, four, five, six, seven, eight, nine, ten, eleven, twelve, thirteen, fourteen, fifteen, etc. nucleotides), which can be the same or different. In certain embodiments of the disclosed methods, a first nucleic acid molecule contains a blunt end to be linked, and a second nucleic acid molecule contains an overhang at the end which is to be linked by a site-specific topoisomerase (e.g., a type IA or a type IB topoisomerase), wherein the overhang includes a sequence complementary to that comprising the blunt end, thereby facilitating strand invasion as a means to properly position the ends for the linking reaction.

As exemplified in Figures 11A-11C, the ds recombinant nucleic acid molecule generated using the methods described herein include those in which one strand (not both strands) is covalently linked at the ends to be linked (i.e. ds recombinant nucleic acid molecules generated using these methods contain a nick at each position where two ends were joined). These embodiments are particularly advantageous in that a polymerase can be used to replicate the ds recombinant nucleic acid molecule by initially replicating the covalently linked strand. For example, a thermostable polymerase such as a polymerase useful for performing an amplification reaction such as PCR can be used to replicate the covalently strand, whereas the strand containing the nick does not provide a suitable template for replication.

Described herein are methods of covalently ligating the ends of two different nucleic acid molecules or two ends of the same nucleic acid molecule, such that the product generated is ligated in both strands and, therefore, does not contain a nick. Representative embodiments of this aspect of the invention are illustrated in Figure 12. For example, in Figure 12A, one of the nucleic acid molecules has topoisomerase molecules attached to the 3' terminus and the 5' terminus of one end such that, when this molecule, which has a 5' overhang, is contacted with a second nucleic acid molecule having a substantially complementary 5' overhang, under suitable conditions, the nucleotides comprising the 5' overhangs can hybridize and the topoisomerases can catalyze ligation of both strands of the nucleic acid molecules. In Figure 12B, each end of the nucleic acid molecules to be linked has a topoisomerase molecule attached to the 3' terminus such that, when the nucleotide sequences are contacted under suitable conditions, nucleotides comprising the 5' overhangs can hybridize and the topoisomerases catalyze ligation (compare Figure 12C, in which each of the nucleic acid molecules to be linked has a topoisomerase attached to the 5'termini of the ends to be linked). Figure 12D illustrates linking three nucleic acid molecules together via a nucleic acid molecule that is topoisomerase-charged at both termini of both ends. Similarly to Figure 11, the examples set forth in Figures 12A-12D show the ends of the nucleic acid molecules that are not being linked as having blunt ends. As discussed with respect to Figure 11, however, the substrate nucleic acid molecules utilized in methods as exemplified in Figure 12 can have any ends as desired, including topoisomerase-charged ends, such that the ends can be ligated to each other, for example, to form circular molecules or to other nucleic acid molecules having an appropriate end, blunt ends, 5' overhangs, 3' overhangs, and the like, as desired. Once nucleic acid molecules are joined by the methods described above, the resulting molecules may then be used in recombination reactions, such as those described elsewhere herein.

A covalently bound topoisomerase, in addition to catalyzing a ligation reaction, also can catalyze the reverse reaction, for example, religation of the 3' nucleotide of the recognition sequence, to which the type IB topoisomerase is linked through the phosphotyrosyl bond, and the nucleotide sequence that, prior to cleavage, comprised the 5' terminus of the nucleic acid molecule, and which, following cleavage, contains a free 5' hydroxy group. As such, methods have been developed for using a type IB topoisomerase to produce recombinant nucleic acid molecules. For example, cloning vectors containing a bound type IB topoisomerase have been developed and are commercially available (Invitrogen Corp., La Jolla CA). Such cloning vectors, when linearized, contain a covalently bound type IB topoisomerase at each 3' end ("topoisomerase charged"). Nucleotide sequences such as those comprising a cDNA library, or restriction fragments, or sheared genomic DNA sequences that are to be cloned into such a vector are treated, for example, with a phosphatase to produce 5' hydroxyl termini, then are added to the linearized topoisomerase-charged vector under conditions that allow the topoisomerase to ligate the nucleotide sequences at the 5' terminus containing the hydroxyl group and the 3' terminus of the vector that contains the covalently bound topoisomerase. A nucleotide sequence such as a PCR amplification product, which is generated containing 5' hydroxyl ends, can be cloned into a topoisomerase-charged vector in a rapid joining reaction (approximately 5 minutes at room temperature). The rapid joining and broad temperature range inherent to the topoisomerase joining reaction makes the use of topoisomerase-charged vectors ideal for high throughput applications, which generally are performed using automated systems.

Type II topoisomerases have not generally been used for generating recombinant nucleic acid molecules or cloning procedures, whereas type IB topoisomerases, as indicated above, are used in a variety of procedures. As disclosed herein, type IA topoisomerases can be used in a variety of procedures similar to those described for the type IB topoisomerases. However, previously described methods of using type IB topoisomerases to ligate two or more nucleotide sequences have suffered from the disadvantage that the bound topoisomerase only effects the joining of the 3' end of the strand to which it is attached and a second strand containing a 5' hydroxyl group. Since the topoisomerase cannot ligate the complementary strands, the nucleic acid molecules that are generated contain nicks. While the presence of such nicks does not prevent the use of the recombinant molecules for transfection of a host cells, as the nicks generally are resolved intracellularly, the presence of such nicks in double stranded nucleic acid molecules significantly limits direct use of the recombinant molecules. For example, a strand of a nucleic acid molecule containing a nick cannot be amplified by PCR because the primer extension reaction terminates at the nick. Thus, nucleic acid constructs prepared using a topoisomerase according to previously described methods generally must be further treated, for example, with a DNA ligase, to obtain a ds recombinant nucleic acid molecule that is covalently linked in both strands and, therefore, useful for subsequent manipulations such as PCR.

Previously described methods for preparing nucleic acid constructs also generally required numerous steps, particularly where more than two nucleotide sequences are to be ligated, and even more so where the sequences must be ligated in a predetermined orientation. For example, the nucleotide sequences to be linked generally are ligated sequentially to produce intermediate constructs, each of which must be cloned, amplified in a host cell, isolated, and characterized. The constructs containing the correct sequences then must be isolated in a sufficient quantity and form such that the next nucleotide sequence can be ligated, and the process of cloning, amplifying, isolating and characterizing performed again to identify the proper construct. Clearly, as the number of different nucleotide sequences to be joined increases, so do the number of essentially repetitive procedures that must be performed, thus resulting in an expensive, laborious and lengthy process.

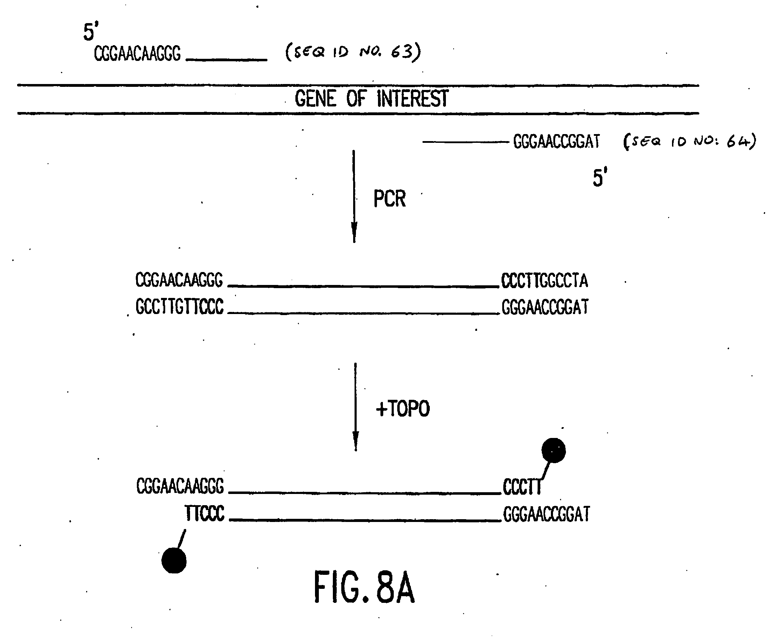

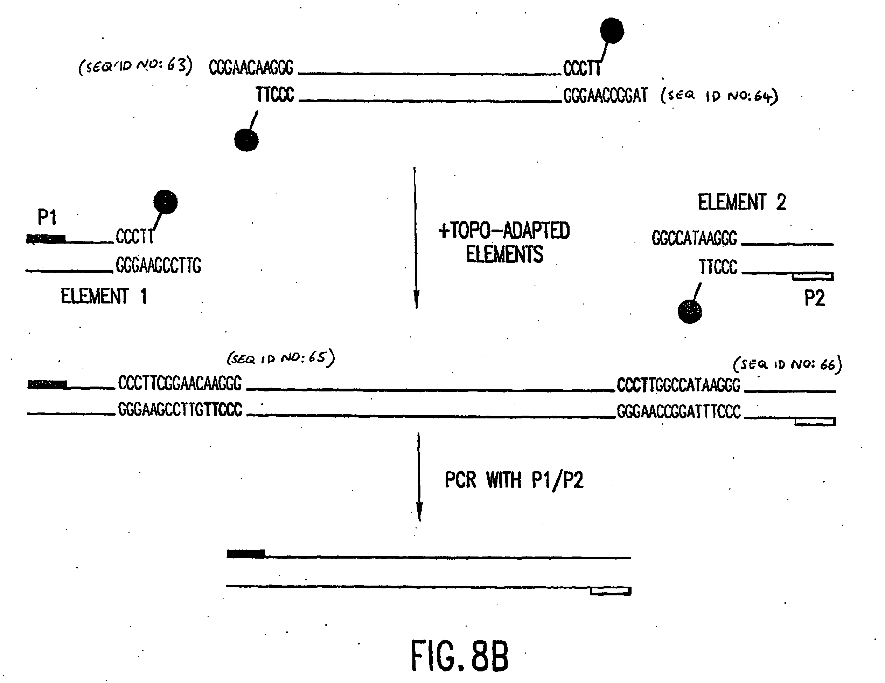

As disclosed herein, an advantage of a method described herein for generating a ds recombinant nucleic acid molecule covalently linked in both strands is that there is no need to perform a separate ligation reaction in order to obtain a functional ds recombinant nucleic acid molecule covalently linked in both strands (see Figures 8 and 12). In addition, a method as described herein can be performed such that, where a number of different nucleic acid molecules are to be covalently linked in a predetermined orientation, there is no requirement that intermediate constructs be cloned, characterized and isolated before proceeding to a subsequent step (see Example 1.B). As such, the methods described herein provide a means to generate a ds recombinant nucleic acid molecule covalently linked in both strands much more quickly and at a substantially lower cost than was possible using previously known methods.

As an additional advantage, the generated ds recombinant nucleic acid molecules covalently linked in both strands are in a form that can be used directly in further procedures, for example, particular procedures involving extension or a primer such as a PCR amplification procedure, or other transcription or translation procedure, because the generated construct does not contain nicks at the sites where the ds nucleotides sequences have been joined. As disclosed herein, a method for generating a ds recombinant nucleic acid molecule covalently linked in one strand, in certain embodiments, also is advantageous in that the generated ds recombinant nucleic acid molecules are in a form that can be used directly in further procedures, for example, particular procedures involving extension of a primer such as a PCR amplification procedure, or other transcription or translation procedure, because in certain embodiments, the generated ds recombinant nucleic acid molecule contains one strand that does not contain a nick at the sites where the ds nucleotides sequences were joined.

The term "nucleotide sequence" or "nucleic acid molecule" is used herein to refer to a discrete nucleic acid molecule. When used as such, the term "nucleotide sequence" is used merely for convenience such that the components in a composition or used in a method described herein can be clearly distinguished. Thus, reference is made, for example, to "nucleic acid molecules", which, in a method described herein, correspond to the reactants (substrates) used to produce a recombinant "nucleic acid molecule" product.

Certain methods described herein are exemplified generally herein with reference to the use of type IB topoisomerase such as the Vaccinia topoisomerase, or a type IA topoisomerase. However, it will be recognized that the methods also can be performed using a topoisomerase other than that exemplified, merely by adjusting the components accordingly. For example, as described in greater detail below, methods are disclosed for incorporating a type IB topoisomerase recognition site at one or both 3' termini of a linear nucleic acid molecule using a PCR primer comprising, at least in part, a nucleotide sequence complementary to the topoisomerase recognition site. In comparison, a topoisomerase recognition site for a type IA or, if desired, type II topoisomerase, can be incorporated into a nucleic acid molecule by using a PCR primer that contains the recognition site.

Cleavage of a nucleic acid molecule by a site specific type IB topoisomerase results in the generation of a 5' overhanging sequence in the strand complementary to and at the same end as that containing the covalently bound topoisomerase. Furthermore, as disclosed herein, PCR primers can be designed that can incorporate a type IB topoisomerase recognition site into a nucleic acid molecule, and that further can produce, upon cleavage of the nucleic acid molecule by the topoisomerase, a 5' overhanging sequence in the complementary strand that has a defined and predetermined sequence. As such, the methods are readily adaptable to generating a ds recombinant nucleic acid molecule having the component nucleic acid molecule operatively linked in a predetermined orientation. In view of the present disclosure, it will be recognized that PCR primers also can be designed such that a type IA topoisomerase recognition site can be introduced into a nucleic acid molecule, including a library of diverse sequences, and, if desired, such that upon cleavage by a site-specific topoisomerase, generates a 3' overhanging sequence.

A method of generating a ds recombinant nucleic acid molecule covalently linked in both strands, as disclosed herein, extends the previously known methods by providing a topoisomerase at or near the terminus of each nucleic acid molecule to be covalently linked. For example, with respect to a type IB topoisomerase, the method provides a topoisomerase recognition site, or a cleavage product thereof (i.e., a covalently bound type IB topoisomerase), at or near the 3' terminus of each linear nucleic acid molecule to be linked. As used herein, the term "topoisomerase recognition site" means a defined nucleotide sequence that is recognized and bound by a site specific topoisomerase. For example, the nucleotide sequence 5'-(C/T)CCTT-3' is a topoisomerase recognition site that is bound specifically by most poxvirus topoisomerases, including vaccinia virus DNA topoisomerase I, which then can cleave the strand after the 3'-most thymidine of the recognition site to produce a nucleotide sequence comprising 5'-(C/T)CCTT-PO₄-TOPO, i.e., a complex of the topoisomerase covalently bound to the 3' phosphate through a tyrosine residue in the topoisomerase (see Shuman, J. Biol. Chem. 266:11372-11379, 1991; Sekiguchi and Shuman, Nucl. Acids Res. 22:5360-5365, 1994; see, also, U.S. Pat. No. 5,766,891; WO 96191497 (PCT/US95/16099); WO 9856943 (PCT/US98/12372)). In comparison, the nucleotide sequence 5'-GCAACTT-3' is the topoisomerase recognition site for type IA *E. coli* topoisomerase III.

Topoisomerase-charged nucleic acid molecules, including those containing a topoisomerase covalently attached to a 5' terminus or 3' terminus or both, of one or both ends of the nucleic acid molecule, can be generated by any of a number of methods. In some cases and under the appropriate conditions, type I topoisomerases can cleave a single stranded nucleotide sequence. For example, a domain comprising the amino-terminal 67 kDa domain of *E. coli* topoisomerase I, which is a type IA topoisomerase, can cleave a single stranded nucleotide sequence containing the topoisomerase recognition site. Where conditions are such that the topoisomerases can cleave a single stranded nucleotide sequence, cleavage of a nucleic acid molecule containing topoisomerase recognition sites at the 5' and 3' termini of one end of nucleic acid molecule can be performed in parallel. Alternatively, where one or both of the topoisomerases requires a nucleic acid molecule for recognition and cleavage, the reactions are performed serially, wherein the more terminal (distal) of the topoisomerase recognition sites is cleaved first, then the more internal (proximal) site, which remains in a double stranded context, is cleaved. For example, a nucleic acid molecule containing an *E. coli* topoisomerase III recognition site at or near a 5' terminus of an end and *a Vaccinia* type IB topoisomerase recognition site at or near the 3' terminus of the same end, and wherein the type IB recognition site is closer to the end than the type IA recognition site, the nucleic acid molecule can be incubated with the *Vaccinia* topoisomerase, to produce a type IB topoisomerase charged nucleic acid molecule, then with the *E. coli* topoisomerase, to produce a nucleic acid molecule having the type IA topoisomerase bound to the 5'terminus and the type IB topoisomerase bound to the 3'terminus. Described herein are methods for producing nucleic acid molecule comprising a topoisomerase attached to one or both termini of at least one end, and further provides such topoisomerase-charged nucleic acid molecules.

As used herein, the term "cleavage product," when used in reference to a topoisomerase recognition site, refers to a nucleotide sequence that has been cleaved by a topoisomerase, generally at its recognition site, and comprises a complex of the topoisomerase covalently bound, in the case of type IA or type II topoisomerase, to the 5' phosphate group of the 5' terminal nucleotide in the topoisomerase recognition site, or in the case of a type IB topoisomerase to the 3' phosphate group of the 3'terminal nucleotide in the topoisomerase recognition site. Such a complex, which comprises a topoisomerase cleaved nucleic acid molecule having the topoisomerase covalently bound thereto, is referred to herein as a "topoisomerase-activated" or a "topoisomerase-charged" nucleotide sequence. Topoisomerase-activated nucleic acid molecules can be used in a method of the invention, as can nucleic acid molecules that contain an uncleaved topoisomerase recognition site and a topoisomerase, wherein the topoisomerase can cleave the nucleic acid molecule at the recognition site and become covalently bound thereto.

In one embodiment of a method of generating a ds recombinant nucleic acid molecule covalently linked in both strands, a topoisomerase recognition site is present at or near the 3' terminus of the end of each nucleotide sequence to be linked such that, in the presence of a type IB topoisomerase, each nucleotide sequence is cleaved to produce a 3' terminus, which contains the topoisomerase covalently bound thereto (see Figure 8). The nucleotide sequences to be covalently linked also can contain a 5' hydroxy group at the same end as that containing the topoisomerase recognition site, or a 5' hydroxyl group can be generated using a phosphatase. Upon contact of such nucleotide sequences, the site specific topoisomerase can ligate each strand containing a 3' phosphate to a respective 5' hydroxyl group, thereby generating a ds recombinant nucleic acid molecule covalently linked in both strands, which can be produced as a linear, -circular, or positively or negatively supercoiled nucleic acid molecule.

The 5' termini of the ends of the nucleotide sequences to be linked by a type IB topoisomerase according to a method described herein may contain complementary 5' overhanging sequences, which can facilitate the initial association of the nucleotide sequences, including, if desired, in a predetermined directional orientation. Alternatively, the 5' termini of the ends of the nucleotide sequences to be linked by a type IB topoisomerase according to a method described herein contain complementary 5' sequences wherein one of the sequences contains a 5' overhanging sequence and the other nucleotide sequence contains a complementary sequence at a blunt end of a 5' terminus, to facilitate the initial association of the nucleotide sequences through strand invasion, including, if desired, in a predetermined directional orientation. The term "5' overhang" or "5' overhanging sequence" is used herein to refer to a strand of a nucleic acid molecule that extends in a 5' direction beyond the terminus of the complementary strand of the nucleic acid molecule. Conveniently, a 5' overhang can be produced as a result of site specific cleavage of a nucleic acid molecule by a type IB topoisomerase (see Example 1).

The 3' termini of the ends of the nucleotide sequences to be linked by a type IA topoisomerase according to a method described herein contain complementary 3' overhanging sequences, which can facilitate the initial association of the nucleotide sequences, including, if desired, in a predetermined directional orientation. Alternatively, the 3'termini of the ends of the nucleotide sequences to be linked by a topoisomerase (e.g., a type IA or a type II topoisomerase) according to a method described herein contain complementary 3' sequences wherein one of the sequences contains a 3' overhanging sequence and the other nucleotide sequence contains a complementary sequence at a blunt end of a 3' terminus, to facilitate the initial association of the nucleotide sequences through strand invasion, including, if desired, in a predetermined directional orientation. The term "3 overhang" or "3 overhanging sequence" is used herein to refer to a strand of a nucleic acid molecule that extends in a 5' direction beyond the terminus of the complementary strand of the nucleic acid molecule. Conveniently, a 3' overhang can be produced upon cleavage by a type IA or type II topoisomerase.

The 3' or 5' overhanging sequences can have any sequence, though generally the sequences are selected such that they allow ligation of a predetermined end of one nucleic acid molecule to a predetermined end of a second nucleotide sequence according to a method described herein (Figure 9C, see, also Example I.B). As such, while the 3' or 5' overhangs can be palindromic, they generally are not because nucleic acid molecules having palindromic overhangs can associate with each other, thus reducing the yield of a ds recombinant nucleic acid molecule covalently linked in both strands comprising two or more nucleic acid molecules in a predetermined orientation. For example, the 5' overhanging sequences of nucleic acid molecules shown in Figure 9A are palindrome and, therefore, the association, for example, of a first CMV element with a second CMV element through the AGCT overhang is just as likely as the association of a CMV element with a GFP element through the AGCT overhang. As such, the efficiency of generating a construct comprising an operatively covalently linked construct containing, in order from 5' to 3', a CMV element, a GFP element and a BGH element would be reduced as compared to the efficiency of generating such a construct using the elements as shown in Figure 9C. The elements shown in Figure 9B contain palindromic overhangs at one end of the GFP element and at the end of the BGH element shown and, therefore, would be less efficient than the elements of Figure 9C, but more efficient than those in Figure 9A, for generating the desired construct.

A nucleotide sequence used in the methods and kits described herein can be designed to contain a bridging phosphorothioate to prevent religation after topoisomerase-cleavage. For example, where the topoisomerase is *E. coli* topoisomerase III, the bridging phosphorothioate can be incorporated between the two thymidines of the GCAACTT cleavage/recognition sequence. When cleaved, the clipped sequence contains a 3'-SH instead of a 3'-OH, thus preventing religation (see Burgin, et al, Nucl. Acids Res. 23:2973-2979, 1995).

A nucleic acid molecule useful in a method or kit described herein can be amplified by an amplification method such as PCR to contain a topoisomerase recognition site at a 3' or 5' terminus of an end. Furthermore, one or both primers used for PCR can be designed such that, upon cleavage of an amplified nucleic acid molecule, the cleaved nucleic acid molecule contains a 5' or 3' overhang at one or both ends. In one embodiment, PCR primers are designed such that the 5' overhanging sequence on a first nucleic acid molecule is complementary to a 5' overhanging sequence on a second (or other) nucleic acid molecule, thereby facilitating the association of the nucleotide sequences, preferably in a predetermined orientation, whereupon they can be covalently linked according to a method described herein. By designing unique overhanging sequences for the different nucleic acid molecule to be linked, any number of nucleic acid molecules can be linked in a desired order and/or orientation.

It should be recognized that PCR is used in two ways with respect to the methods described herein. PCR primers may be designed to impart particular characteristics to a desired nucleic acid molecule, for example, a nucleic acid molecule that encodes a transcriptional or translational regulatory element or a coding sequence of interest such as an epitope tag or cell compartmentalization domain. The PCR primers may be designed such that, upon amplification, the nucleic acid molecule contains a topoisomerase recognition site at one or both ends, as desired. As disclosed herein, the PCR primer also can include an additional sequence such that, upon cleavage of the amplification product by a site specific topoisomerase, the cleaved nucleic acid molecule contains a 5' or 3' overhanging sequence at the topoisomerase cleaved end. By involving a topoisomerase that binds and cleaves a 5' terminus (e.g., by involving a type IA topoisomerase), the PCR primers can be designed to contain a bridging phosphorothioate linkage (see above), which can block religation after topoisomerase cleavage and can assist in the generation of a topoisomerase charged amplification product.

Overhanging sequences generated using PCR can include a single nucleotide overhang that is generated as an artifact of the PCR reaction. For example, a polymerase such at Taq, which does not have a proof-reading function and has an inherent terminal transferase activity, is commonly used, and produces PCR products containing a single, non-template derived 3' A overhang at each end. These amplification products can be linked to topoisomerase charged nucleic acid molecules containing a single 3'T overhang or a single 3' dU overhang, which, for a T/A cloning reaction, can be a vector (see U.S. Pat. Nos. 5,487,993 and 5,856,144), at one or both ends, using the methods described herein.

PCR also is used to amplify a covalently linked ds recombinant nucleic acid molecule covalently linked in one or both strands, generated by a method described herein. For example, as illustrated in Figure 13, a method described herein can generate an expressible ds recombinant nucleic acid molecule from three substrate nucleic acid molecules, including a nucleotide sequence comprising a promoter, a nucleotide sequence comprising a coding sequence, and a nucleotide sequence comprising a polyadenylation signal. The generation of the ds recombinant nucleic acid molecule can be facilitated by the incorporation of complementary 3' (or 5') overhanging sequences at the ends of the ds nucleotides sequences to be joined. For example, the expressible ds recombinant nucleic acid molecule can be generated by contacting a first nucleic acid molecule having a type IA topoisomerase at a 5' terminus of a first end and a type IB topoisomerase at a 3' terminus of a second end with a second nucleic acid molecule and a third double stranded nucleotide sequence. By designing a PCR primer pair containing a first primer that is specific for a portion of the nucleotide sequence comprising the promoter that is upstream from the promoter, and a second primer that is specific for a portion of the nucleotide sequence comprising the polyadenylation signal that is down stream of the signal, only a full length functional ds recombinant nucleic molecule containing the promoter, coding sequence and polyadenylation signal in the correct (predetermined) orientation will be amplified. In particular, partial reaction products, for example, containing only a promoter linked to the coding sequence, and reaction products containing nicks are not amplified. Thus, PCR can be used to specifically design a nucleic acid molecule such that it is useful in a method described herein, and to selectively amplify only those reaction products having the desired components and characteristics.

As used herein, the term "covalently linked," when used in reference to a ds recombinant nucleic acid molecule, means that the nucleic acid molecule is generated from at least two nucleic acid molecules that are ligated together, in both strands, by a topoisomerase mediated ligation. It should be recognized, for example, that a topoisomerase covalently bound to one of the nucleic acid molecules to be covalently linked can be the same as or different from the topoisomerase covalently bound to the other nucleic acid molecule. Thus, a *Vaccinia* topoisomerase can be covalently bound to one nucleic acid molecule and another poxvirus or eukaryotic nuclear type IB topoisomerase can be bound to the other strand. Generally, however, the topoisomerases, where different, are members of the same family, for example, type IA or type IB or type II, although, where the topoisomerases are covalently bound, for example, to a 5' phosphate and generate complementary 3' overhangs, the topoisomerase can be from different families, for example, type IA and type II.

The term "covalently linked" also is used herein in reference to a single stranded or double stranded nucleic acid molecule that is generated from at least two nucleotide sequences that are ligated together in one strand. For example, a ds recombinant nucleic acid molecule that is generated when a first topoisomerase-charged nucleic acid molecule that includes one topoisomerase bound at or near a 5' terminus contacts a second ds nucleotide sequence under conditions such that the topoisomerases can covalently link the 5' terminus of the first nucleic acid molecule to which it is bound, to the 3' terminus of the second nucleic acid molecule, can generate a ds recombinant nucleic acid molecule covalently linked in one strand.

In one embodiment, a ds recombinant nucleic acid molecule covalently linked in both strands generated according to a method described herein does not contain a nick in either strand at the site where two nucleotide sequences are ligated, although it can contain nicks elsewhere in the molecule. In a method for generating a ds recombinant nucleic acid molecule covalently linked in one strand, a ds recombinant nucleic acid molecule is generated that contains a nick at least at the position where ends were linked in the complementary strands. This nicked ds recombinant nucleic acid molecule can be converted to a ds recombinant nucleic acid molecule covalently linked in both strands by introducing the nicked ds recombinant nucleic acid molecule into a cell, or by subjecting the ds recombinant nucleic acid molecule to a ligation reaction, such as using a ligase, as is well known in the art.

The term "recombinant" is used herein to refer to a nucleic acid molecule that is produced by linking at least two nucleotide sequences according to a method described herein. As such, a ds recombinant nucleic acid molecule encompassed within the disclosure herein is distinguishable from a nucleic acid molecule that may be produced in nature, for example, during meiosis. For example, a ds recombinant nucleic acid molecule covalently linked in both strands generated according to a method described herein can be identified by the presence of the two topoisomerase recognition sites, one present in each of the complementary strands, at or near the site at which the nucleic acid molecules were joined.

A method described herein can be performed by contacting a first nucleic acid molecule having a first end and a second end, wherein at the first end or second end or both, the first nucleic acid molecule has a topoisomerase recognition site, or cleavage product thereof, at or near the 3' terminus and has (or can be made to have, for example, by contact with a phosphatase) a hydroxyl group at the 5' terminus of the same end; at least a second nucleic acid molecule having a first end and a second end, wherein at the first end or second end or both, the at least second nucleic acid molecule has a topoisomerase recognition site, or cleavage product thereof, at or near the 3' terminus and has (or can be made to have) a hydroxyl group at the 5' terminus of the same end; and a topoisomerase, under conditions such that the components are in contact and the topoisomerase can effect its activity. Upon contact of the topoisomerase with the first and second (or other) nucleic acid molecules, and cleavage, where necessary, each nucleotide sequence comprises at the cleavage site a covalently bound topoisomerase at the 3' terminus and has, or can have, a hydroxyl group at the 5' terminus such that, upon contact, the first and at least second nucleotide sequences are covalently linked in both strands. Accordingly, described herein is a ds recombinant nucleic acid molecule covalently linked in both strands produced by such a method.

As used herein, the term "at or near," when used in reference to the proximity of a topoisomerase recognition site to the 3' (type IB) or 5' (type IA or type II) terminus of a nucleotide sequence, means that the site is within about 1 to 100 nucleotides from the 3' terminus or 5' terminus, respectively, generally within about 1 to 20 nucleotides from the terminus, and particularly within about 2 to 12 nucleotides from the respective terminus. An advantage of positioning the topoisomerase recognition site within about 10 to 15 nucleotides of a terminus is that, upon cleavage by the topoisomerase, the portion of the sequence downstream of the cleavage site can spontaneously dissociate from the remaining nucleotide sequence, which contains the covalently bound topoisomerase (referred to generally as "suicide cleavage"; see, for example, Shuman, *supra*, 1991; Andersen et al., *supra,* 1991). Where a topoisomerase recognition site is greater than about 12 to 15 nucleotides from the terminus, the nucleotide sequence upstream or downstream of the cleavage site can be induced to dissociate from the remainder of the sequence by modifying the reaction conditions, for example, by providing an incubation step at a temperature above the melting temperature of the portion of the duplex including the topoisomerase cleavage site.

An additional advantage of constructing a first or second (or other) nucleic acid molecule to comprise, for example, a type IB topoisomerase recognition site about 2 to 15 nucleotides from one or both ends is that a 5' overhang is generated following cleavage of the nucleic acid molecule by a site specific topoisomerase. Such a 5' overhanging sequence, which would contain 2 to 15 nucleotides, respectively, can be designed using a PCR method as disclosed herein to have any sequence as desired. Thus, where a cleaved first nucleic acid molecule is to be covalently linked to a selected second (or other) nucleic acid molecule according to a method described herein, and where the selected sequence has a 5' overhanging sequence, the 5' overhang on the first nucleic acid molecule can be designed to be complementary to the 5' overhang on the selected second (or other) ds sequence such that the two (or more) sequences are covalently linked in a predetermined orientation due to the complementarity of the 5' overhangs. As discussed above, similar methods can be utilized with respect to 3' overhanging sequences generated upon cleavage by, for example, a type IA or type II topoisomerase.

As used herein, reference to a nucleotide sequence having "a first end" and "a second end" means that the nucleotide sequence is linear. A substrate nucleic acid molecule can be linear or circular, including supercoiled, although, as a result of cleavage by one or more topoisomerases, a linear topoisomerase-charged nucleic acid molecule generally is produced. For example, a circular nucleic acid molecule containing two type IB topoisomerase recognition sites within about 100 nucleotides of each other and in the complementary strands, preferably within about twenty nucleotides of each other and in the complementary strands, can be contacted with a site specific type IB topoisomerase such that each strand is cleaved and the intervening sequence dissociates, thereby generating a linear nucleic acid molecule having a topoisomerase covalently bound to each end.

It should be recognized that reference to a first end or a second end of a nucleic acid molecule is not intended to imply any particular orientation of the nucleotide sequence, and is not intended to imply a relative importance of the ends with respect to each other. Where a nucleotide sequence having a first end and second end is a double stranded nucleotide sequence, each end contains a 5' terminus and a 3' terminus. Thus, reference is made herein, for example, to a nucleotide sequence containing a topoisomerase recognition site at a 3' terminus and a hydroxyl group at the 5' terminus of the same end, which can be the first end or the second end.

A method described herein can be performed using only a first nucleic acid molecule and a second nucleic acid molecule, or can additionally include a third, fourth or more nucleic acid molecules as desired. Generally, each such nucleotide sequence contains a topoisomerase recognition site, or a cleavage product thereof, at or near at least one 3' or 5' terminus, and can contain a hydroxyl group at the 5' terminus of the same end, or a hydroxyl group can be generated using a phosphatase. Where a nucleotide sequence does not contain a topoisomerase recognition site at or near an end to be linked to a second nucleotide sequence, a topoisomerase recognition site can be introduced into the nucleotide sequence using a method as disclosed herein, for example, by PCR amplification of the sequence using a primer comprising a complement of the topoisomerase recognition site.

The terms "first nucleotide sequence," "second nucleotide sequence," "third nucleotide sequence," and the like, are used herein only to provide a means to indicate which of several nucleotide sequences is being referred to. Thus, absent any specifically defined characteristic with respect to a particular nucleotide sequence, the terms "first," "second," "third" and the like, when used in reference to a nucleotide sequence, or a population or plurality of nucleotide sequences, are not intended to indicate any particular order, importance or other information about the nucleotide sequence. Thus, where an exemplified method refers, for example, to using PCR to amplify a first nucleic acid molecule such that the amplification product contains a topoisomerase recognition site at one or both ends, it will be recognized that, similarly, a second (or other) nucleic acid molecule also can be so amplified.

The term "at least a second nucleotide sequence" is used herein to mean one or more nucleotide sequences in addition to a first nucleotide sequence. Thus, the term can refer to only a second nucleotide sequence, or to a second nucleotide sequence and a third nucleotide sequence (or more). As such, the term "second (or other) nucleotide sequence" or second (and other) nucleotide sequences" is used herein in recognition of the fact that the term "at least a second nucleotide sequence" can refer to a second, third or more nucleotide sequences. It should be recognized that, unless indicated otherwise, a nucleotide sequence encompassed within the meaning of the term "at least a second nucleotide sequence" can be the same or substantially the same as a first nucleotide sequence. For example, a first and second nucleic acid molecule can be the same except for having complementary 5' overhanging sequences produced upon cleavage by a topoisomerase such that the first and second nucleic acid molecules can be covalently linked using a method described herein. As such, a method described herein can be used to produce a concatenate of first and second nucleic acid molecules, which, optionally, can be interspersed, for example, by a third nucleic acid molecule such as a regulatory element, and can contain the covalently linked sequences in a predetermined directional orientation, for example, each in a 5' to 3' orientation with respect to each other.

As disclosed herein, a method described herein provides a means to covalently link, two or more ds nucleotides in a predetermined directional orientation. The term "directional orientation" or "predetermined directional orientation" or "predetermined orientation" is used herein to refer to the covalent linkage, of two or more nucleotide sequences in a particular order. Thus, a method described herein provides a means, for example, to covalently link, a promoter regulatory element upstream of a coding sequence, and to covalently link a polyadenylation signal downstream of the coding region to generate a functional expressible ds recombinant nucleic acid molecule; or to covalently link two coding sequences such that they can be transcribed and translated in frame to produce a fusion polypeptide.

A method described herein also can be performed by contacting a first nucleic acid molecule having a first end and a second end, wherein at the first end or second end or both, the first nucleic acid molecule has a type IB topoisomerase covalently bound at the 3' terminus (topoisomerase-charged) and has (or can be made to have) a hydroxyl group at the 5' terminus of the same end; and at least a second type IB topoisomerase-charged nucleic acid molecule, which has (or can be made to have) a hydroxyl group at the 5' terminus at the same end. Upon contact of the topoisomerase-activated first and at least second nucleotide sequences at the ends containing the topoisomerase and a 5' hydroxyl group, phosphodiester bonds are formed in each strand, thereby generating a ds recombinant nucleic acid molecule covalently linked in both strands.

Methods are described herein for linking two or more (e.g., two, three, four, five, six, seven, etc.) nucleotide sequences, wherein the linked ds recombinant nucleic acid molecule is covalently linked in one strand, but not both strands, (i.e. the ds recombinant nucleic acid molecule contains a nick in one strand at each position where two ends were joined to generate the ds recombinant nucleic acid molecule). Further, one or more of the nucleotide sequences may comprise one or more recombination sites. Using the schematic shown in Figure 11A for purposes of illustration, the disclosure herein includes methods for linking at least two nucleotide sequences comprising contacting a first nucleic acid molecule having a first end and a second end, wherein at the first end at the second end or at both ends, the first nucleic acid molecule has a site-specific type IA topoisomerase covalently bound to the 5' termini; and a second nucleic acid molecule which does not have topoisomerase covalently bound to either termini of at least one end. Further, the second nucleotide sequence will typically have hydroxyl groups at the 3' termini of the end being joined to the first nucleic acid molecule. In many instances, the two nucleotide sequences to be joined will have either 3' or 5'overhangs with sufficient sequence complementarity to allow for hybridization. In related embodiments, the first and second nucleic acid molecules described above may be first and second ends of the same nucleic acid molecule. Thus, connection of the two ends results in the formation of a circularized molecule. Once nucleic acid molecules are joined by the methods described above, the resulting molecules may then be used in recombination reactions, such as those described elsewhere herein. The disclosure herein further includes nucleic acid molecules prepared by methods described herein, compositions comprising such nucleic acid molecules, and methods for using such nucleic acid molecules.

Using the schematic shown in Figure 11B for purposes of illustration, the disclosure herein includes methods for joining three or more nucleotide sequences. While any number of variations of the disclosure herein are possible, three nucleotide sequences may be joined by the use of a linker molecule which contains topoisomerases at or near both the 5' and 3' termini of one end, and optionally one or more recombination site. Thus, upon joining of the three nucleotide sequences, a single nucleotide sequence is formed which contains a first strand with no nicks at the junction points, and a second strand with nicks at the junction points. This process has the advantage of employing a single topoisomerase modified molecule to join three nucleotide sequences together. Once nucleic acid molecules are joined by the methods described above, the resulting molecules may then be used in recombination reactions, such as those described elsewhere herein. The disclosure herein further includes nucleic acid molecules prepared by methods described herein, compositions comprising such nucleic acid molecules, and methods for using such nucleic acid molecules.

Methods for covalently linking both strands of two or more (e.g., two, three, four, five, six, seven, etc.) nucleic acid molecules are described herein. Using the schematic shown in Figure 12A for purposes of illustration, the invention includes methods for linking at least two nucleotide sequences comprising contacting a first nucleic acid molecule having a first end and a second end, wherein at the first end at the second end or at both ends, the first nucleic acid molecule has two topoisomerases (e.g., a type IA and a type IB topoisomerase) one each covalently bound to the 3'and 5'termini; and a second nucleic acid molecule which does not have topoisomerase covalently bound to either termini of at least one end. Further, the second nucleotide sequence will often have hydroxyl groups at the 5' and 3' termini of the end being joined to the first nucleic acid molecule. In many instances, the two nucleotide sequences to be joined will have either 3' or 5' overhangs with sufficient sequence complementarity to allow for hybridization, and, optionally, one or more recombination sites. The first and second nucleic acid molecules as described above can be first and second ends of the same nucleic acid molecule. Thus, connection of the two ends results in the formation of a circularized molecule. Once nucleic acid molecules are joined by the methods described above, the resulting molecules may then be used in recombination reactions, such as those described elsewhere herein. The disclosure herein further includes nucleic acid molecules prepared by methods described herein, compositions comprising such nucleic acid molecules, and methods for using such nucleic acid molecules.

Using the schematic shown in Figure 5D for purposes of illustration, the disclosure herein includes methods for joining three or more nucleotide sequences. While any number of variations of the disclosure herein are possible, three nucleotide sequences may be joined by the use of a linker molecule which contains topoisomerases at or near both the 5'and 3'termini of each end and, optionally, one or more recombination sites. Thus, upon joining of the three nucleotide sequences, a single nucleotide sequence is formed which contains no nicks at the junction points. This process has the advantage of employing a single topoisomerase modified molecule to join three nucleotide sequences together. Once nucleic acid molecules are joined by the methods described above, the resulting molecules may then be used in recombination reactions, such as those described elsewhere herein. The disclosure herein further includes nucleic acid molecules prepared by methods described herein, compositions comprising such nucleic acid molecules, and methods for using such nucleic acid molecules.

Methods for performing topoisomerase mediated joining reactions and recombination reactions which can be performed in either a single tube or multiple tubes are described herein. For instance, all of the components necessary to perform both topoisomerase mediated joining reactions and recombination reactions can be combined in one tube and both reactions can occur essentially simultaneously. Examples of topoisomerase/recombination reactions which can be performed in either a single tube or in multiple tubes are shown in Figures 35-40. Thus, described herein are single tube reactions in which (1) one or more nucleic acid molecules or two ends of one nucleic acid molecule are linked to each other by a topoisomerase mediated reaction and (2) one or more recombination sites undergo recombination with one or more other recombination sites. Any number of toposiomerase mediated joining reaction and/or recombination reactions may occur in processes described herein. Further, these reactions may occur in any order. One or more nucleic acid molecules in reaction mixtures described herein may contain (1) one or more recombination sites and (2) one or more topoisomerases or one or more topoisomerase recognition sites.

As explained below in Example 9, in certain instances, topoisomerases have been found to inhibit particular recombination reactions. In such instances, nucleic acid molecules which have undergone toposiomerase mediated joining reaction(s) may be separated from topoisomerases present in the reaction mixture and then may used as substrates for recombination reaction(s). Often in such instances, the topoisomerase mediated joining reaction(s) and the recombination reaction(s) will occur in separate tubes. Examples of process by which products of topoisomerase mediated joining reactions may be separated from topoisomerase include, but are not limited to, phenol/chloroform extraction, typically followed by precipitation of the nucleic acid (*e.g.*, ethanol precipitation), and chromatography (*e*.*g*., column chromatography).

Alternatively, topoisomerases present in the reaction mixture may be inactivated, for example, by heating (*e*.*g*., heating to about 65°C for about 60 min., about 70°C for about 60 min., about 75°C for about 60 min., about 70°C for about 40 min., about 75°C for about 40 min., about 80°C for about 40 min., about 80°C for about 30 min., about 85°C for about 20 min., about 90°C for about 15 min., about 95°C for about 5 min. or about 99°C for about 1 min.) or by the use of proteases (*e*.*g*., proteinase K). In this instance, it will generally be possible for the topoisomerase mediated joining reaction(s) and the recombination reaction(s) to occur in the same tube.

In specific embodiments of single tube reactions, two or more nucleic acid segments, each comprising one or more topoisomerases or toposiomerase recognition sites are joined to each other using a topoisomerase mediated joining reaction (*e*.*g*., a topoisomerase mediated joining reaction). After which, the tube is heated to about 85°C for about 20 min. and one or more recombinases are added. Further, if one or more of the two or more nucleic acid segments do not comprise recombination sites or if recombination with additional nucleic acid segments is desired, then nucleic acid segments which comprise one or more recombination sites may be added. Typically, the recombination sites present in the tube will be ones which are capable of recombining with each other.

In other specific embodiments of single tube reactions, two or more nucleic acid segments undergo recombination catalyzed by one or more recombinases. After recombination has occurred, toposiomerase is then added to the tube to facilitate topoisomerase mediated joining of nucleic acid segments. As above, additional nucleic acid segments may, optionally, be added to the reaction mixture along with the topoisomerase. Further, when nucleic acid segments to which one or more toposiomerases are attached are added to the reaction mixture, it will often not be necessary to add additional topoisomerase. Thus, in particular embodiments, topoisomerase modified nucleic segments may be added to the above reaction mixtures and, depending on the particular reaction conditions, additional topoisomerase may or may not be added.

Methods for preparing nucleic acid molecules which contain one or more (*e*.*g*., one, two, three, four, five, six, etc.) multiple cloning sites are described herein. For example, one or more nucleic acid segments used in methods described herein may comprise one or more multiple cloning sites. As another example, multiple cloning sites may be added to nucleic acid segments used to prepare nucleic acid molecules by methods described herein or to nucleic acid molecules prepared by methods described herein by the attachment of linkers which contain one or more multiple cloning sites. The disclosure herein includes nucleic acid molecules prepared by methods described herein which contain one or more multiple cloning sites, as well as the use of one or more these multiple cloning sites to modify nucleic acid molecules prepared by methods described herein. Also disclosed herein are nucleic acid molecules produced by the methods described above, as well as uses of these molecules and compositions comprising these molecules.

### VIRAL VECTORS

Methods for preparing nucleic acid molecules having regions of viral nucleic acids, as well as nucleic acid molecules prepared by such methods and compositions comprising these nucleic acid molecules are described herein.

Adenoviruses are viral vectors that can be used, for example, in gene therapy. Adenoviruses are especially attractive vehicles for delivering genes to respiratory epithelia and the use of such vectors are included. Adenoviruses naturally infect respiratory epithelia where they cause a mild disease. Other targets for adenovirus-based delivery systems are liver, the central nervous system, endothelial cells, and muscle. Adenoviruses have the advantage of being capable of infecting non-dividing cells. Kozarsky and Wilson, *Current Opinion in Genetics and Development 3*:499-503 (1993), present a review of adenovirus-based gene therapy. Bout et al., Human Gene Therapy 5:3-10 (1994), demonstrated the use of adenovitrus vectors to transfer genes to the respiratory epithelia of rhesus monkeys. Other instances of the use of adenoviruses in gene therapy can be found in Rosenfeld et al., Science 252:431-434 (1991); Rosenfeld et al., Cell 68:143-155 (1992); Mastrangeli. et al., J. Clin. Invest. 91:225-234 (1993); PCT Publication Nos. WO94/12649 and WO 96/17053; U.S. Patent No. 5,998,205; and Wang et al., Gene Therapy 2:775-783 (1995).

Adeno-associated virus (AAV) and Herpes viruses, as well as vectors prepared from these viruses have also been proposed for use in gene therapy (Walsh et al., 1993, Proc. Soc. Exp. Biol. Med. 204:289-300; U.S. Patent No. 5,436,146; Wagstaff et al., Gene Ther. 5:1566-70 (1998)). Herpes viral vectors are particularly useful for applications where gene expression is desired in nerve cells.

Described herein are methods for preparing nucleic acid molecules which have one or more functional properties of viral vectors (*e.g.*, adenoviral vectors, alphaviral vectors, herpes viral vectors, adeno-associated viral vectors, etc.). Methods described herein include the joining of nucleic acid segments, wherein one or more of the nucleic acid segments contains regions which confer upon product nucleic acid molecules the ability to function as viral vectors (*e*.*g*., the ability to replicate in specific host cells, the ability to be packaged into viral particles, etc.).

Described herein are methods for preparing adenoviral vectors by joining at least one (e.g., one, two, three, four, etc.) nucleic acid segment which comprises adenoviral sequences to one or more other nucleic acid segments. Specific examples of adenoviral vectors, and nucleic acid segments which can be used to prepare adenoviral vectors are disclosed in U.S. Patent Nos. 5,932,210, 6,136,594, and 6,303,362. Adenoviral vector prepared by methods described herein may be replication competent or replication deficient.

One example of an adenoviral vector may be prepared by joining a nucleic acid segment comprising adenoviral nucleic acid to one or more other nucleic acid segments. For example, when a replication deficient adenoviral vector is desired, the adenoviral nucleic acid may have deletions of all or part of one or more of the following regions: the E1a region, the E1b region, and/or the E3 region. Adenoviral vectors which contain deletions in these regions are described, for example, in U.S. Patent No. 6,136,594. The disclosure herein further includes adenoviral vectors prepared by methods described herein, as well as uses of these vectors and compositions comprising these vectors. One example of a use of adenoviral vectors prepared by methods described herein includes the delivery of nucleic acid segments to cells of a mammal (*e.g.*, a human). Thus, described herein are methods for preparing vector suitable for use in gene therapy protocols. Typically, such vectors will be replication deficient.

Adenoviral vectors described herein may comprise substantially the entire adenoviral genome with the exception that are deletions of all or part of one or more of the following regions: the E1a region, the E1b region, and/or the E3 region. In further specific embodiments, non-adenoviral nucleic acid may be present in one or more of the E1a region, the E1b region, and/or the E3 region.

In particular embodiments, adenoviral vectors prepared by methods described herein will contain at least one origin of replication and/or a selection marker which allows for amplification of the vector in prokaryotic cells, such as *E. coli.*

Adeno-associated viral vectors and Herpes viral vectors may be prepared by methods described herein which are similar to those described above. Thus, described herein are methods for preparing such vectors, as well as vectors produced by these methods, uses of these vectors, and compositions comprising these vectors.

Described herein are methods for preparing alphaviral vectors (*e*.*g*., Sindbis virus vectors, Semliki Forest virus vectors, Ross River virus vectors, Venezuelan equine encephalitis virus vectors, Western equine encephalitis virus vectors, Eastern equine encephalitis virus vectors, etc.), as well as alphaviral vectors prepared by such methods, methods employing these alphaviral vectors and compositions comprising these alphaviral vectors.

Described herein are methods for preparing alphaviral vectors by joining at least one nucleic acid segment which comprises alphaviral sequences to one or more other nucleic acid segments. Specific examples of alphaviral vectors and nucleic acids which can be used to prepare alphaviral vectors are described in U.S. Patent Nos. 5,739,026 and 6,224,879, the GibcoBRL's Instruction Manual No. 10179-018, "SFV Gene Expression System", and Invitrogen's Sindbis Expression System manual, catalog no. K750-01 (version E).

In specific embodiments, alphaviral vector sequences used in methods described herein to prepare alphaviral vectors will comprise one or more of the following components: one or more packaging signals (which may or may not be of alphaviral origin), one or more subgenomic promoters, and/or nucleic acid encoding one or more non-structural protein (*e*.*g*., nsp1, nsp2, nsp3, nsp4, etc.).

Alphaviral vectors may be introduced into cells as DNA or RNA molecules. When DNA forms of such vectors are introduced into cells, expression control sequences (*e*.*g*., inducible, repressible or constitutive expression control sequences) may then be used to generate RNA molecules from which one or more non-structural proteins may be translated. In specific embodiments, these non-structural proteins will form an RNA-dependent RNA polymerase which will amplify RNA molecules corresponding to all or part of the transcript generated from the DNA form of the alphaviral vector. Thus, these non-structural proteins may catalyze the Production of additional copies of RNA molecules from RNA templates, resulting in RNA amplification. Further, a nucleic acid segment for which high levels of expression is desired may be operably linked to a subgenomic promoter, thus resulting in the production of high levels of RNA corresponding to the nucleic acid segment.

In one exemplary embodiment, alphaviral vectors prepared by methods described herein comprise DNA wherein an inducible promoter directs transcription of an RNA molecule which encodes nsp1, nsp2, nsp3, and nsp4 of a Sindbis virus and a Sindbis subgenomic promoter operatively linked to a nucleic acid segment which is not of Sindbis viral origin. Also described herein are alphaviral vectors prepared by methods described herein, methods of using such alphaviral vectors, and compositions comprising such alphaviral vectors.

Described herein are methods for joining nucleic acid segments wherein one or more of the nucleic acid segments contains one or more (e.g., one, two, three, four, etc.) viral packaging signal (*e.g.*, one or more packaging signal derived from a virus referred to above). These packaging signals can be used to direct the packaging of nucleic acid molecules prepared by methods described herein. One method for preparing packaged nucleic acid molecules is by the introduction or expression of nucleic acid molecules described herein into packaging cell lines which express proteins suitable for the production of virus-like particles. Also described herein are packaged nucleic acid molecules as described herein, methods for preparing packaged nucleic acid molecules as described herein and compositions comprising packaged nucleic acid molecules as described herein.

Described herein are compositions, and kits containing such compositions, including kits containing component useful for performing methods described herein. A composition described herein may comprise isolated components characteristic of a step of a method described herein. For example, a composition described herein can comprise two or more of the same or different topoisomerase-charged nucleic acid molecules. As used herein, the term "different," when used in reference to the nucleic acid molecules of a composition described herein, means that the nucleic acid molecules share less than 95% sequence identity with each when optimally aligned, generally less than 90% sequence identity, and usually less than 70% sequence identity. Thus, nucleic acid molecules that, for example, differ only in being polymorphic variants of each other or that merely contain different 5' or 3' overhanging sequences are not considered to be "different" for purposes of a composition described herein. In comparison, different nucleic acid molecules are exemplified by a first sequence encoding a polypeptide and second sequence comprising a regulatory element, or a first sequence encoding a first polypeptide a second sequence encoding a non-homologous polypeptide.

Where a composition described herein comprises more than two different isolated nucleic acid molecules or more than two different topoisomerase-charged nucleic acid molecules, each of the nucleic acid molecules is different from each other, i.e., they are all different from each other. However, it will be recognized that each of the nucleic acid molecules, for example, a sequence referred to as a first nucleic acid molecule, generally comprises a population of such nucleotide sequences, which are identical or substantially identical to each other. Thus, it should be clear that the term "different" is used in comparing, for example, a first (or population of first) nucleic acid molecules with a second (and other) nucleic acid molecule. A composition comprising two or more different topoisomerase-charged nucleic acid molecules can further comprise a topoisomerase. Examples of such nucleic acid molecules comprising the components of a composition are disclosed herein and include, for example, coding sequences, transcriptional regulatory element, translational regulatory elements, elements encoding a detectable or selectable markers such as an epitope tag or an antibiotic resistance gene, elements encoding polypeptide domains such as cell compartmentalization domains or signal peptides, and the like.

As used herein, the term "isolated" means that a molecule being referred to is in a form other than that in which it exists in nature. In general, an isolated nucleotide sequence, for example, can be any nucleotide sequence that is not part of a genome in a cell, or is separated physically from a cell that normally contains the nucleotide sequence. It should be recognized that various compositions described herein comprise a mixture of isolated nucleic acid molecules. As such, it will be understood that the term "isolated" only is used in respect to the isolation of the molecule from its natural state, but does not indicate that the molecule is an only constituent.

A composition as described herein can comprise two different nucleic acid molecules, each of which contains a topoisomerase recognition site at or near one or both ends, and a site specific topoisomerase, which can bind to and cleave the nucleic acid molecules at the topoisomerase recognition site. Optionally, at least one of the different nucleic acid molecules can be a topoisomerase-charged nucleic acid molecule. Preferably, the topoisomerase covalently bound to the topoisomerase-charge nucleic acid molecule is of the same family as the topoisomerase in the composition.

Various combinations of components can be used in a method described herein. For example, the method can be performed by contacting a topoisomerase-activated first nucleic acid molecule, which optionally comprises one or more recombination sites; a second nucleic acid molecule having a first end and a second end, wherein at the first end or second end or both, the second nucleotide sequence has a topoisomerase recognition site at or near the 3' terminus, and a hydroxyl group at the 5' terminus of the same end; and a topoisomerase. Where the 5' terminus of one or both ends to be linked has a 5' phosphate group, a phosphatase also can be contacted with the components of the reaction mixture. Upon such contacting, the topoisomerase can cleave the second nucleotide sequence to produce a topoisomerase-activated second nucleic acid molecule, the phosphatase, if necessary, can generate a 5' hydroxyl group at the same end, and the second nucleic acid molecule then can be covalently linked to the topoisomerase-activated first nucleic acid molecule. As such, it will be recognized that a composition described herein can comprise any of various combinations of components useful for performing a method described herein. Once nucleic acid molecules are joined by the methods described above, the resulting molecules may then be used in recombination reactions, such as those described elsewhere herein. The disclosure herein further includes nucleic acid molecules prepared by methods described herein, compositions comprising such nucleic acid molecules, and methods for using such nucleic acid molecules.

In general, a method described herein for generating a ds recombinant nucleic acid molecule covalently linked in both strands is based on the determination that a ds recombinant nucleic acid molecule covalently linked in both strands can be produced by contacting a first nucleic acid molecule with a second nucleic acid molecule, wherein the first and second sequences each have, at the ends to be linked, a topoisomerase recognition site, for example, 5'-(C/T)CCTT-3' (Shuman, *supra,* 1991; U.S. Pat. No. 5,766,891). Upon cleavage, the site specific topoisomerase is covalently bound at the 3' terminus. Where the cleaved nucleotide sequences also contain a 5' hydroxy group at the same end as the bound topoisomerase, and the ends of the two nucleotide sequences associate, the topoisomerase on each 3' terminus can covalently link that terminus to a 5' hydroxyl group on the associated nucleotide sequence (see Figure 1).

As used herein, reference to contacting a first nucleotide sequence and at least a second nucleotide sequence "under conditions such that all components are in contact" means that the reaction conditions are appropriate for the topoisomerase-cleaved ends of the nucleotide sequences to come into sufficient proximity such that a topoisomerase can effect its enzymatic activity and covalently link the 3' or 5' terminus of a first nucleotide sequence to a 5' or 3' terminus, respectively,-of a second nucleotide sequence. Examples of such conditions, which include the reaction temperature, ionic strength, pH, and the like, are disclosed herein, and other appropriate conditions as required, for example, for particular 5' overhanging sequences of the termini generated upon topoisomerase cleavage, can be determined empirically or using formulas that predict conditions for specific hybridization of nucleotide sequences, as is well known in the art (see, for example, (Sambrook et al., Molecular Cloning: A laboratory manual (Cold Spring Harbor Laboratory Press 1989); Ausubel et al., Current Protocols in Molecular Biology, John Wiley and Sons, Baltimore, MD (1987, and supplements through 1995)).

A method described herein may provide a means to render an open reading from a cDNA or an isolated genomic DNA sequence expressible by operatively linking one or more regulatory elements to the putative coding sequence. Accordingly, a first nucleic acid molecule comprising an open reading frame can be amplified by PCR using a primer pair that generates an amplified first nucleic acid molecule having a topoisomerase recognition site at one or both ends and, optionally, one or more recombination sites, as desired, such that, upon cleavage by the site specific topoisomerase, one or both ends contains a defined 5' or 3' overhang or is blunt. Where both ends of the amplified first nucleic acid molecule are so constructed, the 5' or 3' overhanging sequences generally, but not necessarily, are different from each other. The amplified first nucleic acid molecule then can be contacted with a second nucleic acid molecule comprising a desired regulatory element such as a promoter and, in certain embodiments, (a) one or more topoisomerase recognition sites, and with a topoisomerase and/or (b) one or more recombination sites, under conditions which facilitate recombination, such that the second nucleotide sequence is operatively covalently linked to the 5' end of the coding sequence according to a method described herein.

In such a method, a second (or other) nucleic acid molecule also can comprise two or more regulatory elements, for example, a promoter, an internal ribosome entry site and an ATG initiator methionine codon, or the like, or other sequence of interest, for example, an sequence encoding an epitope tag, in operative linkage with each other, and which can be operatively covalently linked to the 5' end of a first nucleic acid molecule comprising a coding sequence. Such a method can further include contacting a third nucleic acid molecule comprising, for example, a polyadenylation signal, which can be operatively covalently linked according to a method described herein to the 3'end of the coding sequence, thereby generating an expressible ds recombinant nucleic acid molecule. As such, a method described herein provides a means for generating a functional ds recombinant nucleic acid molecule that can be transcribed, translated, or both as a functional unit. As disclosed herein, the inclusion of complementary 5' or 3' overhanging sequences generated by topoisomerase cleavage at the termini of the nucleic acid molecules to be linked together by the site specific topoisomerase facilitates the generation of a ds recombinant nucleic acid molecule having a desired directional orientation of the nucleotide sequences in the construct.

A method described herein may be performed such that the first nucleic acid molecule or a second (or other) nucleic acid molecule, or combination thereof, is one of a plurality of nucleotide sequences. As used herein, the term "plurality," when used in reference to a first or at least a second nucleotide sequence, means that the nucleotide sequences are related but different. For purposes of the present disclosure herein, the nucleotide sequences of a plurality are "related" in that each nucleotide sequence in the plurality contains at least a topoisomerase recognition site, or a cleaved form thereof, at one or more termini and/or at least one recombination site. Furthermore, the nucleotide sequences of a plurality are "different" in that they can comprise, for example, a cDNA library, a combinatorial library of nucleotide sequences, a variegated population of nucleotide sequences, or the like. Methods of making cDNA libraries, combinatorial libraries, libraries comprising variegated populations of nucleotide sequences, and the like are well known in the art (see, for example, U.S. Pat. No. 5,837,500; U.S. Pat No. 5,622,699; U.S. Pat. No. 5,206,347; Scott and Smith, Science 249:386-390, 1992; Markland et al., Gene 109:13-19, 1991; O'Connell et al., Proc. Natl. Acad. Sci., USA 93:5883-5887, 1996; Tuerk and Gold, Science 249:505-510, 1990; Gold et al., Ann. Rev. Biochem. 64:763-797, 1995).

Described herein is a method of generating a ds recombinant nucleic acid molecule covalently linked in both strands by amplifying a portion of a first nucleotide sequence using a PCR primer pair, wherein at least one primer of the primer pair encodes a topoisomerase recognition site or a complement thereof and, optionally, one or more recombination sites, thereby producing a first nucleic acid molecule having a first end and a second end, wherein the first end or second end or both has a topoisomerase recognition site at the 3' terminus and/or the 5'terminus; and contacting the first nucleic acid molecule; at least a second nucleic acid molecule having a first end and a second end, wherein the first end or second end or both has a topoisomerase recognition site at the 3' terminus and/or the 5' terminus, or a cleavage product thereof; and a topoisomerase (see Figure 1). When contacted under conditions such that an end of the first nucleic acid molecule having a topoisomerase recognition site and an end of the at least second nucleic acid molecule having a topoisomerase recognition site can associate, a ds recombinant nucleic acid molecule covalently linked in both strands is generated. Once nucleic acid molecules are joined by the methods described above, the resulting molecules may then be used in recombination reactions, such as those described elsewhere herein. The disclosure herein further includes nucleic acid molecules prepared by methods described herein, compositions comprising such nucleic acid molecules, and methods for using such nucleic acid molecules.

As disclosed herein, a PCR method using primers designed to incorporate one or more topoisomerase recognition sites and, optionally, one or more recombination sites at one or both ends of an amplified nucleic acid molecule provides a convenient means for producing nucleic acid molecules useful in a method described herein. In certain embodiments, at least one of the primers of a primer pair is designed such that it comprises, in a 5' to 3' orientation, a nucleotide sequence complementary to a topoisomerase recognition site, and a nucleotide sequence complementary to the 3' end of a target nucleic acid molecule to be amplified (i.e., a target specific region). In addition, the primer can contain, in a position 5' to the complement of the topoisomerase recognition site, a desired nucleotide sequence of any length (generally about 1 to 100 nucleotide, usually about 2 to 20 nucleotides, and particularly about 4 to 12 nucleotides), which, upon cleavage of the amplification product by a site specific topoisomerase, forms a desired 5' overhang. The second primer of the PCR primer pair can be complementary to a desired sequence of the nucleotide sequence to be amplified, and can comprise a complement to a topoisomerase recognition site, a sequence that would generate a 5' overhang upon cleavage by a site specific topoisomerase, or any other sequence, as desired.

Such a primer can comprise or encode any other sequence of interest, including, for example, a site specific integration recognition site such as an att site, a lox site, or the like, or, as discussed above, can simply be used to introduce a topoisomerase recognition site into a nucleic acid molecule comprising such a sequence of interest. A ds recombinant nucleic acid molecule generated according to a method described herein and containing a site specific integration recognition site such as an att site or lox site can be integrated specifically into a desired locus such as into a vector, a gene locus, or the like, that contains the required integration site, for example, an att site or lox site, respectively, and upon contact with the appropriate enzymes required for the site specific event, for example, lambda Int and IHF proteins or Cre recombinase, respectively. The incorporation, for example, of attB or attP sequences into a ds recombinant nucleic acid molecule covalently linked in both strands according to a method described herein allows for the convenient manipulation of the nucleic acid molecule using the GATEWAY™ Cloning System (Invitrogen Corp., La Jolla CA).

In one embodiment, a construct generated according to a method described herein may be further amplified by a PCR reaction or other amplification reaction. Direct PCR of a ds recombinant nucleic acid molecule generated according to a method described herein is possible because the construct is covalently linked in at least one strand. As such, PCR can be used to generate a large amount of the construct. More importantly, as indicated above, PCR provides an *in vitro* selection method for obtaining only a desired product generated according to a method described herein, without obtaining partial reaction products. For example, a method described herein can be used to generate a ds recombinant nucleic acid molecule covalently linked in both strands comprising, operatively linked in a 5' to 3' orientation, a first nucleic acid molecule comprising a promoter, a second nucleic acid molecule comprising a coding region, and a third nucleic acid molecule comprising a polyadenylation signal.

As disclosed herein, a construct having a predetermined orientation can be generated by including complementary 5' overhanging sequences on the ends of the nucleic acid molecules to be joined. By selecting a PCR primer pair including a first primer complementary to the first nucleic acid molecule and upstream of the promoter sequence, and a second primer complementary to the third nucleic acid molecule and downstream of the polyadenylation signal, a functional amplification product comprising the promoter, coding region and polyadenylation signal can be generated. In contrast, partial reaction products that lack either the first nucleic acid molecule or third ds nucleotide is not amplified because either the first or second primer, respectively, would not hybridize to the partial product. In addition, a construct lacking the second nucleic acid molecule would not be generated due to the lack of complementarity of the 5' overhanging sequences of the first and third nucleic acid molecules. As such, a method described herein provides a means to obtain a desired functional ds recombinant nucleic acid molecule covalently linked in both strands.

The use of PCR in such a manner further provides a means to screen a large number of nucleic acid molecules generated according to a method of the invention in order to identify constructs of interest. Since methods for utilizing PCR in automated high throughput analyses are routine and well known, it will be recognized that the methods described herein can be readily adapted to use in a high throughput system. Using such a system, a large number of constructs can be screened in parallel, and partial or incomplete reaction products can be identified and disposed of, thereby preventing a waste of time and expense that would otherwise be required to characterize the constructs or examine the functionality of the constructs in further experiments.

The methods described herein have broad application to the field of molecular biology. As discussed in greater detail below, the methods described herein can be used, for example, to label DNA or RNA probes, to perform directional cloning (see Example 1.B), to generate sense or antisense RNA molecules (see Example 2.A), to prepare bait or prey constructs for performing a two hybrid assay (see Example 2.C), to prepare linear expression elements (see Examples 2.A and 2.B), and to prepare constructs useful for coupled *in vitro* transcription/translation assays (see Example 2.B). For example, a method of generating ds recombinant nucleic acid molecules covalently linked in both strands provides a means to generate linear expression elements (LEEs), which consist of a linear nucleic acid molecule comprising two or more nucleotide sequences such as a promoter or other regulatory element linked to an open reading frame (see Example 1). LEEs have been reported to efficiently transfect cells, thus bypassing a requirement for cloning the expression element in a vector (Sykes and Johnston, Nat. Biotechnol. 17:355-359, 1999). The components of a LEE can be noncovalently linked, or can be covalently linked via a ligation reaction. The preparation of noncovalently linked LEEs requires using PCR primers containing deoxyuridine residues to amplify each nucleotide sequence component, then treating the PCR products with uracil-DNA glycosylase to generate overhanging ends that can hybridize. However, the efficiency of transfection using such noncovalently linked LEEs is variable, and, in some' cases, much lower than the efficiency of covalently linked LEEs (Sykes and Johnston, *supra,* 1999). Furthermore, such LEEs are not suitable for use as templates for PCR amplification because the primer extension reaction cannot proceed past nicks in the template and, therefore, is terminated producing incomplete reaction products.

A method described herein provides a straightforward and simple means to generate covalently linked LEEs, thereby avoiding the inconvenient and additional steps previously described for preparing a LEE, as well as reducing variability in transfection efficiency as observed using noncovalently linked LEEs. For example, a first nucleic acid molecule, which encodes an open reading frame of interest, can be amplified by PCR as disclosed herein to contain a topoisomerase recognition site, or cleavage product thereof, on one or both ends. Furthermore, the PCR primers can be designed such that, upon cleavage of the amplified first nucleic acid molecule by a site specific topoisomerase, the cleavage product contains a predetermined and desired 5' overhanging sequence. A second nucleotide sequence (and a third or more, as desired), in addition to containing a topoisomerase recognition site, or cleavage product thereof, can include or encode a regulatory element, for example, a promoter, an enhancer, a silencer, a splice acceptor site, a translation start site, a ribosome recognition site or internal ribosome entry site, a polyadenylation signal, an initiator methionine codon, or a STOP codon, or can encode any other desired sequence such as an epitope tag or cell compartmentalization domain. Preferably, the second (or other) nucleic acid molecule to be covalently linked to the first nucleic acid molecule has a 5' overhanging sequence that is complementary to the 5' overhang at the end of the first nucleic acid molecule to which it is to be linked. Upon contact of such nucleotide sequences in presence of a topoisomerase a promoter, for example, can be operatively covalently linked to the 5'terminus of the open reading frame, and a polyadenylation signal can be operatively covalently linked to the 3'terminus of the open reading frame, thereby generating a covalently linked functional LEE (see Example 1).

Examples of regulatory elements useful in the are disclosed herein and include transcriptional regulatory elements, translational regulatory elements, elements that facilitate the transport or localization of a nucleotide sequence or polypeptide in (or out of) a cell, elements that confer a detectable phenotype, and the like. Transcriptional regulatory elements include, for example, promoters such as those from cytomegalovirus, Moloney leukemia virus, and herpes virus, as well as those from the genes encoding metallothionein, skeletal actin, phosphoenolpyruvate carboxylase, phosphoglycerate, dihydrofolate reductase, and thymidine kinase, as well as promoters from viral long terminal repeats (LTRs) such as Rous sarcoma virus LTR and operators; enhancers, which can be constitutively active such as an immunoglobulin enhancer, or inducible such as SV40 enhancer; and the like. For example, a metallothionein promoter is a constitutively active promoter that also can be induced to a higher level of expression upon exposure to a metal ion such as copper, nickel or cadmium ion. In comparison, a tetracycline (tet) inducible promoter is an example of a promoter that is induced upon exposure to tetracycline, or a tetracycline analog, but otherwise is inactive. A transcriptional regulatory element also can be a tissue specific regulatory element, for example, a muscle cell specific regulatory element, such that expression of an encoded product is restricted to the muscle cells in an individual, or to muscle cells in a mixed population of cells in culture, for example, an organ culture. Muscle cell specific regulatory elements including, for example, the muscle creatine kinase promoter (Sternberg et al., Mol. Cell. Biol. 8:2896-2909, 1988) and the myosin light chain enhancer/promoter (Donoghue et al., Proc. Natl. Acad. Sci., USA 88:5847-5851, 1991) are well known in the art. Other tissue specific promoters, as well as regulatory elements only expressed during particular developmental stages of a cell or organism are well known in the art.

The regulatory elements contained in the nucleotide sequences used in or produced by the practice of the disclosure herein can be one or more operators. A number of operators are known in the art. An example of an operator suitable for use with the disclosure herein is the tryptophan operator of the tryptophan operon of *E. coli.* The tryptophan repressor, when bound to two molecules of tryptophan, binds to the *E. coli* tryptophan operator and, when suitably positioned with respect to the promoter, blocks transcription. Another example of an operator suitable for use with the disclosure herein is operator of the *E. coli* tetracycline operon. Components of the tetracycline resistance system of *E. coli* have also been found to function in eukaryotic cells and have been used to regulate gene expression. For example, the tetracycline repressor, which binds to tetracycline operator in the absence of tetracycline and represses gene transcription, has been expressed in plant cells at sufficiently high concentrations to repress transcription from a promoter containing tetracycline operator sequences (Gatz et al., Plants 2:397-404 (1992)). The tetracycline regulated expression systems are described, for example in U.S. Patent No. 5,789,156. Additional examples of operators which can be used with the disclosure herein include the *Lac* operator and the operator of the molybdate transport operator/promoter system of *E. coli* (*see, e*.*g*., Cronin et al., Genes Dev. 15:1461-1467 (2001) and Grunden et al., J. Biol. Chem., 274:24308-24315 (1999)).

Described herein are methods for preparing nucleic acid molecules that contain one or more operators which can be used to regulate expression in prokaryotic or eukaryotic cells. As one skilled in the art would recognize, when a nucleic acid molecule which contains an operator is placed under conditions in which transcriptional machinery is present, either *in vivo* or *in vitro*, regulation of expression will often be modulated by contacting the nucleic acid molecule with a repressor and one or more metabolites which facilitate binding of an appropriate repressor to the operator. Thus, the disclosure herein further provides methods for preparing nucleic acid molecules which encode repressors which modulate the function of operators, as well as nucleic acid molecules produced by these methods, compositions comprising these molecules, and uses of these molecules and compositions.

Regulatory or other elements useful in generating a construct according to a method described herein can be obtained in various ways. In particular, many of the elements are included in commercially available vectors and can be isolated therefrom and can be modified to contain a topoisomerase recognition site at one or both ends, for example, using a PCR method as disclosed herein. In addition, the sequences of or encoding the elements useful herein generally are well known and disclosed in publications. In many cases, the elements, for example, many transcriptional and translational regulatory elements, as well as cell compartmentalization domains, are relatively short sequences and, therefore, are amenable to chemical synthesis of the element or a nucleotide sequence encoding the element. Thus, an element comprising a composition described herein, useful in generating a ds recombinant nucleic acid molecule according to a method described herein, or included within a kit described herein, can be chemically synthesized and, if desired, can be synthesized to contain a topoisomerase recognition site at one or both ends of the element and, further, to contain an overhanging sequence following cleavage by a site specific topoisomerase.

A topoisomerase-charged vector can be generated in the following manner (Genome Res. 9: 383-392, 1999): A vector is linearized with a restriction enzyme that leaves "sticky ends". Using a ligase such as T4 DNA ligase, adapter oligonucleotides are ligated to both ends, and both strands, of the linearized DNA. The adapter oligonucleotides contain and position a 5'-CCCTT-3' *Vaccinia* topoisomerase type I recognition sequence such that it can be cleaved by topoisomerase and trap the covalent topoisomerase-DNA complex at each 3' end of the vector. The adapted vector is then incubated with purified *Vaccinia* topoisomerase and an annealing oligonucleotide that complete the "topoisomerase sites" at each end of the vector. The annealing oligonucleotide acts to leave a break, or nick, in the "bottom" strand opposite the last T in the 5'-CCCTT-3' containing oligonucleotide. The oligonucleotide adapter fragments that are "downstream" of the topoisomerase cleavage site (the "leaving groups") are released upon topoisomerase cleavage and are removed in the topoisomerase-vector purification process. In the absence of the 5' hydroxyl from the "leaving group", topoisomerase is trapped in a covalent complex with the DNA ends to produce a topoisomerase-charged vector.

Where nucleic acid molecules are to be covalently linked according to a method described herein, the nucleotide sequences generally are operatively linked such that the recombinant nucleic acid molecule that is generated has a desired structure and performs a desired function or encodes a desired expression product. As used herein, the term "operatively linked" means that two or more nucleotide sequences are positioned with respect to each other such that they act as a unit to effect a function attributable to one or both sequences or a combination thereof. The term "operatively covalently linked" is used herein to refer to operatively linked nucleotide sequences generated according to a method described herein for generating a ds recombinant nucleic acid molecule covalently linked in one or both strands. For example, a nucleotide sequence containing an open reading frame can be operatively linked to a promoter such that the promoter confers its regulatory effect on the open reading frame similarly to the way in which it would effect expression of an open reading frame that it normally is associated with in a genome in a cell. Similarly, two or more nucleotide sequences comprising open reading frames can be operatively linked in frame such that, upon transcription and translation, a chimeric fusion polypeptide is produced.

Although a ds recombinant nucleic acid molecule covalently linked in one or both strands, generated according to a method described herein generally is linear, the construct generated also can be a circularized ds recombinant nucleic acid molecule. Furthermore, a circular ds recombinant nucleic acid molecule can be generated such that it has the characteristics of a vector, and contains, for example, regulatory elements required for replication in a prokaryotic host cell, a eukaryotic host cell, or both, and can contain a nucleotide sequence encoding a polypeptide that confers antibiotic resistance or the like. An advantage of such a method is that the generated ds recombinant nucleic acid molecule, which is circularized according to a method described herein, can be transformed or transfected into an appropriate host cell, wherein the construct is amplified. Thus, in addition to an *in vitro* method such as PCR, which can be used to generate large amounts of a linear ds recombinant nucleic acid molecule generated according to a method described herein an *in vivo* method using a host cell can be used for obtaining a large amount of a circularized product generated according to a method described herein. Such elements including bacterial origins of replication, antibiotic resistance genes, and the like, which comprise a topoisomerase recognition site according to the disclosure herein, can be useful components to include in a kit as disclosed herein.

It should be recognized that a linear ds recombinant nucleic acid molecule covalently linked in one or both strands, also can be cloned into a vector, which can be a plasmid vector or a viral vector such as a bacteriophage, baculovirus, retrovirus, lentivirus, adenovirus, vaccinia virus, semliki forest virus and adeno-associated virus vector, all of which are well known and can be purchased from commercial sources (Promega, Madison WI; Stratagene, La Jolla CA; OIBCO/BRL, Gaithersburg MD). If desired, the vector can be linearized and modified according to a method described herein, for example, using a PCR method, to contain a topoisomerase recognition site, or cleavage product thereof, at one or both 3' termini, or can be constructed by one skilled in the art (see, generally, Meth. Enzymol., Vol. 185, Goeddel, ed. (Academic Press, Inc., 1990); Jolly, Canc. Gene Ther. 1:51-64, 1994; Flotte, J. Bioenerg. Biomemb. 25:37-42, 1993; Kirshenbaum et al., J. Clin. Invest. 92:381-387,1993).

Viral expression vectors can be particularly useful where a method described herein is practiced for the purpose of generating a ds recombinant nucleic acid molecule covalently linked in one or both strands, that is to be introduced into a cell, particularly a cell in a subject. Viral vectors provide the advantage that they can infect host cells with relatively high efficiency and can infect specific cell types or can be modified to infect particular cells in a host.

Viral vectors have been developed for use in particular host systems and include, for example, baculovirus vectors, which infect insect cells; retroviral vectors, other lentivirus vectors such as those based on the human immunodeficiency virus (HIV), adenovirus vectors, adeno-associated virus (AAV) vectors, herpesvirus vectors, vaccinia virus vectors, and the like, which infect mammalian cells (see Miller and Rosman, BioTechniques 7:980-990, 1992; Anderson et al., Nature 392:25-30 Suppl., 1998; Verma and Somia, Nature 389:239-242,1997; Wilson, New Engl. J. Med. 334:1185-1187 (1996)). For example, a viral vector based on an HIV can be used to infect T cells, a viral vector based on an adenovirus can be used, for example, to infect respiratory epithelial cells, and a viral vector based on a herpesvirus can be used to infect neuronal cells. Other vectors, such as AAV vectors can have greater host cell range and, therefore, can be used to infect various cell types, although viral or non-viral vectors also can be modified with specific receptors or ligands to alter target specificity through receptor mediated events.

Described herein is a method which can be used to operatively covalently link a first nucleic acid molecule containing an open reading frame to a second (and other) nucleic acid molecule containing an open reading frame such that a nucleic acid molecule encoding a chimeric polypeptide is generated. The chimeric polypeptide comprises a fusion polypeptide, in which the two (or more) encoded peptides (or polypeptides) are translated into a single product, i.e., the peptides are covalently linked through a peptide bond. For example, a first nucleic acid molecule can encode a cell compartmentalization domain, such as a plasma membrane localization domain, a nuclear localization signal, a mitochondrial membrane localization signal, an endoplasmic reticulum localization signal, or the like, or a protein transduction domain such as the human immunodeficiency virus TAT protein transduction domain, which can facilitate translocation of a peptide linked thereto into a cell (see Schwarze et al., Science 285:1569-1572, 1999; Derossi et al., J. Biol. Chem. 271:18188, 1996; Hancock et al., EMBO J. 10:4033-4039, 1991; Buss et al., Mod, Cell. Biol. 8:3960-3963, 1988; U.S. Pat. No. 5,776,689 ). Such a domain can be useful to target a fusion polypeptide comprising the domain and a polypeptide encoded by a second nucleic acid molecule, to which it is covalently linked according to a method described herein, to a particular compartment in the cell, or for secretion from or entry into a cell. As such, the disclosure herein provides a means to generate ds recombinant nucleic acid molecules covalently linked in both strands that encode a chimeric polypeptide.

A fusion polypeptide expressed from a nucleic acid molecule generated according to a method described herein also can comprise a peptide having the characteristic of a detectable label or a tag such that the express fusion polypeptide can be detected, isolated, or the like. For example, a nucleic acid molecule containing a topoisomerase recognition site, or cleavage product thereof, as disclosed herein, can encode an enzyme such as alkaline phosphatase, -galactosidase, chloramphenicol acetyltransferase, luciferase, or other enzyme; or can encode a peptide tag such as a polyhistidine sequence (e.g., hexahistidine), a V5 epitope, a c-mye epitope; a hemagglutinin A epitope, a FLAG epitope, or the like. Expression of a fusion polypeptide comprising a detectable label can be detected using the appropriate reagent, for example, by detecting light emission upon addition of luciferin to a fusion polypeptide comprising luciferase, or by detecting binding of nickel ion to a fusion polypeptide comprising a polyhistidine tag. Similarly, isolation of a fusion polypeptide comprising a tag can be performed, for example, by passing a fusion polypeptide comprising a myc epitope over a column having an anti-c-myc epitope antibody bound thereto, then eluting the bound fusion polypeptide, or by passing a fusion polypeptide comprising a polyhistidine tag over a nickel ion or cobalt ion affinity column and eluting the bound fusion polypeptide. Methods for detecting or isolating such fusion polypeptides will be well known to those in the art, based on the selected detectable libel or tag (see, for example, Hopp et al., BioTechnology 6:1204, 1988; U.S. Pat. No. 5,011,912).

Described herein is a method which also can be used to detectably label a nucleotide sequence with a chemical or small organic or inorganic moiety such that the nucleotide sequence is useful as a probe. For example, a nucleic acid molecule, which has a topoisomerase recognition site, or cleavage product thereof, at a 3' terminus, can have bound thereto a detectable moiety such as a biotin, which can be detected using avidin or streptavidin, a fluorescent compound (e.g., Cy3, Cy5, Fam, fluorescein, or rhodamine), a radionuclide (e.g., sulfur-35, technicium-99, phosphorus-32, or tritium), a paramagnetic spin label (e.g., carbon-13), a chemiluminescent compound, or the like, such that, upon generating a covalently linked double stranded recombinant nucleic acid molecule according to a method described herein, the generated nucleic acid molecule will be labeled. Methods of detectably labeling a nucleotide sequence with such moieties are well known in the art (see, for example, Hermanson, "Bioconjugate Techniques" (Academic Press 1996) ). Furthermore, a detectable label can be used to allow capture of a ds nucleic acid molecule that is generated. Finally, a detectable label, for example biotin, can be used to block ligation of a topoisomerase-charged end of a first nucleic acid molecule to a labeled end of a second nucleic acid molecule, thus providing a method to direct ligation to the unlabelled end of the second nucleic acid molecule. It should be recognized that such elements as disclosed herein or otherwise known in the art, including nucleotide sequences encoding cell compartmentalization domains, or detectable labels or tags, or comprising transcriptional or translation regulatory elements can be useful components of a kit as disclosed herein.

A method is described herein which provides a means to conveniently generate ds recombinant nucleic acid molecules that encode chimeric polypeptides useful, for example, for performing a two hybrid assay. In such a method, the first nucleic acid molecule encodes a polypeptide, or a relevant domain thereof, that is suspected of having or being examined for the ability to interact specifically with one or more other polypeptides. The first nucleic acid molecule is modified as disclosed herein to contain a topoisomerase recognition site at one or both ends and, if desired, a 5' overhanging sequence. The second nucleic acid molecule, to which the first nucleic acid molecule is to be covalently-linked according to a method described herein, can encode a transcription activation domain or a DNA binding domain (Example 2.C), and contains a topoisomerase recognition site, or cleavage product thereof, and a 5' overhanging sequence complementary to that at the end of the first nucleic acid molecule to which it is to be linked. Upon contact with a topoisomerase, if the nucleotide sequences are not already topoisomerase-charged, a first hybrid useful for performing a two hybrid assay (see, for example, Fields and Song, Nature 340:245-246, 1989; U.S. Pat. No. 5,283,173; Fearon et al., Proc, Natl, Acad. Sci., USA 89:7958-7962, 1992; Chien et al., Proc, Natl, Acad. Sci., USA 88:9578-9582, 1991; Young, Biol. Reprod. 58:302-311(1998)), or modified form of a two hybrid assay such as the reverse two hybrid assay (Leanna and Hannink, Nucl. Acids Res. 24:3341-3347, 1996), the repressed transactivator system (U.S. Pat. No.5,885,779), the protein recruitment system (U.S. Pat. No. 5,776,689), and the like, is generated. Similar methods are used to generate the second hybrid protein, which can comprise a plurality of polypeptides to be tested for the ability to interact with the polypeptide, or domain thereof, of the first hybrid protein.

Similarly, such a method of generating a chimeric protein can be performed according to a method described herein for generating a ds recombinant nucleic acid molecule covalently linked in one strand, using first and second nucleic acid molecules comprising a site-specific topoisomerase recognition site (e.g., a type IA or a type II topoisomerase recognition site), or cleavage product thereof, at least at one 5' terminus of an end to be joined, wherein the nucleic acid molecules can further comprise complementary 3' overhangs upon cleavage by the topoisomerase.

Similarly, such a method of generating a chimeric protein can be performed according to a method described herein for generating a ds recombinant nucleic acid molecule covalently linked in both strands using first and second nucleic acid molecules comprising a topoisomerase recognition site, or cleavage product thereof, at least at the 5' terminus of the ends to be joined, wherein the nucleic acid molecules can further comprise complementary 3' overhangs upon cleavage by the topoisomerase; or one of the first or second nucleic acid molecules can comprise topoisomerase recognition sites, or cleavage products thereof, at the 5' terminus and the 3' terminus of at least one end, and the other nucleic acid molecule can contain a 3' hydroxyl group and a 5' hydroxyl group at the end to be joined, and wherein, upon cleavage by the topoisomerases, the topoisomerase-charged nucleic acid molecule can contain a 5' or 3' overhang that is complementary to, and facilitates hybridization to, a 5' or 3' overhang, respectively, or a blunt end, at the end of the other nucleic acid molecule to be joined.

Also described herein is a method for the directional insertion of DNA fragments into cloning or expression vectors with the ease and efficiency of topoisomerase-mediated cloning. This also has advantages over current cloning systems because it decreases the laborious screening process necessary to identify cloned inserts in the desired orientation. This consists, in its simplest form, of a linearized expression vector having a single topoisomerase molecule covalently attached at both 3' ends. At least one end of the linearized vector contains a 5' single-stranded overhang, while the opposite end can be either blunt, possess a single 3' T extension for T/A cloning, or may itself contain a second 5' single-stranded overhang sequence. These single-stranded sequence overhangs are alternatively referred to herein as "SSS" and may consist of any convenient sequence.

Construction of a topoisomerase-charged cloning vector according to the disclosure herein may be accomplished, for example, by endonuclease digestion of the vector (which may be a pDONR vector (*see* Figure 32) or a pDEST vector (*see* Figure 33)), followed by complementary annealing of synthetic oligonucleotides and site-specific cleavage of the heteroduplex by Vaccinia topoisomerase I. Digestion of a vector with any compatible endonuclease creates specific sticky ends. Custom oligonucleotides may be annealed to these sticky ends, and possess sequences that, following topoisomerase I modification, form custom ends of the vector (*see* Figures 32 and 33). The sequence and length of the SSS will vary based on the desires of the user.

In one use of the TOPO SSS vectors described herein the DNA fragment to be inserted into the vector is a PCR product. Following PCR amplification with custom primers, the product can be directionally inserted into a topoisomerase I charged cloning vector having a SSS on one or both ends of the insertion site. The custom primers may be designed such that at least one primer of a given primer pair contains an additional sequence at its 5' end. The added sequence may be designed to be complementary to the sequence of the single-stranded overhang in the vector. The complementarity between the 5' single-stranded overhang in the vector and the 5' end of the PCR product mediates the directional insertion of the PCR product into the topoisomerase-mediated vector. Specifically, since only one end of the vector and one end of the PCR product possess complimentary SSS regions, the insertion of the product is directional. Topoisomerase I catalyzes the ligation of the PCR product to the vector.

Also described herein is a modified cloning vector, having an overhanging single stranded piece of DNA, (the SSS) charged with topoisomerase, or "TOPO SSS vector". The modified vector allows the directional insertion of PCR amplified, or otherwise suitable, open reading frames (ORF) for subsequent expression, and takes advantage of the efficiency of topoisomerase-mediated cloning.

As noted above, topoisomerases are a class of enzymes that modify the topological state of DNA via the breakage and rejoining of DNA strands, (Shuman et al., US Patent No. '5,766,891). Vaccinia virus encodes a 314 aa type I topoisomerase enzyme capable of site-specific single-strand nicking of double stranded DNA, as well as 5' hydroxyl driven religation. Site-specific type I topoisomerases include, but are not limited to, viral topoisomerases such as pox virus topoisomerase. Examples of pox virus topoisomerases include shope fibroma virus and ORF virus. Other site-specific topoisomerases are well known to those skilled in the art and can be used to practice the disclosure herein.

Shuman teaches that Vaccinia topoisomerase binds to duplex DNA and cleaves the phosphodiester backbone of one strand while exhibiting a high level of sequence specificity. Cleavage occurs at a consensus pentapyrimidine element 5'-(C/T)CCTT-3' or related sequences in the scissile strand. In one embodiment the scissile bond is situated in the range of 2-12 bp from the 3' end of the duplex DNA. In another embodiment cleavable complex formation by Vaccinia topoisomerase requires six duplex nucleotides upstream and two nucleotides downstream of the cleavage site. Examples of Vaccinia topoisomerase cleavable sequences include, but are not limited to, +6/-6 duplex GCCCTTATTCC (SEQ ID NO:29), +8/-4 duplex TCGCCCTTATC (SEQ ID NO:30), +10/-2 duplex TGTCGCCCTTAT (SEQ ID NO:31), +11/-1 duplex GTGTCGCCCTTA (SEQ ID NO:32).

Examples of other site-specific type I topoisomerases are well known in the art. These enzymes are encoded by many organisms including, but not limited to *Saccharomyces cerevisiae, Saccharomyces pombe* and *Tetrahymena,* however these species' topoisomerase I enzymes have less specificity for a consensus sequence than does Vaccinia's. (Lynn, R. M., Bjornsti, M., Caron, P. R. and Wang, J. C., (1989) Peptide sequencing and site-directed mutagenesis identify tyrosine-727 as the active site tyrosine of Saccharomyces cerevisiae DNA topoisomerase I, Proc. Natl. Acad. Sci. USA, 86: 3559-3563), (Eng, W., Pandit, S. D., and Stemglanz, R., (1989) Mapping of the active site tyrosine of eukaryotic DNA topoisomerase I, J. Biol. Chem., 264 13373-13376) and (Busk, H., Thomsen, B., Bonven, B. J., Nielsen, O. F., and Westergaard, O. (1987) Preferential relaxation of supercoiled DNA containing a hexadecameric recognition for topoisomerase I, Nature, 327: 638-640), respectively.

As used herein the term donor signifies a duplex DNA which contains a 5'-CCCTT cleavage site near the 3' end, and the term acceptor signifies a duplex DNA which contains a 5'-OH terminus. Once covalently activated by topoisomerase the donor will be transferred to those acceptors to which it has SSS complementation.

Topoisomerase-modified vectors may be further adapted to contain at least one 5' single-stranded overhang sequence to facilitate the directional insertion of DNA segments. In a preferred embodiment, the segment to be cloned is a PCR product constituting an open reading frame (ORF) which will be expressed from the resultant recombinant vector. The primers used for amplifying the ORF are designed such that at least one primer of the primer pair contains an additional sequence at its 5' end. This sequence is designed to be complementary to the sequence of the 5' single-stranded overhang present in the topoisomerase-modified vector described herein.

Certain preferred, but non-exclusive, embodiments according to the disclosure herein are described in detail below in Examples 5-8.

Nucleic acid molecules assembled using methods described herein either may be used directly or may be amplified and then used for any number of purposes. With preference to Figure 34, nucleic acid segments to be assembled using methods described herein may be generated by any number of methods. For example, these segments may be obtained by any method know in the art. In instances where the nucleic acid segments do not have one or more (*e*.*g*., one, two, three, four, etc.) termini and/or regions suitable for assembly using methods described herein, such termini and/or regions may be added. Suitable termini and/or regions may be added, for example, by amplifying nucleic acids using PCR or by the addition of one or more (*e.g.,* one, two, three, four, etc.) adapter linkers (*e*.*g*., adapter linkers which contain one or more topoisomerase recognition sites). Nucleic acid segments having suitable termini and/or regions may then be assembled using methods described elsewhere herein.

As shown in Figure 34, once assembled, the linked nucleic acid segments may be amplified (*e*.*g*., *in vivo* or *in vitro*) and then used in any number of methods or processes, many of which are described elsewhere herein. Alternatively, the assembled nucleic acid segments may be used directly for applications such as *in vitro* transcription/translation, recombinational cloning, or for transforming or transfecting cells. Thus versatile compositions and methods for manipulating nucleic acids are described herein.

Described herein are methods for linking nucleic acid molecules using topoisomerase and recombination. In particular embodiments of the invention, nucleic acid molecules undergo one or more (*e*.*g*., one, two, three, four, five, six, seven, eight, nine, ten, etc.) recombination reactions and are then linked to one or more (*e.g.,* one, two, three, four, five, six, seven, eight, nine, ten, etc.) other nucleic acid molecules by methods involving covalent linking of strands catalyzed by one or more (*e*.*g*., one, two, three, four, etc.) topoisomerases. In other embodiments, nucleic acid molecules are linked to other nucleic acid molecules by methods involving covalent linking of strands catalyzed by one or more (*e*.*g*., one, two, three, four, etc.) topoisomerases and then undergo one or more (*e*.*g*., one, two, three, four, five, six, seven, eight, nine, ten, etc.) recombination reactions. As one skilled in the art would recognize, the disclosure herein is not tied to any particular order of topoisomerase-mediated linkage of nucleic acid molecules or recombination reactions. Thus, in general, the disclosure herein is directed to compositions and methods for performing both recombination reactions and linking nucleic acid segments using topoisomerases.

Also described herein are adapter-linker molecules for use in accordance with the methods and compositions described herein. The adapter linkers that are provided by, and that may be used in connection with, the disclosure herein can contain both a topoisomerase site and a recombination site. One example of a process described herein is set out schematically in Figure 35. Figure 35 shows a process which involves the connection of a topoisomerase-adapted nucleic acid segment ("adapter linker") which contains a single recombination site to another nucleic acid segment, referred to as an insert. These two nucleic acid segments may be connected by any topoisomerase-mediated process described herein.

Adapter linkers described herein may comprise (1) one or more recombination sites and/or (2) one or more topoisomerase recognition sites or one or more topoisomerases. In particular embodiments, at least one of the one or more recombination sites of the adapter linkers will be located within zero, one, two, three, four, five, six, seven, eight, nine, ten, fifteen, or twenty nucleotides of at least one of the one or more topoisomerase recognition site or one or more topoisomerase. Recombination sites present in adapter linkers described herein may be *att*L, *att*B, *att*P, or *att*L recombination sites. The topoisomerase recognition sites recognition may be recognition sites for type IB topoisomerases, type IA topoisomerases or type II topoisomerases, or the topoisomerases are type IB topoisomerases, type IA topoisomerases or type II topoisomerases. In addiction, topoisomerase recognition sites or topoisomerases may be located, with respect to recombination sites, in adapter linkers described herein such that upon recombination, particular recombination sites become associated with the product molecules. For example, a topoisomerase recognition site may be located on either end of an *att*L site in an adapter linker such that when the linker is attached to a nucleic acid molecule and recombination occurs, either an *att*B or an *att*P site is generated on the nucleic acid molecule to which the adapter linker was attached. Thus, adapter linkers may contain toposiomerase recognition sites and/or topoisomerases positioned, with respect to recombination sites, such that upon ligation to a nucleic acid molecule and recombination any number of variations of recombination sites are present on the product nucleic acid molecules. Examples of such recombination sites include *att*L, *att*B, *att*P, and *att*R recombination sites.

Described herein are methods for linking any number of nucleic acid segments using adapter linkers which contain recombination sites having the same or different specificities, as well as adapter linkers which contain recombination sites having the same or different specificities and kits which contain such adapter linkers. For example, three separate PCR products, referred to as segments A, B, and C, may be linked to adapter linkers such that *att*L1 and *att*L3 sites are present at the ends of segment A, *att*R3 and *att*R4 sites are present at the ends of segment B, and *att*L4 and *att*L2 sites are present at the ends of segment C. Thus, upon recombination with a linearized vector which contains *att*R1 and *att*R2 recombination sites at or near the termini, all three PCR products are joined to each other and inserted into the vector to generate a circularized nucleic acid molecule. Any number of variations of the above are possible.

Also described herein are sets of two or more (*e*.*g*., two, three, four, five, six, seven eight, nine, etc.) adapter linkers which contain (1) one or more recombination sites having the same or different specificities and/or (2) one or more topoisomerases or toposiomerase recognition sites, as well as methods for using these sets of adapter linkers to generate nucleic acid molecules which contain one or more recombination sites, compositions comprising such adapter linker sets or individual member of these sets, nucleic acid molecules which have been adapted with one or more adapter linkers of these sets, and methods for using these nucleic acid molecules.

After topoisomerase-mediated assembly, the assembled nucleic acid molecule may be recombined with another nucleic acid segment which contains one or more (*e*.*g*., one, two, three, four, etc.) suitable recombination sites. The recombination sites shown in Figure 35 are *att*L1 and *att*R1 sites but any suitable recombination sites may be used (*e.g*., *lox* sites, *att*R sites, *att*L sites, *att*B sites, *att*P sites, etc.). Additional suitable recombination sites are described elsewhere herein.

Described herein are methods for generating nucleic acid molecules using topoisomerase recognition sites and recombination sites with recombine with each other. Also described herein are nucleic acid molecules prepared by and used in methods described herein, as well as methods for using nucleic acid molecules generated by methods described herein.

Described herein are methods for generating nucleic acid molecules using multiple (*e*.*g*., two, three, four, five, six, seven, eight, nine, ten, etc.) recombination sites and topoisomerase recognition sites, as well as nucleic acid molecules prepared by and used in such methods. Further, these recombination sites may have multiple (*e*.*g*., two, three, four, five, six, seven, eight, nine, ten, etc.) specificities. In addiction, the topoisomerase recognition sites may be designed to generate termini which will result in the connection of these termini to different nucleic acid segments. For example, these termini may be designed to generate different "sticky ends" upon cleavage with a topoisomerase.

Another example of methods described above is shown in Figure 36. Figure 36 shows a process in which two nucleic acid segments are connected using a process which involves topoi somerase-mediated covalent linkage of strands of the termini of the nucleic acid segments. The resulting nucleic acid molecule then undergoes recombination, which results in (1) the topoisomerase assembled nucleic acid molecule becoming linked to a nucleic acid segment which contains an origin of replication and (2) replacement of a negative selection marker (*e*.*g*., a *ccd*B gene) with a promoter. The recombined nucleic acid product is then connected to a nucleic acid segment which is topoisomerase adapted at both termini and contains a positive selection marker. This last step results in the nucleic acid molecule being circularized.

The circularized nucleic acid end product shown in Figure 36 may be introduced into host cells, which may be prokaryotic (*e*.*g*., bacterial) or cukaryotic (*e*.*g.,* yeast, plant, animal (including mammalian, such as human)) cells such as those described elsewhere herein. Further, cells which contain this end product can be selected for using positive and negative selection. Thus, for example, cells which have acquired a nucleic acid molecule wherein the negative selection marker has not been replaced by the promoter will be selected against. Also described herein are methods and compositions similar to those set out in Figures 35 and 36 in which any number of the steps and components are varied. Examples of steps and components which may be varied are described elsewhere herein. Also described herein are methods for using nucleic acid molecules generated by methods described above.

As one skilled in the art would recognize, nucleic acid segments used in processes such as those shown in Figures 35 and 36 could contain any number of different elements. For example, a positive selection marker could be substituted for the promoters shown in Figure 36. Further, the insert shown in Figure 35 may contain nucleic acid which has any number of functionalities. In particular, when the insert contains a regions which is transcribed, the transcript can be a mRNA or an RNA which serves a function in the absence of translation. Examples of RNA which serves a function in the absence of translation include transfer RNAs (*e*.*g*., suppressor tRNAs), antisense RNAs, ribosomal RNAs, and ribozymes. Additionally, more than one of the nucleic acid segments connected and/or recombined by methods ef as described herein may contain all or part of one or more (*e*.*g*., one, two, three, four, five, six, seven, etc.) open reading frames. In such instances, nucleic acid segments may be connected to each other such that transcription and translation result in the production of one or more fusion proteins. Additional nucleic acid elements which can be used in methods described herein are described elsewhere herein.

Once a nucleic acid molecule, such as the end product of the process shown in Figure 35, has been generated by methods described herein, the nucleic acid molecule may optionally be connected to one or more (*e*.*g*., one, two, three, four, etc.) other nucleic acid molecules or may be circularized by joining of the termini to each other. Further, when three or more nucleic acid molecules are connected to each other by methods described herein, the termini of various intermediate molecules or the end product may be joined to each other to circularize these molecules.

Described herein are compositions and methods for performing homologous recombination and for producing transgenic non-human animals. Gene targeting by homologous recombination between an exogenous DNA construct and cognate chromosomal sequences allows precise modifications to be made at predetermined sites in the genome. Gene targeting is well-established in, *e*.*g*., mouse embryonic stem (ES) cells, and has been used to effect modifications in a large number of murine genes. (*See e*.*g*., Brandon et al., Curr. Biol. 5:625-634,758-765, 873-881 (1995)). Gene targeting can also be accomplished in somatic cells. (*See e*.*g*., Itzhaki et al., Nat. Genet. 15:258-265 (1997)). Cells that have been modified by gene targeting via homologous recombination can then be manipulated by methods known in the art to establish transgenic non-human animals.

One example of a composition that can be used in homologous recombination applications is the end product nucleic acid molecule set out in Figure 37. Figure 37 further shows an example of a method for preparing such compositions. In particular, Figure 37 shows the linkage of topoisomerase adapted nucleic acid segments to a non-topoisomerase adapted nucleic acid segment. In this instance, the nucleic acid segment which the designer of the nucleic acid end product seeks to integrate into a chromosome, referred to here as an insert, is flanked by regions which contain (1) a positive selection marker and (2) a negative selection marker positioned between two recombination sites. Recombination may then be used to replace the two negative selection markers with nucleic acid having homology to a chromosomal region into which the end product is to integrate (labeled "HR1" and "HR2" in Figure 37).

Regions of homology used in the practice of the disclosure herein will vary with the chromosomes of cells into which nucleic acid molecules are to integrate. Further, in many instances, regions of homology will be selected to facilitate integration into cells of a particular organism. Such an organism may be unicellular organism (*e*.*g*., a yeast, a protozoan, etc.) or multicellular organism (*e*.*g*., a plant, an animal, etc.).

Described herein are nucleic acid molecules and compositions for performing homologous recombination and cells produced via homologous recombination involving these molecules and compositions. Methods described herein can be used in the linking of multiple nucleic acid segments. Figure 38, for example, shows a schematic representation of the linking of four nucleic acid segments using toposiomerase to generate a linear nucleic acid molecule with recombination sites (labeled "L1" and "L2") located near the termini. In the first step, topoisomerase adapted nucleic acid segment which contains an *att*L1 recombination site and an *att*L2 recombination site are linked to two other nucleic acid segments using topoisomerase. In this particular instance, each strand of the termini which are joined to each other is covalently linked to a topoisomerase molecule. Thus, upon toposiomerase mediated linkage of the nucleic acid strands, no nicks are present at the junction points. In the second step, the topoisomerase assembled nucleic acid segments are contacted with another nucleic acid segment which contains an origin of replication (labeled "ori"), a positive selection marker (labeled "PM"), an *att*R1 recombination site, and an *att*R2 recombination site in the presence of LR CLONASE™ under conditions which allow for recombination between the *att*L and *att*R recombination sites. In certain such methods, for example, TOPO-adapted vectors are incubated with one or more nucleic acid segments (*e*.*g.,* one or more PCR products) at room temperature (*e*.*g*., about 20-20°C) for about 5-30 (and preferably about 10) minutes; the reaction is then heat-treated by incubation at about 80°C for about 20 minutes, and the reaction mixture then used in a standard LR reaction according to manufacturer's instructions (Invitrogen Corporation), except the incubation time for the LR reaction is increased to about 3 hours. Recombination results in the formation of a circular nucleic acid molecule which contains the various starting nucleic acid segments separated from the origin and selection marker by *att*B1 and *att*B2 recombination sites. As one skilled in the art would recognize, any suitable recombination sites could be used in place of the *att* recombination sites shown in this figure. Also described herein are compositions comprising such nucleic acids, compositions used for producing such nucleic acids, and uses of such nucleic acids and compositions in the recombination and topoisomerase-mediated joining methods described elsewhere herein.

Described herein are nucleic acid molecules suitable for performing cloning reactions in which a first nucleic acid molecule, which shares one or more region of homology with a second nucleic acid molecule, is used to insert nucleic acid from the second nucleic acid molecule into the fist nucleic acid molecule. Also described herein are compositions and methods for performing such cloning reactions.

One example of a process referred to above is RecE/T cloning, which is described in PCT Publication WO 01/04288. Typically, in RecE/T cloning, a linear first nucleic acid molecule (*e*.*g*., a vector) is introduced into a cell which contains (1) regions at the termini that share homology with two separate, nearby regions (*e*.*g*., nucleic acid regions which are about 20 to about 30, about 20 to about 40, about 20 to about 50, about 30 to about 40, about 40 to about 50, about 40 to about 60, about 40 to about 80, about 50 to about 90, etc. nucleotides in length) of a nucleic acid molecule present in the cell (*e*.*g*., a plasmid, a bacterial artificial chromosome, a natural chromosome, etc.), referred to here as "a second nucleic acid molecule", (2) a selection marker, and (3) an origin of replication. The linear first nucleic acid molecule will generally only replicate if it becomes circularized. Further, the first nucleic acid molecule will typically become circularized when it has undergone recombination with the second nucleic acid molecule and acquired nucleic acid from the second nucleic acid molecule which is intervening between the regions of homology. In such embodiments, the regions of homology in the first nucleic acid molecule will typically be in a reverse orientation as compared to the second nucleic acid molecule. Generally, the cell in which recombination occurs will be one which expresses a recombinase such as RecE/T or RedAlpha/Beta. Thus described herein are in part, methods for performing RecE/T cloning, nucleic acid molecules prepared by such methods, compositions comprising such nucleic acid molecules, and methods for using such nucleic acid molecules and compositions.

Modifications of the RecE/T process may be employed to generate a number of different end products. For example, when the regions of homology are arranged in various ways, the first nucleic acid molecule can be designed to (1) insert into the second nucleic acid molecule, or (2) delete nucleic acid from the second nucleic acid molecule. Typically, when insertion of the second nucleic acid molecule into the second nucleic acid molecule is desired, the regions of homology of the first nucleic acid molecule will be in the same orientation with respect to the regions of homology in the second nucleic acid molecule. Further, when deletion of nucleic acid from the second nucleic acid molecule is desired, the regions of homology of the first nucleic acid molecule will generally be in an inverse orientation with respect to the regions of homology in the second nucleic acid molecule. Also, when insertion of the first nucleic acid molecule into the second nucleic acid molecule is desired, typically the first nucleic acid molecule will not contain an origin of replication. Described herein are methods for performing the above processes. Also described herein are nucleic acid molecules and compositions for use in the above processes.

The disclosure herein can also be used to link two nucleic acid segments in a single step process using topoisomerase and recombination sites to generate a circular nucleic acid molecule. An example of this variant is depicted in Figure 39 where one of the nucleic acid segments contains an *att*L1 recombination site (labeled "L1"), a promoter (labeled "P"), and toposiomerase molecule covalently linked to one terminus. The other nucleic acid segment contains an *att*R1 recombination site (labeled "R1"), an open reading frame (labeled "ORF"), an origin of replication (labeled "ORI"), a positive selection marker (labeled "PM"), and topoisomerase molecule covalently linked to one terminus. Thus, when these two nucleic acid segments are contacted with each other in the presence of LR CLONASE™ under conditions which allow for recombination between the *att*L and *att*R recombination sites and topoisomerase mediated linkage of nucleic acid strands, a circular molecule is formed having the structure indicated. In certain such methods, for example, TOPO-adapted vectors are incubated with one or more nucleic acid segments (*e*.*g.,* one or more PCR products) at room temperature (*e.g.,* about 20-20°C) for about 5-30 (and preferably about 10) minutes; the reaction is then heat-treated by incubation at about 80°C for about 20 minutes, and the reaction mixture then used in a standard LR reaction according to manufacturer's instructions (Invitrogen Corporation), except the incubation time for the LR reaction is increased to about 3 hours. As one skilled in the art would recognize, any suitable recombination sites could be used in place of the *att* recombination sites shown in this figure.

The disclosure herein can also be used to link two nucleic acid segments using toposiomerase mediated methods to generate a circular nucleic acid molecule. A schematic representation of one embodiment of this aspect of the invention is illustrated in Figure 40. As shown in Figure 40, the circular molecule contains an open reading frame (labeled "ORF") positioned between *att*L1 and *att*L2 recombination site (labeled "L1" and "L2"). The topoisomerase assembled product then undergoes recombination with another circular molecule which contains *att*R1 and *att*R2 recombination sites to generate a third circular nucleic acid molecule which contains the open reading frame positioned between *att*B1 and *att*B2 recombination sites. Further, the open reading frame is operably linked to a promoter. Thus, the final nucleic acid molecule produced by this process is an expression construct. As one skilled in the art would recognize, any suitable recombination sites could be used in place of the *att* recombination sites shown in this figure.

As disclosed herein, a first nucleic acid molecule can be one of a plurality of nucleotide sequences, for example, a cDNA library, a combinatorial library of nucleotide sequences, or a population of variegated nucleotide sequences. As such, a particularly useful embodiment described herein is in generating recombinant polynucleotides encoding chimeric polypeptides for performing a high throughput two hybrid assay for identifying protein-protein interactions that occur among populations of polypeptides (see U.S. Pat. No. 6,057,101 and U.S. Pat. No. 6,083,693). In such a method, two populations (pluralities) of nucleotide sequences encoding polypeptides are examined, each plurality having a complexity of from a few related but different nucleotide sequences to as high as tens of thousands of such sequences. By performing a method described herein, for example, using a PCR primer pair to amplify each nucleotide sequence in the plurality, wherein at least one primer of the PCR primer pair comprises (a) at least one topoisomerase recognition site or complement thereof or (b) at least one recombination site, covalently linked recombinant polynucleotides encoding a population of chimeric bait polypeptides and a population of chimeric prey polypeptides readily can be generated by contacting the amplified pluralities of nucleotide sequences, each of which comprises (a) at least one topoisomerase recognition site, with at least one topoisomerase and a nucleotide sequence, which contains at least one topoisomerase recognition site and encodes a transcription activation domain or a DNA binding domain or (b) at least one recombination site site, with at least one topoisomerase and a nucleotide sequence, which contains at least one recombination site and encodes a transcription activation domain or a DNA binding domain.

In practicing a method described herein, a first nucleic acid molecule also can encode a ribonucleic acid (RNA) molecule, which can function, for example, as a riboprobe, an antisense nucleotide sequence, a ribozyme, or a triplexing nucleotide sequence, or can be used in an *in vitro* translation reaction, and the second nucleic acid molecule can encode a regulatory element useful for expressing an RNA from the first nucleotide sequence (see Example 2.A). For example, where it is desired to produce a large amount of RNA, a second nucleic acid molecule component for performing a method described herein can comprise an RNA polymerase promoter such as a T7, T3 or SP6 RNA polymerase promoter. Where the RNA molecule is to be expressed in a cell, for example, an antisense molecule to be expressed in a mammalian cell, the second (or other) nucleic acid molecule can include a promoter that is active in a mammalian cell, particularly a tissue specific promoter, which is active only in a target cell. Furthermore, where the RNA molecule is to be translated, for example, in a coupled *in vitro* transcription/translation reaction, the first nucleotide sequence or second (or other) nucleotide sequence can contain appropriate translational regulatory elements (see Example 2.B).

Methods described herein may also be used to produce constructs which allow for silencing of genes *in vivo*. One method of silencing genes involves the production of double-stranded RNA, termed RNA interference (RNAi). (*See, e*.*g*., Mette et al., EMBO J., 19:5194-5201 (2000)). The mechanism by which RNAi is believed to function, which is reviewed in Fjose et al., Biotechnol. Annu. Rev. 7:31-57 (2001), appears to be based on the ability of double stranded RNA to induce the degradation of specific RNA molecules. This mechanism is reported to involve the conversion of double-stranded RNA into short RNAs that direct ribonucleases to homologous RNA targets (*e*.*g*., mRNA targets). Methods described herein can be used in a number of ways to produce molecules such as RNAi. Thus, expression products of nucleic acid molecules described herein can be used to silence gene expression.

One example of a nucleic acid molecule designed to produce RNAi is a molecule in which a nucleic acid segment is linked to one or more promoters such that RNA corresponding to both strands are produced as two separate transcripts or as part of the same transcript For example, a nucleic acid molecule could be prepared using methods described herein wherein two copies of an open reading frame are connected by an intervening nucleic acid segment with two promoters that drive transcription in different directions. Thus, one of the promoters drives transcription of sense strand mRNA and the other promoter drives transcription of antisense mRNA. Another example of a nucleic acid molecule which could be used to produce RNAi is one in which an open reading frame is flanked on each end by promoters which drive transcription of the open reading frame in opposing directions. As a third example, doubles stranded RNA can be produced from a nucleic acid molecule which encode RNA having a "snapback" region (*e*.*g*., a region that is six, seven, eight, nine ten, etc. nucleotides in length) at one terminus. Thus, an RNA transcript of this type will form a hairpin turn at or near one terminus. When such an RNA molecule is incubated, under appropriate conditions, in the presence of an RNA dependent RNA polymerase, the double stranded region formed by the hairpin can be used to prime second strand synthesis to form double stranded RNA molecule.

Nucleic acid segments designed to produce RNAi, such as the nucleic acid molecules described above, need not correspond to the full-length gene or open reading frame. For example, when the nucleic acid segment corresponds to all or part of an ORF or encode an RNA molecule which does not correspond to all or part of an ORF, the segment may only correspond to part of the ORF (*e*.*g*., about 15, about 16, about 17, about 18, about 19, about 20, about 21, about 22, about 23, about 24, about 25, about 26, about 27, about 28, about 29, about 30, about 40, about 50, about 60, etc. nucleotides at the 5' or 3' end of the ORF).

Thus, described herein are methods for preparing nucleic acid molecules comprising at least three segments. In some embodiments, at least two of these segments share at least one region of sequence identity (*e*.*g*., a region at least about 15, at least about 16, at least about 17, at least about 18, at least about 19, at least about 20, at least about 21, at least about 22, at least about 23, at least about 24, at least about 25, at least about 26, at least about 27, at least about 28, at least about 29, at least about 30, at least about 40, at least about 50, at least about 60, at least about 70, at least about 80, at least about 90, at least about 100 nucleotides, etc. nucleotides in length). In other embodiments, one nucleic acid segment is flanked by region which can confer transcription of the interior portion of the molecule in opposing directions (*e*.*g*., to produce sense and antisense transcripts). Also described herein are nucleic acid molecules prepared by methods described herein and the use of such molecules to either inhibit gene expression or facilitate the degradation of specific RNA molecules.

Also described herein are methods for preparing nucleic acid molecules which can be used to express antisense RNA (*e*.*g*., antisense mRNA). Methods similar to those described above for the production of nucleic acid molecules which can be used for RNAi may be employed; however, only the antisense strand will typically be transcribed in molecules prepared by methods described herein which may be used to generate antisense RNA.

In related embodiments, promoters which drive transcription of the sense RNA or antisense RNA can be either constitutive (e.g., CMV promoter, SV40 promoter, etc.), inducible (e.g., a metallothionein promoter, etc.), or repressible. Thus, for example, two different inducible promoters can be used to drive transcription of sense RNA and antisense RNA. In such an instance, promoter activation can be used to induce production of sense RNA, antisense RNA, or both sense RNA and aritisense RNA. Further, the amount of sense RNA and/or antisense RNA produced can be related by using, for example, graduated induction and/or derepression of the promoters.

Gene silencing methods involving the use of compounds such as RNAi and antisense DNA, for examples, are particularly useful for identifying gene functions. More specifically, gene silencing methods can be used to reduce or prevent the expression of one or more genes in a cell or organism. Phenotypic manifestations associated with the selective inhibition of gene functions can then be used to assign role to the "silenced" gene or genes. As an example, Chuang et al., Proc. Natl. Acad. Sci. (USA) 97:4985-4990 (2000), have demonstrated that *in vivo* production of RNAi can alter gene activity in *Arabidopsis thaliana.* Thus, described herein are methods for regulating expression of nucleic acid molecules in cells and tissues comprising the expression of RNAi and antisense RNA. Also described herein are methods for preparing nucleic acid molecules which can be used to produce RNA corresponding to one or both strands of a DNA molecule.

Described herein are methods for regulating expression of nucleic acid molecules *in vivo* (e.g., in cells and tissues) and/or *in vitro* comprising the expression of sense RNA and/or antisense RNA. Also described herein are methods for preparing nucleic acid molecules which can be used to produce RNA corresponding to one or both strands of a nucleic acid molecule (e.g., a DNA molecule). Also described herein are compositions for performing the methods described above and nucleic acid molecules produced by the above methods (e.g., RNA and DNA molecules).

Described herein are compounds and methods for gene silencing involving ribozymes. In particular, antisense RNA/ribozymes fusions, which comprise 1) antisense RNA corresponding to a target gene and 2) one or more ribozymes that cleave RNA (e.g., hammerhead ribozyme, hairpin ribozyme, delta ribozyme, *Tetrahymena* L-21 ribozyme, etc.). Also described herein are vectors that express such fusions, methods for producing such vectors, and methods for using such vector to suppress gene expression.

Expression of antisense molecules fused to ribozymes can be used, for example, to cleave specific RNA molecules in a cell because the antisense RNA portion of the transcript can be designed to hybridize to particular mRNA molecules. Further, the ribozyme portion of the transcript can be designed to cleave the RNA molecule to which it has hybridized. For example, the ribozyme can be one which cleaves double stranded RNA (e.g., a *Tetrahymena* L-21 ribozyme).

Methods described herein can be particularly useful for generating an expressible ds recombinant nucleic acid molecule that can be inserted in a site specific manner into a target DNA sequence. The target DNA sequence can be any DNA sequence, particularly a genomic DNA sequence, and preferably a gene for which some or all of the nucleotide sequence is known. The method can be performed utilizing a first nucleic acid molecule, which has a first end and a second end and encodes a polypeptide, for example, a selectable marker, wherein the first nucleic acid molecule comprises at least one topoisomerase recognition site and/or at least one recombination site or cleavage product thereof at the 3' terminus of each end and, optionally, a hydroxyl group at the 5' terminus of each end, and wherein, preferably, the 5' termini comprise 5' overhanging sequences, which are different from each other; and covalently linking the first nucleic acid molecule to first and second PCR amplification products according to a method described herein. The first and second amplification products are generated from sequences upstream and downstream of the site at which the construct is to be inserted, and each amplification product contains at least one topoisomerase recognition site and optionally at least one recombination site, preferably, a 5' overhanging sequence, which is generated following contact with the site specific topoisomerase. Preferably, the first and second amplification products have different 5'overhanging sequences such that each can be linked to a predetermined end of the first nucleic acid molecule. Such a method similarly can be performed using a ds amplification product comprising at least one topoisomerase recognition site and, optionally, at least one recombination site, or cleavage product thereof, at the 5' terminus of one or both ends, wherein, upon cleavage by the topoisomerase, the topoisomerase-charged molecule can comprise a 3' overhang at one or both ends containing the topoisomerase. In addition, the method can be performed using a ds amplification product comprising topoisomerase recognition sites and, optionally, recombination sites, or cleavage products thereof, at or near the 5' terminus and the 3'terminus of one or both ends, wherein, upon cleavage by the topoisomerases, the topoisomerase-charged nucleic acid molecule preferably contains a 5' or 3' overhang at one or both ends containing the topoisomerases. Once nucleic acid molecules are joined by the methods described above, the resulting molecules may then be used in recombination reactions, such as those described elsewhere herein.

The first and second amplification products may be generate using two sets of PCR primer pairs. The two sets of PCR primer pairs may be selected such that, in the presence of an appropriate polymerase such as Taq polymerase and a template comprising the sequences to be amplified, the primers amplify portions of a target DNA sequence that are upstream of and adjacent to, and downstream of and adjacent to, the site for insertion of the selectable marker. In addition, the sets of PCR primer pairs may be designed such that the amplification products contain a topoisomerase recognition site and, following cleavage by the site specific topoisomerase, a 5' overhanging sequence at the end to be covalently linked to the selectable marker. As such, the first PCR primer pair includes 1) a first primer, which comprises, in an orientation from 5' to 3', a nucleotide sequence complementary to a 5' overhanging sequence of the end of the selectable marker to which the amplification product is to be covalently linked, a nucleotide sequence complementary to a topoisomerase recognition site, and a nucleotide sequence complementary to a 3' sequence of a target DNA sequence upstream of the insertion site; and 2) a second primer, which comprises a nucleotide sequence of the target genomic DNA upstream of the 3' sequence to which the first primer is complementary, i.e., downstream of the insertion site. The second PCR primer pair includes 1) a first primer, which comprises, from 5' to 3', a nucleotide sequence complementary to the 5' overhanging sequence of the end of the selectable marker to which it is to be covalently linked, a nucleotide sequence complementary to a topoisomerase recognition site, and a nucleotide sequence of a 5' sequence of a target DNA sequence, wherein the 5' sequence of the target genomic DNA is downstream of the 3'sequence of the target DNA sequence to which the first primer of the first PCR primer pair is complementary; and the second primer of the second primer pair comprises a nucleotide sequence complementary to a 3'sequence of the target DNA sequence that is downstream of the 5' sequence of the target genomic DNA contained in the first primer. The skilled artisan will recognize that the sequences of the primer that are complementary to the target genomic DNA are selected based on the sequence of the target DNA. These priers may further comprise one or more recombination sites.

Upon contact of the nucleic acid molecule comprising the selectable marker, the first and second amplification products, and a topoisomerase (if the molecules are not topoisomerase-charged), a ds recombinant nucleic acid molecule covalently linked in both strands is generated according to a method described herein. The generated ds recombinant nucleic acid molecule can be further amplified, if desired, using PCR primers that are specific for an upstream and downstream sequence of the target genomic DNA, thus ensuring that only functional constructs are amplified. The generated ds recombinant nucleic acid molecule is useful for performing homologous recombination in a genome, for example, to knock-out the function of a gene in a cell, or to confer a novel phenotype on the cell containing the generated recombinant nucleic acid molecule. The method can further be used to produce a transgenic non-human organism having the generated ds recombinant nucleic acid molecule stably maintained in its genome.

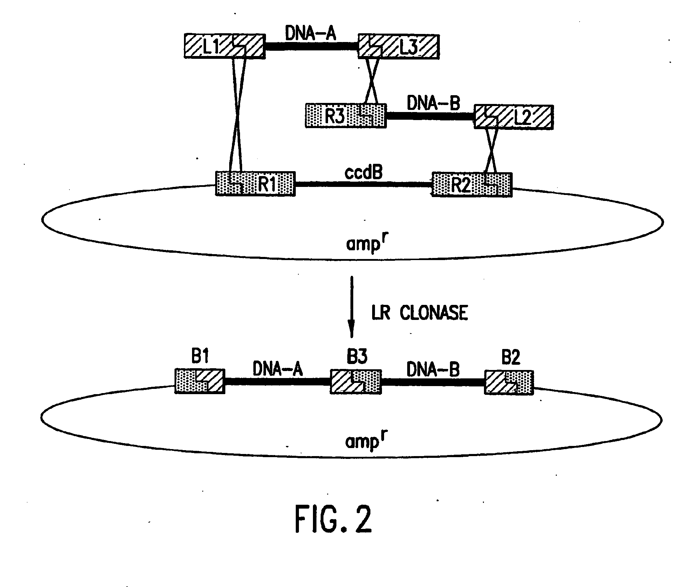

Described herein is a method which is also useful for covalently linking, an adapter or linker sequence to one or both ends of a nucleic acid molecule of interest, including to each of a plurality of nucleic acid molecules. For example, where it is desired to put linkers on both ends of a first nucleic acid molecule, the method can be performed by contacting a topoisomerase with a first nucleic acid molecule, which has a topoisomerase recognition site, or cleavage product thereof, at one or both 3' or 5' termini and which can include hydroxyl groups at both 5' termini and one or more recombination sites; and a second nucleic acid molecule and at least a third double stranded nucleotide sequence, each of which can include a topoisomerase recognition site, or cleavage product thereof at the appropriate 3' or 5' terminus and which can also include, where desirable, a 5' hydroxyl group at the same terminus and one or more recombination sites. An appropriate terminus is the terminus to which the linker is to be covalently linked in at least one strand to the first nucleotide sequence. In one embodiment, one or both linker sequences contain an overhanging sequence that is complementary to a sequence at the 5' terminus of the end of the first nucleic acid molecule to which the linker is to be covalently linked, thereby facilitating the initial association of the nucleotide sequences in the proper (predetermined) orientation (see, for example, Figure 2 and Example 1.B). In performing such a method, the linker sequences comprising the second and at least third nucleotide sequence can be the same or different.

Figure 14 shows one example of a process for preparing a nucleic acid molecule containing a topoisomerase (e.g., a type IA topoisomerase) bound to the 5' terminus of one end of the sequence, and wherein the same end further comprise a 3' overhang (see (4) in Figure 14). In step A, a nucleotide sequence to be modified with topoisomerase is digested with a restriction enzyme that generates a "sticky" end. The restricted nucleotide sequence is then contacted in step B with a linear, single stranded nucleotide sequence which contains a topoisomerase attached the 5' terminus and a ligase (e.g., a DNA ligase such as T4 DNA ligase). The linear, single stranded nucleotide sequence also contains a region at the 3' terminus which shares sufficient sequence complementarity to the "sticky" end generated by the restriction enzyme, such that the two molecules will hybridize. Thus, in step B, the two nucleotide sequences are ligated to each other. In step C, the product of the second step is contacted with a third nucleotide sequence which shares sequence complementarity to portions of the linear, single stranded nucleic acid molecule generated in step B, and a ligase. The product of step C, shown in (4), is a nucleic acid molecule containing a topoisomerase attached to the 5' terminus of one end and a 3' overhang on the same end. It will be recognized that numerous variations of the exemplified method are described herein. For example, similar processes can be performed to prepare nucleic acid molecules which comprise topoisomerase attached to the 3' terminus of one end or which have a 5' overhang or are blunt ended at the end to which a topoisomerase is attached. In another example, the nucleotide sequence labeled number 3 in Figure 14 can be produced in the following manner: a nucleic acid molecule can be digested with a restriction enzyme to generate a nucleic acid molecule with a single-stranded 5' overhang that includes a type IA topoisomerase recognition site. The nucleic acid molecule with the single stranded overhang can then be contacted with type IA topoisomerase to generate a type IA topoisomerase-charged nucleic acid molecule.

Figure 15 shows two illustrations of the disclosure herein, wherein single stranded or double stranded DNA is covalently linked to single stranded RNA. Where single stranded DNA is joined to single stranded RNA, the 3' end of the ribonucleotide sequence is covalently linked to the 5' end of the deoxyribonucleotide sequence. Where double stranded DNA is joined to single stranded RNA, the 3' terminus of the ribonucleotide sequence shares sufficient sequence complementarity to the 3'overhang of the deoxyribonucleotide sequence such that the two molecules hybridize. As above, the 3' end of the ribonucleotide sequence is also covalently linked to the 5' end of the deoxyribonucleotide sequence. As will be recognized, numerous variations of the above are within the scope of the disclosure herein. For example, the RNA molecule can be double stranded. In another example, all of the nucleotide sequences can be deoxyribonucleotide sequences and/or can comprise one or more recombination sites.

Described herein is a ds recombinant nucleic acid molecule having, or which can be made to have, a first end and a second end, each end including a 5' terminus and a 3' terminus, wherein the vector comprises a site-specific type IA topoisomerase recognition site at or near a 5' terminus of the first end, the second end, or both the first end and the second end. The ds recombinant nucleic acid molecule can further include a type IB topoisomerase recognition site at or near a 3' termini of an end that does not include a type IA topoisomerase recognition site. The ds recombinant nucleic acid molecule can be a vector.

Also described herein is a topoisomerase-charged ds recombinant nucleic acid molecule having a first end and a second end, each end having a 5' terminus and a 3' terminus, wherein a site-specific type IA topoisomerase is bound at the 5' terminus of the first end, the second end, or both the first end and the second end. For example, the topoisomerase-charged ds recombinant nucleic acid molecule can include a type IA topoisomerase bound at the 5' termini of each of the first and second ends. The topoisomerase-charged nucleic acid ds recombinant nucleic acid molecule can include a type IB topoisomerase bound at a 3'termini of an end not bound by a type IA topoisomerase. The topoisomerase-charged ds recombinant nucleic acid molecule can be a vector.

### KITS

Described herein are kits, which contain components useful for conveniently practicing the methods described herein. A kit described herein may contain a first nucleic acid molecule, which encodes a polypeptide, particularly a selectable marker, and contains a topoisomerase recognition site at each end. Preferably, the first nucleotide sequence comprises a topoisomerase-activated nucleotide sequence. More preferably, the topoisomerase-charged first nucleotide sequence comprises a 5' overhanging sequence at each end, and most preferably the 5' overhanging sequences are different from each other. Optionally, each of the 5' termini comprises a 5' hydroxyl group.

In addition, the kit can contain at least a nucleotide sequence (or complement thereof) comprising a regulatory element, which can be an upstream or downstream regulatory element, or other element, and which contains a topoisomerase recognition site at one or both ends. Preferably, the kit contains a plurality of nucleic acid molecules, each comprising a different regulatory element or other element, for example, a sequence encoding a tag or other detectable molecule or a cell compartmentalization domain. The different elements can be different types of a particular regulatory element, for example, constitutive promoters, inducible promoters and tissue specific promoters, or can be different types of elements including, for example, Transcriptional and translational regulatory elements, epitope tags, and the like. Such nucleic acid molecules can be topoisomerase-activated, and can contain 5' overhangs or 3' overhangs that facilitate "operatively covalently linking the elements in a predetermined orientation, particularly such that a polypeptide such as a selectable marker is expressible *in vitro* or in one or more cell types.

The kit also can contain primers, including first and second primers, such that a primer pair comprising a first and second primer can be selected and used to amplify a desired ds recombinant nucleic acid molecule covalently linked in one or both strands, generated using components of the kit. For example, the primers can include first primers that are complementary to elements that generally are positioned at the 5' end of a generated ds recombinant nucleic acid molecule, for example, a portion of a nucleic acid molecule comprising a promoter element, and second primers that are complementary to elements that generally are positioned at the 3' end of a generated ds recombinant nucleic acid molecule, for example, a portion of a nucleic acid molecule comprising a transcription termination site or encoding an epitope tag. Depending on the elements selected from the kit for generating a ds recombinant nucleic acid molecule covalently linked in both strands, the appropriate first and second primers can be selected and used to amplify a full length functional construct.

A kit described herein may contain a plurality of different elements, each of which can comprise one or more recombination sites and/or can be topoisomerase-activated at one or both ends, and each of which can contain a 5' overhanging sequence or a 3'overhanging sequence or a combination thereof. The 5' or 3' overhanging sequences can be unique to a particular element, or can be common to plurality of related elements, for example, to a plurality of different promoter element. Preferably, the 5' overhanging sequences of elements are designed such that one or more elements can be operatively covalently linked to provide a useful function, for example, an element comprising a Kozak sequence and an element comprising a translation start site can have complementary 5' overhangs such that the elements can be operatively covalently linked according to a method described herein.

The plurality of elements in the kit can comprise any elements, including transcription or translation regulatory elements; elements required for replication of a nucleotide sequence in a bacterial, insect, yeast, or mammalian host cell; elements comprising recognition sequences for site specific nucleic acid binding proteins such as restriction endonucleases or recombinases; elements encoding expressible products such as epitope tags or drug resistance genes; and the like. As such, a kit as described herein provides a convenient source of different elements that can be selected depending, for example, on the particular cells that a construct generated according to a method described herein is to be introduced into or expressed in. The kit also can contain PCR primers, including first and second primers, which can be combined as described above to amplify a ds recombinant nucleic acid molecule covalently linked in one or both strands, generated using the elements of the kit. Optionally, the kit further contains a site specific topoisomerase in an amount useful for covalently linking in at least one strand, a first nucleic acid molecule comprising a topoisomerase recognition site to a second (or other) nucleic acid molecule, which can optionally be topoisomerase-activated nucleic acid molecules or nucleotide sequences that comprise a topoisomerase recognition site.

A kit described herein may contain a first nucleic acid molecule, which encodes a selectable marker, and contains a topoisomerase recognition site and/or a recombination site at each end; a first and second PCR primer pair, which can produce a first and second amplification products that can be covalently linked in one or both strands, to the first nucleic acid molecule in a predetermined orientation according to a method described herein. Such a generated construct can be introduced into a cell and can incorporate into the genome of the cell by homologous recombination in a site specific manner, where it can be stably maintained and can express a heterologous polypeptide in the cell or can knock-out a target gene function. A target gene to be knocked-out, for example, can be any gene for which at least part of the sequence is known or can be readily determined and the function of which it is desired to disrupt, for example, an oncogene, a gene involved in apoptosis, a gene encoding a seine/threonine or a tyrosine kinase, or any other gene.

The first PCR primer pair in a kit described herein useful for generating a ds recombinant nucleic acid molecule covalently linked in both strands, includes a first primer that comprises, in an orientation from 5' to 3', a nucleotide sequence complementary to a 5' overhanging sequence of a nucleic acid molecule to which it is to be covalently linked (for example, an end of the nucleic acid molecule encoding the selectable marker), a nucleotide sequence complementary to a topoisomerase recognition site and/or a recombination site, and a nucleotide sequence complementary to a 3' sequence of the target DNA sequence. The first PCR primer pair also includes a second primer that comprises a nucleotide sequence of the target DNA sequence upstream of the 3' sequence to which the first primer is complementary.

The second PCR primer pair of a kit useful for generating a ds recombinant nucleic acid molecule covalently linked in both strands, includes a first primer that comprises, from 5' to 3', a nucleotide sequence complementary to a 5' overhanging sequence of a nucleic acid molecule to which it is to be covalently linked, a nucleotide sequence complementary to a topoisomerase recognition site and/or a recombination site, and a nucleotide sequence of a 5' sequence of the target DNA sequence, wherein the 5' sequence of the target gene is downstream of the 3' sequence of the target DNA sequence to which the first primer of the first primer pair is complementary. The second PCR primer pair also includes a second primer that comprises a nucleotide sequence complementary to a 3' sequence of the target gene that is downstream of the 5' sequence of the target DNA sequence contained in the first primer.

A kit described herein useful for generating a ds recombinant nucleic acid molecule covalently linked in both strands contains a first nucleic acid molecule, which encodes a transcription activation domain and comprises a topoisomerase recognition site, or cleavage product thereof, at a 3' terminus; and a second nucleic acid molecule, which encodes a DNA binding domain and comprises a topoisomerase recognition site and/or a recombination site, or cleavage product thereof, at a 3' terminus. Upon cleavage by the site specific topoisomerase, the first or second nucleic acid molecule can have a 5' overhang, or both sequences can have 5' overhangs, which are the same or are different from each other. Where the nucleic acid molecules have a 5' overhang, the overhang generally is complementary to a nucleic acid molecule to which first or second nucleic acid molecule is to be covalently linked according to a method described herein. The kit also can contain one or a pair of adapters, linkers or the like, which can comprise a topoisomerase recognition site, or cleavage product thereof, at one or both 3' termini, and, optionally, a hydroxyl group at the same terminus/termini. Such adapters, linkers, or the like are selected such that they contain a 5' overhang that is complementary to one or the other of the two nucleic acid molecules described above and part of the kit.

Similarly, a kit described herein can contain one or a pair of adapters, linkers or the like, which comprise a topoisomerase recognition site and/or a recombination site, or cleavage product thereof, at one or both 5' termini, and, optionally, a hydroxyl group at the same terminus (or termini). Such adapters, linkers, or the like are selected such that they contain a 3' overhang that is complementary to one or the other of the two nucleic acid molecules described above and part of the kit. In addition, the kit can contain one or a pair of adapters, linkers or the like, which comprise a topoisomerase recognition site, or cleavage product thereof, at one or both 5' and/or 3' termini, and, optionally, a hydroxyl group at the same terminus/termini.

Adapters, linkers, or the like generally are selected such that they contain a 5' and/or a 3' overhang that is complementary to one or the other of the two nucleic acid molecules as disclosed herein and part of the kit. Such adapters, linkers, or the like can be joined to the ends of nucleic acid molecules that are to covalently linked to one or the other of the first or second nucleic acid molecules provided with the kit, thus facilitating the construction of chimeric polynucleotides encoding the bait and prey polypeptides useful in a two hybrid assay. Such a kit also can contain a PCR primer or primer pair, which can be used to prepare an amplified plurality of nucleotide sequences comprising a topoisomerase recognition site, or cleavage product thereof (see Table 1 and Example 1).

A PCR primer pair in a kit described herein, which can be used for generating a ds recombinant nucleic acid molecule covalently linked in one strand, can include a first primer that comprises, in an orientation from 5' to 3', a nucleotide sequence of a 5' overhanging sequence of a nucleic acid molecule to which it is to be linked (for example, an end of the nucleic acid molecule encoding the selectable marker), a topoisomerase recognition site (e.g., a type IA or type II topoisomerase recognition site) and, optionally, a recombination site, and a nucleotide sequence complementary to a 5' sequence of the target DNA sequence. The PCR primer pair also includes a second primer that comprises a nucleotide sequence of the target DNA sequence downstream of the 5' sequence to which the first primer is complementary.

A kit described herein may contain a first nucleic acid molecule, which encodes a transcription activation domain and comprises a site-specific topoisomerase recognition site (e.g., a type IA or a type II topoisomerase recognition site) and, optionally, a recombination site, or cleavage product thereof, at a 5' terminus; and a second nucleic acid molecule, which encodes a DNA binding domain and comprises a site-specific topoisomerase recognition site (e.g., a type IA or a type II topoisomerase recognition site), or cleavage product thereof, at a 5' terminus. Upon cleavage by the site specific topoisomerase, the first or second nucleic acid molecule can have a 3' overhang, or both sequences can have 3' overhangs, which are the same or are different from each other. Where the nucleic acid molecules have a 3' overhang, the overhang generally is complementary to a nucleic acid molecule to which first or second nucleic acid molecule is to be linked according to a method described herein. The kit also can contain one or a pair of adapters, linkers or the like, which comprise a site-specific topoisomerase recognition site (e.g., a type IA or a type II topoisomerase recognition site) and, optionally, a recombination site, or cleavage product thereof, at one or both 5' termini, and which can contain a 5' overhang that is complementary to one or the other of the two nucleic acid molecules of the kit.

A ds recombinant nucleic acid molecule covalently linked in one or both strands, and generated according to a method described herein, can be used for various purposes, including, for example, for expressing a polypeptide in a cell, for diagnosing or treating a pathologic condition, or the like. As such, described herein is a medicament, which can be useful for treating a pathologic condition by expressing a polypeptide in one or more cells or by expressing an antisense molecule, or the like. Such a ds recombinant nucleic acid molecule can be provided to a cell by contacting the cell *ex vivo,* then administering the cell to the subject, such a method also allowing for selection and/or expansion of the cells containing the ds recombinant nucleic acid molecule prior to such administration, or can be provided directly to the subject. For administration to a living subject, the ds recombinant nucleic acid molecule, which is covalently linked in one or both strands, generally is formulated in a composition suitable for administration to the subject. Thus, described herein are compositions containing a ds recombinant nucleic acid molecule covalently linked in one or both strands, generated according to a method described herein. As disclosed herein, such nucleic acid molecules are useful as medicaments for treating a subject suffering from a pathological condition.

A composition for administration generally is formulated using one or more pharmaceutically acceptable carriers as well known in the art and include, for example, aqueous solutions such as water or physiologically buffered saline or other solvents or vehicles such as glycols, glycerol, oils such as olive oil or injectable organic esters. A pharmaceutically acceptable carrier can contain physiologically acceptable compounds that act, for example, to stabilize or to increase the absorption of the conjugate. Such physiologically acceptable compounds include, for example, carbohydrates, such as glucose, sucrose or dextrans, antioxidants, such as ascorbic acid or glutathione, chelating agents, low molecular weight proteins or other stabilizers or excipients. One skilled in the art would know that the choice of a pharmaceutically acceptable carrier, including a physiologically acceptable compound, depends, for example, on the route of administration of the composition, which can be, for example, orally or parenterally such as intravenously, and by injection, intubation, or other such method known in the art. A composition as described herein also can contain a second reagent such as a diagnostic reagent, nutritional substance, toxin, or therapeutic agent, for example, a cancer chemotherapeutic agent.

The ds recombinant nucleic acid molecule covalently linked in one or both strands, can be incorporated within an encapsulating material such as into an oil-in-water emulsion, a microemulsion, micelle, mixed micelle, liposome, microsphere or other polymer matrix (see, for example, Gregoriadis, Liposome Technology, Vol. 1 (CRC Press, Boca Raton, FL 1984); Fraley, et al., Trends Biochem. Sci., 6:77 (1981) ). Liposomes, for example, which consist of phospholipids or other lipids, are nontoxic, physiologically acceptable and metabolizable carriers that are relatively simple to make and administer. "Stealth" liposomes (see, for example, U.S. Pat. Nos. 5,882,679; 5,395,619; and 5,225,212 ) are an example of such encapsulating materials particularly useful for preparing a pharmaceutical composition, and other "masked" liposomes similarly can be used, such liposomes extending the time that a nucleic acid molecule remains in the circulation. Cationic liposomes, for example, also can be modified with specific receptors or ligands (Morishita et al., J. Clin. Invest., 91:2580-2585 (1993)). The nucleic acid molecule also can be introduced into a cell by complexing it with an adenovirus-polylysine complex (see, for example, Michael et al., J. Biol. Chem. 268:6866-6869 (1993)). Such compositions can be particularly useful for introducing a nucleic acid molecule into a cell *in vivo* or *in vitro*, including *ex vivo*, wherein the cell containing the nucleic acid molecule is administered back to the subject (see U.S. Pat. No. 5,399,346 ). A nucleic acid molecule generated according to a method described herein also can be introduced into a cell using a biolistic method (see, for example, Sykes and Johnston, *supra*, 1999).

### Host Cells

Described herein are host cells comprising one or more of the nucleic acid molecules or vectors described herein, particularly those nucleic acid molecules and vectors described in detail herein. Representative host cells that may be used include, but are not limited to, bacterial cells, yeast cells, plant cells and animal cells. Preferred bacterial host cells include Escherichia spp. cells (particularly E. coli cells and most particularly E. coli strains DH10B, Stb12, DH5(, DB3, DB3.1 (preferably E. coli LIBRARY EFFICIENCY® DB3.1™ Competent Cells; Invitrogen Corporation, Carlsbad, CA), DB4 and DB5 (see U.S. Application No. 09/518,188, filed March 2, 2000), Bacillus spp. cells (particularly B. subtilis and B. megaterium cells), Streptomyces spp. cells, Erwinia spp. cells, Klebsiella spp. cells, Serratia spp. cells (particularly S. marcessans cells), Pseudomonas spp. cells (particularly P. aeruginosa cells), and Salmonella spp. cells (particularly S. typhimurium and S. typhi cells). Preferred animal host cells include insect cells (most particularly Drosophila melanogaster cells, Spodoptera frugiperda Sf9 and Sf21 cells and Trichoplusa High-Five cells), nematode cells (particularly C. elegans cells), avian cells, amphibian cells (particularly Xenopus Jaevis cells), reptilian cells, and mammalian cells (most particularly NIH3T3, CHO, COS, VERO, BHK and human cells). Preferred yeast host cells include Saccharomyces cerevisiae cells and Pichia pastoris cells. These and other suitable host cells are available commercially, for example from Invitrogen Corporation (Carlsbad, California), American Type Culture Collection (Manassas, Virginia), and Agricultural Research Culture Collection (NRRL; Peoria, Illinois).

Methods for introducing the nucleic acid molecules and/or vectors described herein into the host cells described herein, to produce host cells comprising one or more of the nucleic acid molecules and/or vectors described herein, will be familiar to those of ordinary skill in the art. For instance, the nucleic acid molecules and/or vectors described herein may be introduced into host cells using well known techniques of infection, transduction, electroporation, transfection, and transformation. The nucleic acid molecules and/or vectors described herein may be introduced alone or in conjunction with other the nucleic acid molecules and/or vectors and/or proteins, peptides or RNAs. Alternatively, the nucleic acid molecules and/or vectors described herein may be introduced into host cells as a precipitate, such as a calcium phosphate precipitate, or in a complex with a lipid. Electroporation also may be used to introduce the nucleic acid molecules and/or vectors described herein into a host. Likewise, such molecules may be introduced into chemically competent cells such as E. coli. If the vector is a virus, it may be packaged in vitro or introduced into a packaging cell and the packaged virus may be transduced into cells. Hence, a wide variety of techniques suitable for introducing the nucleic acid molecules and/or vectors described herein into cells in accordance with the disclosure herein are well known and routine to those of skill in the art. Such techniques are reviewed at length, for example, in Sambrook, J., et al., Molecular Cloning, a Laboratory Manual, 2nd Ed., Cold Spring Harbor, NY: Cold Spring Harbor Laboratory Press, pp. 16.30-16.55 (1989), Watson, J.D., et al., Recombinant DNA, 2nd Ed., New York: W.H. Freeman and Co., pp. 213-234 (1992), and Winnacker, E.-L., From Genes to Clones, New York: VCH Publishers (1987), which are illustrative of the many laboratory manuals that detail these techniques.

### Polymerases

Polymerases for use as described herein include but are not limited to polymerases (DNA and RNA polymerases), and reverse transcriptases. DNA polymerases include, but are not limited to, Thermus thermophilus (Tth) DNA polymerase, Thermus aquaticus (Taq) DNA polymerase, Thermotoga neopolitana (Tne) DNA polymerase, Thermotoga maritima (Tma) DNA polymerase, Thermococcus litoralis (Tli or VENT™) DNA polymerase, Pyrococcus furiosus (Pfu) DNA polymerase, DEEPVENT™ DNA polymerase, Pyrococcus woosii (Pwo) DNA polymerase, Pyrococcus sp KOD2 (KOD) DNA polymerase, Bacillus sterothermophilus (Bst) DNA polymerase, Bacillus caldophilus (Bca) DNA polymerase, Sulfolobus acidocaldarius (Sac) DNA polymerase, Thermoplasma acidophilum (Tac) DNA polymerase, Thermus flavus (Tfl/Tub) DNA polymerase, Thermus ruber (Tru) DNA polymerase, Thermus brockianus (DYNAZYME™) DNA polymerase, Methanobacterium thermoautotrophicum (Mth) DNA polymerase, mycobacterium DNA polymerase (Mtb, Mlep), E. coli pol I DNA polymerase, T5 DNA polymerase, T7 DNA polymerase, and generally pol I type DNA polymerases and mutants, variants and derivatives thereof. RNA polymerases such as T3, T5, T7 and SP6 and mutants, variants and derivatives thereof may also be used as described herein.

The nucleic acid polymerases used as described herein may be mesophilic or thermophilic, and are preferably thermophilic. Mesophilic DNA polymerases may include Pol I family of DNA polymerases (and their respective Klenow fragments) any of which may be isolated from organism such as E. coli, H. influenzae, D. radiodurans, H. pylori, C. aurantiacus, R. prowazekii, T.pallidum, Synechocystis sp., B. subtilis, L. lactis, S. pneumoniae, M. tuberculosis, M. leprae, M. smegmatis, Bacteriophage L5, phi-C31, T7, T3, T5, SP01, SP02, mitochondrial from S. cerevisiae MIP-1, and eukaryotic C. elegans, and D. melanogaster (Astatke, M. et al., 1998, J. Mol. Biol. 278, 147-165), pol III type DNA polymerase isolated from any sources, and mutants, derivatives or variants thereof, and the like. Thermostable DNA polymerases that may be used in the methods and compositions described herein include Taq, Tne, Tma, Pfu, KOD, Tfl, Tth, Stoffel fragment, VENT™ and DEEPVENT™ DNA polymerases, and mutants, variants and derivatives thereof (U.S. Patent No. 5,436,149; U.S. Patent 4,889,818; U.S. Patent 4,965,188; U.S. Patent 5,079,352; U.S. Patent 5,614,365; U.S. Patent 5,374,553; U.S. Patent 5,270,179; U.S. Patent 5,047,342; U.S. Patent No. 5,512,462; WO 92/06188; WO 92/06200; WO 96/10640; WO 97/09451; Bames, W.M., Gene 112:29-35 (1992); Lawyer, F.C., et al., PCR Meth. Appl. 2:275-287 (1993); Flaman, J.-M, et al., Nucl. Acids Res. 22(15):3259-3260 (1994)).

Reverse transcriptases for use as described herein include any enzyme having reverse transcriptase activity. Such enzymes include, but are not limited to, retroviral reverse transcriptase, retrotransposon reverse transcriptase, hepatitis B reverse transcriptase, cauliflower mosaic virus reverse transcriptase, bacterial reverse transcriptase, Tth DNA polymerase, Taq DNA polymerase (Saiki, R.K., et al., Science 239:487-491 (1988); U.S. Patent Nos. 4,889,818 and 4,965,188), Tne DNA polymerase (WO 96/10640 and WO 97/09451), Tma DNA polymerase (U. S. Patent No. 5,374,553) and mutants, variants or derivatives thereof (see, e.g., WO 97/09451 and WO 98/47912). Preferred enzymes for use in the invention include those that have reduced, substantially reduced or eliminated RNase H activity. By an enzyme "substantially reduced in RNase H activity" is meant that the enzyme has less than about 20%, more preferably less than about 15%, 10% or 5%, and most preferably less than about 2%, of the RNase H activity of the corresponding wildtype or RNase H⁺ enzyme such as wildtype Moloney Murine Leukemia Virus (M-MLV), Avian Myeloblastosis Virus (AMV) or Rous Sarcoma Virus (RSV) reverse transcriptases. The RNase H activity of any enzyme may be determined by a variety of assays, such as those described, for example, in U.S. Patent No. 5,244,797, in Kotewicz, M.L., et al., Nucl. Acids Res. 16:265 (1988) and in Gerard, G.F., et al., FOCUS 14(5):91 (1992). Polypeptides for use as described herein may include, but are not limited to, M-MLV H⁻ reverse transcriptase, RSV H⁻ reverse transcriptase, AMV H⁻ reverse transcriptase, RAV (rous-associated virus) H⁻ reverse transcriptase, MAV (myeloblastosis-associated virus) H⁻ reverse transcriptase and HIV H⁻ reverse transcriptase. (See U.S. Patent No. 5,244,797 and WO 98/47912). It will be understood by one of ordinary skill, however, that any enzyme capable of producing a DNA molecule from a ribonucleic acid molecule (i.e., having reverse transcriptase activity) may be equivalently used in the compositions, methods and kits described herein.

The enzymes having polymerase activity for use as described herein may be obtained commercially, for example from Invitrogen Corporation (Carlsbad, California), Perkin-Elmer (Branchburg, New Jersey), New England BioLabs (Beverly, Massachusetts) or Boehringer Mannheim Biochemicals (Indianapolis, Indiana). Enzymes having reverse transcriptase activity for use as described herein may be obtained commercially, for example from Invitrogen Corporation (Carlsbad, California), Pharmacia (Piscataway, New Jersey), Sigma (Saint Louis, Missouri) or Boehringer Mannheim Biochemicals (Indianapolis, Indiana). Alternatively, polymerases or reverse transcriptases having polymerase activity may be isolated from their natural viral or bacterial sources according to standard procedures for isolating and purifying natural proteins that are well-known to one of ordinary skill in the art (see, e.g., Houts, G.E., et all J. Virol. 29:517 (1979)). In addition, such polymerases/reverse transcriptases may be prepared by recombinant DNA techniques that are familiar to one of ordinary skill in the art (see, e.g., Kotewicz, M.L., et al., Nucl. Acids Res. 16:265 (1988); U.S. Patent No. 5,244,797; WO 98/47912; Soltis, D.A., and Skalka, A.M., Proc. Natl. Acad. Sci. USA 85:3372-3376 (1988)). Examples of enzymes having polymerase activity and reverse transcriptase activity may include any of those described in the present application.

### Methods of Nucleic Acid Synthesis, Amplification and Sequencing

The disclosure herein may be used in combination with any method involving the synthesis of nucleic acid molecules, such as DNA (including cDNA) and RNA molecules. Such methods include, but are not limited to, nucleic acid synthesis methods, nucleic acid amplification methods and nucleic acid sequencing methods. Such methods may be used to prepare molecules (e.g., starting molecules) used as described herein or to further manipulate molecules or vectors produced as described herein.

Nucleic acid synthesis methods described herein may comprise one or more steps. For example, a method for synthesizing a nucleic acid molecule comprising (a) mixing a nucleic acid template (e.g., a nucleic acid molecules or vectors described herein) with one or more primers and one or more enzymes having polymerase or reverse transcriptase activity to form a mixture; and (b) incubating the mixture under conditions sufficient to make a first nucleic acid molecule complementary to all or a portion of the template. The nucleic acid template may be a DNA molecule such as a cDNA molecule or library, or an RNA molecule such as a mRNA molecule. Conditions sufficient to allow synthesis such as pH, temperature, ionic strength, and incubation times may be optimized by those skilled in the art. If desired, recombination sites may be added to such synthesized molecules during or after the synthesis process (see for sample, U.S. Patent Application No. 09/177,387 filed 10/23/98 based on U. S. provisional patent application no. 60/065,930 filed October 24, 1997).

The target or template nucleic acid molecules or libraries may be prepared from nucleic acid molecules obtained from natural sources, such as a variety of cells, tissues, organs or organisms. Cells that may be used as sources of nucleic acid molecules may be prokaryotic (bacterial cells, including those of species of the genera Escherichia, Bacillus, Serratia, Salmonella, Staphylococcus, Streptococcus, Clostridium, Chlamydia, Neisseria, Treponema, Mycoplasma, Borrelia, Legionella, Pseudomonas, Mycobacterium, Helicobacter, Erwinia, Agrobacterium, Rhizobium, and Streptomyces) or eukaryotic (including fungi (especially yeast's), plants, protozoans and other parasites, and animals including insects (particularly Drosophila spp. cells), nematodes (particularly Caenorhabditis elegans cells), and mammals (particularly human cells)).

Of course, other techniques of nucleic acid synthesis which may be advantageously used will be readily apparent to one of ordinary skill in the art.

The disclosure herein may be used in combination with methods for amplifying or sequencing nucleic acid molecules. Nucleic acid amplification methods may include the use of one or more polypeptides having reverse transcriptase activity, in methods generally known in the art as one-step (e.g., one-step RT-PCR) or two-step (e.g., two-step RT-PCR) reverse transcriptase-amplification reactions. For amplification of long nucleic acid molecules (i.e., greater than about 3-5 Kb in length), a combination of DNA polymerases may be used, as described in WO 98/06736 and WO 95/16028.

Amplification methods may comprise one or more steps. For example, a method for amplifying a nucleic acid molecule may comprise (a) mixing one or more enzymes with polymerase activity with one or more nucleic acid templates; and (b) incubating the mixture under conditions sufficient to allow the enzyme with polymerase activity to amplify one or more nucleic acid molecules complementary to all or a portion of the templates. Nucleic acid molecules may be amplified by such methods. If desired, recombination sites may be added to such amplified molecules during or after the amplification process (see for example, U.S. Patent Application No. 09/177,387 filed 10/23/98 based on U. S. provisional patent application no. 60/065,930 filed October 24, 1997).

General methods for amplification and analysis of nucleic acid molecules or fragments are well known to one of ordinary skill in the art (see, e.g., U.S. Pat. Nos. 4,683,195; 4,683,202; and 4,800,159; Innis, M.A., et al., eds., PCR Protocols: A Guide to Methods and Applications, San Diego, California: Academic Press, Inc. (1990); Griffin, H.G., and Griffin, A.M., eds., PCR Technology: Current Innovations, Boca Raton, Florida: CRC Press (1994)). For example, amplification methods which may be used include PCR (U.S. Patent Nos. 4,683,195 and 4,683,202), Strand Displacement Amplification (SDA; U.S. Patent No. 5,455,166; EP 0 684 315), and Nucleic Acid Sequence-Based Amplification (NASBA; U.S. Patent No. 5,409,818; EP 0 329 822).

Typically, these amplification methods comprise: (a) mixing one or more enzymes with polymerase activity with the nucleic acid sample in the presence of one or more primer sequences, and (b) amplifying the nucleic acid sample to generate a collection of amplified nucleic acid fragments, preferably by PCR or equivalent automated amplification technique.

Following amplification or synthesis by the methods described herein, the amplified or synthesized nucleic acid fragments may be isolated for further use or characterization. This step is usually accomplished by separation of the amplified or synthesized nucleic acid fragments by size or by any physical or biochemical means including gel electrophoresis, capillary electrophoresis, chromatography (including sizing, affinity and immunochromatography), density gradient centrifugation and immunoadsorption. Separation of nucleic acid fragments by gel electrophoresis is particularly preferred, as it provides a rapid and highly reproducible means of sensitive separation of a multitude of nucleic acid fragments, and permits direct, simultaneous comparison of the fragments in several samples of nucleic acids. One can extend this approach, in another preferred embodiment, to isolate and characterize these fragments or any nucleic acid fragment amplified or synthesized by the methods described herein. Thus, the disclosure herein is also directed to isolated nucleic acid molecules produced by the amplification or synthesis methods described herein.

In this embodiment, one or more of the amplified or synthesized nucleic acid fragments are removed from the gel which was used for identification (see above), according to standard techniques such as electroelution or physical excision. The isolated unique nucleic acid fragments may then be inserted into standard vectors, including expression vectors, suitable for transfection or transformation of a variety of prokaryotic (bacterial) or eukaryotic (yeast, plant or animal including human and other mammalian) cells. Alternatively, nucleic acid molecules produced by the methods described herein may be further characterized, for example by sequencing (i.e., determining the nucleotide sequence of the nucleic acid fragments), by methods described below and others that are standard in the art (see, e.g., U.S. Patent Nos. 4,962,022 and 5,498,523, which are directed to methods of DNA sequencing).

Nucleic acid sequencing methods may comprise one or more steps. For example, the disclosure herein may be combined with a method for sequencing a nucleic acid molecule comprising (a) mixing an enzyme with polymerase activity with a nucleic acid molecule to be sequenced, one or more primers, one or more nucleotides, and one or more terminating agents (such as a dideoxynucleotides) to form a mixture; (b) incubating the mixture under conditions sufficient to synthesize a population of molecules complementary to all or a portion of the molecule to be sequenced; and (c) separating the population to determine the nucleotide sequence of all or a portion of the molecule to be sequenced.

Nucleic acid sequencing techniques which may be employed include dideoxy sequencing methods such as those disclosed in U.S. Patent Nos. 4,962,022 and 5,498,523.

### Kits

Described herein are kits which may be used in conjunction with the disclosure herein. Kits described herein may contain any number of components but typically will contain at least two components. Kits may comprise one or more containers, which may contain one or more components selected from the group consisting of one or more nucleic acid molecules or vectors described herein, one or more primers, the molecules and/or compounds described herein, supports described herein, one or more polymerases, one or more reverse transcriptases, one or more recombination proteins (or other enzymes for carrying out the methods described herein), one or more topoisomerases, one or more buffers, one or more detergents, one or more restriction endonucleases, one or more nucleotides, one or more terminating agents (e.g., ddNTPs), one or more transfection reagents, pyrophosphatase, and the like. The kits described herein may also comprise instructions for carrying out methods described herein.

For example, a kit as described herein may comprise (1) a first nucleic acid molecule which comprises one or more (*e*.*g*., 1, 2, 3, 4, 5, 6, 7, 8, 9, 10, etc.) recombination sites and/or one or more (*e.g.*, 1, 2, 3, 4, 5, 6, 7, 8, 9, 10, etc.) toposiomerase recognition sites and (2) instructions for covalently linking the first nucleic molecule to another nucleic acid molecule using methods described herein. In particular embodiments, the instructions describe methods for linking two or more nucleic molecules in either one or both strands. In a related embodiment, the first nucleic acid molecule is topoisomerase adapted prior to inclusion in the kit.

Additional kits of the invention can contain, for example, one or more topoisomerase-charged nucleic acid molecule substrates, which can include one or more control nucleic acid sequences which can be useful, for example, to test the accuracy or fidelity of the components of the kit; one or more topoisomerases; one or more compositions comprising one or more topoisomerases; one or more recombinases (or recombination proteins); one or more compositions comprising one or more recombinases (or recombination proteins); one or more primers, which can comprise at least one topoisomerase recognition site and/or at least one recombination site, a nucleotide sequence complementary to at least one topoisomerase recognition site and/or at least one recombination site, or both at least one topoisomerase recognition site and at least one nucleotide sequence complementary to at least one topoisomerase recognition site; one or more cells, which can contain or be useful for containing a nucleic acid molecule of the kit or generated using the kit; one or more reagents, polymers, buffers, or the like, for performing a method using the kit; instructions for performing a method using the kit; and the like.

A kit described herein may contain a nucleic acid molecule having a first end and a second end, and encoding a polypeptide to be expressed, for example, a selectable marker, wherein the nucleic acid molecule comprises a topoisomerase recognition site or cleavage product thereof at the 3' terminus of one or both ends. Optionally, the nucleic acid molecule contains a hydroxyl group at the 5' terminus of one or both of the other ends, i.e., at the ends that do not contain a topoisomerase recognition site or that are not topoisomerase-charged. Further, one or both 5'termini may comprise overhanging sequences, which are different from each other. A kit described herein also can contain a nucleic acid molecule having a first end and a second end, and encoding a polypeptide to be expressed, for example, a selectable marker, wherein the nucleic acid molecule comprises a topoisomerase recognition site or cleavage product thereof at the 5' terminus of one or both ends. Optionally, the nucleic acid molecule contains a hydroxyl group at the 3' terminus of one or both ends, and preferably, one or both 3' termini comprise overhanging sequences, which are different from each other. In addition, a kit as described herein can contain a nucleic acid molecule having a first end and a second end, and encoding a polypeptide to be expressed, for example, a selectable marker, wherein the nucleic acid molecule comprises a topoisomerase recognition site or cleavage product thereof at the 5' terminus and the 3' terminus of one or both ends. As such, it should be recognized that a kit described herein can include any of various combinations of such nucleic acid molecules comprising one or more topoisomerase recognition sites or topoisomerase-charged nucleic acid molecules.

A kit described herein also can contain a nucleic acid molecule comprising a regulatory element or other nucleotide sequence, for example, a coding sequence, and a topoisomerase recognition site and/or a recombination site, or cleavage product thereof, at a 3' terminus of at least a first end and, optionally, a hydroxyl group at the 5' terminus of an end containing the recognition site; or comprising a topoisomerase recognition site or cleavage product thereof at a 5' terminus of at least a first end, and, optionally, a hydroxyl group at the 3' terminus of the end containing the recognition site; or comprising a topoisomerase recognition site at the 5' terminus and 3' terminus of at least a first end. In certain embodiments, the kit may contain a variety of upstream regulatory elements, a variety of downstream regulatory elements, a variety of elements useful detecting or identifying a molecule containing the element, and combinations thereof. For example, the kit can contain a variety of gene promoter elements, which are active constitutively or inducibly and in a few or many different types of cells, elements that permit ribosome binding such as an internal ribosome entry site, an element encoding a Kozak sequence or an initiator methionine, or the like. In addition, or alternatively, the kit can contain a variety of downstream regulatory elements such a polyadenylation signal sequences, sequences that terminate transcription or translation, or the like. Similarly, the kit can contain elements encoding detectable markers such as epitope tags, or the like. The kit may contain a variety of such elements, each of which contains at least one topoisomerase recognition site and/or at least one recombination site. In certain other such aspects, these elements may contain an overhanging sequence such that they can be operably covalently linked to each other or to a nucleic acid molecule encoding a polypeptide such as a selectable marker according to a method described herein.

Optionally, the kit contains element specific primers, which can amplify a construct containing one of the variety of elements included in the kit. Where the kit contains such primers, the nucleic acid molecules comprising the regulatory or other element has a nucleotide sequence that can be specifically recognized by the primer and that results in extension of the primer through and including the regulatory element. In particular, the kit can contain element specific forward and reverse primers, which can be combined to produce a primer pair that amplifies, for example, a construct containing a particular 5' regulatory element and a particular 3' regulatory element of the kit. Such a primer pair can selectively amplify a desired functional covalently linked ds nucleic acid molecule generated according to a method described herein but does not amplify partial reaction products.

A kit as described herein may contain a first nucleic acid molecule, which has a first end and a second end, contains a topoisomerase recognition site, or cleavage product thereof, and/or a recombination site, at or near one or both 3' termini, and encodes a transcription activation domain; and a second nucleic acid molecule, which has a first end and a second end, contains a topoisomerase recognition site, or cleavage product thereof, at or near one or both 3' termini, and encodes a DNA binding domain; or contains a first nucleic acid molecule, which has a first end and a second end, contains a topoisomerase recognition site, or cleavage product thereof, and/or a recombination site, at or near one or both 5' termini, and encodes a transcription activation domain; and a second nucleic acid molecule, which has a first end and a second end, contains a topoisomerase recognition site, or cleavage product thereof, and/or a recombination site, at or near one or both 5' termini, and encodes a DNA binding domain. A kit as described herein also can contain a first nucleic acid molecule, which has a first end and a second end, and encodes a transcription activation domain, and a second nucleic acid molecule, which has a first end and a second end, and encodes a DNA binding domain, wherein at least the first nucleic acid molecule or the second nucleic acid molecule contains a topoisomerase recognition site, or cleavage product thereof, at or near a 5' terminus and at or near 3' terminus of at least one end, and wherein the other ds nucleotide contains a 3' hydroxyl and 5' hydroxyl at the end to be covalently linked to the end of the nucleic acid molecule comprising the recognition sites. Such a kit is useful, for example, for generating covalently linked ds recombinant nucleic acid molecules encoding chimeric polypeptides for performing a two hybrid assay. The kit can further contain a primer pair, which can amplify a nucleotide sequence to be operably linked to the first or second nucleic acid molecule, wherein at least one primer of the primer pair comprises a topoisomerase recognition site, a complement of a topoisomerase recognition site, or both. An amplification product generated using such a primer pair may contain, following cleavage by a site-specific topoisomerase, a 3' or 5' overhanging sequence that is complementary to the first or second nucleic acid molecule to which it is to be covalently linked. Such a kit can facilitate the generation of recombinant polynucleotides that comprise a first or second nucleotide sequence of the kit and encode a chimeric polypeptide useful for performing a two hybrid assay.

Also described herein are additional kits for carrying out the methods described herein, and particularly for use in creating the product nucleic acid molecules described herein. Also described herein are kits for carrying out homologous recombination (particularly gene targeting) according to the methods described herein. Such kits may also comprise further components for further manipulating the recombination site-containing molecules and/or compounds produced by the methods described herein. The kits described herein may comprise one or more nucleic acid molecules described herein (particularly starting molecules comprising one or more recombination sites and optionally comprising one or more reactive functional moieties), one or more molecules and/or compounds described herein one or more supports described herein and/or one or more vectors described herein. Such kits may optionally comprise one or more additional components selected from the group consisting of one or more host cells, one or more nucleotides, one or more polymerases and/or reverse transcriptases, one or more suitable buffers, one or more primers, one or more terminating agents, one or more populations of molecules for creating combinatorial libraries and one or more combinatorial libraries.

A kit as described herein may contain a first nucleic acid molecule, which encodes a polypeptide, particularly a selectable marker, and contains a topoisomerase recognition site at each end. In certain preferred such embodiments, the first nucleic acid molecule is a circular molecule (for example, a plasmid, vector, etc.) and comprises at least one recombination site, and more preferably at least two recombination sites, flanking the one or more, preferably two or more, topoisomerase recognition sites on the molecule. Preferably, the first nucleotide sequence comprises a topoisomerase-activated nucleotide sequence. More preferably, the topoisomerase-charged first nucleotide sequence comprises a 5' overhanging sequence at each end, and most preferably the 5' overhanging sequences are different from each other. Optionally, each of the 5' termini comprises a 5' hydroxyl group.

Kits may also contain at least a nucleotide sequence comprising a regulatory element, which can be an upstream or downstream regulatory element, or other element, which contains one or more topoisomerase recognition sites and, optionally, contains one or more recombination sites at one or both ends. Preferably, the kit contains a plurality of nucleic acid molecules, each comprising a different regulatory element or other element, for example, a sequence encoding a tag or other detectable molecule or a cell compartmentalization domain. The different elements can be different types of a particular regulatory element, for example, constitutive or inducible promoters or tissue specific promoters, or can be different types of elements including, for example, transcriptional and translational regulatory elements, epitope tags, and the like. Such nucleic acid molecules can be topoisomerase-activated, and can contain 5' overhanging sequences that facilitate operably covalently linking the elements in a predetermined orientation, particularly such that a polypeptide such as a selectable marker is expressible in vitro or in one or more cell types.

Such kits also may contain primers, including first and second primers, such that a primer pair comprising a first and second primer can be selected and used to amplify a desired covalently linked ds recombinant nucleic acid molecule generated using components of the kit. For example, the primers can include first primers that are complementary to elements that generally are positioned at the 5' end of a generated ds recombinant nucleic acid molecule, for example, a portion of a nucleic acid molecule comprising a promoter element, and second primers that are complementary to elements that generally are positioned at the 3' end of a generated ds recombinant nucleic acid molecule, for example, a portion of a nucleic acid molecule comprising a transcription termination site or encoding an epitope tag. Depending on the elements selected from the kit for generating a covalently linked ds recombinant nucleic acid molecule, the appropriate first and second primers can be selected and used to amplify a full length functional construct.

A kit as described herein may contain a plurality of different elements, each of which can be topoisomerase-activated at one or both ends, and each of which can contain a 5' overhanging sequence. The 5' overhanging sequences can be unique to a particular element, or can be common to plurality of related elements, for example, to a plurality of different promoter element. Preferably, the 5' overhanging sequences of elements are designed such that one or more elements can be operably covalently linked to provide a useful function, for example, an element comprising a Kozak sequence and an element comprising a translation start site can have complementary 5' overhangs such that the elements can be operably covalently linked according to a method described herein.

The plurality of elements in the kit can comprise any elements, including transcription or translation regulatory elements; elements required for replication of a nucleotide sequence in a bacterial, insect, yeast, or mammalian host cell; elements comprising recognition sequences for site specific nucleic acid binding proteins such as restriction endonucleases or recombinases; elements encoding expressible products such as epitope tags or drug resistance genes; and the like. As such, a kit as described herein provides a convenient source of different elements that can be selected depending, for example, on the particular cells that a construct generated according to a method described herein is to be introduced into or expressed in. The kit also can contain PCR primers, including first and second primers, which can be combined as described above to amplify a covalently linked ds recombinant nucleic acid molecule generated using the elements of the kit. Optionally, the kit further contains one or more topoisomerases (*e*.*g*., one or more site-specific topoisomerases) and/or one or more recombinases (or recombination proteins) in an amount useful for covalently linking a first nucleic acid molecule comprising a topoisomerase recognition site to a second (or other) nucleic acid molecule, which can be topoisomerase-activated nucleic acid molecules or can be nucleotide sequences that comprise a topoisomerase recognition site.

A kit as described herein may contain a first nucleic acid molecule, which encodes a selectable marker, and contains a topoisomerase recognition site at each end; a first and second PCR primer pair, which can produce a first and second amplification products that can be covalently linked to the first nucleic acid molecule in a predetermined orientation according to a method described herein. Such a generated construct can be introduced into a cell and can incorporate into the genome of the cell by homologous recombination in a site specific manner, where it can be stably maintained and can express a heterologous polypeptide in the cell or can knock-out a target gene function. A target gene to be knocked-out, for example, can be any gene for which at least part of the sequence is known or can be readily determined and the function of which it is desired to disrupt, for example, an oncogene, a gene involved in apoptosis, a gene encoding a serine/threonine or a tyrosine kinase, or any other gene.

The first PCR primer pair in a kit as described herein includes a first primer that comprises, in an orientation from 5' to 3', a nucleotide sequence complementary to a 5' overhanging sequence of a nucleic acid molecule to which it is to be covalently linked (for example, an end of the nucleic acid molecule encoding the selectable marker), a nucleotide sequence complementary to a topoisomerase recognition site and/or to a recombination site, and a nucleotide sequence complementary to a 3' sequence of the target DNA sequence.. The first PCR primer pair also includes a second primer that comprises a nucleotide sequence of the target DNA sequence upstream of the 3' sequence to which the first primer is complementary.

The second PCR primer pair of a kit as described herein includes a first primer that comprises, from 5' to 3', a nucleotide sequence complementary to a 5' overhanging sequence of a nucleic acid molecule to which it is to be covalently linked, a nucleotide sequence complementary to a topoisomerase recognition site and optionally, a nucleotide sequence complementary to a recombination site, and a nucleotide sequence of a 5' sequence of the target DNA sequence, wherein the 5' sequence of the target gene is downstream of the 3' sequence of the target DNA sequence to which the first primer of the first primer pair is complementary. The second PCR primer pair also includes a second primer that comprises a nucleotide sequence complementary to a 3' sequence of the target gene that is downstream of the 5' sequence of the target DNA sequence contained in the first primer.

A kit as described herein may contain a first nucleic acid molecule, which encodes a transcription activation domain and comprises a topoisomerase recognition site, or cleavage product thereof, at or near a 3' terminus; and a second nucleic acid molecule, which encodes a DNA binding domain and comprises a topoisomerase recognition site and optionally a recombination site, or cleavage product thereof, at or near a 3' terminus. Upon cleavage by the site specific topoisomerase, the first or second nucleic acid molecule can have a 5' overhang, or both sequences can have 5' overhangs, which are the same or are different from each other. Where the nucleic acid molecules have a 5' overhang, the overhang generally is complementary to a nucleic acid molecule to which first or second nucleic acid molecule is to be covalently linked according to a method described herein.

The kit also can contain one or a pair of adapters, linkers or the like, which comprise a topoisomerase recognition site and, optionally, a recombination site, or cleavage product thereof, at one or both 3' termini, and, optionally, a hydroxyl group at the same terminus/termini. Such adapters, linkers, or the like are selected such that they contain a 5' overhang that is complementary to one or the other of the two nucleic acid molecules described above and part of the kit. Similarly, the kit also can contain one or a pair of adapters, linkers or the like, which comprise a topoisomerase recognition site and, optionally, a recombination site, or cleavage product thereof, at one or both 5' termini, and, optionally, a hydroxyl group at the same terminus/termini. Such adapters, linkers, or the like are selected such that they contain a 3' overhang that is complementary to one or the other of the two nucleic acid molecules described above and part of the kit. In addition, the kit can contain one or a pair of adapters, linkers or the like, which comprise a topoisomerase recognition site, or cleavage product thereof, at or near one or both 5' and/or 3' termini, and, optionally, a hydroxyl group at the same terminus/termini. Such adapters, linkers, or the like are selected such that they contain a 5' and/or a 3' overhang that is complementary to one or the other of the two nucleic acid molecules described above and part of the kit. Such adapters, linkers, or the like can be joined to the ends of nucleic acid molecules that are to covalently linked to one or the other of the first or second nucleic acid molecules provided with the kit, thus facilitating the construction of chimeric polynucleotides encoding the bait and prey polypeptides useful in a two hybrid assay. Such a kit also can contain a PCR primer or primer pair, which can be used to prepare an amplified plurality of nucleotide sequences comprising a topoisomerase recognition site, or cleavage product thereof. Additional kits may optionally comprise one or more additional components such as one or more topoisomerases, one or more recombination proteins, one or more vectors, one or more polypeptides having polymerase activity, and one or more host-cells.

It will be understood by one of ordinary skill in the relevant arts that other suitable modifications and adaptations to the methods and applications described herein are readily apparent from the description of the invention contained herein in view of information known to the ordinarily skilled artisan, and may be made without departing from the scope of the invention or any embodiment thereof. Having now described the present invention in detail, the same will be more clearly understood by reference to the following examples, which are included herewith for purposes of illustration only and are not intended to be limiting of the invention.

### EXAMPLES

### EXAMPLE 1

### Construction of Covalently Linked Double Stranded Recombinant Nucleic Acid Molecules Using Topoisomerase

This experiment demonstrates that topoisomerase can be used to produce covalently linked double stranded (ds) recombinant nucleic acid molecules.

### A. Methods

Except where indicated, experiments were performed using the following methods. PCR was performed in 50 µl reactions, including 10 ng plasmid (template), 100 ng each primer, 2.5 Units Taq DNA polymerase (Sigma), 5 µl 10X PCR buffer, and 4 µl of dNTPs (200 µM each). An initial denaturation was performed by incubating the reaction at 94°C for 4 min; followed by 30 cycles of PCR using 94°C (45 sec) for denaturation, 55°C (45 sec) for primer annealing and 72°C (1 min per kb of target sequence) for extension. After cycling, the reactions were incubated at 72°C (10 min), and then placed at 4°C.

Topoisomerase joining reactions were performed in 5 µl, including 50-100 ng each amplified element (PCR-generated or synthetic), 0.5 µl 500 mM Tris (pH 7.5), and 0.5 µg topoisomerase. Reactions were incubated at room temperature for 5 min, then 1-2 µl of the Topo-linked product was used for linear fragment generation.

Linear fragment generation by PCR was performed in 50 µl reactions, including 1-2 µl of the Topo-linked product (template), 100 ng each primer, 2.5 U Taq DNA polymerase (Sigma), 5 µl 10X PCR buffer, and 4 µl dNTPs (200 µM each). PCR was performed as described above.

The resultant linear fragment was purified using a SNAP Miniprep Kit (Invitrogen) as described by the manufacturer. Essentially, 100 µl PCR product was mixed with 300 µl Binding Buffer, 750 µl isopropanol, and the mixture was applied to a SNAP Miniprep Column/Collection Tube and centrifuged at 7,000 rpm for 30 sec. The column was washed with 700 µl Wash Buffer, centrifuged at 7,000 rpm for 30 sec; then washed with 900 µl 1X Final Wash and centrifuged at 7,000 rpm for 30 sec. The column was then centrifuged at 7,000 rpm for an additional 30 sec to remove all remaining liquid. Water (30 to 50 µl) was added and the column was centrifuged at 7,000 rpm for 30 sec to elute the purified DNA. DNA concentration was determined by spectrophotometry.

### B. Generation of Topoisomerase Linked Linear Nucleic Acid Molecules

PCR primers were designed to examine the directional addition of elements to the coding sequence of green fluorescent protein (GFP; see Figure 9A-C). The CMV promoter (approximately 700 bp) and BGH polyadenylation signal sequence (approximately 380 bp) were amplified from a pCMV/myc/nuc plasmid template, and the GFP element (approximately 700 bp) was amplified from a pcDNA3.1/GFP plasmid template (Invitrogen) using the primers indicated in Figure 9D. The resultant amplification products were joined using topoisomerase as described above, and a portion of the ligation reaction was used as template for PCR with primers F6945 (SEQ ID NO: 11) and F6948 (SEQ ID NO: 15) to amplify the entire construct (CMV+GFP+BGH; approximately 1,700 bp). In addition, 5 µl of the ligation mixture was treated with proteinase K for 30 min at 37°C to remove any bound topoisomerase, and then subjected to electrophoresis on a 3-8% NuPAGE Tris-acetate gel to examine the ligated products.

Only a small amount of ligation product of the correct size (1.7 kb) was observed when the recombinant nucleic acid molecules were generated using elements having palindromic overhanging sequence (Figures 9A or 9B), whereas significant quantities of the desired product were generated using elements having non-palindromic overhangs (Figure 9C). These results demonstrate that the efficiency of generating ds recombinant nucleic acid molecule covalently linked in both strands containing nucleotide sequences operatively linked in a predetermined orientation is related to the nature of the overhang sequence. In particular, the selection of overhanging sequences that lack palindromic regions result in the efficient generation of a desired ds recombinant nucleic acid molecule covalently linked in both strands, whereas the presence of palindromic sequences in the overhangs allows the formation of ligation products other than the intended product, thus decreasing the efficiency of generating a desired product.

### EXAMPLE 2

### Functional Characterization of Topoisomerase-generated ds Recombinant Nucleic Acid Molecules

This example demonstrates that a method described herein provides a means to generate functional ds recombinant nucleic acid molecules covalently linked in both strands.

### A. Expression of Sense and Antisense mRNA from a Topo-ligated Construct

The ability to create a ds recombinant nucleic acid molecule containing functional upstream and downstream elements flanking a gene of interest was examined using two synthetic elements containing either a T7 or a T3 promoter sequence. The elements were made by annealing pairs of synthetic oligonucleotides. The T7 linker was generated by mixing equal molar amounts of T7top (F9304; SEQ ID NO: 20) and T7bottom (F9305; SEQ ID NO: 21) oligonucleotides (Figure 9D). The T3 linker was generated by mixing equal molar amounts of T3top (F9661; SBQ ID NO: 23) and T7bottom (F9662; SEQ ID NO: 24) oligonucleotides (Figure 9D). The mixtures were heated in boiling water for 5 min, then allowed to cool to room temperature. Both elements were designed to contain a topoisomerase recognition site at one end.

The GFP gene was amplified with GFP primers F8418 (SEQ ID NO: 17) and F8420 (SEQ ID NO: 18, Figure 9D; see, also, Figure 9C). Unpurified GFP PCR product (2 µl) was mixed with 50 ng of T7 linker and 50 ng of T3 linker, topoisomerase was added, and the topo-joining reaction was allowed to proceed at room temperature for 5 min. Two µl of the joining reaction was used as template for a 50 µl PCR reaction with primers for the T7 and T3 sequences.

After amplification, a 4 µl aliquot of the PCR reaction was used as template for in vitro transcription. The reaction was performed using a Promega RiboProbe In Vitro Transcription Systems kit according to the manufacturer's instruction. The reaction was allowed to proceed for 60 min at 37°C with T7 or T3 RNA polymerase (final volume, 20 µl). Aliquots of the in vitro transcription reactions were digested with RNase or DNase, then undigested and digested samples were subjected to electrophoresis in a 2% TBE gel. A predominant band of the predicted size (either sense or antisense orientation) was observed in the undigested samples.. No decrease in the product band was noted in samples treated with DNase. The product bands disappeared when samples were treated with RNase indicating the product was RNA. These results demonstrate that topoisomerase can be used according to a method described herein to generate a ds recombinant nucleic acid molecule covalently linked in both strands in a predetermined orientation, and that an RNA transcript can be expressed from such a nucleic acid molecule.

### B. Expression of a Translation Product from a Topo-ligated Construct

The ability of topoisomerase ligated polynucleotide to support coupled in vitro transcription/translation was examined. A ds recombinant nucleic acid molecule was generated according to a method described herein by linking an element containing a T7 promoter (plus a Kozak sequence) to lacZ PCR products of 1kb, 2kb, or 3 kb. Two 2 µl of the generated products were used as template for PCR amplification reactions (primers, SEQ ID NOS: 25-28; Figure 9D). Unpurified aliquots of the amplification reactions (3 µl) were used as templates for coupled transcription/translation with a TNT T7 Quick for PCR DNA Kit according to the manufacturer's instructions (Promega).

Two µl aliquots from each reaction were separated by electrophoresis on a Tris-glycine gel (Novex), then visualized by autoradiography, which revealed protein products that migrated at the expected sizes. These results demonstrate that a method described herein can be used to produce a ds recombinant nucleic acid molecule covalently linked in both strands useful as a template for expressing a polypeptide by a coupled in vitro transcription/translation reaction.

### C. Generation of Topo-ligated Constructs for Performing a Two Hybrid Assay

Two hybrid assays provide a powerful method for detecting protein-protein interactions in vivo. These assays are based on the fact that many eukaryotic transcriptional activators consist of two physically and functionally separable domains, including a DNA binding domain, which binds to a specific DNA sequence, and a transcriptional activation domain, which interacts with the basal transcriptional machinery. The association of a transactivation domain with a DNA binding domain can promote the assembly of a functional RNA polymerase II complex, thereby allowing transcriptional activation, for example, of a detectable reporter gene (Field and Song, Nature 340:245-246, 1989). Where a first protein, X, is fused to a DNA binding domain, for example, a GAL4 binding domain, and a second protein, Y, which can be the same or different from X, is fused into a transactivation domain, for example, a VP16 domain, an interaction of proteins X and Y can be identified by detecting transcription of a reporter gene having a GAL4 promoter.

The ability of a method described herein to generate linear constructs for expressing fusion proteins for performing a mammalian two-hybrid assay was examined. PCR was used to generate GAL4 (F10779 and F12667 primers; SEQ ID NOS: 1 and 3, respectively), VP16 (F10779 and F12668 primers; SEQ ID NOS: 1 and 5, respectively), p53 (F12669 and F12505 primers; SEQ ID NOS: 8 and 4, respectively), T antigen (F12670 and F12505 primers; SEQ ID NOS: 9 and 4, respectively), and SV40pA (F12016 and F561 primers; SEQ ID NOS: 6 and 7, respectively) elements containing topoisomerase sites at the appropriate ends. Topoisomerase was used to create the covalently linked, double stranded constructs GMA+p53+SV40pA and VP16+T antigen+SV40pA, and the resultant ligation products were used as templates for PCR amplification.

Purified GAL4+p53+SV40pA and VP16+T antigen-hSV40pA PCR constructs were co-transfected with a lacZ reporter gene (pGene/lacZ plasmid; (Invitrogen) into CHO cells (6 well plate, 1x10⁵ cells/well). In parallel experiments, the use of plasmid vectors containing the expression constructs was examined, as was the use of PCR reaction mixtures containing the unpurified constructs. Control reactions were performed using GAL4+pA and VP16+pA without inserts (negative controls) or p53+VP16 (positive control). Cells were lysed 48 hr after transfection and reporter gene activity was measured using a beta-galactosidase assay kit.

A high level of reporter gene activity was detected with the positive control (Figure 10, sample 3) and in the sample co-transfected with the reporter gene and the linear GAL4+p53+SV40pA and VP16+T antigen+SV40pA constructs (Figure 10, sample 4). Low level activity (but greater than that of the negative controls; samples 5, 6, 8 and 9) was detected when the plasmid version of the constructs was used (Figure 10, sample 1). Low level activity was also observed in the sample co-transfected with the unpurified, PCR-generated prey and bait constructs (sample 7). These results demonstrate that a method described herein can be used to prepare constructs useful for performing a two hybrid assay.

### EXAMPLE 3

### Production and Use of Directionally Topo-Charged Gateway Vectors

### Introduction

As a combination of Topoisomerase and GATEWAY™ recombinational cloning technologies, directionally Topo-charged Gateway vectors were developed. These tools facilitate easy entry into the Gateway system by alleviating the necessity of adding attB sites (25 base pairs) to either side of a PCR amplified ORF prior to recombination into a Donor vector. Instead, a four base tag recognition sequence (CACC) is added to the 5' end of the ORF and PCR products are then directionally TOPO-cloned to create an Entry or a Gateway compatible expression vector (See Figures 8 and 9).

In the present Example, three Topo-Gateway vectors and one Destination vector were created in all. Two topo entry vectors have been produced: (1) pENTR/D-TOPO® (Figure 22), which allows ORFs directionally cloned between attL sites to be transferred to any of the N-terminal fusion prokaryotic and all of the eukaryotic DEST vectors; and (2) pENTR/SD/D-TOPO® (Figure 23), which allows ORFs to be directionally topo cloned downstream of a prokaryotic ribosome binding site (Shine-Dalgamo). Genes cloned in this manner can be transferred to prokaryotic DEST vectors without N-terminal tags and expressed in bacteria yielding proteins with native N-termini.

One directional Topo Gateway mammalian expression vector has also been constructed, pcDNA/GWD-TOPO® (Figure 19). This vector allows directional cloning of an ORF into a pcDNA 3.1 derivative. ORFs cloned into this vector are expressed in mammalian cells under the control of the CMV promoter. Cloned ORFs are flanked by attB sites in the vector, allowing them to be moved around in the Gateway system via BP and LR Clonase reactions. This vector also encodes a C-terminal V5 tag, the TK poly adenylation signal, and the neomycin (G418) resistance marker for selection of stable clones in mammalian cell lines. Finally, a Gateway Destination vector was constructed from pcDNA/GWD-TOPO® by transferring the ccdB and chloramphenicol resistance cassettes.

These Topo Gateway Entry and Expression vectors improve the ease of entry into the Gateway system by allowing the researcher to directly clone a PCR amplified gene without the necessity of adding attB sites to the primers and performing a BP clonase reaction.

### Materials and Methods

Construction of pcDNA/GWD-TOPO®. pcDNA/GWD-TOPO® was constructed by first replacing the multiple cloning site in pcDNA3.1attB (an early version with the BGH polyadenylation signal). This was done by digesting the parent vector with BsrG I (which cuts within each att site flanking the MCS) and inserting a double stranded oligonucleotide encoding the new MCS (Figure 18). Once the proper insertion was confirmed, the V5/His tag and BGH polyadenylation signal were replaced with a V5 tag followed by three stop codons (TAG, TGA, TAA) and the thymidine kinase (TK) polyadenylation signal from Herpes Simplex Virus. This was accomplished by digesting the vector with AscI and AvrII, purification of the vector fragment, and inserting two fragments encoding the new sequences in a triple ligation (see Figure 19).

Construction of pcDNA-DEST 40. pcDNA-DEST 40 was created from pcDNA/GWD-TOPO® via a BP clonase reaction with pDONR221. pDONR221 was combined with pcDNAGW-DT(sc) and BP clonase (Invitrogen Corporation; Carlsbad, CA) in the appropriate buffer. The reaction was incubated according to the standard protocol and transformants selected for on Kanamycin plates. The product, a pcDNA destination vector containing att P sites flanking the ccdB, ccdA, and chloramphenicol resistance genes was selected on Ampicillin/ chloramphenicol containing media (see Figure 20).

Construction of pENTR/D-TOPO® (sc). pDONOR221 was modified by adding an adaptation sequence cassette between the attP sites by BP recombination with pcDNA/GVM-TOPO®(sc) creating pENTR/D-TOPO® (sc) (Figure 21). pDONR221 was combined with pcDNA/GWD-TOPO® (sc) and BP clonase in the appropriate buffer. The reaction was incubated according to the standard protocol except that DH10BsbcC cells were used for transformation and propagation of pENTR/D-TOPO® (sc). This cell line carries a mutation that allows maintenance of plasmids that carry hairpin structures (e.g. attL sites) that are in close proximity. This plasmid did not support growth of Top 10 cells in selective media.

Creation of pENTR/D-TOPO® and pENTR/SD/D-TOPO®. The vector pENTR/D-TOPO®(sc) was directionally topo charged by sequential digestion with Not I, Asc I, and Xho I followed by ligation with the directional topo adapters Topo-D71 (SEQ ID NO:75), -D72 (SEQ ID NO:81), -D75 (SEQ ID NO:77) and -D76 for pENTR/SD/D-TOPO® or Topo D-73 (SEQ ID NO:76), -D74 (SEQ ID NO:82), -D75 (SEQ ID NO: 77) and -D76 for pENTR/D-TOPO® overnight at 15C (see Figure 26). The adapted vectors were separated from free oligonucleotides by isopropanol precipitation at room temerature. The purified, adapted vector was topo charged by addition of the common annealing oligo Topo D-70 (SEQ ID NO:83, SEQ ID NO:84), T4 Kinase, and recombinant vaccinia topoisomerase I. After incubation at 37C for 15 minutes, charged vector was purified either by agarose gel electrophoresis (NB JC-12, 2001-035, pg. 3) or chromatography on a 25 Q MacroPrep column (BioRad) (NB2000-0342, pg. 45). Directional topo cloning efficiency was assayed by incubation of 1ng purified vector with 5ng directional (CACC) 750 bp test insert for 5 minutes at room temperature. Top 10 chemically competent cells were then transformed with 2 ul of the cloning reaction and grown out on LB plates containing Kanamycin as antibiotic selection.

Topo-Gateway cloning and gene expression. To test the ability of these vectors to support Topo cloning, Gateway cloning and protein production, the gene encoding human HLA class I (accession No. D32129) was amplified by PCR with primers that incorporated the four base CACC tag at its 5' end immediately upstream of the ATG start codon. This PCR product was cloned into both pEIVTR/D-TOPO® and pENTR/SD/D-TOPO®. Ten clones from each HLA reaction were used in colony directional PCR reactions (d-PCR). In this experiment, clones were amplified with a T7 primer (binds 5' to the attL 1 site) and 129 reverse primer (specific for the 3' end of HLA).

In addition to the HLA gene, the gene for chloramphenicol acetyl transferase (CAT) was similarly amplified and cloned into the two entry vectors. After miniprep and digestion analysis, single clones from each reaction were isolated and sequenced using the M13 Forward and M13 Reverse primers. All entry clones were confirmed by sequencing and recombined by L/R Clonase reaction with pcDNA/GW DEST 40 (pENTR- D-TOPO® clones) or pET DEST 42 (pENTR/SD/D-TOPO® clones). Positive clones were confirmed by digestion with NcoI (site appears at the 5' end of directionally adapted ORFs, caCCATGG), and NotI (data not shown). The resulting pcDNA-DEST 40 (HLA and CAT) and pcDNA/GW/D-TOPO® (HLA and CAT) constructs were then used to transfect COS cells. Cells were transfected using Lipofectamine 2000, 8ug DNA and Optimem buffer. Reactions were applied to the cells for 5 hours then the media changed. After an overnight incubation at 37C, the cells were harvested, lysed and run on a 4-20% Tris-Glycine gel using standard procedures. After electrophoresis, proteins were transferred to nitrocellulose membranes, blocked, and probed with V5-HRP antibody and ECL detection.

One positive clone from each pET DEST 42 reaction was used to transform BL21(DE3) cells and grown overnight in LB/Amp. The culture was then diluted 1:25 in the same medium and allowed to grow to O.D.(600mm) = 0.5 at which time expression of recombinant protein was induced by addition of IPTG to a final concentration of 1 mM. After the cultures were allowed to grow 3 hours at 37C, cells were harvested by centrifugation. Aliquots of cell pellets were boiled in NuPage denaturing sample buffer, run on 4-12% NuPage polyacrylamide gels, and stained using SafeStam™ (Invitrogen). As a positive control for expression of test genes in the pET DEST 42 vector, the HLA and CAT genes were directly topo cloned into pET100 CAT and HLA (dTopo, no attB sites). These constructs were used to transfect BL21(DE3) E. coli cells, grown to log phase and induced with IPTG as described above.

### Results and Discussion

Directional cloning efficiency of HLA and CAT clones in pENTR-dTopo and pENTR/SD-dTopo. Directional PCR reactions were designed to ensure that the HLA ORF cloned into pENTR/D-TOPO® and pENTR/SD/D-TOPO® were in the correct orientation. Ten colonies were picked from each of the Topo cloning transformations and put directly into PCR reactions as described in 'Materials and Methods." Eight of ten pENTR/SD-HLA clones tested were correctly oriented while nine of ten pENTR-HLA clones were correct. These tests were done with gel purified vector which had approximately 10-15% no insert background (data not shown).

Alternatively, restriction analysis of the CAT clones was done. Clones were isolated and the DNA digested with NcoI and AscL One of the two NcoI sites in a correctly oriented CAT clone appears at the 5' end of each ORF as part of the Kozac directional adaptation sequence and the first two codons of the CAT gene (caCCATGG). AscI is present in the vector at the 3' end of the ORF. A correctly oriented clone will have two NcoI sites (one at the 5' end and one internal) and will yield 500 bp and 150 bp fragments after a double digest with Asc I. The CAT ORF encodes at its 3' end the sequence, CGCC, which is a one base pair mismatch to the optimum tag sequence. This close homology caused the CAT PCR product to directionally clone with only 50% efficiency (four of eight clones, data not shown).

Sequencing of Entry Clones. Each of the Entry clones chosen for recombination into DEST vectors and subsequent expression were sequenced from both ends to confirm that the adapters and ORFs ligated correctly. M13 forward and reverse primers were used and the reactions were sent to ResGen for sequencing on an ABI 3700 capillary sequencer. From these reactions a minimum of 600 bases of readable sequence were obtained. It is clear that there is some loss of signal as the reaction proceeds through the attL sites but significant signal remains after this point using this procedure (data not shown).

Expression of HLA and CAT in COS cells. Expression from pcDNA/GW/D-TOPO® and pcDNA-DEST 40 was tested by transfection of COS cells with HLA and CAT as the test gene in these constructs. Harvested lysates were probed for V5-tagged recombinant protein by Western blot using the V5 antibody. Data shown in Figure 27 indicates that both the HLA and CAT genes express in these vectors whether the genes were cloned directly via Topo cloning (Figure 27, lanes 3 and 6) or after LR clonase transfer from pENTR/D-TOPO® (Figure 27, lanes 2 and 5).

Bacterial expression of HLA and CAT. The CAT and HLA genes cloned into pENTR/SD/D-TOPO® were transferred via LR Clonase reaction to pDEST-42 (pET, C-terminal V5/His). The results shown in Figure 28 suggest that the CAT gene expressed in bacteria whether it is flanked by attB sites or not (Figure 28, compare lanes 6 and 7). The finding that the CAT gene expresses well in E.coli after being transferred to a pET DEST vector from pENTR/SD/D-TOPO® validates the utility of this system for cloning and expressing ORFs using the Topo-Gateway system.

Interestingly, HLA cloned into pDEST 42 (flanked by attB sites) failed to express in BL21(DE3) cells in two independent experiments (Figure 28, lanes 3 and 4). As seen above, the HLA gene from the same Entry clone expressed well in COS cells when recombined into a mammalian DEST vector. Further, the fact that the pET system was unable to support expression of the HLA gene when it was flanked by attB sites suggests that there can be gene specific variations on expression using the Gateway system at least in bacteria. One factor that may be involved in this result is that HLA expressed from the control vector (pET 100 d-Topo) ran anomalously in the gel (30 kDa instead of the predicted 41 kDa). This human protein may not express well in bacteria in any case and the expression problem may be exacerbated by addition of attB sites. More experimentation will be required to understand this finding.

In conclusion, we have described the construction and testing of two new Topo Gateway Entry vectors, one new Topo Gateway Expression vector and a new DEST vector that followed from that. In all, these new tools that combine the ease and efficiency of Topo cloning and the versatility of the Gateway system permit the cloning and expression of large numbers of genes in many different contexts with a minimum of expense and effort.

### EXAMPLE 4

### Alternative Methods of Topoisomerase Cloning

A TOPO SSS vector may be made by first obtaining a commercially available cloning vector. One such vector is pUni/V5-His version A (Invitrogen Corp, Carlsbad, CA), a circular supercoiled vector that contains uniquely designed elements. These elements include a BGH polyadenylation sequence to increase mRNA stability in eukaryotic hosts, a T7 transcription termination region, an R6Kg DNA replication origin and a kanamycin resistance gene and promoter for antibiotic resistance selection. Additionally, pUni/V5-His version A contains a multiple cloning site, which is a synthetic DNA sequence encoding a series of restriction endonuclease recognition sites. These sites are engineered for cloning of DNA into a vector at a specific position. Also within the vector's multiple cloning site is a loxP site inserted 5' to the endonuclease recognition sites thereby facilitating Cre recombinase-mediated fusion into a variety of other expression vectors, (Echo™ Cloning System, Invitrogen Corporation, Carlsbad, CA). An optional C-terminal V5 epitope tag is present for easy detection of expressed fusion proteins using an Anti-V5 Antibody. An optional C-terminus polyhistidine (6xHis) tag is also present to enable rapid purification and detection of expressed proteins. A bacterial ribosomal binding site downstream from the loxP site makes transcription initiation in E. coli possible. Though this combination of elements is specific for pUni/V5-His version A cloning vector, many similar cloning and expression vectors are commercially available or may be assembled from sequences and by methods well know in the art. pUni/V5-His version A is a 2.2kb double stranded plasmid.

Construction of a topoisomerase I charged cloning vector from pUni/V5-His version A is accomplished by endonuclease digestion of the vector, followed by complementary annealing of synthetic oligonucleotides and site-specific cleavage of the heteroduplex by Vaccinia topoisomerase L SacI and EcoRI are two of the many restriction endonuclease sites present within the multiple cloning site of pUni/V5-His version A. Digestion of pUni/V5-His version A with the corresponding restriction enzymes, SacI and EcoRI will leave cohesive ends on the vector (5'-AGCT-3' and 5'-AATT-3'). These enzymes are readily available from numerous vendors including New England Biolabs (Beverly, MA, Catalogue Nos. RO156S, SacI and RO101S, EcoRI). The digested pUni/V5-His version A is easily separated from the digested fragments using isopropanol precipitation. These and other methods for digesting and isolating DNA are well known to those of ordinary skill in the art (Sambrook, J., Fritsch, E.F., and T. Maniatis. (1989) Molecular Cloning, A Laboratory Manual. Second edition. Cold Spring Harbor Laboratory Press. pp 5.28 - 5.32.)

The purified, digested vector is then incubated with two specific oligonucleotide adapters and T4 DNA ligase. The adapters are oligonucleotide duplexes containing ends that are compatible with the SacI and EcoRI ends of the vector. One of skill in the art will readily appreciate that other adapter oligonucleotides with appropriate sequences can be made for other vectors having different restriction sites. Following incubation with T4 DNA ligase, the vector containing the ligated adapters is purified using isopropanol. The adapter duplex that results from the annealing of TOPO D1 and TOPO D2 has a single-stranded Eco R1 overhang at one end and a 12-nucleotide single-stranded overhang at the other end.

The first adapter oligonucleotide, (TOPO D1), has complementation to the EcoRI cohesive end, 3'-TTAA-5'. Furthermore, TOPO D1 has an additional 24-bp including the topoisomerase consensus pentapyrimidine element 5'-CCCTT located 16-bp upstream of the 3' end. The remaining sequence and size of TOPO D1 adapter oligo is variable, and may be modified to fit a researcher's particular needs. 5'-AATTGATCCCTTCACCGACATAGTACAG-3' (SEQ ID NO:33) may be the full sequence of the adapter used.

The second adapter oligonucleotide, (TOPO D2), must have full complementation to TOPO D1. TOPO D2 complements directly 5' of the EcoRI cohesive flap, extending the bottom strand of the linearized vector. Additionally, TOPO D2 contains the sequence 3'-GTGG, which is the necessary SSS for directional cloning. In this embodiment, the SSS was chosen to complement the Kozak sequence known to help expression of ORFs in eukaryotic cells by increasing the efficiency of ribosome binding on the mRNA, however, sequence and length are highly variable to meet the specific needs of individual users. The complete sequence of TOPO D2 is 3-CTAGGGAAGTGG-5 (SEQ ID NO: 34). Similar to above, the adapter duplex that results from the annealing of oligonucleotides TOPO D4 and TOPO D5 has a single-stranded SacI overhang at one end, and a 12 nucleotide single-stranded overhang at the other end.

The third adapter oligonucleotide (TOPO D5), has complementation to the SacI cohesive end, 3'-TCGA-5'. Similar to TOPO D1, TOPO D5 has additional bases creating a single stranded overhang. The length and sequence can vary based on the needs of the user. TOPO D5's sequence is 5'-AAGGGCGAGCT-3'. (SEQ ID NO: 35)

The fourth adapter oligonucleotide (TOPO D4), has full complementation to TOPO D5, and complements directly 5' of the SacI cohesive flap extending the top strand of the linearized vector. TOPO D4 also contains the topoisomerase consensus sequence 5'-CCCIT. The remaining sequence and size of TOPO D4 adapter oligo is variable and may be modified to fit particular needs. The sequence of TOPO D4 is 3'-GACATGATACAGTTCCCGC-5', (SEQ ID NO:36) which includes an additional 12 bp single stranded overhang.

These adapter oligonucleotides can be chemically synthesized using any of numerous techniques, including the phosphoramadite method, (Caruthers, M. H., Barone, A. D., Beaucage, S. L., Dodds, D. R., Fisher, E. F., McBride, L. J., Matteucci, M., Stabinsky, Z., and Tang, J. Y., (1987) Chemical Synthesis of Deoxyoligonucleotides, Methods Enzymol. 154: 287-313). This and other methods for the chemical synthesis of oligos are well known to those of ordinary skill in the art.

Complementary annealing of the purified digested vector and the adapter oligonucleotides is done by incubation of the DNA in the presence of T4 DNA ligase. Typical ligation reactions are performed by incubation of a cloning vector with suitable DNA fragments in the presence of ligase and an appropriate reaction buffer. Buffers for ligation reactions should contain ATP to provide energy to for the reaction, as well as, reducing reagents like dithiothreitol and pH stabilizers like Tris-HCl. The ratio of concentrations for the cloning vector and the DNA fragments are dependent on each individual reaction, and formulae for their determination are abundant in the literature, (See e.g. Protocols and Applications Guide (1991), Promega Corporation, Madison, WI, p.45). T4 Ligase will catalyze the formation of a phosphodiester bond between adjacent 5'-phosphates and 3'-hydroxyl termini during the incubation. Cohesive end ligation can generally be accomplished in 30 minutes at 12-15° C, while blunt end ligation requires 4-16 hours at room temperature, (Ausubel, F.M., Brent, R., Kingston, R.E., Moore, D.D., Seidman, J.G., Smith, J.A., Struhl, K. (1992) Second Edition; Short Protocols in Molecular Biology, John Wiley & Sons, Inc., New York, NY, pp. 3.14-3.37), however parameter range varies for each experiment. In the current embodiment, purified, digested pUni/V5-His version A and the adapter oligos were incubated in the presence of T4 ligase and a suitable buffer for sixteen hours at 12.5° C. The resulting linearized and adapted vector comprises the purified cloning vector attached to the adapter oligonucleotides through base pair complementation and T4 ligase-catalyzed, phosphodiester bonds.

Efficient modification of the adapted vector with topoisomerase requires the addition of an annealing oligo to generate double stranded DNA on TOPO D1's and TOPO D4's single stranded overhangs. Vaccinia topoisomerase I initially binds non-covalently to double stranded DNA. The enzyme then diffuses along the duplex until locating and covalently attaching to the consensus pentapyrimidine sequence 5'-CCCTT, forming the topoisomerase adapted complex (See Shuman et al., U.S. Patent No 5,766,891). Modification of the adapted vector takes place in the absence of DNA ligase to prevent the formation of phosphodiester bonds between the adapted vector and the annealing oligo, since phosphodiester bonds in the non-scissile strand will prevent the dissociation of the leaving group upon cleavage.

The annealing oligonucleotide (TOPO D3), must have complementation to the single stranded DNA overhangs of TOPO D1 and TOPO D4. In the current embodiment the overhangs both share the following sequence, 5'-GACATAGTACAG-3' (SEQ ID NO:37). Therefore, TOPO D3 has the following sequence, 3-CTGTATCATGTCAAC-5, (SEQ ID NO:38) which comprises full complementation to the adapter oligos' single stranded overhang and an additional 3 bp overhang, 3'-AAC-5'.

Incubation of the adapted vector with the annealing oligo in the presence of topoisomerase will create double stranded DNA to which topoisomerase can non-covalently bind. Bound topoisomerase will search the double stranded DNA by a facilitated diffusion mechanism, until the 5'-CCCTT recognition motif is located. Cleavage of the phosphodiester backbone of the scissile strand 3' of the motif is catalyzed via a nucleophilic attack on the 3' phosphorus atom of the preferred oligonucleotide cleavage sequence 5-CCCTT, resulting in covalent attachment of the DNA to the enzyme by a 3'-phosphotyrosyl linkage, (See Shuman, S., Kane, E. M., Morham, S. G. (1989) Proc. Natl. Acad. Sci. U.S.A. 86, 9793-9796). Cleavage of the scissile strand creates a double stranded leaving group comprising the 3' end adapter oligo, downstream from the 5'-CCCTT motif, and the annealing oligo TOPO D3. Although the leaving group can religate to the topoisomerase-modified end of the vector via 5' hydroxyl-mediated attack of the phosphotyrosyl linkage, this reaction is disfavored when the leaving group is no longer covalently attached to the vector. The addition of T4 polynucleotide kinase and ATP to the cleavage/religation reaction further shifts the equilibrium toward the accumulation of trapped topoisomerase since the kinase can phosphorylate the 5' hydroxyl of the leaving group to prevent the rejoining from taking place, (Ausubel, F.M., Brent, R., Kingston, R.E., Moore, D.D., Seidman, J.G., Smith, J.A., Struhl, K. (1992) Second Edition; Short Protocols in Molecular Biology, John Wiley & Sons, Inc., New York, NY, pp. 3.14-3.30). The resulting linearized vector comprises a blunt end from the TOPO D4/D3 leaving group and a SSS bearing end from the TOPO D1/D3 leaving group. Both of the linearized cloning vector's ends are charged with topoisomerase, enabling fast, efficient and directional topoisomerase mediated insertion of an acceptor molecule.

Although the above example details the modification of pUni/V5-His version A to form the topoisomerase-modified directional cloning vector, a person of ordinary skill in the art will appreciate how to apply these methods to any plasmid, cosmid, virus, or other DNA. It should also be noted that this example demonstrates a vector containing a 5' single-stranded overhang comprising the sequence 5'-GGTG-3', however the design of adapter duplexes and annealing oligonucleotides would allow one of skill in the art to custom design overhangs of any sequence or length at one or both ends of a given vector.

Specifically, any plasmid, cosmid, virus or other DNA can be modified to possess a SSS of any convenient sequence and length. These are the basic steps: the vector is first subjected to a treatment that is known to linearize the DNA. Common procedures include, but are not limited to, restriction digestion and treatment with topoisomerase II. Following linearization, a custom SSS is added. In the above example, complementary oligonucleotides are added to the sticky ends of a restriction digestion giving the desired SSS, however SSS forming oligonucleotides can be added by T4 blunt end ligation, as well. The SSS sequence is exposed by a topoisomerase I mediated, single strand nicking. In turn, this SSS can be used to directionally insert a PCR product comprising one or more complimentary SSS.

Likewise, topoisomerase modification can be applied to any double-stranded plasmid, cosmid, virus or other piece of DNA. Methods for the attachment of topoisomerase I to double stranded DNA are well known in the art, (See Shuman et al., U.S. Patent No 5,766,891). The strategic placement of topoisomerase on to a piece of double stranded DNA is determined by the incorporation of a topoisomerase I consensus sequence, (See Shuman -et al., U.S. Patent No 5,766,891). The topoisomerase I will bind the double stranded DNA, nick the scissile strand thus revealing the predetermined single-stranded overhang sequence, and ligate the incoming PCR product in the correct, SSS mediated orientation.

### EXAMPLE 5

### Production of Custom Topoisomerase I-adapted Vectors

As an example of the application of the disclosure herein to another plasmid, pCR 2.1 (Invitrogen Corporation; Carlsbad, CA) was modified to create a topoisomerase I adapted vector with a custom single stranded sequence.

Plasmid pCR 2.1 is 3.9 kb T/A cloning vector. Within the sequence of this vector are many uniquely designed elements. These elements include an f1 origin, a ColE1 origin, a kanamycin resistance gene, an ampicillin resistance gene, a LacZ-alpha fragment and a multiple cloning sequence located within the LacZ-alpha fragment allowing for blue-white selection of recombinant plasmids. The multiple cloning sequence of pCR 2.1 contains; numerous restriction sites, including but not limited to, HindIII, SpeI and EcoRI; M13 forward and reverse primers and a T7 RNA polymerase promoter.

Construction of the topoisomerase I charged vector possessing a custom single stranded sequence consists of endonuclease digestion followed by complementary annealing of synthetic oligonucleotides and the site specific cleavage of the heteroduplex by Vaccinia topoisomerase I. Digestion of pCR 2.1 with the restriction enzymes HindIII, SpeI and EcoRI leaves HindIII and EcoRI cohesive ends on the vector. The dissociated fragment of pCR 2.1 downstream from the HindIII cleavage site is further cleaved with SpeI in order to reduce its size. By reducing the size of the fragment, the digested vector is easily purified away from the smaller digested pieces by isopropanol precipitation. These enzymes are readily available from numerous vendors including New England Biolabs, (Beverly, MA, Catalogue Nos.; RO104S, HindIII; RO133S, SpeI; RO101S, EcoRI). Methods for the digestion and the isolation of DNA are well known to those skilled in the art, (Sambrook, J., Fritsch, E.F., and T. Maniatis. (1989) Molecular Cloning, A Laboratory Manual. Second edition. Cold Spring Harbor Laboratory Press. pp. 5.28-5.32.)

The purified digested vector is incubated with four adapter oligonucleotides and T4 DNA ligase. These adapter oligonucleotides are designed to have complementation to either the HindIII cohesive end, the EcoRI cohesive end, or to each other. Following incubation with T4 DNA ligase the adapted vector is purified using isopropanol.

The first adapter oligonucleotide, (TOPO H), has complementation to the HindIII cohesive end, 3'-TCGA-5'. Furthermore, TOPO H has an additional 24 bp including the topoisomerase consensus pentapyrimidine element 5'-CCCTT located 19-bp upstream of the 3' end. The remaining sequence and size of TOPO H adapter oligo is variable, and may be modified to fit a researcher's particular needs. 5'-AGCTCGCCCTTATTCCGATAGTG-3' (SEQ ID NO: 39) is the full sequence of the adapter used.

The second adapter oligonucleotide (TOPO 16), must have full complementation to TOPO H. TOPO 16 complements directly 5' of the HindIII cohesive end, extending the bottom strand of the linearized vector. Additionally. TOPO 16 contains the sequence 3'-TAAG, which is the chosen single stranded sequence for directional cloning. The complete sequence of TOPO 16 is 3'-GCGGGAATAAG-5' (SEQ ID NO: 40).

The third adapter oligonucleotide (TOPO 1), has complementation to the EcoRI cohesive end, 3'-TTAA-5'. Similar to TOPO H, TOPO 1 has additional bases containing the topoisomerase I consensus sequence CCCTT located 12 bp upstream of the 3' end. The length and sequence of TOPO 1 can vary based on the needs of the user. TOPO 1's sequence is 5'-AATTCGCCCTTATTCCGATAGTG-3' (SEQ ID NO: 41).

The fourth adapter oligonucleotide (TOPO 2), has full complementation to TOPO 1, and complements directly 5' of the BcoRI cohesive end extending the top strand of the linearized vector. In the current embodiment, the sequence of TOPO 2 is 3'-GCGGGAA-5'.

Complementary annealing of the purified digested vector and the adapter oligonucleotides is done by incubation of the DNA in the presence of T4 DNA ligase. T4 Ligase will catalyze the formation of a phosphodiester bond between adjacent 5'-phosphates and 3'-hydroxyl termini during the incubation. In the current embodiment, purified, digested pCR 2.1 and the adapter oligos were incubated in the presence of T4 ligase and a suitable buffer for sixteen hours at 12.5° C. The resulting linearized and adapted vector comprises the purified cloning vector attached to the adapter oligonucleotides through base pair complementation and T4 ligase-catalyzed, phosphodiester bonds. Ligation techniques are abundant in the literature, (see Ausubel, F.M., et al, (1992) Second Edition; Short Protocols in Molecular Biology, John Wiley & Sons, Inc., New York, NY, pp. 3.14-3.37)

Charging of the adapted vector with topoisomerase requires the addition of annealing oligonucleotides to generate double stranded DNA on TOPO H's and TOPO 1's single stranded overhangs. Charging of the adapted vector takes place in the absence of DNA ligase to prevent the formation of phosphodiester bonds between the adapted vector and the annealing oligo, since phosphodiester bonds in the non-scissile strand will prevent the dissociation of the leaving group upon cleavage.

The annealing oligonucleotide (TOPO 17), must have complementation to the single stranded DNA overhang of TOPO H. In the current embodiment the overhang has the following sequence, 5'-CGATAGTG-3'. Therefore, TOPO 17 has the following sequence, 3'-GCTATCAC-5', which comprises full complementation to the adapter oligo's single stranded overhang.

The annealing oligonucleotide (TOPO 3), must have complementation to the single stranded DNA overhang of TOPO 1. In the current embodiment The overhang has the following sequence, 3'-GTGATAGCCTTA-5' (SEQ ID NO: 42). Therefore, TOPO 3 has the following sequence, 5'-CAACACTATCGGAAT-3', (SEQ ID NO: 43) which comprises full complementation to the adapter oligo's single stranded overhang and an additional 3 bp overhang, 5'-CAA-3'.

Incubation of the adapted vector with the annealing oligo in the presence of topoisomerase will create double stranded DNA to which topoisomerase can non-covalently bind Bound topoisomerase will search the double stranded DNA by a facilitated diffusion mechanism, until the 5'-CCCTT recognition motif is located. Cleavage of the phosphodiester backbone of the scissile strand 3' of the motif will result in the covalent attachment of the DNA to the enzyme by a 3'-phosphotyrosyl linkage, (See Shuman, S., et al (1989) Proc. Natl. Acad. Sci. U.S.A. 86, 9793-9796). Cleavage of the scissile strand creates a double stranded leaving group comprising the 3' end the adapter oligos, downstream from the 5'-CCCTT motif, and the complementary annealing oligonucleotide. The leaving group can religate to the topoisomerase adapted vector through its 5' hydroxyl's attack of the phosphotyrosyl linkage, also catalyzed by topoisomerase. Addition of T4 polynucleotide kinase to the equilibrium reaction prevents the back reaction via the kinase-mediated phosphorylation of the leaving group's 5' hydroxyl, (Ausubel, F.M., et al (1992) Second Edition; Short Protocols in Molecular Biology, John Wiley & Sons, Inc., New York, NY, pp. 3.14-3.30). The resulting linearized vector comprises a blunt end from the TOPO 1/3 leaving group and a single stranded sequence end from the TOPO H/17 leaving group. Both of the linearized cloning vector's ends are charged with topoisomerase, enabling fast, efficient and directional topoisomerase mediated insertion of an acceptor molecule.

### EXAMPLE 6

### Directional Cloning Using Topoisomerase

Described herein is a method for directional cloning of DNA. In such methods, the TOPO SSS vector constructed from pUni/V5-His version A was used for the directional insertion of ORFs from the GeneStorm Expression Ready Clones (Invitrogen Corporation, Carlsbad, CA). The modified pUni vector was selected for the cloning of these ORF's because the single strand added to the vector has homology to the Kozak sequence known to enhance ORF expression. Note, however, that, as before, any plasmid, cosmid, virus or other DNA could be modified to possess the necessary single stranded sequence. Likewise, any DNA fragment could be modified to possess a homologous sequence to any vector SSS. As a point of interest, the sequence of the SSS can effect directional cloning efficiencies. For example, SSSs with low GC content will have lower annealing stability, also SSSs that have high complementation to both ends of a DNA fragment to be cloned will loose the capability to direct these DNA inserts. Thus the sequence of a SSS should be carefully designed to avoid these and similar problems.

This disclosure is particularly useful in the directional insertion of PCR products into vectors constructed according to the disclosure herein. In the PCR amplification of the desired insert, the PCR primers are designed so as to complement identified sequences of the insert(s) that are to be directionally cloned into the TOPO SSS vector. The primer designed to bind upstream of the DNA's coding strand is modified with an additional vector SSS complementation sequence on its 5' end. The resulting PCR product will possess a complementary sequence allowing SSS mediated directional insertion into the TOPO SSS cloning vector and subsequent expression of the product.

One such embodiment comprises introducing to a donor duplex DNA substrate a SSS site by PCR amplifying the donor duplex DNA molecule with the 5' oligonucleotide primer containing the SSS. PCR amplification of a region of DNA is achieved by designing oligonucleotide primers that complement a known area outside of the desired region. In a preferred embodiment the primer that has homology to the coding strand of the double stranded region of DNA will possess an additional sequence of nucleotides complementary to the SSS of the TOPO SSS cloning vector.

Using the disclosure herein in a high throughput format, we selected 82 known ORFs from the GeneStorm expression system (Invitrogen Corporation, Carlsbad, CA) for directional cloning into the TOPO SSS vector, however, any sequence of DNA may be selected as desired by individual users. For each of these ORFs, primers are designed with homology to the coding and the non-coding strands. To clone PCR products in a directional fashion into the modified pUni/V5-His version A TOPO SSS vector as described in Example 4, one primer of a given pair was modified to contain primer of a given pair was modified to contain the nucleotide sequence complementary to the SSS contained within the vector. In the current example, the coding primer contained the added sequence 5'-CACC-3', which complements the 'SSS', 3'-GTGG-5', of the TOPO SSS cloning vector. PCR amplification of the above ORFs with their respective primers will produce double stranded DNA fragments, which possess the SSS at their 5' end. We used pfu polymerase in our PCR amplification, but it is well-known that PCR reactions can be performed with either a non-thermophillic polymerase such as pfu or with a thermophillic polymerase like taq followed by a blunting step to remove the non-template nucleotide these enzymes leave at the end of PCR products.

In the present example, 0.1 µg of each primer was combined with 0.05 µg of DNA containing an ORF in a PCR reaction mix totaling 50 µl total volume. Besides the primers and vector, the reaction mix also contained water, PCR buffer salts, 10mM dNTPs and 1.25 units of *pfu* polymerase. Thermal cycling temperatures were as follows: an initial 94° C denaturation; followed by 25 repetitions of 94° C denaturation, 55° C primer annealing, and 72° C elongation, each at one minute; and ended with a 72° C, fifteen minute elongation. These parameters will vary with each DNA fragment to be amplified, and can be optimized for fragments of varying lengths and composition using methods well known to those of ordinary skill in the art (Ausubel, F.M., Brent, R., Kingston, R.E., Moore, D.D., Seidman, J.G., Smith, J.A., Struhl, K. (1992) Second Edition; Short Protocols in Molecular Biology, John Wiley & Sons, Inc., New York, NY, pp. 15.3-15.4). Techniques for the conversion of 3' overhangs to blunt end termini will also be familiar to those of ordinary skill in the art (Protocols and Applications Guide (1991), Promega Corporation, Madison, WI, pp.43-44).

Incubation of the PCR amplified donor duplex DNA -containing the SSS complementary sequence with the modified pUni/V5-His version A TOPO SSS vector results in the directional cloning of the donor DNA. For example, the eighty-two ORFs from the GeneStorm clone collection (Invitrogen Corporation, Carlsbad, CA) were amplified using SSS adapted primers. Amplification of the 82 GeneStorm ORFs with the described modified primer pairs resulted in PCR products that had the SSS complementary sequence at their 5' end. This ORF PCR product is combined with 10 ng of TOPO SSS cloning vector in either sterile water or a salt solution. The reaction is mixed gently and incubated for 5 minutes at room temperature (22-23°C). After five minutes, we placed the reaction on ice then proceeded to the OneShot® Chemical Transformation or Electroporation (Invitrogen Corporation, Carlsbad, CA, Catalogue Nos. C4040-10 and C4040-50, respectively) (Invitrogen TOPO Cloning Protocol. Invitrogen Corporation Carlsbad, CA). Topoisomerase had joined the adjacent strands of the vector and the product by catalyzing a rejoining reaction (Figure 17). DNA fragments constructed with the SSS at their 5' ends were thus correctly inserted into TOPO SSS cloning vectors with a high efficiency.

Directional insertion of DNA fragments containing 5' SSS occurs with greater than 90% efficiency as shown by sequencing multiple colonies of transformed host cells. In the current example, the TOPO SSS cloning vectors containing the GeneStorm ORFs were incubated with transformation competent *E. coli* host cells. In 74 of the transformation reactions, the directional cloning of the ORFs into the TOPO SSS cloning vector occurred in at least seven of the eight colonies picked, and 59 of these cloning reactions were directional in all eight colonies pricked. The overall directional cloning score was 609 of 656, thus, directional insertion was present in over 93% of the clones picked (see Table 1).

**Table 1. Directional Cloning of ORFs using a TOPO SSS Cloning Vector**

| Positive colonies. dPCR reactions | Clones tested |
|---|---|
| 8/8 | 59 |
| 7/8 | 15 |
| 6/8 | 2 |
| 5/8 | 1 |
| 4/8 | 3 |
| 3/8 | 2 |

### EXAMPLE 7

### Directional Cloning of a Reporter Gene

In a similar example, using the above described modified pCR2.1 TOPO SSS vector, a PCR-generated ORF encoding the gene encoding the reporter molecule Green Fluorescent Protein (GFP) was directionally cloned in frame with the lacZ a fragment present in the vector. The primers used to amplify the GFP gene contained the requisite SSS complementation sequence 5'-ATTC-3', and the known sequence for translation initiating methionine, 5'-ATG-3'. Using the necessary cloning steps noted above, the PCR amplified GFP was inserted into the vector and transformed cells were grown on solid Agar plates. Glowing colonies represented a correctly inserted PCR product (see Table 2).

**Table 2. In-frame and Directional Insertion of GFP Into Modified pGR2.1 TOPO SSS Cloning Vector.**

| 5' sequence of PCR Product | Percentage of Correct Inserts | Total White Colonies |
|---|---|---|
| 5'-ATTCATG-3' (homologous) | 86% | 457 |
| 5'-CAAGATG-3' (non-homologous) | 35% | 118 |
| 5'-ATTCGGATG-3' (frame shift) | 0% | 268 |
| VECTOR ONLY | 0% | 31 |

These data represent a substantial improvement over the current state of the art in cloning, and furthermore allow in cloning that is highly compatible with high throughput techniques. Given directional cloning efficiencies greater than 90%, a user need only screen two colonies for each cloned DNA fragment. Thus, on a 96-well plate, 48 separate clones can be screened for directional insertion, 400% more than current cloning techniques. Use of this will streamline many high-throughput-gene-expression operations, and allow them to be run at a fraction of their current costs.

### EXAMPLE 8

### Directional Topoisomerase Cloning of Blunt-end PCR Products into Entry Vectors

### Overview

In additional embodiments, the compositions, kits and methods of the described herein combine a highly efficient, 5-minute cloning strategy ("TOPO® Cloning;" Invitrogen Corporation, Carlsbad, CA) to directionally clone blunt-end PCR products into vectors for entry into the recombinational cloning system (e.g., the GATEWAY™ System available from Invitrogen Corporation, Carlsbad, CA). Using this cloning strategy as described herein, blunt-end PCR products clone directionally at greater than 90% efficiency, with no ligase, post-PCR procedures, or restriction enzymes required.

For optimal expression of a PCR product after recombination with the GATEWAY™ destination vector of interest, any suitable expression vector may be used. Examples include, but are not limited to, the pENTR Directional TOPO^{®} vectors available commercially (Invitrogen Corporation; Carlsbad, CA), which have a number of benefits including the following:

| Vector | Benefits |
|---|---|
| pENTR/D-TOPO^{®} | •For efficient expression of a gene of interest after recombination with a GATEWAY^{™} destination vector |
| pENTR/SD/D-TOPO^{®} | •Contains a T7 gene 10 translational enhancer and a ribosome binding site for optimal expression of native protein after recombination with a prokaryotic GATEWAY^{™} destination vector • Also suitable for efficient expression of a gene of interest in other host cell systems (e.g., mammalian, insect, yeast) after recombination with a suitable GATEWAY™ destination vector |

These pENTR/D-TOPO^{®} and pENTR/SD/D-TOPO^{®} vectors are designed to facilitate rapid, directional TOPO^{®} Cloning of blunt-end PCR products for entry into the GATEWAY™ System. Features of these vectors include:
- attL1 and attL2 sites for site-specific recombination of the entry clone with a GATEWAY™ destination vector;
- Directional TOPO^{®} Cloning site for rapid and efficient directional cloning of blunt-end PCR products;
- rrnB transcription termination sequences to prevent basal expression of the PCR product of interest in E coli;
- Kanamycin resistance gene for selection in E. coli;
- pUC origin for high-copy replication and maintenance of the plasmid in E. coli; and
- T7 gene 10 translation enhancer and ribosome binding site for efficient translation of the PCR product in prokaryotic systems (pENTR/SD/D-TOPO^{®} only).

Using these pENTR Directional TOPO^{®} vectors in conjunction with the GATEWAY™ recombinational cloning system described herein, genes of interest contained in blunt-end PCR products may be readily expressed by following several simple steps:
1. the blunt-end PCR product is cloned (using topoisomerase in the "TOPO^{®} Cloning" procedures described herein) into one of the pENTR TOPO^{®} vectors described above, to generate an entry clone;
2. an expression construct is generated by performing a recombination reaction between this entry clone and a GATEWAY™ destination vector of choice (such as those described elsewhere herein); and
3. the expression construct is introduced into an appropriate host cell (e.g., a bacterial, mammalian, yeast, insect, or other appropriate host cell, the choice depending on the specific destination vector chosen for production of the expression construct above), and the recombinant protein encoded by the gene of interest on the PCR product (and now contained on the expression construct) is expressed using expression conditions appropriate for the particular host cell system.

### Directional TOPO^{®} Cloning

Topoisomerase I from Vaccinia virus binds to duplex DNA at specific sites (CCCTT) and cleaves the phosphodiester backbone in one strand (Shuman, 1991). The energy from the broken phosphodiester backbone is conserved by formation of a covalent bond between the 3' phosphate of the cleaved strand and a tyrosyl residue (Tyr-274) of topoisomerase I. The phospho-tyrosyl bond between the DNA and enzyme can subsequently be attacked by the 5' hydroxyl of the original cleaved strand, reversing the reaction and releasing topoisomerase (Shuman, 1994). TOPO^{®} Cloning exploits this reaction to efficiently clone PCR products.

Directional joining of double-stranded DNA using TOPO^{®}-charged oligonucleotides occurs by adding a 3' single-stranded end (overhang) to the incoming DNA (Cheng and Shuman, 2000). This single-stranded overhang is identical to the 5' end of the TOPO^{®}-charged DNA fragment. This approach has been modified by adding a 4 nucleotide overhang sequence to the TOPO^{®}-charged DNA and adapting it to a "whole vector" format.

In this system, PCR products are directionally cloned by adding four bases to the forward primer (CACC). The overhang in the cloning vector (GTGG) invades the 5' end of the PCR product, anneals to the added bases, and stabilizes the PCR product in the correct orientation. Inserts can be cloned in the correct orientation with efficiencies equal to or greater than 90%.

### Methods

Designing PCR Primers. The design of the PCR primers to amplify a gene of interest is critical for expression. Depending on the pENTR TOPO^{®} vector being used, several considerations must be kept in mind during design of PCR primers, including:
- the sequences required to facilitate directional cloning;
- the sequences required for proper translation initiation of the PCR product; and
- whether or not the PCR product is to be fused in frame with an N- or C-terminal tag after recombination of the entry clone with a GATEWAY™ destination vector.

Guidelines to Design the Forward PCR Primer. When designing the forward PCR primer, the following points must be considered.

To enable directional cloning, the forward PCR primer MUST contain the sequence, CACC, at the 5' end of the primer. The four nucleotides, CACC, base pair with the overhang sequence, GTGG, in each pENTR TOPO^{®} vector.

If the PCR product is to be expressed in mammalian cells (following recombination of the entry clone with a GATEWAY™ destination vector), the sequence of interest must include a Kozak translation initiation sequence with an ATG initiation codon for proper initiation of translation (Kozak, 1987; Kozak, 1991; Kozak, 1990). An example of a Kozak consensus sequence is (G/A)NNATGG. Other sequences are possible, but the G or A at position -3 and the G at position +4 are the most critical for function (shown in bold). The ATG initiation codon is shown underlined. Note: If the sequence of interest does not contain an initiation codon within the context of a Kozak sequence, the forward PCR primer may be designed so as to contain a Kozak sequence at the 5' end of the primer (see below).

If the PCR product is to be expressed in prokaryotic cells without an N-terminal fusion tag (following recombination of the entry clone with a GATEWAY™- destination vector), the PCR product should be TOPO^{®} Cloned into a pENTR/SD/D-TOPO^{®} entry vector. As noted above, pENTR/SD/D-TOPO^{®} contains a T7 gene 10 translational enhancer and a ribosome binding site (RBS) to enable efficient translation of the PCR product in E. coli. To ensure optimal spacing for proper translation, the forward PCR primer should be designed such that that the ATG initiation codon of the PCR product directly follows the CACC necessary for directional cloning (see below).

Example of Forward Primer Design. Below is the DNA sequence of the N-terminus of a theoretical protein and the proposed sequence for a corresponding forward PCR primer. The ATG initiation codon is underlined.

| | |
|---|---|
| DNA sequences: | 5'-ATG GA TCT GAT AAA (SEQ ID NO: 44) G |
| Proposed Forward PCR primers: | 5'-C ACC GA TCT GAT AAA ATG G (SEQ ID NO:45) |

If the forward PCR primer is designed as noted above, then (a) the ATG initiation codon falls within the context of a Kozak sequence (see boxed sequence), allowing proper translation initiation of the PCR product in mammalian cells (note that the first three base pairs of the PCR product following the 5' CACC overhang will constitute a functional codon); and (b) the ATG initiation codon is properly spaced from the RBS (in pENTR/SD/D-TOPO^{®} only), allowing proper translation of the PCR product in prokaryotic cells.

Guidelines to Design the Reverse Primer. When designing your reverse PCR primer, consider the following points below. See Figures 26 and 27 for diagrams of the TOPO^{®} Cloning sites for pENTR/D-TOPO^{®} and pENTR/SD/D-TOPO^{®}, respectively.

To ensure that the PCR product clones directionally with high efficiency, the reverse PCR primer MUST NOT be complementary to the overhang sequence GTGG at the 5' end. A one base pair mismatch can reduce the directional cloning efficiency from 90% to 50%, increasing the likelihood that the ORF will be cloned in the opposite orientation (see "example A" below). We have not observed evidence of PCR products cloning in the opposite orientation from a two base pair mismatch.

If the PCR product is to be fused in frame with a C-terminal tag (following recombination of the entry clone with a GATEWAY™- destination vector), then the reverse PCR primer should be designed so as to remove the native stop codon in the gene of interest (see "example B" below).

If the PCR product is NOT to be fused in frame with a C-terminal tag (following recombination of the entry clone with a GATEWAY™-destination vector), then the native sequence containing the stop codon should be included in the reverse primer, or it should be ensured that the stop codon is upstream from the reverse PCR primer binding site (see "example B" below).

Example A of Reverse Primer Design. Below is the sequence of the C-terminus of a theoretical protein. The protein should be fused in frame with a C-terminal tag (following recombination of the entry clone with a GATEWAY™- destination vector). The stop codon is underlined. DNA sequence: AAG TCG GAG CAC TCG ACG ACG GTG TAG-3' One solution is to design the reverse PCR primer to start with the codon just upstream of the stop codon, but the last two codons contain GTGG (underlined below), which is identical to the 4 bp overhang sequence. As a result, the reverse primer will be complementary to the 4 bp overhang sequence, increasing the probability that the PCR product will clone in the opposite orientation. This situation should be avoided. DNA sequence: AAG TCG GAG CAC TCG ACG ACG GTG TAG-3' (SEQ ID NO: 46) Proposed Reverse PCR primer sequence: TG AGC TGC TGC CAC AAA-5' (SEQ ID NO: 47)

Another solution is to design the reverse primer so that it hybridizes just downstream of the stop codon, but still includes the C-terminus of the ORF. Note that the stop codon will need to be replaced with a codon for an innocuous amino acid such as glycine, alanine, or lysine.

Example B of Reverse Primer Design. Below is the sequence for the C-terminus of a theoretical protein. The stop codon is underlined. ...GCG GTT AAG TCG GAG CAC TCG ACG ACT GCA TAG-3' (SEQ ID NO: 48) To fuse the ORF in frame with a C-terminal tag (supplied by the destination vector after recombination), remove the stop codon by starting with nucleotides homologous to the last codon (TGC) and continue upstream. The reverse primer will be:
5'-TGC AGT CGT CGA GTG CTC CGA CTT-3' (SEQ ID NO: 49)
This will amplify the C-terminus without the stop codon and allow the ORF to be joined in frame with a C-terminal tag. If it is not desirable to join the ORF in frame with a C-terminal tag, the reverse primer should simply be designed to include the stop codon:
5'-CTA TGC AGT CGT CGA GTG CTC CGA CTT-3' (SEQ ID NO: 50)
Important: It must be remembered that the pENTR TOPO^{®} vectors accept blunt-end PCR products. 5' phosphates should not be added to the primers for PCR, as this will prevent ligation into the pENTR TOPO^{®} vectors. In addition, it is recommended that the oligonucleotides be gel-purified prior to use, especially if they are long (> 30 nucleotides).

### Producing Blunt-End PCR Products

Once a PCR strategy has been chosen and primers synthesized according to the guidance presented above, the blunt-end PCR product can be produced. Any thermostable, proofreading polymerase may be used for this purpose, including ThermalAce™, PLATINUM^{®}, *Pfx, Pfu*, or Vent^{®} for PCR. To produce blunt-end PCR products, the instructions and recommendations of the manufacturer of the polymerase should be followed. It is important to optimize PCR conditions to produce a single, discrete PCR product. Gel purification of PCR fragments, according to methods outlined below, is also recommended.

### Producing PCR Products

To produce amplification products via PCR, 25 µl or 50 µl PCR reaction mixtures are set up using the following guidelines:

Follow the manufacturer's instructions for the DNA polymerase that is being used

Use the cycling parameters suitable for the primers and template.

Use a 7 to 30 minute final extension to ensure that all PCR products are completely extended.

After cycling, the tube should be placed on ice or stored at -20°C for up to 2 weeks.

### Checking the PCR Product

To verify quality and quantity of the PCR product, 5 µl to 10 µl should be removed from each PCR reaction and analyzed by agarose gel electrophoresis for the following:

The presence of a single, discrete band of the correct size. If there is not a single, discrete band, consult the manufacturer's recommendations for optimizing PCR reactions with the chosen polymerase. Alternatively, the desired product may be gel purified (see below).

Estimate the concentration of the PCR product. For TOPO^{®} Cloning, a 5:1 molar ratio of PCR product to TOPO^{®} vector is recommended to obtain the highest cloning efficiency. For example, 20 ng of a 500bp PCR product, or 10 ng of a 1000bp PCR product, may be used in a TOPO^{®} Cloning reaction. The concentration of the PCR product may need to be adjusted before proceeding to TOPO^{®} Cloning.
Note: If ThermalAce™ polymerase is being used to produce the blunt-end PCR product, it should be noted that ThermalAce™ can generate higher yields than other proofreading polymerases. When generating PCR products in the 0.5 to 1.0 kb range, we generally dilute the PCR reaction 1:5 in 1X ThermalAce™ buffer before performing the TOPO^{®} Cloning reaction. For PCR products larger than 1.0 kb, dilution may not be required.

### Setting Up the TOPO^{®} Cloning Reaction

### Introduction

Once you have produced the desired PCR product, you are ready to TOPO^{®} Clone it into the pENTR TOPO^{®} vector and transform the recombinant vector into TOP10 *E*. *coli.* It is important to have everything you need set up and ready to use to ensure that you obtain the best possible results. We suggest that you read the sections entitled Setting Up the TOPO^{®} Cloning Reaction (pages 10-11) and Transforming OneShot^{®} TOP10 Competent Cells (pages 12-14) before beginning. If this is the first time you have TOPO^{®} Cloned, perform the control reactions on pages 22-24 in parallel with your samples.

If you are TOPO^{®} Cloning in HTP format (see below), you may transform TOP10 *E. coli* using Bulk TOP10 cells (500 reaction kits) or MultiShot™ TOP10 cells (480 reaction kits). Depending on which kit you are using, see the TOPO^{®} Cloning and transformation protocols on pages 15-16 or 17-18.

Note: Recent experiments demonstrate that including salt (200 mM NaCl, 10 mM MgCl₂) in the TOPO^{®} Cloning reaction may result in an increase in the number of transformants. From these results, we recommend adding salt to the TOPO^{®} Cloning reaction. A stock salt solution is provided in the kit for this purpose. Please note that the amount of salt added to the TOPO^{®} Cloning reaction varies depending on whether you plan to transform chemically competent cells (provided) or electrocompetent cells (see page x for ordering information). For this reason two different TOPO^{®} Cloning reactions are provided to help you obtain the best possible results. Please read the following information carefully.

### Transforming Chemically Competent E. coli

For TOPO^{®} Cloning and transformation into chemically competent *E. coli*, adding sodium chloride and magnesium chloride to a final concentration of 200 mM NaCl, 10 mM MgCl₂ in the TOPO^{®} Cloning reaction increases the number of colonies over time. A Salt Solution (1.2 M NaCl, 0.06 M MgCl₂) is provided to adjust the TOPO^{®} Cloning reaction to the recommended concentration of NaCl and MgCl₂.

### Transforming Electrocompetent E. coli

For TOPO^{®} Cloning and transformation of electrocompetent *E. coli,* salt may also be included in the TOPO^{®} Cloning reaction, but the amount of salt must be reduced to 50 mM NaCl, 2.5 mM MgCl₂ to prevent arcing when electroporating. Dilute the Salt Solution 4-fold with water to prepare a 300 mM NaCl, 15 mM MgCl₂ solution for convenient addition to the TOPO^{®} Cloning reaction.

### Setting Up the TOPO^{®} Cloning Reaction

The table below describes how to set up your TOPO^{®} Cloning reaction (6 µl) for eventual transformation into either chemically competent One Shot^{®} TOP0 *E. coli* (provided) or electrocompetent *E. coli*. Additional information on optimizing the TOPO^{®} Cloning reaction for your needs can be found on page 21. If you generated your PCR product using ThermalAce- polymerase, please note that you may need to dilute your PCR reaction before proceeding (see page 9).

Note: The blue color of the TOPO^{®} vector solution is normal and is used to visualize the solution.

**Table 3. Setting Up a TOPO^{®} Cloning Reaction Mixture.**

| Reagents* | Chemically Competent *E. coli* | Electrocompetent *E. coli* |
|---|---|---|
| Fresh PCR product | 0.5 to 4 µl | 0.5 to 4 µl |
| Salt Solution | 1 µl | - |
| Dilute Salt Solution (1:4) | - | 1 µl |
| Sterile Water | add to a final volume of 5 µl | add to a final volume of 5 µl |
| TOPO^{®} vector | 1 µl | 1 µl |

| | | |
|---|---|---|
| *Store all reagents at -20°C when finished. Salt solutions and water can be stored at room temperature or 4°C. | | |

### Performing the TOPO^{®} Cloning Reaction

Mix reaction gently and incubate for 5 minutes at room temperature (22-23°C).

Note: For most applications, 5 minutes will yield plenty of colonies for analysis. Depending on your needs, the length of the TOP011 Cloning reaction can be varied from 30 seconds to 30 minutes. For routine subcloning of PCR products, 30 seconds may be sufficient. For large PCR products (> 1 kb) or if you are TOPO^{®} Cloning a pool of PCR products, increasing the reaction time may yield more colonies.

Place the reaction on ice and proceed to Transforming One Shot7 TOP10 Competent Cells, next page. Note: You may store the TOPO7 Cloning reaction at -20°C overnight.

### Transforming One Shot^{®} TOP10 Competent Cells

### Introduction

Once you have performed the TOPO^{®} Cloning reaction, you will transform your pENTR TOPO^{®} construct into competent *E. coli.* One Shot^{®} TOP10 Chemically Competent *E. coli* (Box 2) are included with the 20 reaction kit to facilitate transformation, however, you may also transform electrocompetent cells (see page x for ordering information). Protocols to transform chemically competent or electrocompetent *E. coli* are provided in this section.

### Materials Supplied by the User

In addition to general microbiological supplies (i.e. plates, spreaders), you will need the following reagents and equipment.
(a) 42°C water bath (or electroporator with cuvettes, optional)
(b) LB plates containing 50 µg/ml kanamycin (two for each transformation)
(c) 37 °C shaking and non-shaking incubator
There is no blue-white screening for the presence of inserts. Most transformants will contain recombinant plasmids with the PCR product of interest cloned in the correct orientation. Sequencing primers are included in the kit to sequence across an insert in the multiple cloning site to confirm orientation and reading frame.

### Preparing for Transformation

For each transformation, you will need one vial of competent cells and two selective plates.

Equilibrate a water bath to 42°C (for chemical transformation) or set up your electroporator if you are using electrocompetent *E. coli.*

For electroporation, dilute a small portion of the Salt Solution 4-fold to prepare Dilute Salt Solution (e.g. add 5 µl of the Salt Solution to 150 sterile water).

Warm the vial of SOC medium from Box 2 to room temperature.

Warm LB plates containing 50 µg/ml kanamycin at 37°C for 30 minutes.

Thaw on ice 1 vial of One Shot^{®} TOP10 cells from Box 2 for each transformation.

Important: Please note that directional TOPO^{®} Cloning generally yields 5 to 10-fold fewer colonies than traditional bidirectional TOPO TA Cloning^{®}. When directionally TOPO^{®} Cloning a 750 bp test insert, we generally obtain 1800-3000 colonies using the protocol on pages 22-23. Although fewer total colonies are obtained, greater than 90% of the colonies will contain plasmid with your PCR insert in the correct orientation.

### One Shot^{®} TOP10 Chemical Transformation Protocol

1. Add 2 µl of the TOPO^{®} Cloning reaction from Performing the TOPO^{®} Cloning Reaction, Step 2, page 11 into a vial of One Shot^{®} TOP10 Chemically Competent *E. coli* and mix gently. Do not mix by pipetting up and down.
2. Incubate on ice for 5 to 30 minutes.
   Note: Longer incubations on ice seem to have a minimal effect on transformation efficiency. The length of the incubation is at the user's discretion.
3. Heat-shock the cells for 30 seconds at 42° C without shaking.
4. Immediately transfer the tubes to ice.
5. Add 250 µl of room temperature SOC medium.
6. Cap the tube tightly and shake the tube horizontally (200 rpm) at 37° C for 30 minutes.
7. Spread 50-200 µl from each transformation on a prewarmed selective plate and incubate overnight at 37° C. We recommend that you plate two different volumes to ensure that at least one plate will have well-spaced colonies.
8. An efficient TOPO7 Cloning reaction may produce several hundred colonies. Pick ∼5 colonies for analysis (see Analyzing Transformants, page 19).

### Transformation by Eletroporation

Use ONLY electrocompetent cells for electroporation to avoid arcing. Do not use the One Shot^{®} TOP10 chemically competent cells for electroporation.
1. Add 2 µl of the TOPO7 Cloning reaction from Performing the TOPO7 Cloning Reaction, Step 2, page 11 into a 0.1 cm cuvette containing 50 µl of electrocompetent E coli and mix gently. Do not mix by pipetting up and down. Avoid formation of bubbles.
2. Electroporate your samples using your own protocol and your electroporator.
   Note: If you have problems with arcing, see below.
3. Immediately add 250 µl of room temperature SOC medium.
4. Transfer the solution to a 15 ml snap-cap tube (i.e. Falcon) and shake for at least 1 hour at 37° C to allow expression of the kanamycin resistance gene.
5. Spread 20-100 µl from each transformation on a prewarmed selective plate and incubate overnight at 37° C. To ensure even spreading of small volumes, add 20 µl of SOC. We recommend that you plate two different volumes to ensure that at least one plate will have well-spaced colonies.
6. An efficient TOPO7 Cloning reaction may produce several hundred colonies. Pick ∼5 colonies for analysis (see Analyzing Transformants, page 19).

Addition of the Dilute Salt Solution in the TOPO^{®} Cloning Reaction brings the final concentration of NaCl and MgCl₂ in the TOPO^{®} Cloning Reaction to 50 mM and 2.5 mM, respectively. To prevent arcing of your samples during electroporation, the volume of cells should be between 50 and 80 µl (0.1 cm cuvettes) or 100 to 200 µl (0.2 cm curettes).

If you experience arcing during transformation, try one of the following suggestions:

Reduce the voltage normally used to charge your electroporator by 10%

Reduce the pulse length by reducing the load resistance to 100 ohms

Ethanol precipitate the TOPO^{®} Cloning reaction and resuspend in water prior to electroporation.

### High-Throughput Applications

The 480 and 500 reaction pENTR and pENTR/SD Directional TOPO^{®} Cloning Kits are specifically designed to allow production of GATEWAY™ entry clones for use in high-throughput (HTP) applications. In these kits, the pENTR TOPO^{®} vector is provided in bulk and chemically competent TOP10 *E. coli* are provided in a choice of two formats:

Cells are provided in bulk aliquots of 5 ml to allow simple transfer of the cells from a sterile trough into a 96-well plate containing the TOPO7 Cloning reaction (Catalog nos. K2400-500 and K2420-500).

Cells are provided pre-aliquoted in 96-well plates (in 12-well stripwells) to allow addition of the TOPO7 Cloning reaction to the cells (Invitrogen Corporation, Carlsbad, CA; Catalog nos. K2400-480 and K2420-480).
HTP TOPO^{®} Cloning and Transformation with Bulk Cells

### Description

In this protocol, the TOPO^{®} Cloning reaction is set up in a 96-well U bottom, polystyrene plate (Costar, Catalog no. 3366, 330 µl/well) and the TOP10 competent cells are placed in a trough for dispensing.

### Before Starting

Chill a 96-well metal heating block (VWR, Catalog no. 13259-260) on ice until the block is cold.

Bring a vial of SOC to room temperature.

Pre-heat a heat block or thermocycler containing a 96-well metal block to 42°C.

Note: You can also use a water bath, but be careful not to contaminate the cells.
- Thaw 1 tube (5 ml) of TOP10 chemically competent *E. coli* on ice (30-60 minutes).
- Warm LB agar plates containing 50 µg/ml kanamycin to 37°C. If you plan to include a pUC19 control to test the transformation efficiency of the cells, you will need LB agar plates containing 50-100 µg/ml ampicillin. Controls: For your convenience a 50 µl aliquot of competent cells is provided to perform a test TOPO^{®} Cloning and transformation reaction. In addition, you can include the pUC19 plasmid as an internal control (see Procedure below).

### Procedure

1. Set up the 6 µl TOPO^{®} Cloning reaction in each well as follows. If you include pUC19 as a control, leave 2-3 wells empty.

| | |
|---|---|
| PCR product | 1 µl |
| Salt Solution | 1 µl |
| Sterile Water | 3 µl |
| pENTR TOPO^{®} vector | 1 µl |
| Final Volume | 6 µl |

2. Incubate 5-10 minutes at room temperature.
3. Place the 96-well plate on the cooling block for 5 minutes.
4. If you are including pUC19, add 1 µl (10 pg) of the plasmid to 2-3 empty wells.
5. Pour thawed TOP10 *E. coli* into a sterile trough and immediately dispense 45 µl/well. Gently pipet up and down 1-2 times to mix.
6. Cover the plate with Parafilm^{®} and incubate it on the chilled block for 20 minutes.
7. Transfer the plate to either the pre-warmed heat block or the thermocycler and heat-shock the cells at 42°C for 30 seconds.
8. Transfer the plate back to the cooling block and press down to ensure the plate is in complete contact with the cooling block. Incubate for 1 minute.
9. Remove the Parafilm^{®} and add 150 µL/well of SOC.
10. Re-cover the plate and incubate the plate at 37°C for 1 hour.
Note: Gentle shaking (125 RPM) is optional.
11. Plate 50 µL from each well onto LB agar plates containing 50 µg/ml kanamycil. For the pUC19 controls, plate 10µl of the transformation mixture plus 20 µl of SOC on LB plates containing 100 µg/ml ampicillin.
Incubate overnight at 37°C.
12. The next day, select 5-10 colonies and process as desired.

### Too Many Colonies

If you obtain too many colonies, reduce the amount of bacterial culture plated and/or dilute the transformation with additional SOC.

### HTP TOPO Cloning and Transformation with MultiShot™ Cells

### Description

In this protocol, the TOPO^{®} Cloning reaction is set up in a 96-well plate and 2 µl are transferred to each well of a 96-well MultiShot™ plate containing 15 µl of chemically competent TOP10 *E. coli* per well. Before Starting
- Chill two 96-well metal heating blocks (VWR, Catalog no. 13259-260) on ice until the blocks are cold.
- Bring a vial of SOC to room temperature.
- Warm LB agar plates containing 50 µg/ml kanamycin to 37°C. If you plan to include a pUC19 control to test the transformation efficiency of the cells, you will need LB agar plates containing 50-100 µg/ml ampicillin.
- Pre-heat a heat block or thermocycler containing a 96-well metal block to 42°C.
- Note: You can also use a water bath, but be careful not to contaminate the cells.
- If you are using a thermocycler, program the machine to hold the temperature at 42°C.

Controls: A test plate containing 1 row (12 wells) of TOP10 cells is included to perform test TOPO^{®} Cloning reactions and transformations. In addition, you can include the pUC19 plasmid as an internal control (see Procedure below).

### Procedure

1. In a 96-well plate, set up the following 6 µl TOPO^{®} Cloning reaction in each well.

| | |
|---|---|
| PCR product | 1 µl |
| Salt Solution | 1 µl |
| Sterile Water | 3 µl |
| pENTR TOPO^{®} vector | 1 µl |
| Final Volume | 6 µl |

2. Incubate 5-10 minutes at room temperature.
3. Place the 96-well plate on one of the cooling blocks for 5 minutes.
4. Remove a 96-well MultiShot™ plate of chemically competent TOP10 *E. coli* from the freezer and place it in the second cooling block. Cells should thaw within 30 seconds.
5. Carefully remove the aluminum foil seal.
6. Use a multi-channel pipet to add 2 µl of each TOPO^{®} Cloning reaction (∼3.3 ng) to each well of the 96-well plate containing cells. Keep the volume around 2 µl for uniform results. For the pUC19 control, add 1 µl (10 pg) of the DNA.
7. Cover the cells with the supplied plastic lid and incubate the cells and DNA in the chilled block for 20 minutes.
8. Transfer the cell plate to either the pre-warmed heat block or thermocycler and heat-shock for 30 seconds at 42°C.
9. Transfer the cell plate back to a cooling block, press the plate into the block and allow the plate to cool for 1 minute.
10. Remove the plastic lid and add 90 µl SOC to each well.
11. Cover the plate with the lid and incubate the plate at 37 °C for 1 hour. Note: Gentle shaking (125 RPM) is optional.
12. Plate 100 µl from each well onto LB agar plates containing 50 µg/ml kanamycin. For the pUC19 controls, plate 10 µl of the transformation mixture plus 20 µl of SOC on LB plates containing 100 µg/ml ampicillin. Incubate overnight at 37°C.

NOTE: If you obtain too many colonies, you can reduce the amount of cells plated or dilute the TOPO^{®} Cloning reactions with sterile water or TE buffer prior to adding the reaction to the cells.

### Analyzing Transformants

### Analyzing Positive Clones

1. Pick 5 colonies and culture them overnight in LB or SOB medium containing 50-100 µg/ml kanamycin.
2. Isolate plasmid DNA using your method of choice. If you need ultra-pure plasmid DNA for automated or manual sequencing, we recommend using the S.N.A.P.J MidiPrep Kit (Catalog no. K1910-01).
3. Analyze the plasmids by restriction analysis to confirm the presence and correct orientation of the insert. Use a restriction enzyme or a combination of enzymes that cut once in the vector and once in the insert.

### Sequencing

You may sequence your construct to confirm that your gene is cloned in the correct orientation. The M13 Forward (-20) and M13 Reverse primers are included in the kit to help you sequence your insert. The M13 Forward (-20) and M13 Reverse primers are also available separately from Invitrogen (see page x for ordering information).

Important: If you download the sequence for pENTR/D-TOPO^{®} or pENTR/SD/D-TOPO^{®} from the Invitrogen Corporation Web site (see description for Figures 26 and 27 herein), note that the overhang sequence (GTGG) will be shown already hybridized to CACC. No DNA sequence analysis program allows us to show the overhang without the complementary sequence.

### Analyzing Transformants by PCR

You may analyze positive transformants using PCR. For PCR primers, use a combination of the M13 Forward (-20) primer or the M13 Reverse primer and a primer that hybridizes within your insert. You will have to determine the amplification conditions. If you are using this technique for the first time, we recommend performing restriction analysis in parallel. Artifacts may be obtained because of mispriming or contaminating template.

The protocol below is provided for your convenience. Other protocols are suitable.
1. Prepare a PCR cocktail consisting of PCR buffer, dNTPs, primers, and Taq polymerase. Use a 20 µl reaction volume. Multiply by the number of colonies to be analyzed (e.g. 5).
2. Pick 5 colonies and resuspend them individually in 20 µl of the PCR cocktail (remember to make a patch plate to preserve the colonies for further analysis).
3. Incubate reaction for 10 minutes at 94°C to lyse cells and inactivate nucleases.
4. Amplify for 20 to 30 cycles.
5. For the final extension, incubate at 72°C for 10 minutes. Store at -4°C.
6. Visualize by agarose gel electrophoresis.

Important: If you have problems obtaining transformants or the correct insert, perform the control reactions described on page 22-24. These reactions will help you troubleshoot your experiment. Long-Term Storage

Once you have identified the correct clone, be sure to purify the colony and make a glycerol stock for long term storage. We recommend that you store a stock of plasmid DNA at -20°C.
1..Streak the original colony out for single colony on LB plates containing 50 µg/ml kanamycin.
2. Isolate a single colony and inoculate into 1-2 ml of LB containing 50 µg/ml kanamycin.
3. Grow until culture reaches stationary phase.
4. Mix 0.85 ml of culture with 0.15 ml of sterile glycerol and transfer to a cryovial.
5. Store at -80°C.

### Recombining the Entry Construct with a Destination Vector

Once you have obtained your entry clone, you may recombine the PENT TOPO^{®} construct with any GATEWAY™ destination vector of choice to generate an expression clone. This "LR" recombination reaction is mediated by LR CLONASE™, a cocktail of recombination proteins. LR CLONASE™ Enzyme Mix is available from Invitrogen Corporation (Carlsbad, CA). In certain such methods, for example, TOPO-adapted vectors are incubated with one or more nucleic acid segments (*e*.*g*., one or more PCR products) at room temperature (*e*.*g*., about 20-20°C) for about 5-30 (and preferably about 10) minutes; the reaction is then heat-treated by incubation at about 80°C for about 20 minutes, and the reaction mixture then used in a standard LR reaction according to manufacturer's instructions (Invitrogen Corporation), except the incubation time for the LR reaction is increased to about 3 hours. Optimizing the TOPO^{®} Cloning Reaction

Speeding up the Cloning Process. The high efficiency of TOPO^{®} Cloning allows you to streamline the cloning process. If you routinely clone PCR products and wish to speed up the process, consider the following:
- Incubate the TOPO^{®} Cloning reaction for only 30 seconds instead of 5 minutes.

You may not obtain the highest number of colonies, but with the high efficiency of TOPO^{®} Cloning, most of the transformants will contain your insert.
- After adding 3 gl of the TOPO^{®} Cloning reaction to chemically competent cells, incubate on ice for only 5 minutes.

Increasing the incubation time to 30 minutes does not significantly improve transformation efficiency.

Obtaining More Transformants. If you are TOPO^{®} Cloning large PCR products, toxic genes, or cloning a pool of PCR products, you may need more transformants to obtain the clones you want. To increase the number of colonies:
- Incubate the salt-supplemented TOPO^{®} Cloning reaction for 20 to 30 minutes instead of 5 minutes.

Increasing the incubation time of the salt-supplemented TOPO^{®} Cloning reaction allows more molecules to ligate, increasing the transformation efficiency. Addition of salt appears to prevent topoisomerase I from rebinding and nicking the DNA after it has ligated the PCR product and dissociated from the DNA.
- Titrate the amount of PCR product used in the TOPO7 Cloning reaction for maximum colony output.

### Cloning Dilute PCR Products

To clone dilute PCR products, you may:
- Increase the amount of the PCR product
- Incubate the TOPO^{®} Cloning reaction for 20 to 30 minutes
- Concentrate the PCR product

### Performing the Control Reactions

### Introduction

We recommend performing the following control TOPO^{®} Cloning reactions the first time you use the 20 reaction kit to help you evaluate your results. Performing the control reactions involves producing a control PCR product using the reagents included in the kit and using this product directly in a TOPO^{®} Cloning reaction.

### Before Starting

For each transformation, prepare two LB plates containing 50 µg/ml kanamycin.

### Producing the Control PCR Product

Use your thermostable, proofreading polymerase and the appropriate buffer to amplify the control PCR product. Follow the manufacturer's recommendations for the polymerase you are using.
1. To produce the 750 bp control PCR product, set up the following 50 µl PCR:

| | |
|---|---|
| Control DNA Template (100 ng) | 1 µl |
| 10X PCR Buffer (appropriate for enzyme) | 5 µl |
| DNTP Mix | 0.5 µl |
| Control PCR Primers (0.1 µg/µl each) | 1 µl |
| Sterile Water | 41.5 µl |
| Thermostable polymerase (1-2.5 units/µl) | 1 µl |
| Total Volume | 50 µl |

2. Overlay with 70 µl (1 drop) of mineral oil.
3. Amplify using the following cycling parameters:

| Stop | Time | Temperature | Cycles |
|---|---|---|---|
| Initial Denaturation | 2 minutes | 94°C | 1X |
| Denaturation | 1 minute | 94°C | 25X |
| Annealing | 1 minute | 55°C | |
| Extension | 1 minute | 72°C | |
| Final Extension | 7 minutes | 72°C | 1X |

4. Remove 10 • 1 from the reaction and analyze by agarose gel electrophoresis. A discrete 750 bp band should be visible- Proceed to the Control TOPO7 Cloning Reactions, next page.

### Control TOPO^{®} Cloning Reactions

Using the control PCR product produced on the previous page and the pENTR is TOPO^{®} vector, set up two 6 pl TOPO^{®} Cloning reactions as described below.
1. Set up control TOPO^{®} Cloning reactions:

| Reagent | "Vector Only" | "Vector + PCR Insert" |
|---|---|---|
| Sterile Water | 4 µl | 3 µl |
| Salt Solution or Dilute Salt Solution | 1 µl | 1 µl |
| Control PCR Product | - | 1 µl |
| pENTR TOPO^{®} vector | 1 µl | 1 µl |

2. Incubate at room temperature for 5 minutes and place on ice.
3. Transform 3 µl of each reaction into separate vials of One Shot^{®} TOP10 cells (page 13).
4. Spread 100-200 µl of each transformation mix onto LB plates containing 50 µg/ml kanamycin. Be sure to plate two different volumes to ensure that at least one plate has well-spaced colonies.
5. Incubate overnight at 37°C.

### Analysis of Results

Hundreds of colonies from the vector + PCR insert reaction should be produced. To analyze the transformations, isolate plasmid DNA and digest with the appropriate restriction enzyme as listed below. The table below lists the digestion patterns that you should see for inserts that are cloned in the correct orientation or in the reverse orientation

| Vector | Restriction Enzyme | Expected Digestion Patterns (bp) |
|---|---|---|
| pENTR / D-TOPO^{®} | | Correct orientation: Reverse orientation: Empty vector: |
| pENTR / DS / D-TOPO^{®} | | Correct orientation: Reverse orientation: Empty vector: |

Greater than 90% of the colonies should contain the 750 bp insert in the correct orientation.
Relatively few colonies should be produced in the vector-only reaction.

### Transformation Control

pUC19 plasmid is included to check the transformation efficiency of the One Shot^{®} TOP10 competent cells. Transform one vial of One Shot^{®} TOP10 cells with 10 pg of pUC19 using the protocol on page 13. Plate 10 µl of the transformation mixture plus 20 µl of SOC on LB plates containing 100 µg/ml ampicillin. Transformation efficiency should be -1 x 10⁹ cfu/µg DNA.

### Factors Affecting Cloning Efficiency

Please note that lower cloning efficiencies will result from the following variables. Most of these are easily corrected, but if you are cloning large inserts, you may not obtain the expected 90% directional cloning efficiency.

| Variable | Solution |
|---|---|
| Low efficiency of directional cloning | Forward primer should contain CACC at the 5' end. |
| | Reverse primer is complementary to the overhang at the 5' end. Re-design primer to avoid base pairing to the overhang. |
| pH >9 in PCR amplification reaction | Check the pH of the PCR amplification reaction and adjust with 1 M Tris-HCl, pH 8. |
| Incomplete extension during PCR | Be sure to include a final extension step of 7 to 30 minutes during PCR. Longer PCR products will need a longer-extension time. |
| Cloning large inserts (>1 kh) | Increase amount of insert or gel-purify as described on pages 25-26. |
| Excess (or overly dilute) PCR product | Reduce (or concentrate) the amount of PCR product. |
| PCR cloning artifacts ("false positives") | TOPO^{®} Cloning is very efficient for small fragments (<100 bp) present in certain PCR reactions. Gel-purify your PCR product (pages 25-26) or optimise your PCR. |

### Gel Purifying PCR Products .

### Introduction

Smearing, multiple banding, primer-dimer artifacts, or large PCR products (>3 kb) may necessitate gel purification. If you wish to purify your PCR product, be extremely careful to remove all sources of nuclease contamination. There are many protocols to isolate DNA fragments or remove oligonucleotides. Please refer to Current Protocols in Molecular Biology, Unit 2.6 (Ausubel et al., 1994) for the most common protocols. Three simple protocols are provided below.

Note: cloning efficiency may decrease with purification of the PCR product (*e*.*g*. PCR product too dilute). You may wish to optimise your PCR to produce a single band (see Producing Blunt-End PCR Products, page 9). Using the S.N.A.P.™ Gel Purification Kit

The S.N.A.P.™ Gel Purification Kit available from Invitrogen (Catalog no. K1999-25) allows you to rapidly purify PCR products from regular agarose gels.
1. Electrophorese amplification reaction on a 1 to 3% regular TAE agarose gel. (Note: Do not use TBE to prepare agarose gels. Borate interferes with the sodium iodide step, below.)
2. Cut out the gel slice containing the PCR product and melt it at 65°C in 2 volumes of the 6 M sodium iodide solution.
3. Add 1.5 volumes Binding Buffer.
4. Load solution (no more than I ml at a time) from Step 3 onto a S.N.A.P.™ column. Centrifuge 1 minute at 3000 x g in a microcentrifuge and discard the supernatant.
5. If you have solution remaining from Step 3, repeat Step 4.
6. Add 900 µl of the Final Wash Buffer.
7. Centrifuge 1 minute at full speed in a microcentrifuge and discard tile flowthrough.
8. Repeat Step 7.
9. Elute the purified PCR product in 40 µl of TE or sterile water.
   Use 4 µl for the TOPO^{®} Cloning reaction and proceed as described on page 11.

### Quick S.N.A.P.™ Method

An even easier method is to simply cut out the gel slice containing your PCR product, place it on top of the S.N.A.P.™ column bed, and centrifuge at full speed for 10 seconds. Use 1-2 µl of the flow-through in the TOPO^{®} Cloning reaction (page 11). Be sure to make the gel slice as small as possible for best results.

### Low-Melt Agarose Method

If you prefer to use low-melt agarose, use the procedure below. Please note that gel purification will result in a dilution of your PCR product and a potential loss of cloning efficiency.
1. Electrophorese as much as possible of your PCR reaction on a low-melt agarose gel (0.8 to 1.2%) in TAE buffer.
2. Visualize the band of interest and excise the band.
3. Place the gel slice in a microcentrifuge tube and incubate the tube at 65°C until the gel slice melts.
4. Place the tube at 37°C to keep the agarose melted.
5. Add 4 µl of the melted agarose containing your PCR product to the TOPO^{®} Cloning reaction as described on page 11.
6. Incubate the TOPO^{®} Cloning reaction at 37 °C for 5 to 10 minutes. This is to keep the agarose melted.
7. Transform 2 to 4 µl directly into OneShot^{®} TOP10 cells using the method on page 13.

Note: the cloning efficiency may decrease with purification of the PCR product. You may wish to optimize your PCR to produce a single band.

### EXAMPLE 9

### Optimization of Reaction Conditions for TOPO Joining Reactions Using GATEWAY™ Vectors

To use TOPO Cloning procedures in conjunction with GATEWAY vectors, the optimal conditions for the combined reactions were investigated. In carrying out these studies, several questions were addressed. Sufficiency of Template for BP Reaction, and Inhibition of BP Reaction by TOPO Reaction Components

To address these issues, TOPO Tools was used as described elsewhere herein to generate attB1+CAT+attB2 templates. Secondary PCR was then performed to generate sufficient template for testing studies, and BP reactions were performed using the products. The following reaction conditions were used for each step of the process:

| TOPO Joining Reaction: | BP Reaction: |
|---|---|
| X ng of PCR product (see below) | 2 µl salt-free buffer |
| 1 µl topoisomerase | 1 µl TOPO Joining Product |
| 0.5 µl of 500 mM Tris | 0.5 µl of pDONR222 (300 ng/µl) |
| 1 µl of 40 mM NaCl | 2 µl of BP Clonase (Invitrogen) |
| 37°C for 15 min | room temp for 25 min → Proteinase |
| Transformation (chemical) | K treatment |

Following BP reactions, mixtures were chemically transformed into chemically competent *E. coli* cells (*e*.*g*., TOP10; Invitrogen Corporation) and cells were plated to determine recombination efficiency.

### Results

| | 1 | 2 | 3 | 4 | 5 | 6 |
|---|---|---|---|---|---|---|
| Colonies | 149 | 270 | 514 | 0 | 0 | 0 |
| Template Used | 0.8 ng | 1.6 ng | 4 ng | 1.6 ng | 4 ng | 0 ng |
| TOPO Joining? | No | No | No | Yes | Yes | No |

These results demonstrate that TOPO Tools generates sufficient template for the subsequent BP reaction. In addition, these results demonstrate that TOPO joining inhibits the subsequent BP reaction. Effect of Presence of attB1 and attB2 Adapters on BP Reactions

In this portion of the studies, the effects of the presence of excess attB1 and attB2 adapters in the reaction mixtures on the subsequent BP reaction were examined. To address this issue, different amounts of attB1 and attB2 adapters were added to templates (attB1+CAT+attB2, 20 ng), and BP reactions were performed under standard conditions (60 minutes at room temperature). Following BP reactions, mixtures were chemically transformed into chemically competent *E. coli* cells (*e*.*g*., TOP10; Invitrogen Corporation) and cells were plated to determine recombination efficiency.

### Results:

| | 1 | 2 | 3 | 4 | 5 | 6 |
|---|---|---|---|---|---|---|
| Adapter amount (ng) | 20 | 10 | 5 | 2.5 | 1 | 0 |
| No. of colonies formed | 270 | 475 | 760 | 590 | 340 | 460 |

These results demonstrate that the presence of an excess of attB1 and attB2 adapters has no significant effect on the transformation efficiencies observed, indicating that the BP reaction is not significantly influenced by the presence of attB1 and attB2 adapters in the reaction mixture.

### Removal of Inhibitors from TOPO Joining Reactions

To address the optimal methods for removing inhibitors from TOPO Joining reactions prior to use of the products in BP reactions, various treatment methods were assessed. TOPO Joining reactions were performed using the following reaction mixtures, incubated at room temperature for 5 minutes:

| | |
|---|---|
| attB1+attB2 (20 ng/µl each) | 2 µl |
| CAT (100 ng/µl) | 1.7 µl |
| attB1+CAT+attB2 product (10 ng/µl) | 1 µl |
| 500 mM Tris | 0.5 µl |
| Topoisomerase (1 µg/µl) | 1 µl |

Following TOPO Joining reactions, seven different samples of the reaction mixtures were treated under one of the following conditions prior to carrying out BP reactions:
(1) add 1 µl of 0.6% SDS + 3 mM EDTA to one reaction, 37°C for 15 min;
(2) add 4 µl of 0.6% SDS + 3 mM EDTA to four reactions, 37°C for 15 min, then SNAP purify into 20 µl of water,
(3) add 4 µl of 0.6% SDS + 3 mM EDTA + 1 µl of proteinase K (2 µg/µl) to 4 reactions, 37°C for 15 minutes, then SNAP purify into 20 µl of water;
(4) add 0.8 µl of 2.5 M NaCl to one reaction, 37°C for 17 minutes;
(5) add 3.2 µl of 2.5 M NaCl to four reactions, 37°C for 15 min, then SNAP purify into 20 µl of water;
(6) add 3.2 µl of 2.5 M NaCl and 1 µl of 2 µg/µl proteinase K to 4 reactions, 37°C for 15 min, then SNAP purify into 20 µl of water (positive control; 0.8 ng template used);
(7) (negative control; no template used).

BP reactions were performed using salt-free buffer for 60 min at room temperature. For unpurified mixtures, 1 µl of TOPO Joining reaction mixture was used per 10 µl of BP reaction. For purified mixtures, 5.5 µl of TOPO Joining reaction mixture was used per 10 µl of BP reaction. Following BP reactions, mixtures were chemically transformed into chemically competent *E. coli* cells (*e*.*g*., TOP10; Invitrogen Corporation) and cells were plated to determine recombination efficiency.

### Results

| | 1 | 2 | 3 | 4 | 5 | 6 | 7 | 8 |
|---|---|---|---|---|---|---|---|---|
| Treatment | SDS | SDS | SDS | NaCl | NaCl | NaCl | (+) | (-) |
| Proteinase K | - | - | + | - | - | + | | |
| Purification | - | + | + | - | + | + | | |
| No. of Colonies | 6 | 515 | 400 | 0 | 550 | 657 | 179 | 0 |

These results demonstrate that: (1) purification is not necessary to carry out the BP reaction efficiently; (2) treatment of reaction mixtures with proteinase K is not required following TOPO Joining reactions for maximum efficiency of subsequent BP reactions; and (3) SDS treatment and NaCl treatment of reaction mixtures give the same transformation efficiencies (and therefore have the same effects upon the BP reaction).

### Optimization of BP Reaction Temperature

To determine the optimum reaction temperature for carrying out BP reactions following TOPO Joining, attB1+CAT+attB2 PCR product was used as the template for BP reactions conducted under various temperatures. Following BP reactions, mixtures were chemically transformed into chemically competent *E. coli* cells (*e*.*g*., TOP10; Invitrogen Corporation) and cells were plated to determine recombination efficiency.

### Results

| BP Reaction Temperature | 42°C | 37°C | Room Temp | 14°C |
|---|---|---|---|---|
| No. of Colonies (+ Template) | 3 | 337 | 588 | 195 |
| No. of Colonies (no Template) | 0 | 4 | 0 | 0 |

These results demonstrate that room temperature (about 20-25°C) is the optimal reaction temperature for carrying out BP reactions. Optimization of Molar Ratio of attB1:insert:attB2

To determine the optimal molar ratio for attB1, insert and attB2 templates in the BP reaction, these templates were mixed in various molar ratios and BP reactions carried out under optimal conditions described above. Following BP reactions, mixtures were chemically transformed into chemically competent *E. coli* cells (*e*.*g*., TOP10; Invitrogen Corporation) and plated to determine recombination efficiency.

### Results

| Ratio of attB1:insert:attB2 | 2:1:2 | 1.5:1:1.5 | 1:1:1 | 1:2:1 | 0 (control) |
|---|---|---|---|---|---|
| No. of Colonies | 81 | 93 | 165 | 154 | 9 |

These results demonstrate that a ratio of attB1:insert:attB2 at 1:1:1 is optimal for carrying out BP reactions.

### Determination of Effect of Salt on BP Reaction

To determine whether the presence of salt in the BP reaction solution influences the recombination efficiency, BP reactions were carried out in salt-free buffers, or in standard BP reaction buffers containing salt.

### Results (No. of colonies formed)

| | | |
|---|---|---|
| Buffer Salt | - | + |
| + template | 108 | 109 |
| - template (neg. control) | 1 | 0 |

These results demonstrate that the presence or absence of salt in the reaction buffer during the BP reaction has no impact upon the recombination efficiency. Determination of Optimal Number of TOPO Joining Reactions

In the next series of experiments, the question of whether one TOPO Joining reaction is sufficient to provide optimal recombination efficiency for BP reactions after purification was examined. A single TOPO Joining reaction was carried out using the following reaction mixture:

| | |
|---|---|
| attB1 and attB2 (20 ng/µl each) | 0.5 µl |
| CAT (100 ng/µl) | 1.7 µl |
| 500 mM Tris | 0.5 µl |
| Topoisomerase (1 µg/µl) | 1 µl |
| dH₂O | sufficient to bring final volume to 5 µl |

The reaction mixture was incubated at 37°C for 15 minutes, then 1 µl of 0.6% SDS + 3 mM EDTA was added; the mixture was incubated at 37°C for 15 minutes, and then purified using a SNAP column (see above) into 20 µl of water. A BP reaction was then carried out using the product of this TOPO Joining reaction as follows:

| | |
|---|---|
| standard BP reaction buffer | 2 µl |
| pDONR222 (300 ng/µl) | 0.5 µl |
| TOPO Joining product (from above) | 5.5 µl |
| BP Clonase | 2 µl |

The reaction mixture was incubated at room temperature for 60 minutes, then 1 µl of 2 µg/µl proteinase K was added; the mixture was incubated at 37°C for 15 minutes, and then at 75°C for 15 minutes. 4 µl of this reaction mixture was then used for chemical transformation into chemically competent *E. coli* cells (*e*.*g*., TOP10; Invitrogen Corporation) and cells were then plated to determine recombination efficiency.

### Results (No. of Colonies Formed):

| + Template | - Template (neg. control) |
|---|---|
| 188 | 0 |

These results demonstrate that one TOPO Joining reaction provides sufficient template to carry out an efficient BP reaction.

### Optimization of Purification Methods

Experiments were also conducted to determine whether the SNAP purification column (Invitrogen Corporation) or the CONCERT purification system (Invitrogen Corporation) differed in providing optimal purified template for carrying out BP reactions after TOPO Joining. TOPO Joining reactions and BP reactions were conducted as described above, except that some samples were purified using SNAP columns, and other samples were purified using the CONCERT plasmid purification system after conducting the TOPO Joining reaction. Purified samples were then carried through a standard BP reaction, and reaction mixtures were then used either for transformation via chemical transformation or electroporation. Following transformation, cells were plated to determine recombination efficiency.

### Results (No. of Colonies Formed)

| Transformation Method | SNAP | CONCERT | No template (neg. control) |
|---|---|---|---|
| Chemical | 188 | 254 | 0 |
| Electroporation | 8220 | 11,460 | 672 |

These results demonstrate that both SNAP and CONCERT purification systems work well to provide purified template for BP reactions after TOPO Joining reactions.

### Optimal Conditions

Based on the results of the above experiments taken together, it was determined that the optimal conditions for combination TOPO Joining-Gateway reactions are as follows:
(1) TOPO Joining Reaction
(a) attB1/insert at 1:1 molar ratio, in 5 µl reaction volume
(b) incubate at 37°C for 15 minutes
(c) add 1 µl of 0.6% SDS + 3 mM EDTA; incubate at 37°C for 15 minutes
(d) purify with SNAP column or CONCERT system into 20 µl of dH₂O

(2) BP Reaction
(a) prepare reaction mixture:

| | | |
|---|---|---|
| (i) | purified TOPO Joining product | 5.5 µl; |
| (ii) | standard BP reaction buffer | 2 µl; |
| (iii) | pDONR222 (30 ng/µl) | 0.5 µl; |
| (iv) | BP Clonase | 2 µl; |

(b) incubate reaction mixture at room temperature for 60 minutes;
(c) add 1 µl of 2 µg/µl proteinase K;
(d) incubate at 37°C for 15 minutes;
(e) incubate at 75°C for 15 minutes;
(3) Transformation
(a) use 2-4 µl of reaction mixture from BP reaction, and carry out either chemical transformation or electroporation.

To demonstrate the efficacy of these optimized conditions, experiments were conducted using CAT and lacZ inserts of various sizes subjected to TOPO Joining and subsequent BP reactions, followed by transformation and plating.

### Results

### Chemical Transformation

| Insert | CAT | lacZ (1kb) | lacZ (1.5 kb) | lacZ (2kb) | lacZ (3.2kb) | none |
|---|---|---|---|---|---|---|
| No. of Colonies | 188 | 180 | 182 | 177 | 71 | 3 |
| Right-sized Clone | 10/10 | 18/18 | 16/16 | 17/18 | 18/18 | -- |

### Electrical Transformation

| Insert | CAT | lacZ (1kb) | lacZ (1.5 kb) | lacZ (2kb) | lacZ (3.2kb) | none |
|---|---|---|---|---|---|---|
| No. of Colonies | 8222 | 7335 | 7320 | 7500 | 6150 | 510 |

These results, taken together, demonstrate that the conditions described above are optimal for combination TOPO Joining-Gateway reactions on inserts of various sizes.

### EXAMPLE 10

### Construction of a Mammalian Expression Cassette Without Secondary PCR Methods

### Preparation of elements and gene of interest

The following primer sets (see Table 4 below) and templates were used for PCR amplification of elements and gene of interest: ,
(A) Primer set: Sequence #1 and #2; template: pcDNA 4/TetO. PCR product: 5' element.
(B) Primer set: Sequence #3 and #4; template: pcDNA 3.2/V5. PCR product: 3' element.
(C) Primer set: Sequence #5 and #6; template: pcDNA 3.1/CAT. PCR product: CAT insert.

**Table 4. Primers Used for Construction of Expression Cassette.**

| | |
|---|---|
| Sequence #1 | GTTGACATTGATTATTGACTAG (SEQ ID NO: 51) |
| Sequence #2 | GTTCCGAAGGGTTAACGCTAGAGTCCGGAGGC (SEQ ID NO: 52) |
| Sequence #3 | GACTCAAAGGGAAGGTAAGCCTATCCCTAAGG (SEQ ID NO:53) |
| Sequence #4 | GCGCAGATCTGCTATGGCAG (SEQ ID NO:54) |
| Sequence #5 | CGGAACAAGGGACCATGGAGAAAAAAATCACTGGATA (SEQ ID NO:55) |
| Sequence #6 | TGAGTCAAGGGCGCCCCGCCCTGCTGCCACTCATCG (SEQ ID NO:56) |
| Sequence #7 | GGGGACAAGTTTGTACAAAAAAGCAGGCTTCCCTTC-GGAAC (SEQ ID NO:57) |
| Sequence #8 | GTTCCGAAGGGAAGCCTGCTTTTTTGTACAAACTTGT-CCCC (SEQ ID NO:58) |
| Sequence #9 | GACTCAAAGGGACCCAGCTTTCTTGTACAAAGTGGT-CCCC (SEQ ID NO:59) |
| Sequence #10 | GGGGACCACTTTGTACAAGAAAGCTGGGTCCCTTTG-AGTC (SEQ ID NO:60) |
| Sequence #11 | CACGACGTTGTAAAACGACG (SEQ ID NO:61) |
| Sequence #12 | ATGTAATACGACTCACTATAGG (SEQ ID NO:62) |

Platinum *Taq* DNA polymerase High Fidelity (Invitrogen Corporation; Carlsbad, CA) was used for PCR. The PCR conditions were as follows:

| Components | Volume | Final Concentration |
|---|---|---|
| dH₂O | 35.5 µl | |
| 10 mM dNTP mixture (2.5 mM each) | 4 µl | 0.2 mM each |
| 10X High Fidelity PCR Buffer | 5 µl | 1X |
| 50 mM MgSO₄ | 2 µl | 2 mM |
| Primer 1 (100 ng/µl) | 1 µl | |
| Primer 2 (100 ng/µl) | 1 µl | |
| Template (10ng/µl) | 1 µl | |
| Platinum *Taq* High Fidelity (5 U/µl) | 0.5 µl | |

94°C: 4 min (1 cycle)
94 °C 30 sec ->55 °C 30 sec ->68 °C 1 min (30 cycles)
68 °C 10 min (1 cycle)
4°C (to completion)

The following conditions were used to purify PCR generated fragments:

### Reagent: SNAP MiniPrep kit (Invitrogen Corporation).

### Steps

(1) Mix 50 µl PCR product with 150 µl Binding Buffer. Mix well.
(2) Add 350 µl of Isopropanol. Mix well.
(3) Load the sample onto a SNAP MiniPrep Column.
(4) Centrifuge at 14000 rpm for 1 min. Discard the column flow through.
(5) Add 500 µl of Wash Buffer and centrifuge at 14000 rpm for 1 min. Discard the column flow through.
(6) Add 700 µl of 1X Final Wash Buffer and centrifuge at 14000 rpm for 1 min. Discard the column flow through.
(7) Dry the column by centrifuge at 14000 rpm for 1 min.
(8) Transfer the column to a new centrifuge tube. Add 50 µl of dH₂O to the column. Incubate at room temperature for 2-5 min. Centrifuge at 14000 rpm for 1 min. Collect the flow through.
(9) DNA concentration measurement by UV absorbance at 260 nm.

### TOPO Joining reaction

For production of expression cassettes with secondary PCR, the following joining conditions were used:

| | |
|---|---|
| 5' element (700 bp) | 75 ng |
| 3' element (350 bp) | 35 ng |
| 500 mM Tris (pH7.5) | 0.5 µl |
| Topoisomerase (1 µg/µl) | 0.5 µl |
| CAT insert (700 bp) | 150 ng |
| dH2O | enough to bring final volume to 5 µl |

The reaction was performed at room temperature for 5-15 min. Half volume of the reaction was used as template for the second round PCR with primer set sequence #1 and sequence #4. PCR conditions were the same as above except that the extension time was 2 min. After PCR, DNA was purified as mentioned above. Purified DNA was used for transfection.

For production of expression cassette without secondary PCR, the following joining conditions were used:

| | |
|---|---|
| 5' element (700 bp) | 510 ng |
| 3' element (350 bp) | 230 ng |
| 500 mM Tris (pH7.5) | 1.5 µl |
| Topoisomerase (1 µg/µl) | 3 µl |
| CAT insert (700 bp) | 450 ng |
| dH2O | enough to bring final volume to 15 µl |

The reaction was performed at 37°C for 15 min. Proteinase K was added to a final concentration of 50 µg/ml and the mixture was incubated at 37°C for 10 min. The treated DNA was ready for transfection.

### Gene expression study

Three cell lines (suspension TRex-CHO, adherent TRex-CHO and adherent TRex-293 cell lines) were used as model cell lines to test these expression cassettes. Standard cell culture methods were used. Twenty-four well cell culture plates were used. Lipofectamine 2000 was used as transfection reagent. Twenty-four hours after transfection, tetracycline was added at a final concentration of 1 µg/ml. For control experiment, no tetracycline was added. Cells were incubated for another 24 hours before lysis. Western blot was used for transfer of proteins and anti-V5 or anti-CAT antibody was used for detection.

### Results and Discussion

The purpose of this study was to demonstrate that expression cassettes could be generated without secondary PCR. In this study, we compared the expression data generated from an expression cassette produced using a secondary PCR step to that obtained using an expression cassette produced without a secondary PCR step. For the expression cassette produced with secondary PCR, about 1.2 µg/well of DNA was used for transfection into 24-well plate format. For the expression cassette without secondary PCR, the product from one joining reaction was used (about 1.2 µg/well). The detection data showed that functional expression cassettes can be produced using the methods described herein, without using a secondary PCR step (Figure 30).

### EXAMPLE 11

### Generation of Gateway Compatible Cassettes With Topo Tools Methods

### Preparation of adaptors

Equal amounts of sequence #7 and sequence #8 (*see* Table 4, above) were mixed in 40 mM NaCl and the mixture was denatured at 95°C for 5 min and slowly cooled to room temperature to form the attB1adaptor. Equal amounts of sequence #9 and sequence #10 (*see* Table 4, above) were mixed in 40 mM NaCl and the mixture was denatured at 95°C for 5 min and slowly cooled to room temperature to form the attB2 adaptor. TOPO Joining

CAT insert was generated as in example 10. The joining conditions were as optimized above (*see* Examples 9 and 10):

| | |
|---|---|
| attB1 adaptor (40 bp) | 10 ng |
| attB2 adaptor (40 bp) | 10 ng |
| 500 mM Tris (pH7.5) | 0.5 µl |
| Topoisomerase (1 µg/µl) | 1 µl |
| CAT insert (700 bp) | 170 ng |
| dH₂O | sufficient to bring final volume to 5 µl |

The reaction was performed at 37°C for 15 min. SDS and EDTA were added to a final concentration of 0.1% and 0.5 mM respectively. The mixture was incubated at 37°C for 15 min.

### Purification

Water (15 µl) was added to the treated mixture. DNA was purified with SNAP MiniPrep kit (Invitrogen).

### Steps

(1) Mix the treated product with 60 µl Binding Buffer. Mix well.
(2) Add 140 µl of Isopropanol. Mix well.
(3) Load the sample onto a SNAP MiniPrep Column.
(4) Centrifuge at 14000 rpm for 1 min. Discard the column flow through.
(5) Add 500 µl of Wash Buffer and centrifuge at 14000 rpm for 1 min. Discard the column flow through.
(6) Add 700 µl of 1X Final Wash Buffer and centrifuge at 14000 rpm for 1 min. Discard the column flow through.
(7) Dry the column by centrifuge at 14000 rpm for 1 min.
(8) Transfer the column to a new centrifuge tube. Add 20 µl of dH₂O to the column. Incubate at room temperature for 2-5 min. Centrifuge at 14000 rpm for 1 min. Collect the flow through.

| BP reaction | |
|---|---|
| BP reaction buffer | 2 µl |
| Purified product | 5.5 µl |
| pDONR 222 (300 ng/µl) | 0.5 µl |
| BP clonase | 2 µl |

The reaction mixture was incubated at room temperature for 60 min then 1 µl of Proteinase K (2 µg/µl) was added. The mixture was incubated at 37°C for 15 min followed by 15 min at 75°C to inactive the enzyme.

### Transformation

The treated mixture was transformed into TOP10 competent cells (chemical) or eletroporated into ElectroMax competent cells. Cells were plated onto LP-Kanamycin plates and incubated at 37°C overnight. The number of colonies was counted. To make sure that insert was present in these colonies, we designed primer sets (sequence #11 and #12) to do colony PCR. If insert was present, the PCR product would have produced a band of about 700 bp; if no insert was present, however, the PCR product band would be about 2.2 kb in size.

### Results and Discussion

In this study, we wanted to demonstrate that PCR products produced with TOPO Tools sticky ends can be directly joined to attB1 and attB2 adaptors. The joined product can be directly used in the BP recombination reaction to create GATEWAY^{™} entry clones (Table 5).

**Table 5. Colonies Generated from BP Reaction.**

| Transformation Type | attB1-Cat-attB2 | Vector only |
|---|---|---|
| Chemical | 188 | 0 |
| Electroporation | 8220 | 672 |

To further confirm the insert was present in these colonies, we picked 18 positive colonies and 2 negative colonies to do PCR. PCR results showed that right-sized product was present in all 18 colonies checked (Figure 31).

The present invention has been described in some detail by way of illustration and example for purposes of clarity of understanding, it will be obvious to one of ordinary skill in the art that the same can be performed by modifying or changing the invention within a wide and equivalent range of conditions, formulations and other parameters without affecting the scope of the invention or any specific embodiment thereof, and that such modifications or changes are intended to be encompassed within the scope of the appended claims.

The following commonly owned, co-pending U.S. patent applications are referenced: U.S. Provisional Appl. No. 60/254,510, filed December 8, 2000; U.S. Appl. No. 09/732,914, filed December 11, 2000; U.S. Provisional Appl. No. 60/291,972, filed May 21, 2001; U.S. Provisional Appl. No. 60/318,902, filed September 14, 2001; and U.S. Provisional Appl. No. 60/326,092, filed September 28, 2001.

## Claims

1. An isolated, linear nucleic acid molecule comprising, in order:
(i) at least one first Type I topoisomerase recognition site;
(ii) at least one first site recognised by a site-specific recombinase;
(iii) at least one nucleic acid segment;
(iv) at least one second site recognised by a site-specific recombinase; and
(v) at least one second Type I topoisomerase recognition site;
wherein said first and second sites recognised by a site specific recombinase do not recombine with one another

2. The nucleic acid molecule of claim 1 , wherein the separation between said first Type I topoisomerase recognition site and said first site recognised by a site-specific recombinase is about 0 to about 100 nucleotides; preferably about 0 to about 50 nucleotides.

3. The nucleic acid molecule of claim 2, wherein the separation between said first Type I topoisomerase recognition site and said first site recognised by a site-specific recombinase is about 0 to about 30 nucleotides.

4. The nucleic acid molecule of claim 3, wherein the separation between said first Type I topoisomerase recognition site and said first site recognised by a site-specific recombinase is about 0 to about 20 nucleotides.

5. The nucleic acid molecule of claim 4, wherein the separation between said first Type I topoisomerase recognition site and said first site recognised by a site-specific recombinase is about 0 to about 10 nucleotides.

6. The nucleic acid molecule of any preceding claim, wherein said first and second sites recognised by a site-specific recombinase are selected from the group consisting of:
(a) *att*B sites;
(b) *att*P sites;
(c) *att*L sites;
(d) *att*R sites;
(e) *lox* sites;
(f) *psi* sites;
(g) *dif* sites;
(h) *cer* sites; and
(i) *frf* sites
and mutants, variants and derivatives of the sites recognised by recombination proteins of (a), (b), (c), (d), (e), (f), (g), (h) or (I) which retain the ability to undergo recombination.

7. The nucleic acid molecule of claim 6, wherein said first and second sites recognised by recombination proteins are *lox* sites.

8. The nucleic acid molecule of claim 7, wherein said first and second sites recognised by recombination proteins are *lox*P or *lox*P511 sites.

9. The nucleic acid molecule of claim 1, wherein said first and second sites recognised by recombination proteins are *att* sites.

10. The nucleic acid molecule of claim 9, wherein said first and second sites recognised by recombination proteins are selected from the group consisting of *att*B sites, *att*P sites, *att*L sites and *att*R sites.

11. The nucleic acid molecule of any preceding claim, wherein said at least one nucleic acid segment comprises at least one open reading frame.

12. The nucleic acid molecule of claim 11, wherein said open reading frame encodes an antibiotic resistance gene.

13. The nucleic acid molecule of any preceding claim, wherein said type I topoisomerase is a type IB topoisomerase.

14. The nucleic acid molecule of claim 13, wherein said IB topoisomerase is selected from the group consisting of eukaryotic nuclear type 1 topoisomerase and a poxvirus topoisomerase.

15. The nucleic acid molecule of claim 14, wherein said IB topoisomerase is a poxvirus topoisomerase and is produced by or isolated from a virus selected from the group consisting of vaccinia virus, Shope fibroma virus, ORF virus, fowlpox virus, molluscum contagiosum virus and *Amsacta rnoorel* entomopoxvirus.

16. The nucleic acid molecule of claim 1, wherein said first or second site recognised by recombination proteins is separated from said topoisomerase recognition site by about 10 to about 30 nucleotides.

17. The nucleic acid molecule of claim 1, wherein said first or second site recognised by recombination proteins is separated from said topoisomerase recognition site by about 20 to about 50 nucleotides.

18. The nucleic acid molecule of claim 1, wherein said first or second site recognised by recombination proteins is separated from said topoisomerase recognition site by about 40 to about 80 nucleotides.

19. The nucleic acid molecule of claim 1, wherein said first or second site recognised by recombination proteins is separated from said topoisomerase recognition site by about 70 to about 100 nucleotides.

20. A host cell comprising the isolated nucleic acid molecule of any of claims 1 to 19.

21. A kit comprising the isolated nucleic acid molecule of any of claims 1 to 19.

22. The kit of claim 21 further comprising one or more components selected from the group consisting of one or more topoisomerases, one or more recombination proteins, one or more vectors, one or more polypeptides having polymerase activity, and one or more host cells.

23. An *in vitro* method of cloning a nucleic acid molecule comprising:
a) obtaining a first nucleic acid molecule to be cloned
b) mixing said first nucleic acid molecule to be cloned *in vitro* with a second nucleic acid molecule, said second nucleic acid molecule comprising an isolated, linear nucleic acid molecule comprising, in order:
i) at least one first Type I topoisomerase recognition site;
ii) at least a first site recognised by a site-specific recombinase;
iii) at least one nucleic acid segment;
iv) at least a second site recognised by a site-specific recombinase; and
v) at least one second Type I topoisomerase recognition site;
wherein said first and second sites recognised by a site-specific recombinase do not recombine with each other; and
c) incubating said mixture together with at least one topoisomerase under conditions such that said first nucleic acid molecule to be cloned is inserted into said second nucleic acid molecule between said first and second Type I topoisomerase recognition sites, thereby producing a first product molecule comprising said first nucleic add molecule to be cloned between said first and second sites recognised by a site-specific recombinase.

24. The method of claim 23, wherein the second nucleic acid molecule is a vector.

25. The method of claim 23, wherein said first nucleic acid molecule to be cloned is a linear nucleic acid molecule.

26. The method of claim 25, wherein said linear nucleic acid molecule Is a blunt- end nucleic acid molecule.

27. The method of claim 25, wherein said first nucleic acid molecule to be cloned is a PCR product.

28. The method of any of claims 23 to 27, wherein said first nucleic acid molecule to be cloned comprises at least one open reading frame.

29. The method of any of claims 23 to 28, further comprising contacting said first product molecule with at least one third nucleic acid molecule comprising at least a third and a fourth site recognised by a slte-speclfic recombinase that do not recombine with each other, under conditions favouring recombination between said first and third and between said second and fourth sites recognised by a site-specific recombinase, thereby producing at least one second product molecule.

30. The method of claim 29, wherein the third nucleic acid molecule is a vector.

31. The method of any of claims 23 to 29, further comprising inserting said first product molecule into a host cell.

32. The method of any of claim 30 or claim 31, further comprising inserting said second product molecule Into a host cell.

33. The method of either claim 24 or claim 30, wherein said vector is an expression vector.

34. The method of any of claims 22 to 33, wherein said second nucleic acid molecule comprises at least one additional nucleic acid sequence selected from the group consisting of a selectable marker, a cloning site, a restriction site, a promoter, an operator, an operon, an origin of replication and a gene or partial gene.

35. The method of any of claims 23 to 34, wherein said first and second sites recognised by a site-specific recombinase are selected from the group consisting of: (a) *att*B sites, (b) *att*P sites, (c) *att*L sites, (d) *att*R sites, (e) *lox* sites, (f) psi sites, (g) *dif* sites, (h) *cer* sites, (i) *frf* sites and mutants, variants and derivatives of the sites recognised by a site-specific recombinase of (a), (b), (c), (d), (e), (f), (g), (h) or (i) which retain the ability to undergo recombination.

36. The method of any of claims 29 to 35, wherein said third and fourth sites recognised by a site-specific recombinase are selected from the group consisting of: (a) *att*B sites, (b) *att*P sites, (c) *att*L sites, (d) *att*R sites, (e) *lox* sites, (f) psi sites, (g) *dif* sites, (h) *cer* sites, (i) *frf* sites and mutants, variants and derivatives of the sites recognised by a site-specific recombinase of (a), (b), (c), (d), (e), (f), (g), (h) or (i) which retain the ability to undergo recombination.

37. The method of either of claim 35 or claim 36, wherein said *lox* sites are selected form the group from the group consisting of *lox*P sites and *lox*P511 sites.

38. The method of any of claims 29 to 37, wherein said product nucleic acid molecule and said third nucleic acid molecule are combined in the presence of at least one site-specific recombinase.

39. The method of claim 38, wherein said site-specific recombinase is selected from the group consisting of: (a) Cre; (b) Int; (c) IHF; (d) Xis; (e) Fis; (f) Hin; (g) Gin; (h) Cin; (i) Tn3 resolvase; (j) TndX; (k) XerC; and (l) XerD.

40. The method of claim 39, wherein said site-specific recombinase is Cre.

41. The method of claim 39, wherein said site-specific recombinase is selected from the group consisting of Int, Xis, IHF and Fis.

## Patentansprüche

1. Ein isoliertes, lineares Nukleinsäuremolekül, das in der Reihenfolge folgendes umfasst:
(i) mindestens eine erste Erkennungsstelle für Topoisomerase vom Typ I;
(ii) mindestens eine erste Stelle, die von einer ortsspezifischen Rekombinase erkannt wird;
(iii) mindestens ein Nukleinsäuresegment;
(iv) mindestens eine zweite Stelle, die von einer ortsspezifischen Rekombinase erkannt wird; und
(v) mindestens eine zweite Erkennungsstelle für Topoisomerase vom Typ I;
wobei die ersten und zweiten Stellen, die von einer ortsspezifischen Rekombinase erkannt werden, miteinander nicht rekombinieren.

2. Das Nukleinsäuremolekül nach Anspruch 1, wobei die Trennung zwischen der ersten Erkennungsstelle für Topoisomerase vom Typ I und der ersten Stelle, die von einer ortsspezifischen Rekombinase erkannt wird, etwa 0 bis etwa 100 Nukleotide; vorzugsweise etwa 0 bis etwa 50 Nukleotide beträgt.

3. Das Nukleinsäuremolekül nach Anspruch 2, wobei die Trennung zwischen der ersten Erkennungsstelle für Topoisomerase vom Typ I und der ersten Stelle, die von einer ortsspezifischen Rekombinase erkannt wird, etwa 0 bis etwa 30 Nukleotide beträgt.

4. Das Nukleinsäuremolekül nach Anspruch 3, wobei die Trennung zwischen der ersten Erkennungsstelle für Topoisomerase vom Typ I und der ersten Stelle, die von einer ortsspezifischen Rekombinase erkannt wird, etwa 0 bis etwa 20 Nukleotide beträgt.

5. Das Nukleinsäuremolekül nach Anspruch 4, wobei die Trennung zwischen der ersten Erkennungsstelle für Topoisomerase vom Typ I und der ersten Stelle, die von einer ortsspezifischen Rekombinase erkannt wird, etwa 0 bis etwa 10 Nukleotide beträgt.

6. Das Nukleinsäuremolekül nach einem vorstehenden Anspruch, wobei die ersten und zweiten Stellen, die von einer ortsspezifischen Rekombinase erkannt werden, aus der Gruppe ausgewählt sind, die aus folgendem besteht:
(a) attB-Stellen;
(b) attP-Stellen;
(c) attL-Stellen;
(d) attR-Stellen;
(e) lox-Stellen;
(f) psi-Stellen;
(g) dif-Stellen;
(h) cer-Stellen; und
(i) frf-Stellen
und Mutanten, Varianten und Derivaten der Stellen, die von Rekombinationsproteinen aus (a), (b), (c), (d), (e), (f), (g), (h) oder (i), die die Fähigkeit zur Rekombination beibehalten, erkannt werden.

7. Das Nukleinsäuremolekül nach Anspruch 6, wobei die ersten und zweiten Stellen, die von Rekombinationsproteinen erkannt werden, lox-Stellen sind.

8. Das Nukleinsäuremolekül nach Anspruch 7, wobei die ersten und zweiten Stellen, die von Rekombinationsproteinen erkannt werden, loxP- oder loxP511-Stellen sind.

9. Das Nukleinsäuremolekül nach Anspruch 1, wobei die ersten und zweiten Stellen, die von Rekombinationsproteinen erkannt werden, att-Stellen sind.

10. Das Nukleinsäuremolekül nach Anspruch 9, wobei die ersten und zweiten Stellen, die von Rekombinationsproteinen erkannt werden, aus der Gruppe ausgewählt sind, die aus attB-Stellen, attP-Stellen, attL-Stellen und attR-Stellen besteht.

11. Das Nukleinsäuremolekül nach einem vorstehenden Anspruch, wobei das mindestens eine Nukleinsäuresegment mindestens einen offenen Leserahmen umfasst.

12. Das Nukleinsäuremolekül nach Anspruch 11, wobei der offene Leserahmen für ein Antibiotikaresistenzgen kodiert.

13. Das Nukleinsäuremolekül nach einem vorstehenden Anspruch, wobei die Topoisomerase vom Typ I eine Topoisomerase vom Typ IB ist.

14. Das Nukleinsäuremolekül nach Anspruch 13, wobei die IB-Topoisomerase aus der Gruppe ausgewählt ist, die aus eukaryotischer nukleärer Topoisomerase vom Typ I und einer Pockenvirus-Topoisomerase besteht.

15. Das Nukleinsäuremolekül nach Anspruch 14, wobei die IB-Topoisomerase eine Pockenvirus-Topoisomerase ist und von einem Virus erzeugt oder daraus isoliert wird, das aus der Gruppe ausgewählt ist, die aus Vaccinia-Virus, Shope-Fibromvirus, ORF-Virus, Geflügelpockenvirus, Molluscum contagiosum-Virus und Insekten-Pockenvirus aus Amsacta moorei besteht.

16. Das Nukleinsäuremolekül nach Anspruch 1, wobei die erste oder zweite Stelle, die von Rekombinationsproteinen erkannt wird, von der Erkennungsstelle für Topoisomerase um etwa 10 bis etwa 30 Nukleotide getrennt ist.

17. Das Nukleinsäuremolekül nach Anspruch 1, wobei die erste oder zweite Stelle, die von Rekombinationsproteinen erkannt wird, von der Erkennungsstelle für Topoisomerase um etwa 20 bis etwa 50 Nukleotide getrennt ist.

18. Das Nukleinsäuremolekül nach Anspruch 1, wobei die erste oder zweite Stelle, die von Rekombinationsproteinen erkannt wird, von der Erkennungsstelle für Topoisomerase um etwa 40 bis etwa 80 Nukleotide getrennt ist.

19. Das Nukleinsäuremolekül nach Anspruch 1, wobei die erste oder zweite Stelle, die von Rekombinationsproteinen erkannt wird, von der Erkennungsstelle für Topoisomerase um etwa 70 bis etwa 100 Nukleotide getrennt ist.

20. Eine Wirtszelle, die das isolierte Nukleinsäuremolekül aus einem der Ansprüche 1 bis 19 umfasst.

21. Ein Kit, das das isolierte Nukleinsäuremolekül aus einem der Ansprüche 1 bis 19 umfasst.

22. Das Kit nach Anspruch 21, das ferner eine oder mehr als eine Komponente umfasst, die aus der Gruppe ausgewählt ist, die aus einer oder mehr als einer Topoisomerase, einem oder mehr als einem Rekombinationsprotein, einem oder mehr als einem Vektor, einem oder mehr als einem Polypeptid mit Polymeraseaktivität und einer oder mehr als einer Wirtszelle besteht.

23. Ein In-Vitro-Verfahren zum Klonen eines Nukleinsäuremoleküls, das folgendes umfasst:
a) Gewinnen eines ersten Nukleinsäuremoleküls, das geklont werden soll
b) Vermischen des ersten Nukleinsäuremoleküls, das geklont werden soll, in vitro mit einem zweiten Nukleinsäuremolekül, wobei das zweite Nukleinsäuremolekül ein isoliertes, lineares Nukleinsäuremolekül umfasst, das in der Reihenfolge folgendes umfasst:
(i) mindestens eine erste Erkennungsstelle für Topoisomerase vom Typ I;
(ii) mindestens eine erste Stelle, die von einer ortsspezifischen Rekombinase erkannt wird;
(iii) mindestens ein Nukleinsäuresegment;
(iv) mindestens eine zweite Stelle, die von einer ortsspezifischen Rekombinase erkannt wird; und
(v) mindestens eine zweite Erkennungsstelle für Topoisomerase vom Typ I;
wobei die ersten und zweiten Stellen, die von einer ortsspezifischen Rekombinase erkannt werden, miteinander nicht rekombinieren; und
c) Inkubieren des Gemischs zusammen mit mindestens einer Topoisomerase unter solchen Bedingungen, dass das erste Nukleinsäuremolekül, das geklont werden soll, in das zweite Nukleinsäuremolekül zwischen den ersten und zweiten Erkennungsstellen für Topoisomerase vom Typ I insertiert wird, wodurch ein erstes Produktmolekül erzeugt wird, das das erste Nukleinsäuremolekül, das geklont werden soll, zwischen den ersten und zweiten Stellen, die von einer ortsspezifischen Rekombinase erkannt werden, umfasst.

24. Das Verfahren nach Anspruch 23, wobei das zweite Nukleinsäuremolekül ein Vektor ist.

25. Das Verfahren nach Anspruch 23, wobei das erste Nukleinsäuremolekül, das geklont werden soll, ein lineares Nukleinsäuremolekül ist.

26. Das Verfahren nach Anspruch 25, wobei das lineare Nukleinsäuremolekül ein Nukleinsäuremolekül mit glattem Ende ist.

27. Das Verfahren nach Anspruch 25, wobei das erste Nukleinsäuremolekül, das geklont werden soll, ein PCR-Produkt ist.

28. Das Verfahren nach einem der Ansprüche 23 bis 27, wobei das erste Nukleinsäuremolekül, das geklont werden soll, mindestens einen offenen Leserahmen umfasst.

29. Das Verfahren nach einem der Ansprüche 23 bis 28, das ferner das Inkontaktbringen des ersten Produktmoleküls mit mindestens einem dritten Nukleinsäuremolekül, das mindestens eine dritte und eine vierte Stelle umfasst, die von einer ortsspezifischen Rekombinase erkannt werden, die miteinander nicht rekombinieren, unter Bedingungen umfasst, die eine Rekombination zwischen den ersten und dritten und zwischen den zweiten und vierten Stellen, die von einer ortsspezifischen Rekombinase erkannt werden, begünstigen, wodurch mindestens ein zweites Produktmolekül erzeugt wird.

30. Das Verfahren nach Anspruch 29, wobei das dritte Nukleinsäuremolekül ein Vektor ist.

31. Das Verfahren nach einem der Ansprüche 23 bis 29, das ferner das Insertieren des ersten Produktmoleküls in eine Wirtszelle umfasst.

32. Das Verfahren nach einem der Ansprüche 30 oder 31, das ferner das Insertieren des zweiten Produktmoleküls in eine Wirtszelle umfasst.

33. Das Verfahren nach einem der Ansprüche 24 oder 30, wobei der Vektor ein Expressionsvektor ist.

34. Das Verfahren nach einem der Ansprüche 22 bis 33, wobei das zweite Nukleinsäuremolekül mindestens eine zusätzliche Nukleinsäuresequenz umfasst, die aus der Gruppe ausgewählt ist, die aus einer auswählbaren Markierung, einer Klonierungsstelle, einer Restriktionsstelle, einem Promotor, einem Operator, einem Operon, einem Replikationsursprung und einem Gen oder unvollständigen Gen besteht.

35. Das Verfahren nach einem der Ansprüche 23 bis 34, wobei die ersten und zweiten Stellen, die von einer ortsspezifischen Rekombinase erkannt werden, aus der Gruppe ausgewählt sind, die aus folgendem besteht: (a) attB-Stellen, (b) attP-Stellen, (c) attL-Stellen, (d) attR-Stellen, (e) lox-Stellen, (f) psi-Stellen, (g) dif-Stellen, (h) cer-Stellen, (i) frf-Stellen und Mutanten, Varianten und Derivate der Stellen, die von einer ortsspezifischen Rekombinase aus (a), (b), (c), (d), (e), (f), (g), (h) oder (i), die die Fähigkeit zur Rekombination beibehalten, erkannt werden.

36. Das Verfahren nach einem der Ansprüche 29 bis 35, wobei die dritten und vierten Stellen, die von einer ortsspezifischen Rekombinase erkannt werden, aus der Gruppe ausgewählt sind, die aus folgendem besteht: (a) attB-Stellen, (b) attP-Stellen, (c) attL-Stellen, (d) attR-Stellen, (e) lox-Stellen, (f) psi-Stellen, (g) dif-Stellen, (h) cer-Stellen, (i) frf-Stellen und Mutanten, Varianten und Derivate der Stellen, die von einer ortsspezifischen Rekombinase aus (a), (b), (c), (d), (e), (f), (g), (h) oder (i), die die Fähigkeit zur Rekombination beibehalten, erkannt werden.

37. Das Verfahren nach einem der Ansprüche 35 oder 36, wobei die lox-Stellen aus der Gruppe ausgewählt sind, die aus loxP-Stellen und loxP511-Stellen besteht.

38. Das Verfahren nach einem der Ansprüche 29 bis 37, wobei das Produkt-Nukleinsäuremolekül und das dritte Nukleinsäuremolekül in Gegenwart von mindestens einer ortsspezifischen Rekombinase kombiniert werden.

39. Das Verfahren nach Anspruch 38, wobei die ortsspezifische Rekombinase aus der Gruppe ausgewählt ist, die aus folgendem besteht: (a) Cre; (b) Int; (c) IHF; (d) Xis; (e) Fis; (f) Hin; (g) Gin; (h) Cln; (i) Tn3-Resolvase; (j) TndX; (k) XerC; und (l) XerD.

40. Das Verfahren nach Anspruch 39, wobei die ortsspezifische Rekombinase Cre ist.

41. Das Verfahren nach Anspruch 39, wobei die ortsspezifische Rekombinase aus der Gruppe ausgewählt ist, die aus Int, Xis, IHF und Fis besteht.

## Revendications

1. Molécule d'acide nucléique linéaire, isolée comprenant, dans l'ordre :
(i) au moins un premier site de reconnaissance de topoisomérase de type I ;
(ii) au moins un premier site reconnu par une recombinase spécifique de site ;
(iii) au moins un segment d'acide nucléique ;
(iv) au moins un second site reconnu par une recombinase spécifique de site ; et
(v) au moins un second site de reconnaissance de topoisomérase de type I ;
lesdits premier et second sites reconnus par une recombinase spécifique de site ne se recombinant pas entre eux.

2. Molécule d'acide nucléique selon la revendication 1, dans laquelle la séparation entre ledit premier site de reconnaissance de topoisomérase de type I et ledit premier site reconnu par une recombinase spécifique de site est d'environ 0 à environ 100 nucléotides, de préférence d'environ 0 à environ 50 nucléotides.

3. Molécule d'acide nucléique selon la revendication 2, dans laquelle la séparation entre ledit premier site de reconnaissance de topoisomérase de type I et ledit premier site reconnu par une recombinase spécifique de site est d'environ 0 à environ 30 nucléotides.

4. Molécule d'acide nucléique selon la revendication 3, dans laquelle la séparation entre ledit premier site de reconnaissance de topoisomérase de type I et ledit premier site reconnu par une recombinase spécifique de site est d'environ 0 à environ 20 nucléotides.

5. Molécule d'acide nucléique selon la revendication 4, dans laquelle la séparation entre ledit premier site de reconnaissance de topoisomérase de type I et ledit premier site reconnu par une recombinase spécifique de site est d'environ 0 à environ 10 nucléotides.

6. Molécule d'acide nucléique selon l'une quelconque des revendications précédentes, dans laquelle lesdits premier et second sites reconnus par une recombinase spécifique de site sont choisis dans le groupe constitué par :
(a) les sites *att*B ;
(b) les sites *att*P ;
(c) les sites *att*L ;
(d) les sites *att*R ;
(e) les sites *lox* ;
(f) les sites *psi* ;
(g) les sites *dif* ;
(h) les sites *cer* ; et
(i) les sites *frf*
et des mutants, variants et dérivés des sites reconnus par des protéines de recombinaison de (a), (b), (c), (d), (e), (f), (g), (h) ou (i) qui conservent l'aptitude à subir une recombinaison.

7. Molécule d'acide nucléique selon la revendication 6, dans laquelle lesdits premier et second sites reconnus par des protéines de recombinaison sont les sites *lox*.

8. Molécule d'acide nucléique selon la revendication 7, dans laquelle lesdits premier et second sites reconnus par des protéines de recombinaison sont les sites *lox* ou *lox*P511.

9. Molécule d'acide nucléique selon la revendication 1, dans laquelle lesdits premier et second sites reconnus par des protéines de recombinaison sont les sites *att*.

10. Molécule d'acide nucléique selon la revendication 9, dans laquelle lesdits premier et second sites reconnus par des protéines de recombinaison sont choisis dans le groupe constitué par les sites *att*B, les sites *att*P, les sites *att*L et les sites *att*R.

11. Molécule d'acide nucléique selon l'une quelconque des revendications précédentes, dans laquelle ledit au moins un segment d'acide nucléique comprend au moins un cadre de lecture ouvert.

12. Molécule d'acide nucléique selon la revendication 11, dans laquelle ledit cadre de lecture ouvert code pour un gène de résistance à un antibiotique.

13. Molécule d'acide nucléique selon l'une quelconque des revendications précédentes, dans laquelle ladite topoisomérase de type I est une topoisomérase de type IB.

14. Molécule d'acide nucléique selon la revendication 13, dans laquelle ladite topoisomérase IB est choisie dans le groupe constitué par une topoisomérase de type 1 nucléaire eucaryote et une topoisomérase de poxvirus.

15. Molécule d'acide nucléique selon la revendication 14, dans laquelle ladite topoisomérase IB est une topoisomérase de poxvirus et est produite par ou isolée à partir d'un virus choisi dans le groupe constitué par le virus de la vaccine, le virus du fibrome de Shope, le virus de l'ecthyma contagieux, le virus de la variole aviaire, le virus de *Molluscum contagiosum* et l'entomopoxvirus *Amsacta moorei.*

16. Molécule d'acide nucléique selon la revendication 1, dans laquelle ledit premier ou second site reconnu par des protéines de recombinaison est séparé dudit site de reconnaissance de topoisomérase par environ 10 à environ 30 nucléotides.

17. Molécule d'acide nucléique selon la revendication 1, dans laquelle ledit premier ou second site reconnu par des protéines de recombinaison est séparé dudit site de reconnaissance de topoisomérase par environ 20 à environ 50 nucléotides.

18. Molécule d'acide nucléique selon la revendication 1, dans laquelle ledit premier ou second site reconnu par des protéines de recombinaison est séparé dudit site de reconnaissance de topoisomérase par environ 40 à environ 80 nucléotides.

19. Molécule d'acide nucléique selon la revendication 1, dans laquelle ledit premier ou second site reconnu par des protéines de recombinaison est séparé dudit site de reconnaissance de topoisomérase par environ 70 à environ 100 nucléotides.

20. Cellule hôte comprenant la molécule d'acide nucléique isolée selon l'une quelconque des revendications 1 à 19.

21. Coffret comprenant la molécule d'acide nucléique isolée selon l'une quelconque des revendications 1 à 19.

22. Coffret selon la revendication 21, comprenant en outre un ou plusieurs composants choisis dans le groupe constitué par une ou plusieurs topoisomérases, une ou plusieurs protéines de recombinaison, un ou plusieurs vecteurs, un ou plusieurs polypeptides ayant une activité polymérase et une ou plusieurs cellules hôtes.

23. Procédé *in vitro* de clonage d'une molécule d'acide nucléique comprenant les opérations consistant à :
a) obtenir une première molécule d'acide nucléique devant être clonée
b) mélanger ladite première molécule d'acide nucléique devant être clonée *in vitro* avec une seconde molécule d'acide nucléique, ladite seconde molécule d'acide nucléique comprenant une molécule d'acide nucléique linéaire, isolée comprenant, dans l'ordre :
i) au moins un premier site de reconnaissance de topoisomérase de type I ;
ii) au moins un premier site reconnu par une recombinase spécifique de site ;
iii) au moins un segment d'acide nucléique ;
iv) au moins un second site reconnu par une recombinase spécifique de site ; et
v) au moins un second site de reconnaissance de topoisomérase de type I ;
lesdits premier et second sites reconnus par une recombinase spécifique de site ne se recombinant pas entre eux ; et
c) laisser incuber ledit mélange conjointement avec au moins une topoisomérase dans des conditions telles que ladite première molécule d'acide nucléique devant être clonée est introduite dans ladite seconde molécule d'acide nucléique entre lesdits premier et second sites de reconnaissance de topoisomérase de type I, produisant ainsi une première molécule produit comprenant ladite première molécule d'acide nucléique devant être clonée entre lesdits premier et second sites reconnus par une recombinase spécifique de site.

24. Procédé selon la revendication 23, dans lequel la seconde molécule d'acide nucléique est un vecteur.

25. Procédé selon la revendication 23, dans lequel ladite première molécule d'acide nucléique devant être clonée est une molécule d'acide nucléique linéaire.

26. Procédé selon la revendication 25, dans lequel ladite molécule d'acide nucléique linéaire est une molécule d'acide nucléique à extrémités franches.

27. Procédé selon la revendication 25, dans lequel ladite première molécule d'acide nucléique devant être clonée est un produit de PCR.

28. Procédé selon l'une quelconque des revendications 23 à 27, dans lequel ladite première molécule d'acide nucléique devant être clonée comprend au moins un cadre de lecture ouvert.

29. Procédé selon l'une quelconque des revendications 23 à 28, comprenant en outre la mise en contact de ladite première molécule produit avec au moins une troisième molécule d'acide nucléique comprenant au moins des troisième et quatrième sites reconnus par une recombinase spécifique de site qui ne se recombinent pas entre eux, dans des conditions favorisant une recombinaison entre lesdits premier et troisième et entre lesdits second et quatrième sites reconnus par une recombinase spécifique de site, produisant ainsi au moins une seconde molécule produit.

30. Procédé selon la revendication 29, dans lequel la troisième molécule d'acide nucléique est un vecteur,

31. Procédé selon l'une quelconque des revendications 23 à 29, comprenant en outre l'introduction de ladite première molécule produit dans une cellule hôte.

32. Procédé selon l'une quelconque de la revendication 30 ou de la revendication 31, comprenant en outre l'introduction de ladite seconde molécule produit dans une cellule hôte.

33. Procédé selon l'une ou l'autre de la revendication 24 ou de la revendication 30, dans lequel ledit vecteur est un vecteur d'expression.

34. Procédé selon l'une quelconque des revendications 22 à 33, dans lequel ladite seconde molécule d'acide nucléique comprend au moins une séquence d'acide nucléique supplémentaire choisie dans le groupe constitué par un marqueur sélectionnable, un site de clonage, un site de restriction, un promoteur, un opérateur un opéron, une origine de réplication et un gène ou gène partiel.

35. Procédé selon l'une quelconque des revendications 23 à 34, dans lequel lesdits premier et second sites reconnus par une recombinase spécifique de site sont choisis dans le groupe constitué par : (a) les sites *att*B, (b) les sites *att*P, (c) les sites *att*L, (d) les sites *att*R, (e) les sites *lox*, (f) les sites psi, (g) les sites *dif*, (h) les sites *cer*, (i) les sites *frf* et des mutants, variants et dérivés des sites reconnus par une recombinase spécifique de site de (a), (b), (c), (d), (e), (f), (g), (h) ou (i) qui conservent l'aptitude à subir une recombinaison.

36. Procédé selon l'une quelconque des revendications 29 à 35, dans lequel lesdits troisième et quatrième sites reconnus par une recombinase spécifique de site choisis dans le groupe constitués par : (a) les sites *att*B, (b) les sites *att*P, (c) les sites *att*L, (d) les sites *att*R, (e) les sites *lox*, (f) les sites psi, (g) les sites *dif*, (h) les sites cer, (i) les sites *frf* et des mutants, variants et dérivés des sites reconnus par une recombinase spécifique de site de (a), (b), (c), (d), (e), (f), (g), (h) ou (i) qui conservent l'aptitude à subir une recombinaison.

37. Procédé selon l'une ou l'autre de la revendication 35 ou de la revendication 36, dans lequel lesdits sites *lox* sont choisis dans le groupe constitué par les sites *lox*P et les sites *lox*P511.

38. Procédé selon l'une quelconque des revendications 29 à 37, dans lequel ladite molécule d'acide nucléique produit et ladite troisième molécule d'acide nucléique sont combinées en présence d'au moins une recombinase spécifique de site.

39. Procédé selon la revendication 38, dans lequel ladite recombinase spécifique de site est choisie dans le groupe constitué par : (a) Cre ; (b) Int ; (c) IHF ; (d) Xis ; (e) Fis ; (f) Hin ; (g) Gin ; (h) Cin ; (i) la résolvase Tn3 ; (j) TndX ; (k) XerC ; et (1) XerD.

40. Procédé selon la revendication 39, dans lequel ladite recombinase spécifique de site est Cre.

41. Procédé selon la revendication 39, dans lequel ladite recombinase spécifique de site est choisie dans le groupe constitué par Int, Xis, IHF et Fis.
